# EUROPEAN PATENT APPLICATION

(11) **EP 4 043 461 A1**
(43) Date of publication of application: **17.08.2022**
(21) Application number: 20872056.5
(22) Date of filing: 30.09.2020
(51) Int. Cl.: C07D 471/04, C07D 513/04, C07D 213/81, A61P 37/00, A61P 35/00, A61K 31/4353, A61K 31/395

(54) **COMPOUND AS SMALL MOLECULE INHIBITOR PD-1/PD-L1 AND APPLICATION THEREOF**

(30) Priority: 30.09.2019 CN 201910943193; 02.09.2020 CN 202010911882
(71) Applicant: Medshine Discovery Inc., Nanjing, Jiangsu 210032 (CN)
(72) Inventor: WU, Lingyun, Shanghai 200131 (CN); WANG, Cailin, Shanghai 200131 (CN); XU, Xiongbin, Shanghai 200131 (CN); TONG, Haijun, Shanghai 200131 (CN); CHEN, Shuhui, Shanghai 200131 (CN)
(74) Representative: Dehns
(86) International application number: PCT/CN2020/119511
(87) International publication number: WO 2021/063404

(57) **Abstract**

A biaryl compound. Specifically disclosed is a compound represented by formula (I) and a pharmaceutically acceptable salt thereof.

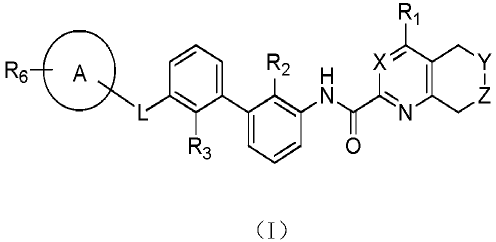

## Description

### Technical Field

The present disclosure relates to a class of small molecule PD-1/PD-L1 inhibitor. Specifically disclosed is a compound represented by formula (I), a pharmaceutically acceptable salt thereof or an isomer thereof.

### Background

Programmed cell death 1 (PD-1), also known as CD279, is an important immunosuppressive molecule in the CD28/CTLA-4 receptor family. It is a membrane protein containing 268 amino acid residues and is widely expressed on the surface of various immune cells such as T cells, macrophages, and B cells, the ligands of which are PD-L1 and PD-L2. PD-L1 is a protein encoded by the CD274 gene and mainly expressed on the surface of tumor cells, dendritic cells and macrophages. After the binding of PD-1 and PD-L1, the PD-1/PD-L1 signaling pathway is activated, which in turn inhibits the activation of T cells, resulting in the incapacitation of T cells to incapacitate, and contributing to the immune escape of tumor cells. PD-L2, another ligand of PD-1, is mainly expressed on the surface of dendritic cells, macrophages and B cells and is associated with inflammation and autoimmune diseases.

PD-1 negatively regulates immune responses by binding to its ligand PD-L1 and dephosphorylating multiple key molecules in the TCR signaling pathway. In healthy organisms, the activation of PD-1/PD-L1 signaling pathway can avoid the surrounding tissue damage caused by excessive immune response, thereby reducing the occurrence of autoimmune diseases. However, under the induction of the tumor microenvironment, the expressions of PD-1 and PD-L1 are abnormally increased, and tumor cells can successfully escape the recognition and attack of the immune system by the binding of these PD-L1 molecules to PD-1 on T cells. PD-L1 monoclonal antibody can block this "tumor immune escape mechanism" and restore the patient's own immune system's anti-cancer function.

Currently, the marketed drugs targeting the PD-1/PD-L1 pathway are all monoclonal antibodies (mAbs). In 2014, the U.S. Food and Drug Administration (FDA) approved the first two monoclonal antibodies (Pembrolizumab and Nivolumab) targeting the PD-1 molecule to the market. Over the next few years, three additional mAbs targeting the PD-L1 molecule (Atezolizumab, Durvalumab, and Avelumab) appeared on the market. All of the above are biological macromolecules, which have their own significant defects, such as being easily decomposed by proteases, poor *in vivo* stability, and need to be administered by injection; product quality is not easy to control, and production technology requirements are high; large-scale preparation and purification are difficult, high production cost and easy to produce immunogenicity. The small molecule PD-1/PD-L1 inhibitor is still in the early stage of research and development. Aurigene polypeptide small molecule PD-L1 inhibitor CA-170 (WO2015033299, WO2019087087) has entered clinical phase I, BMS benzyl phenyl ether small molecule PD-1/PD-L1 inhibitors (WO2015034820, WO2015160641) are still in preclinical research stage. In addition, Incyte (WO2018119224, WO2018119263) and Gilead (US20180305315, WO2019160882) are also reported having small molecule PD-1/PD-L1 inhibitors. However, small molecule drugs can pass through the cell membrane and act on intracellular targets, with advantages of convenient storage and transportation, low production cost, no immunogenicity and suitability for oral administration. Therefore, small molecule PD-1/PD-L1 inhibitors have broad application prospects.

### Content of the present invention

The present disclosure provides a compound represented by formula (I), an isomer or a pharmaceutically acceptable salt thereof, 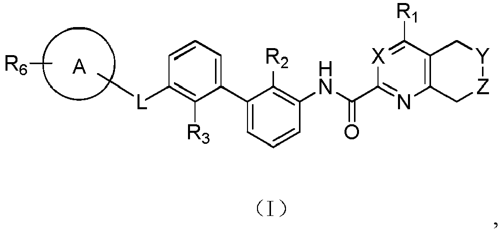 wherein,
ring A is selected from phenyl, 5- to 10- membered heteroaryl and 5- to 10-membered heterocyclenyl, the phenyl, 5- to 10- membered heteroaryl and 5- to 10-membered heterocyclenyl are optionally substituted by 1, 2 or 3 Rₐ;
L is selected from single bond, -C(=O)NH-, -CH=CH-, -O-CH₂- and -NH-;
X is selected from -CH- and N;
when Y is selected from -CH₂- and -CH(CH₃)-, then Z is selected from NR₄;
or, when Y is selected from NR₅, then Z is selected from-CH₂-;
R₁ is selected from H, C₁₋₃ alkyl, C₁₋₃ alkyl-O-, pyridyl-C₁₋₃ alkyl-O- and C₁₋₃ alkyl-NH-, the C₁₋₃ alkyl, C₁₋₃ alkyl-O-, pyridyl-C₁₋₃ alkyl-O- and C₁₋₃ alkyl-NH- are optionally substituted by 1, 2 or 3 R_{b};
R₂ and R₃ are independently selected from H, F, Cl, Br, I, CN, C₁₋₃ alkyl, C₂₋₆ alkenyl and C₂₋₆ alkynyl, the C₁₋₃ alkyl, C₂₋₆ alkenyl and C₂₋₆ alkynyl are optionally substituted by 1, 2 or 3 R_{c};
R₆ is selected from H, C₁₋₆ alkyl, -C₀₋₆ alkyl-C₃₋₆ cycloalkyl, -C₀₋₆ alkyl-3- to 10- membered heterocycloalkyl, -C₀₋₆ alkyl-NH- C₁₋₆ alkyl, -C₀₋₆ alkyl-NH-C₀₋₆ alkyl-C₃₋₆cycloalkyl, -C₀₋₆ alkyl-NH-C₀₋₆ alkyl-3- to 6- membered heterocycloalkyl and -C₀₋₆ alkyl-O-C₁₋₆ alkyl, the C₁₋₆ alkyl, -C₀₋₆ alkyl-C₃₋₆ cycloalkyl, -C₀₋₆ alkyl-3- to 6-membered heterocycloalkyl, -C₀₋₆ alkyl-NH- C₁₋₆ alkyl, -C₀₋₆ alkyl-NH-C₀₋₆ alkyl-C₃₋₆cycloalkyl, -C₀₋₆ alkyl-NH-C₀₋₆ alkyl-3- to 6- membered heterocycloalkyl and -C₀₋₆ alkyl-O-C₁₋₆ alkyl are optionally substituted by 1, 2 or 3 R_{d};
R₄ and R₅ are independently selected from H, C₁₋₆ alkyl, C₁₋₆ alkyl-NH-, C₃₋₈ cycloalkyl, -C₀₋₆ alkyl-C₃₋₆ cycloalkyl and 3- to 8- membered heterocycloalkyl, the C₁₋₆ alkyl, C₁₋₆ alkyl-NH-, C₃₋₈ cycloalkyl, -C₀₋₆ alkyl-C₃₋₆ cycloalkyl and 3- to 8-membered heterocycloalkyl are optionally substituted with 1, 2 or 3 Rₑ;
Rₐ, R_{b}, R_{c}, R_{d} and Rₑ are independently selected from H, F, Cl, Br, I, OH, CN, NH₂, COOH. 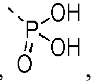 C₁₋₆ alkyl, -O-C₁₋₆ alkyl, -C₀₋₆ alkyl-NH-C₁₋₆ alkyl, -C₀₋₆ alkyl-NH-C₀₋₆ alkyl-3- to 6-membered heterocycloalkyl, -N(C₁₋₆ alkyl)₂, -C₀₋₆ alkyl-C₃₋₆ cycloalkyl and -C₀₋₆ alkyl-3- to 6- membered heterocycloalkyl, the C₁₋₆ alkyl, -O-C₁₋₆ alkyl, -C₀₋₆ alkyl-NH-C₁₋₆ alkyl, -C₀₋₆ alkyl-NH-C₀₋₆ alkyl-3- to 6- membered heterocycloalkyl, -N(C₁₋₆ alkyl)₂, -C₀₋₆ alkyl-C₃₋₆ cycloalkyl and -C₀₋₆ alkyl-3- to 6-membered heterocycloalkyl are optionally substituted by 1, 2 or 3 R;
R is independently selected from F, Cl, Br, I, OH, CN, NH₂, COOH, CH₃, CH₃CH₂, CH₃O, NHCH₃, N(CH₃)₂ and CH₃(CH₃)₂;
the 5- to 10- membered heteroaryl, 5- to 10- membered heterocycloalkenyl, 3-to 6- membered heterocycloalkyl, 3- to 8- membered heterocycloalkyl and 3- to 10-membered heterocycloalkyl independently comprise 1 , 2, 3 or 4 heteroatoms or heteroatom groups independently selected from -NH-, N, -S- and -O-.

In some embodiments of the present disclosure, the compound represented by the above-mentioned formula (I), the isomer or the pharmaceutically acceptable salt thereof, 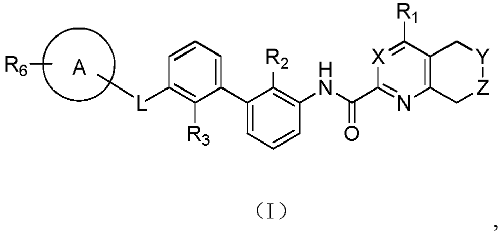 wherein,
ring A is selected from phenyl, 5- to 10- membered heteroaryl and 5- to 10-membered heterocyclenyl, the phenyl, 5- to 10- membered heteroaryl and 5- to 10-membered heterocyclenyl are optionally substituted by 1, 2 or 3 Rₐ;
L is selected from single bond, -C(=O)NH-, -CH=CH-, -O-CH₂- and -NH-;
X is selected from -CH- and N;
when Y is selected from -CH₂- and -CH(CH₃)-, then Z is selected from NR₄;
or, when Y is selected from NR₅, then Z is selected from-CH₂-;
R₁ is selected from H, C₁₋₃ alkyl, C₁₋₃ alkyl-O- and C₁₋₃ alkyl-NH-, the C₁₋₃ alkyl, C₁₋₃ alkyl-O- and C₁₋₃ alkyl-NH- are optionally substituted by 1, 2 or 3 R_{b};
R₂ and R₃ are independently selected from H, F, Cl, Br, I, CN, C₁₋₃ alkyl, C₂₋₆ alkenyl and C₂₋₆ alkynyl, the C₁₋₃ alkyl, C₂₋₆ alkenyl and C₂₋₆ alkynyl are optionally substituted by 1, 2 or 3 R_{c};
R₆ is selected from H, C₁₋₆ alkyl, -C₀₋₆ alkyl-C₃₋₆ cycloalkyl, -C₀₋₆ alkyl-3- to 10- membered heterocycloalkyl, -C₀₋₆ alkyl-NH- C₁₋₆ alkyl, -C₀₋₆ alkyl-NH-C₀₋₆ alkyl-C₃₋₆cycloalkyl, -C₀₋₆ alkyl-NH-C₀₋₆ alkyl-3- to 6- membered heterocycloalkyl and -C₀₋₆ alkyl-O-C₁₋₆ alkyl, the C₁₋₆ alkyl, -C₀₋₆ alkyl-C₃₋₆ cycloalkyl, -C₀₋₆ alkyl-3- to 6-membered heterocycloalkyl, -C₀₋₆ alkyl-NH- C₁₋₆ alkyl, -C₀₋₆ alkyl-NH-C₀₋₆ alkyl-C₃₋₆cycloalkyl, -C₀₋₆ alkyl-NH-C₀₋₆ alkyl-3- to 6- membered heterocycloalkyl and -C₀₋₆ alkyl-O-C₁₋₆ alkyl are optionally substituted by 1, 2 or 3 R_{d};
R₄ and R₅ are independently selected from H, C₁₋₆ alkyl, C₁₋₆ alkyl-NH-, C₃₋₈ cycloalkyl, -C₀₋₆ alkyl-C₃₋₆ cycloalkyl and 3- to 8- membered heterocycloalkyl, the C₁₋₆ alkyl, C₁₋₆ alkyl-NH-, C₃₋₈ cycloalkyl, -C₀₋₆ alkyl-C₃₋₆ cycloalkyl and 3- to 8-membered heterocycloalkyl are optionally substituted with 1, 2 or 3 Rₑ;
Rₐ, R_{b}, R_{c}, R_{d} and Rₑ are independently selected from H, F, Cl, Br, I, OH, CN, NH₂, COOH, 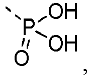 C₁₋₆ alkyl, -O-C₁₋₆ alkyl, -C₀₋₆ alkyl-NH-C₁₋₆ alkyl, -C₀₋₆ alkyl-NH-C₀₋₆ alkyl-3- to 6-membered heterocycloalkyl, -N(C₁₋₆ alkyl)₂, -C₀₋₆ alkyl-C₃₋₆ cycloalkyl and -C₀₋₆ alkyl-3- to 6- membered heterocycloalkyl, the C₁₋₆ alkyl, -O-C₁₋₆ alkyl, -C₀₋₆ alkyl-NH-C₁₋₆ alkyl, -C₀₋₆ alkyl-NH-C₀₋₆ alkyl-3- to 6- membered heterocycloalkyl, -N(C₁₋₆ alkyl)₂, -C₀₋₆ alkyl-C₃₋₆ cycloalkyl and -C₀₋₆ alkyl-3- to 6-membered heterocycloalkyl are optionally substituted by 1, 2 or 3 R;
R is independently selected from F, Cl, Br, I, OH, CN, NH₂, COOH, CH₃, CH₃CH₂, CH₃O, NHCH₃, N(CH₃)₂ and CH₃(CH₃)₂;
the 5- to 10- membered heteroaryl, 5- to 10- membered heterocycloalkenyl, 3-to 6- membered heterocycloalkyl, 3- to 8- membered heterocycloalkyl and 3- to 10-membered heterocycloalkyl independently comprise 1 , 2, 3 or 4 heteroatoms or heteroatom groups independently selected from -NH-, N, -S- and -O-.

In some embodiments of the present disclosure, the compound represented by the above-mentioned formula (I), the isomer or the pharmaceutically acceptable salt thereof, 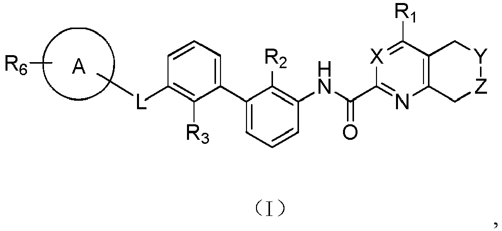 wherein,
ring A is selected from phenyl, 5- to 10- membered heteroaryl and 5- to 10-membered heterocyclenyl, the phenyl, 5- to 10- membered heteroaryl and 5- to 10-membered heterocyclenyl are optionally substituted by 1, 2 or 3 Rₐ;
L is selected from single bond, -C(=O)NH-, -CH=CH-, -O-CH₂- and -NH-;
X is selected from C and N;
when Y is selected from C, then Z is selected from NR₄;
or, when Y is selected from NR₅, then Z is selected from C;
R₁ is selected from H, C₁₋₃ alkyl, C₁₋₃ alkyl-O- and C₁₋₃ alkyl-NH-, the C₁₋₃ alkyl, C₁₋₃ alkyl-O- and C₁₋₃ alkyl-NH- are optionally substituted by 1, 2 or 3 R_{b};
R₂ and R₃ are independently selected from H, F, Cl, Br, I, CN, C₁₋₃ alkyl, C₂₋₆ alkenyl and C₂₋₆ alkynyl, the C₁₋₃ alkyl, C₂₋₆ alkenyl and C₂₋₆ alkynyl are optionally substituted by 1, 2 or 3 R_{c};
R₆ is selected from H, C₁₋₆ alkyl, -C₀₋₆ alkyl-C₃₋₆ cycloalkyl, -C₀₋₆ alkyl-3- to 6- membered heterocycloalkyl, -C₀₋₆ alkyl-NH-C₁₋₆ alkyl, -C₀₋₆ alkyl-NH-C₀₋₆ alkyl-C₃₋₆ cycloalkyl, -C₀₋₆ alkyl-NH-C₀₋₆ alkyl-3- to 6- membered heterocycloalkyl and -C₀₋₆ alkyl-O-C₁₋₆ alkyl, the C₁₋₆ alkyl, -C₀₋₆ alkyl-C₃₋₆ cycloalkyl, -C₀₋₆ alkyl-3- to 6-membered heterocycloalkyl, -C₀₋₆ alkyl-NH-C₁₋₆ alkyl, -C₀₋₆ alkyl-NH-C₀₋₆ alkyl-C₃₋₆ cycloalkyl, -C₀₋₆ alkyl-NH-C₀₋₆ alkyl-3- to 6- membered heterocycloalkyl and -C₀₋₆ alkyl-O-C₁₋₆ alkyl are optionally substituted by 1, 2 or 3 R_{d};
R₄ and R₅ are independently selected from H, C₁₋₆ alkyl, C₁₋₆ alkyl-NH-, C₃₋₈ cycloalkyl and 3- to 8- membered heterocycloalkyl, the C₁₋₆ alkyl, C₁₋₆ alkyl-NH-, C₃₋₈ cycloalkyl and 3- to 8- membered heterocycloalkyl are optionally substituted by 1, 2 or 3 Rₑ;
Rₐ, R_{b}, R_{c}, R_{d} and Rₑ are independently selected from H, F, Cl, Br, I, OH, CN, NH₂, COOH, 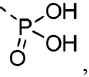 C₁₋₆ alkyl, -O-C₁₋₆ alkyl, -C₀₋₆ alkyl-NH-C₁₋₆ alkyl, -C₀₋₆ alkyl-NH-C₀₋₆ alkyl-3- to 6-membered heterocycloalkyl, -N(C₁₋₆ alkyl)₂, -C₀₋₆ alkyl-C₃₋₆ cycloalkyl and -C₀₋₆ alkyl-3- to 6- membered heterocycloalkyl, the C₁₋₆ alkyl, -O-C₁₋₆ alkyl, -C₀₋₆ alkyl-NH-C₁₋₆ alkyl, -C₀₋₆ alkyl-NH-C₀₋₆ alkyl-3- to 6- membered heterocycloalkyl, -N(C₁₋₆ alkyl)₂, -C₀₋₆ alkyl-C₃₋₆ cycloalkyl and -C₀₋₆ alkyl-3- to 6-membered heterocycloalkyl are optionally substituted by 1, 2 or 3 R;
R is independently selected from F, Cl, Br, I, OH, CN, NH₂, COOH, CH₃, CH₃CH₂, CH₃O, NHCH₃, N(CH₃)₂ and CH₃(CH₃)₂;
the 5- to 10- membered heteroaryl, 5- to 10- membered heterocycloalkenyl, 3-to 6- membered heterocycloalkyl and 3- to 8- membered heterocycloalkyl independently comprise 1, 2, 3 or 4 heteroatoms or heteroatom groups independently selected from -NH-, N, -S- and -O-.

In some embodiments of the present disclosure, the above-mentioned compound, the isomer or the pharmaceutically acceptable salt thereof, which is selected from 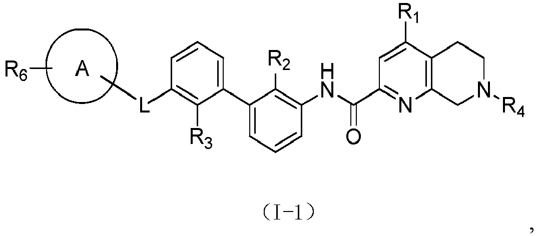 wherein R₁, R₂, R₂, R₃, R₄, R₆, L and ring A are as defined in the present disclosure.

In some embodiments of the present disclosure, the above-mentioned compound, the isomer or the pharmaceutically acceptable salt thereof, which is selected from 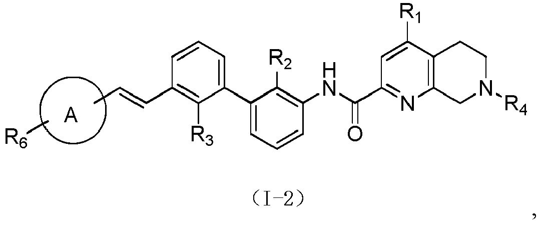 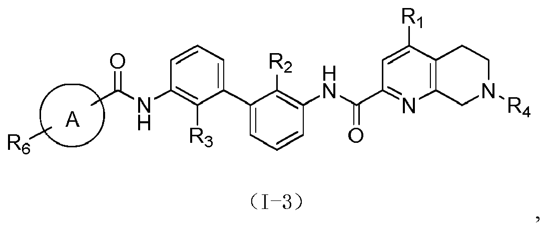 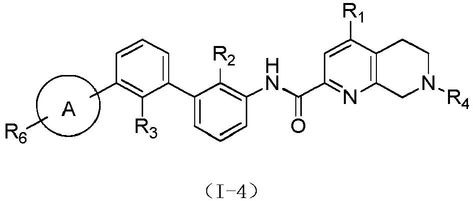 and 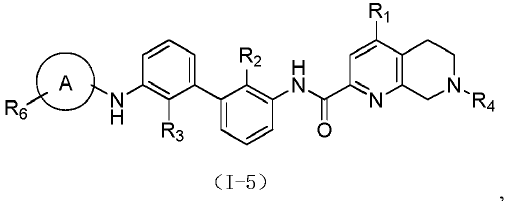 wherein R₁, R₂, R₂, R₃, R₄, R₆ and ring A are as defined in the present disclosure.

In some embodiments of the present disclosure, the above-mentioned compound, the isomer or the pharmaceutically acceptable salt thereof, which is selected from 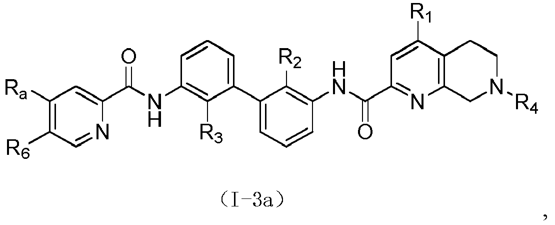 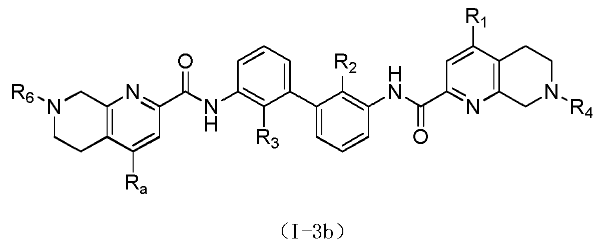 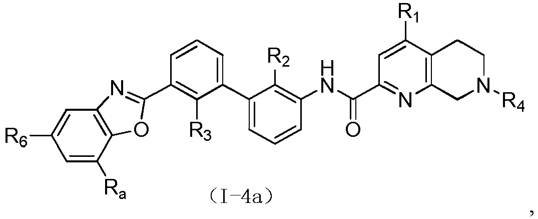 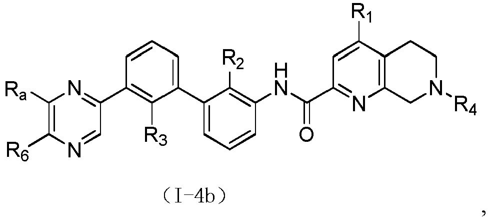 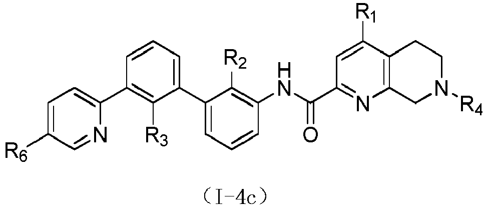 and 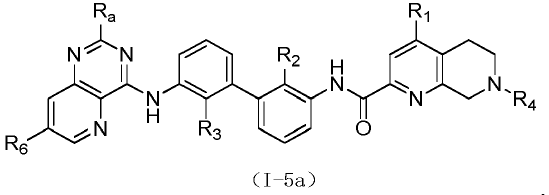 wherein R₁, R₂, R₂, R₃, R₄, R₆ and Rₐ are as defined in the present disclosure.

In some embodiments of the present disclosure, the above-mentioned compound , the isomer or the pharmaceutically acceptable salt thereof, which is selected from 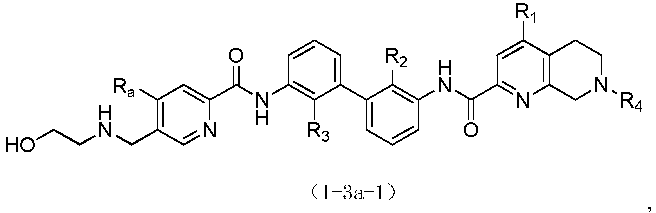 wherein R₁, R₂, R₂, R₃, R₄ and Rₐ are as defined in the present disclosure.

In some embodiments of the present disclosure, in the above-mentioned compound, the isomer or the pharmaceutically acceptable salt thereof, wherein, Rₐ, R_{b}, R_{c} and Rₑ are independently selected from H, F, Cl, Br, I, OH, CN, NH₂, COOH, CH₃, CF₃, CHF₂, CH₂F, CH₃CH₂, CH₂OH, 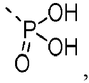 CH₃O, NHCH₃, N(CH₃)₂, CH₃(CH₃)₂, 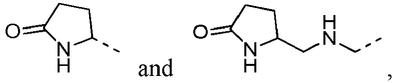 other variables are as defined in the present disclosure.

In some embodiments of the present disclosure, in the above-mentioned compound, the isomer or the pharmaceutically acceptable salt thereof, wherein, Rₐ, R_{b}, R_{c} and Rₑ are independently selected from F, Cl, Br, I, OH, CN, NH₂, COOH, CH₃, CH₃CH₂, CH₃O, NHCH₃, N(CH₃)₂, CH₃(CH₃)₂, 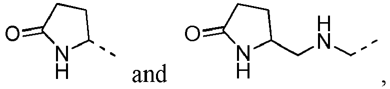 other variables are as defined in the present disclosure.

In some embodiments of the present disclosure, in the above-mentioned compound, the isomer or the pharmaceutically acceptable salt thereof, wherein, R_{d} is selected from F, Cl, Br, I, OH, CN, NH₂, COOH, CH₃, CH₃CH₂, CH₂OH, CH₃O, NHCH₃, N(CH₃)₂, CH₃(CH₃)₂, other variables are as defined in the present disclosure.

In some embodiments of the present disclosure, in the above-mentioned compound, the isomer or the pharmaceutically acceptable salt thereof, wherein, R_{d} is selected from F, Cl, Br, I, OH, CN, NH₂, COOH, CH₃, CH₃CH₂, CH₃O, NHCH₃, N(CH₃)₂, CH₃(CH₃)₂, other variables are as defined in the present disclosure.

In some embodiments of the present disclosure, R_{d} is selected from F, Cl, Br, I, OH, CN, NH₂, COOH, CH₃, CH₃CH₂, CH₂OH, CH₃O, NHCH₃, N(CH₃)₂, CH₃(CH₃)₂, other variables are as defined in the present disclosure.

In some embodiments of the present disclosure, in the above-mentioned compound, the isomer or the pharmaceutically acceptable salt thereof, wherein, R₁ is selected from H, CN, CH₃, CH₃O-, pyridyl-CH₂O- and CH₃CH₂NH-, the CH₃, CH₃O-, pyridyl-CH₂O- and CH₃CH₂-NH- are optionally substituted by 1, 2 or 3 R_{b}, other variables are as defined in the present disclosure.

In some embodiments of the present disclosure, in the above-mentioned compound, the isomer or the pharmaceutically acceptable salt thereof, wherein, R₁ is selected from CN, CF₃, -OCHF₂, CH₃, CH₃O-, -NHCH₂CH₂OH, 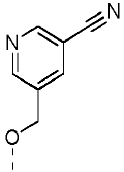 and 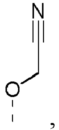 other variables are as defined in the present disclosure.

In some embodiments of the present disclosure, R₁ is selected from H, CH₃, CH₃O- and CH₃CH₂NH-, the CH₃, CH₃O- and CH₃CH₂NH- are optionally substituted by 1, 2 or 3 R_{b}, other variables are as defined in the present disclosure.

In some embodiments of the present disclosure, R₁ is selected from CH₃, CH₃O- and HOCH₂CH₂NH-, other variables are as defined in the present disclosure.

In some embodiments of the present disclosure, in the above-mentioned compound, the isomer or the pharmaceutically acceptable salt thereof, wherein, R₁ is selected from H, CH₃ and CH₃CH₂NH-, the CH₃ and CH₃CH₂NH- are optionally substituted by 1, 2 or 3 R_{b}, other variables are as defined in the present disclosure.

In some embodiments of the present disclosure, in the above-mentioned compound, the isomer or the pharmaceutically acceptable salt thereof, wherein, R₁ is selected from CH₃ and HOCH₂CH₂NH-, other variables are as defined in the present disclosure.

In some embodiments of the present disclosure, in the above-mentioned compound, the isomer or the pharmaceutically acceptable salt thereof, wherein, R₂ and R₃ are independently selected from Cl, CN and CH₃, other variables are as defined in the present disclosure.

In some embodiments of the present disclosure, in the above-mentioned compound, the isomer or the pharmaceutically acceptable salt thereof, wherein, R₆ is selected from H, -CH₂CH₃, 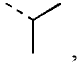 -CH₂NHCH₃, -CH₂NHCH₂CH₃, 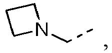 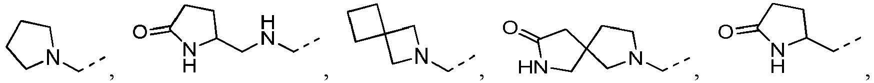 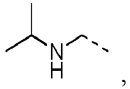 the -CH₂CH₃, 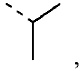 -CH₂NHCH₃, -CH₂NHCH₂CH₃, 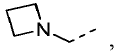 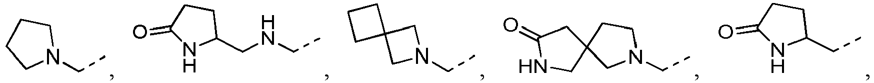 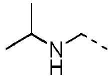 are optionally substituted by 1, 2 or 3 R_{d}, other variables are as defined in the present disclosure.

In some embodiments of the present disclosure, in the above-mentioned compound, the isomer or the pharmaceutically acceptable salt thereof, wherein, R₆ is selected from H, 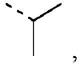 -CH₂CH₂OH, -CH₂NHCH₂CH₂OH, 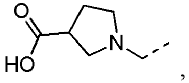 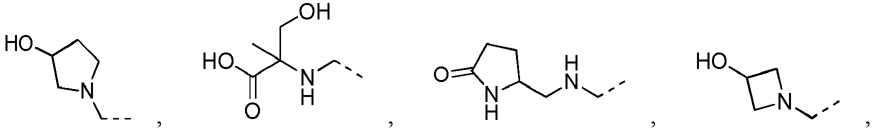 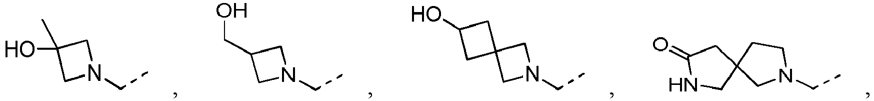 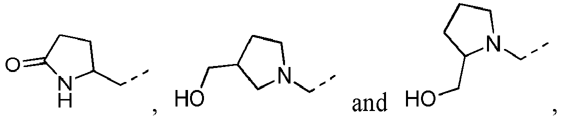 other variables are as defined in the present disclosure.

In some embodiments of the present disclosure, R₆ is selected from H, - CH₂CH₃, 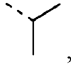 -CH₂NHCH₃, -CH₂NHCH₂CH₃, 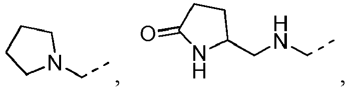 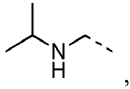 the -CH₂CH₃, 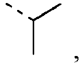 -CH₂NHCH₃, -CH₂NHCH₂CH₃, 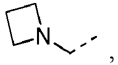 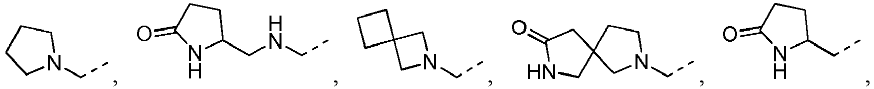 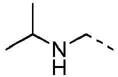 are optionally substituted by 1, 2 or 3 R_{d}, other variables are as defined in the present disclosure.

In some embodiments of the present disclosure, R₆ is selected from H, - CH₂CH₂OH, -CH₂NHCH₂CH₂OH, 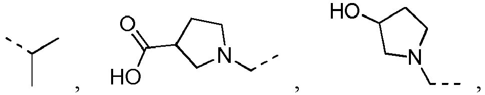 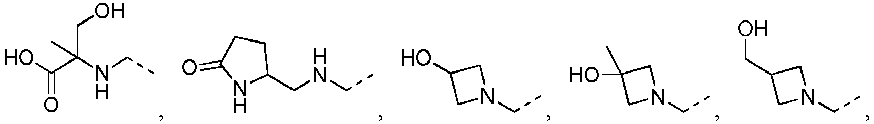 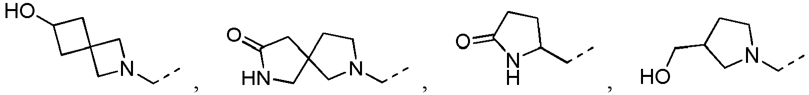 and 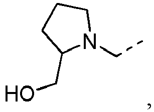 other variables are as defined in the present disclosure.

In some embodiments of the present disclosure, in the above-mentioned compound, the isomer or the pharmaceutically acceptable salt thereof, wherein, R₄ and R₅ are independently selected from H, CH₃, CH₂CH₃, 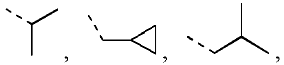 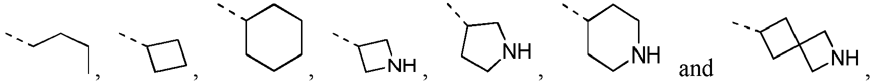 the CH₃, CH₂CH₃, 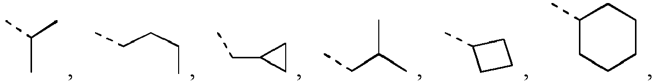 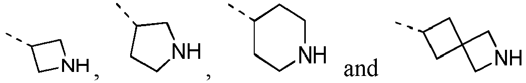 are optionally substituted with 1, 2 or 3 Rₑ, and other variables are as defined in the present disclosure.

In some embodiments of the present disclosure, in the above-mentioned compound, the isomer or the pharmaceutically acceptable salt thereof, wherein, R₄ and R₅ are independently selected from H, CH₃, CH₂CH₃, 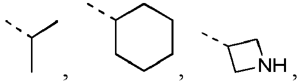 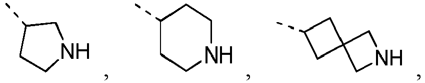 CH₂CH₂OH, 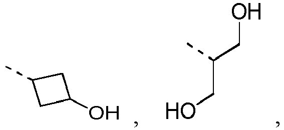 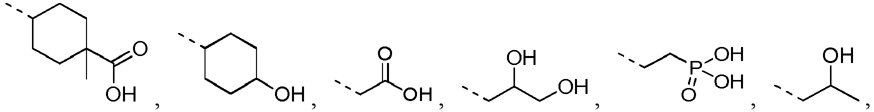 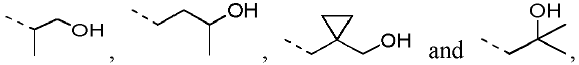 other variables are as defined in the present disclosure.

In some embodiments of the present disclosure, R₄ and R₅ are independently selected from H, CH₃, CH₂CH₃, 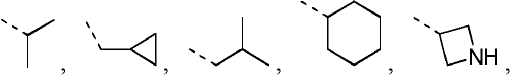 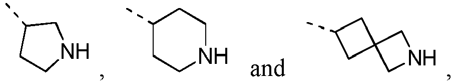 the CH₃, CH₂CH₃, 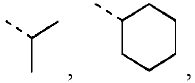 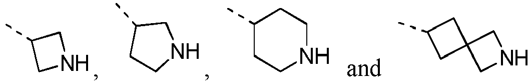 are optionally substituted by 1, 2 or 3 Rₑ, and other variables are as defined in the present disclosure.

In some embodiments of the present disclosure, R₄ and R₅ are independently selected from H, CH₃, CH₂CH₃, 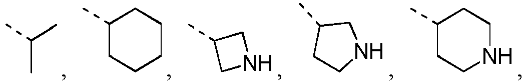 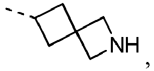 CH₂CH₂OH, 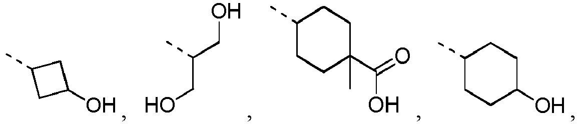 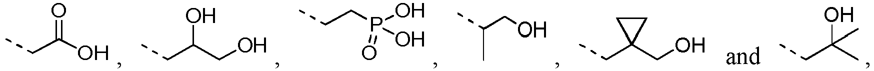 other variables are as defined in the present disclosure.

In some embodiments of the present disclosure, in the above-mentioned compound, the isomer or the pharmaceutically acceptable salt thereof, wherein, the structural moiety 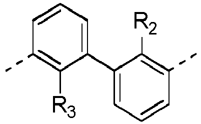 is selected from 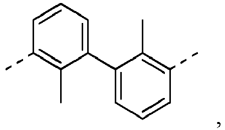 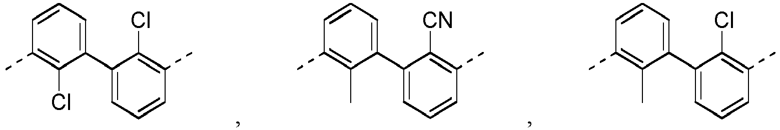 and 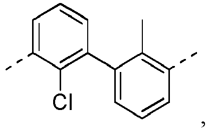 other variables are as defined in the present disclosure.

In some embodiments of the present disclosure, in the above-mentioned compound, the isomer or the pharmaceutically acceptable salt thereof, wherein, ring A is selected from benzo[*d]*oxazolyl, pyrrolidine -2-keto, pyridyl, 5,6,7,8-tetrahydro-1,7-diazanaphthyl, phenyl, 4,5,6,7-tetrahydro-2*H*-pyrazolo[3,4-c]pyridyl, 4,5,6,7-tetrahydrothiazolo[5,4-c]pyridyl, pyrazinyl and pyrido[3,2-*d*]pyrimidinyl, the benzo[d]oxazolyl, pyrrolidine -2-keto, pyridyl and 5,6,7,8-tetrahydro-1,7-diazanaphthyl, phenyl, 4,5,6,7-tetrahydro-2*H*-pyrazolo[3,4-c]pyridyl, 4,5,6,7-tetrahydrothiazolo[5,4-c]pyridyl, pyrazinyl and pyrido[3,2-*d*]pyrimidinyl are optionally substituted by 1, 2 or 3 Rₐ, other variables are as defined in the present disclosure.

In some embodiments of the present disclosure, in the above-mentioned compound, the isomer or the pharmaceutically acceptable salt thereof, wherein, ring A is selected from 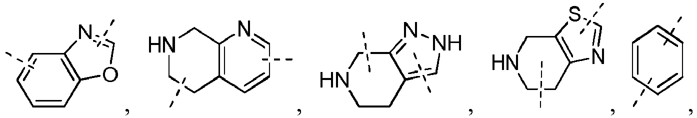 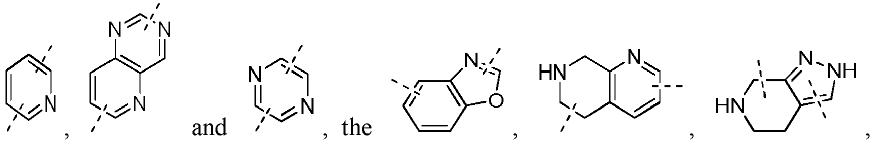 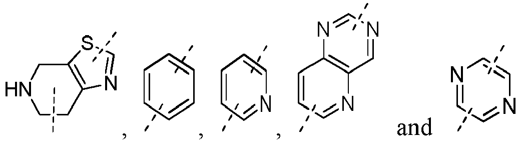 are optionally substituted by 1, 2 or 3 Rₐ, other variables are as defined in the present disclosure.

In some embodiments of the present disclosure, in the above-mentioned compound, the isomer or the pharmaceutically acceptable salt thereof, wherein, ring A is selected from 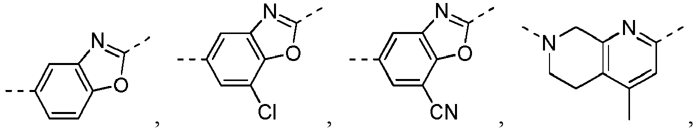 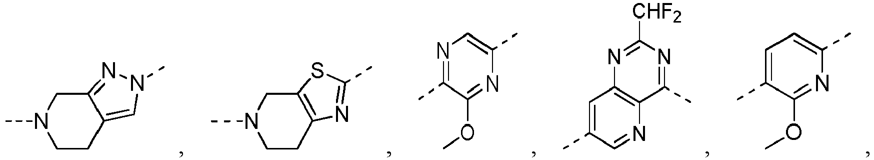 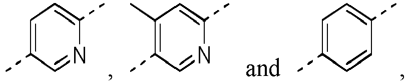 other variables are as defined in the present disclosure.

In some embodiments of the present disclosure, ring A is selected from benzo[d]oxazolyl, pyrrolidine -2-keto, pyridyl, 5,6,7,8-tetrahydro 1,7-diazanaphthyl, phenyl, 4,5,6,7-tetrahydro-2*H*-pyrazolo[3,4-c]pyridyl, 4,5,6,7-tetrahydrothiazolo[5,4-c]pyridyl, pyrazinyl and pyrido[3,2-*d*]pyrimidinyl, the benzo[d]oxazolyl, pyrrolidine - 2-keto, pyridyl and 5,6,7,8- tetrahydro-1,7-diazanaphthyl, phenyl, 4,5,6,7-tetrahydro-2*H*-pyrazolo[3,4-c]pyridyl, 4,5,6,7-tetrahydro hydrothiazolo[5,4-c]pyridyl, pyrazinyl and pyrido[3,2-d]pyrimidinyl are optionally substituted by 1, 2 or 3 Rₐ, other variables are as defined in the present disclosure.

In some embodiments of the present disclosure, ring A is selected from 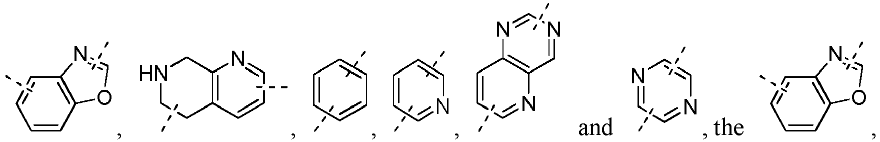 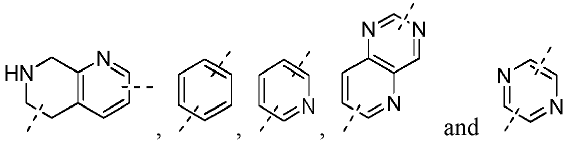 are optionally substituted by 1, 2 or 3 Rₐ, other variables are as defined in the present disclosure.

In some embodiments of the present disclosure, ring A is selected from 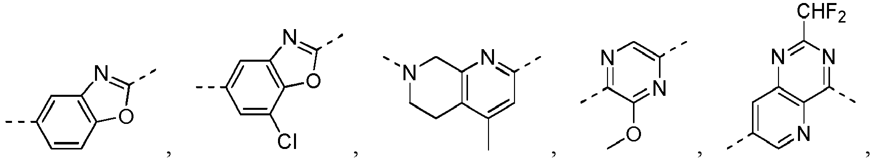 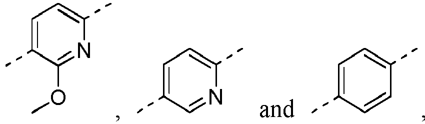 other variables are as defined in the present disclosure.

In some embodiments of the present disclosure, in the above-mentioned compound, the isomer or the pharmaceutically acceptable salt thereof, wherein, ring A is selected from benzo[d]oxazolyl, pyrrolidine-2-keto, pyridyl and 5,6,7,8-tetrahydro-1,7-diazanaphthyl, the benzo[d]oxazolyl, pyrrolidine-2-keto, pyridyl and 5,6,7,8-tetrahydro-1,7-diazanaphthyl are optionally substituted by 1, 2 or 3 Rₐ, other variables are as defined in the present disclosure.

In some embodiments of the present disclosure, in the above-mentioned compound, the isomer or the pharmaceutically acceptable salt thereof, wherein, the structural moiety 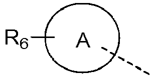 is selected from 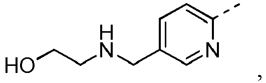 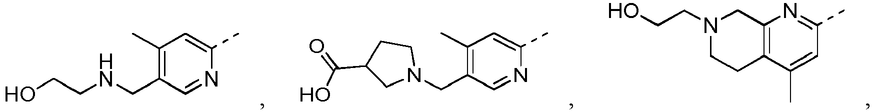 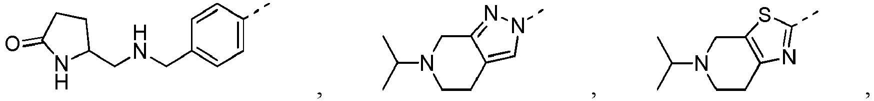 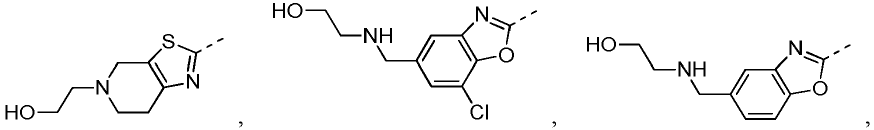 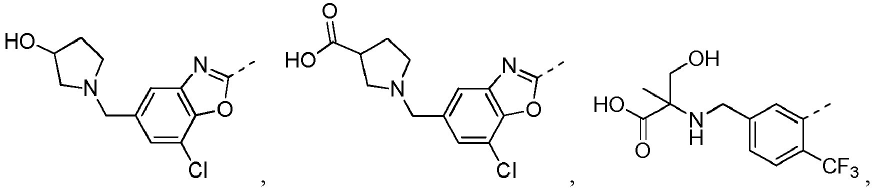 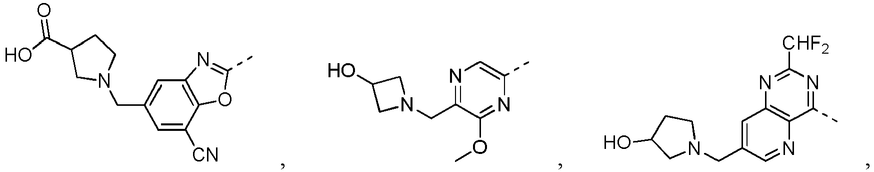 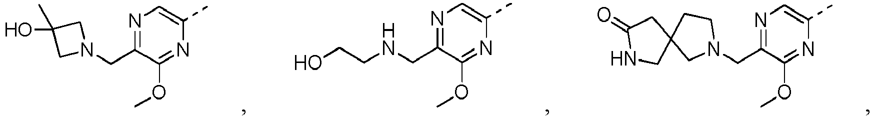 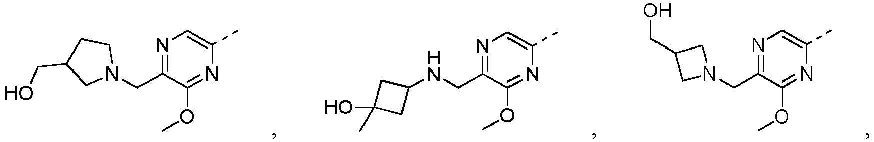 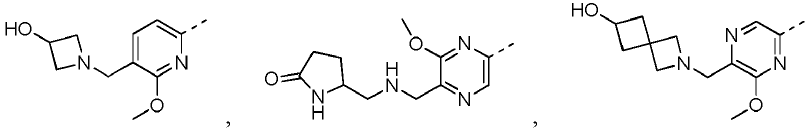 and 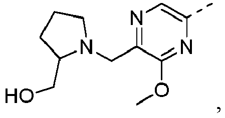 other variables are as defined in the present disclosure.

In some embodiments of the present disclosure, in the above-mentioned compound, the isomer or the pharmaceutically acceptable salt thereof, wherein, the structural moiety 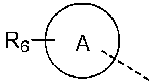 is selected from 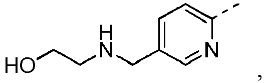 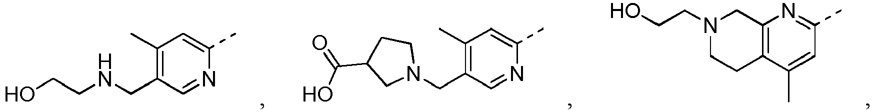 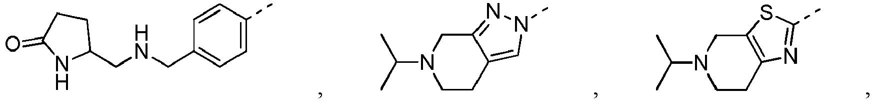 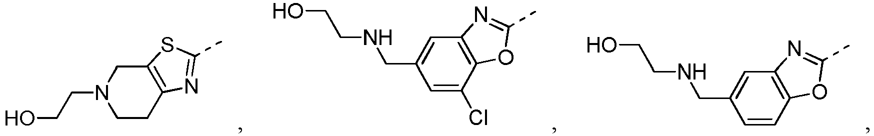 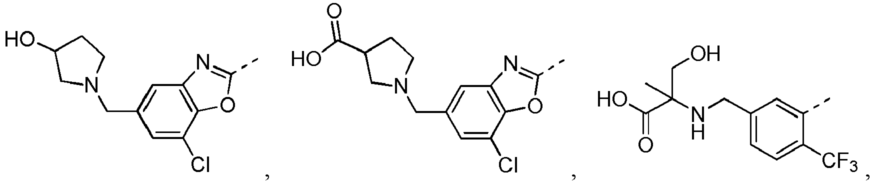 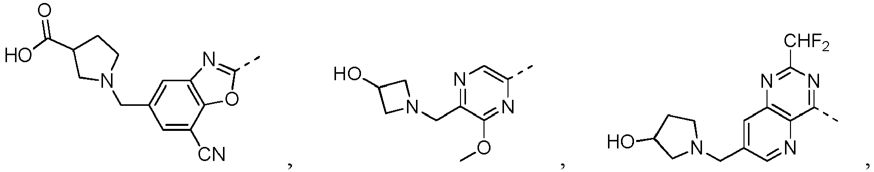 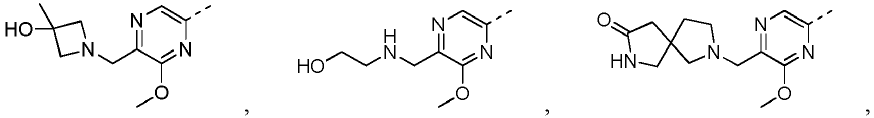 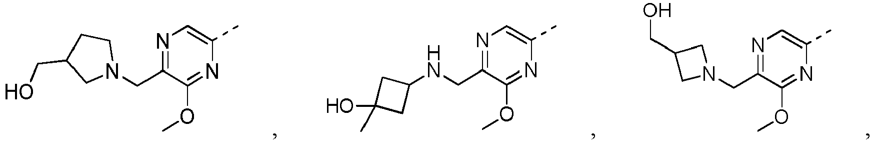 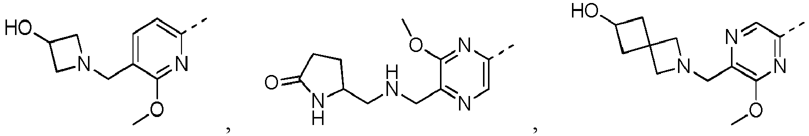 and 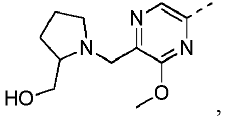 other variables are as defined in the present disclosure.

In some embodiments of the present disclosure, in the above-mentioned compound, the isomer or the pharmaceutically acceptable salt thereof, wherein, the structural moiety 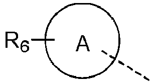 is selected from 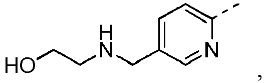 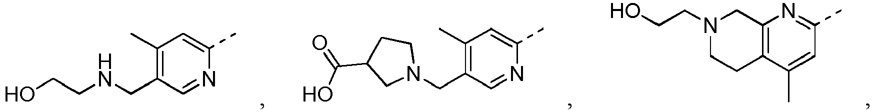 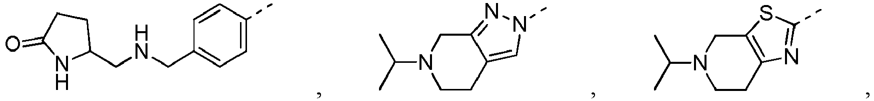 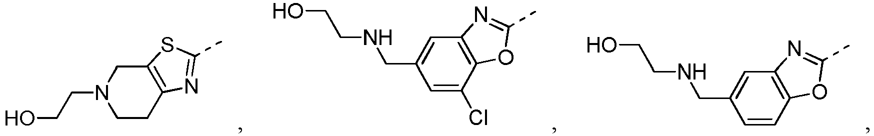 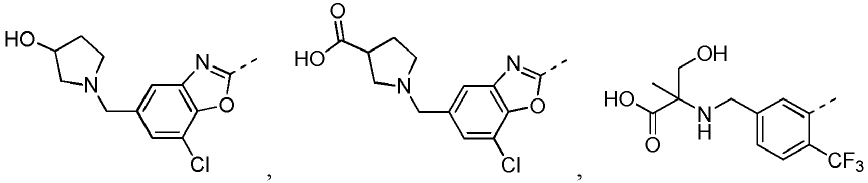 and 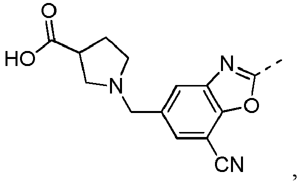 other variables are as defined in the present disclosure.

There are also some technical solutions of the present disclosure which are obtained by any combinations of the above-mentioned variables.

The present disclosure also provides a compound of the following formula, an isomer or a pharmaceutically acceptable salt thereof,

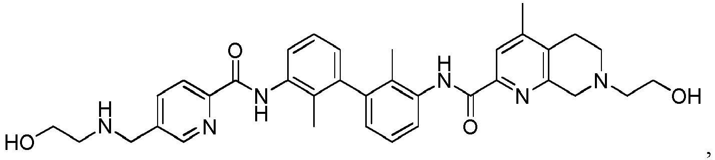

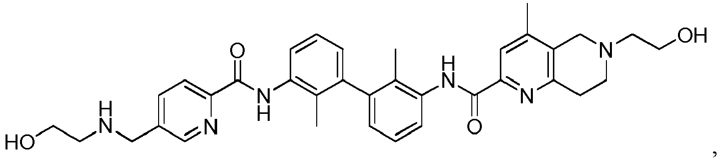

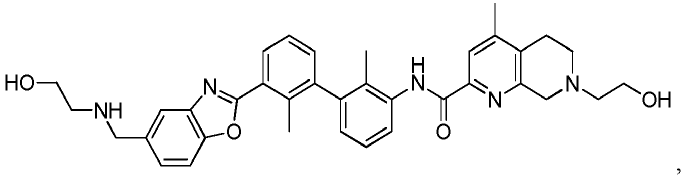

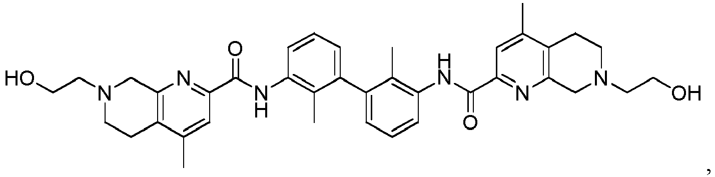

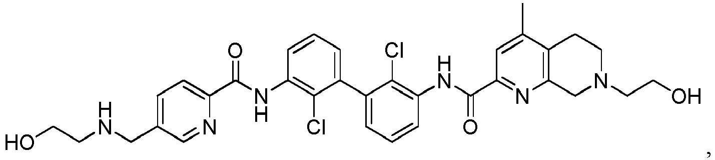

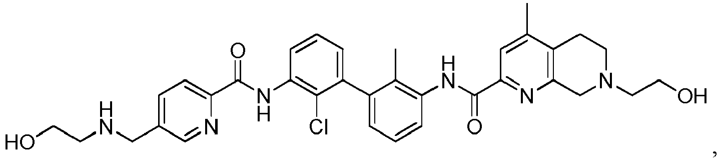

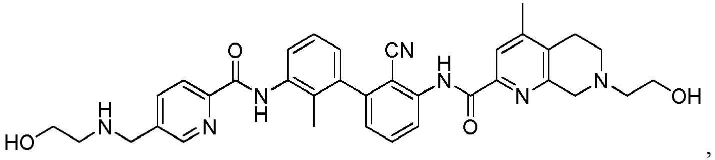

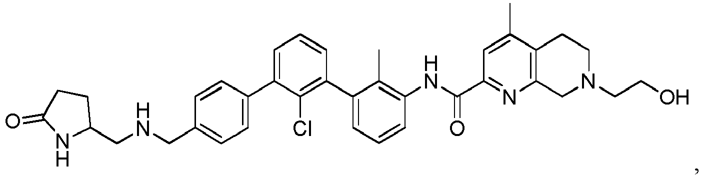

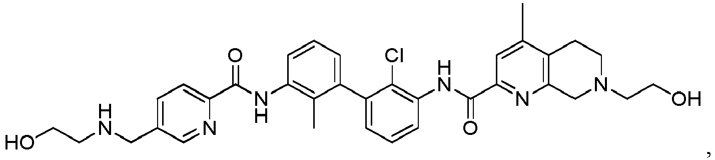

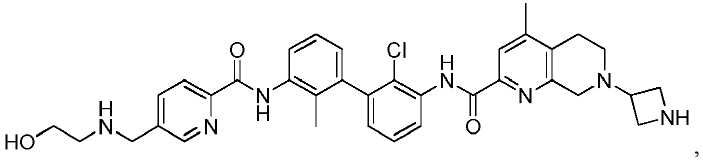

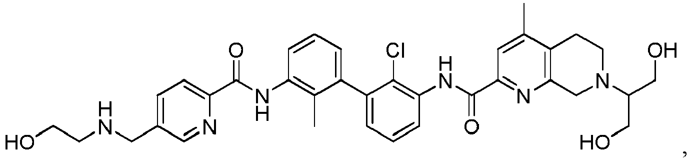

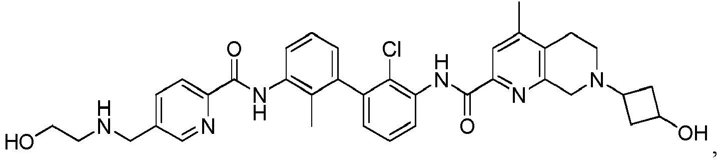

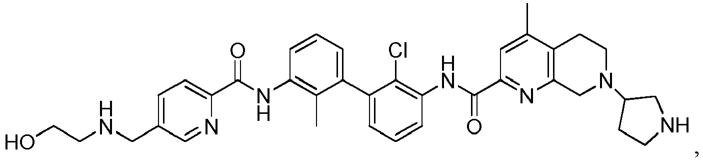

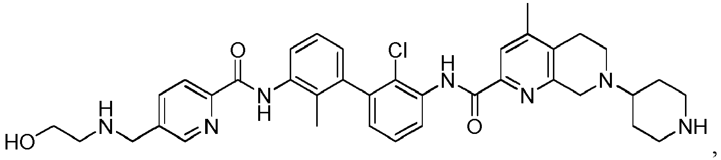

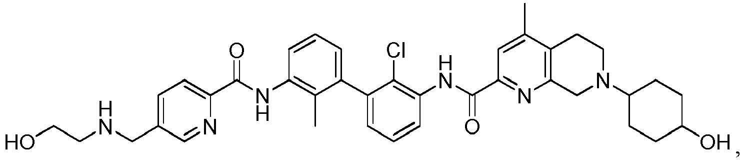

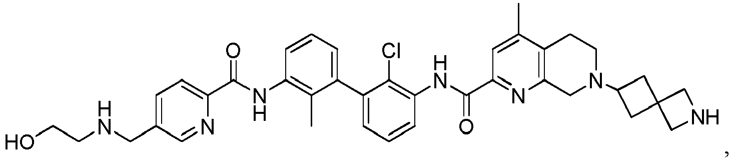

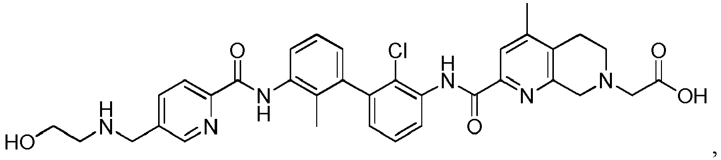

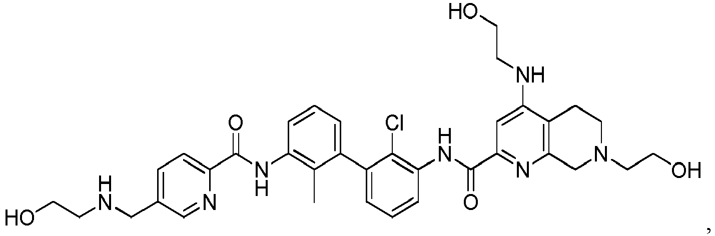

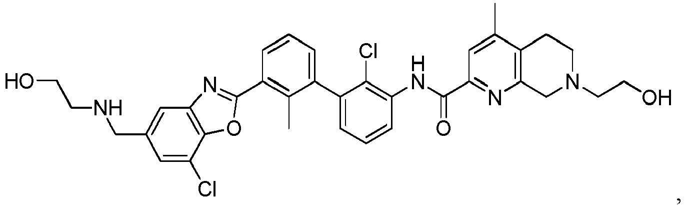

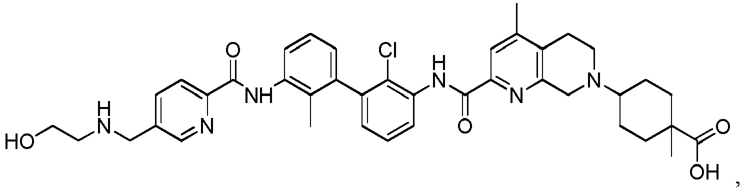

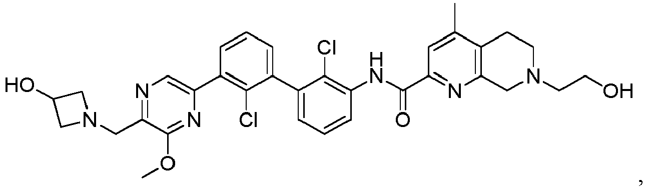

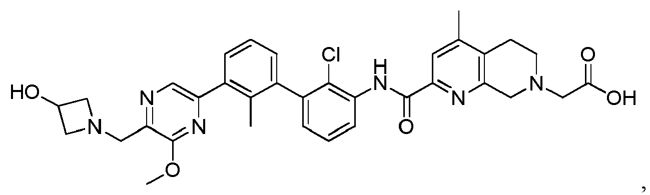

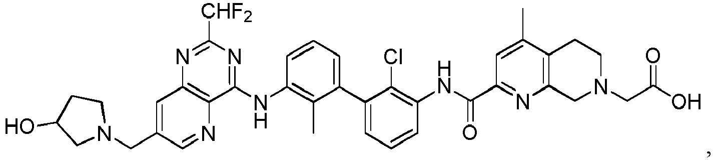

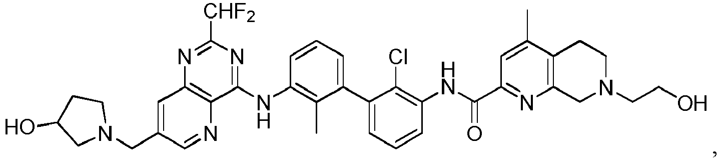

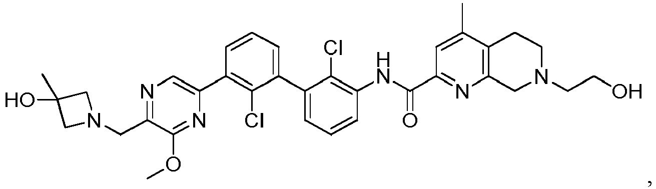

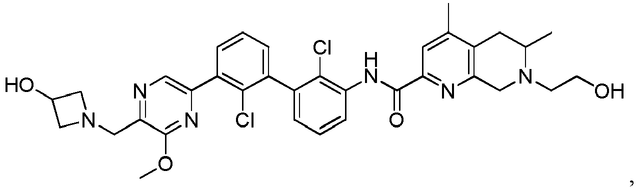

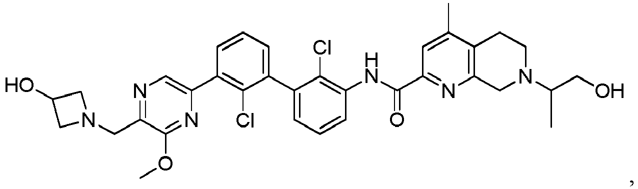

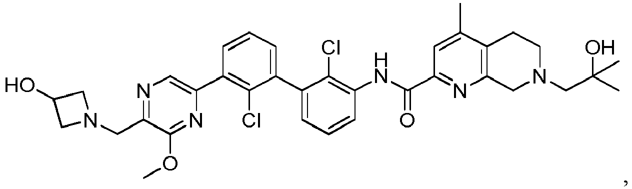

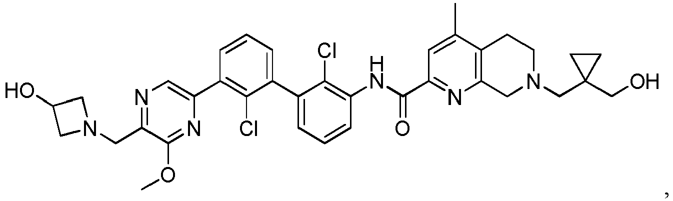

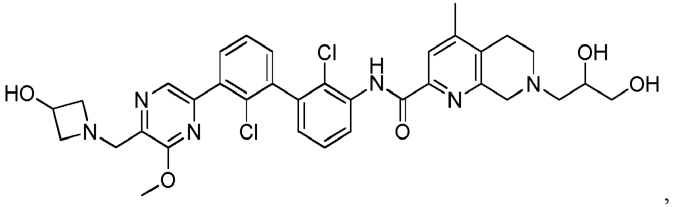

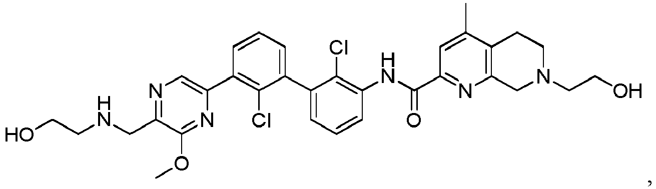

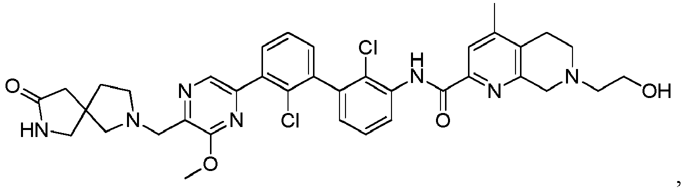

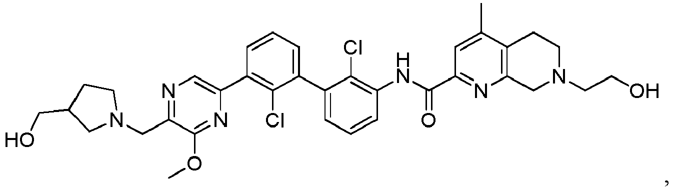

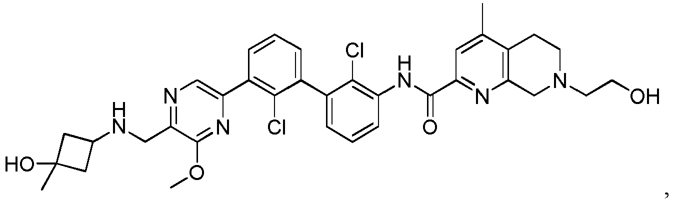

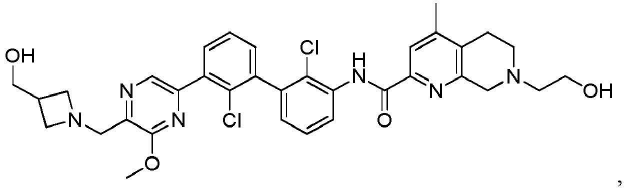

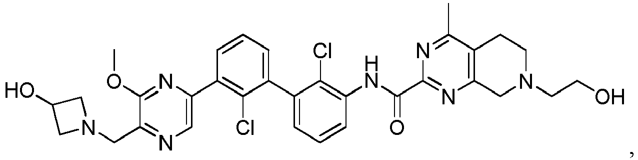

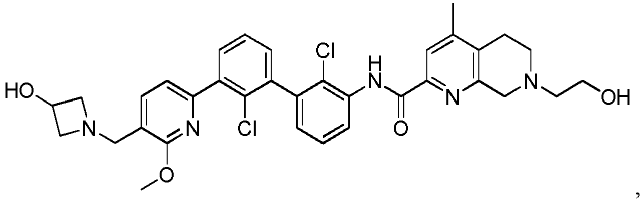

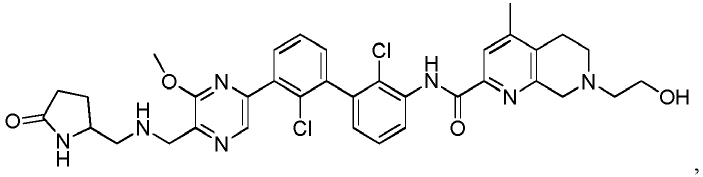

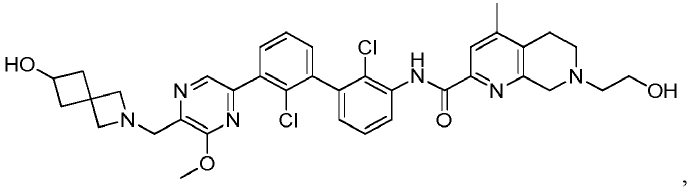

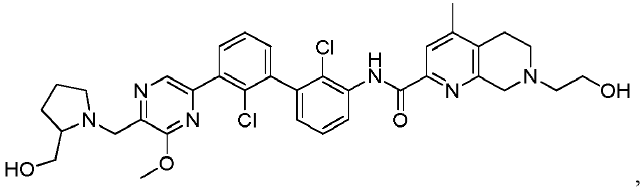

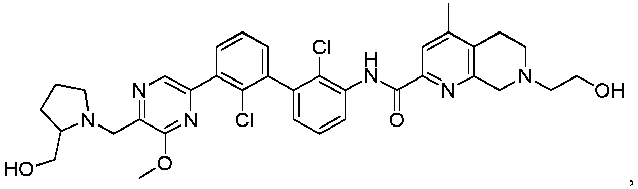

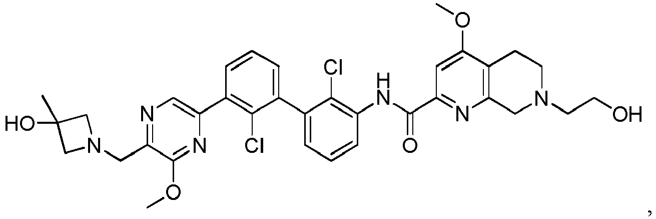

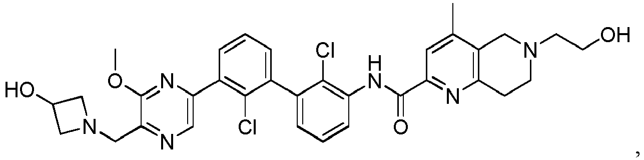

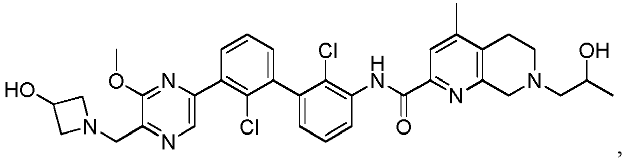

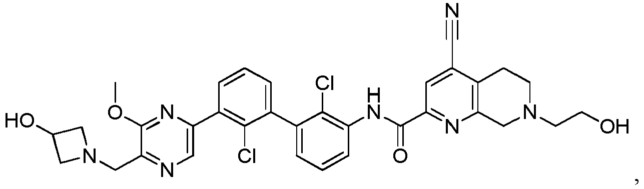

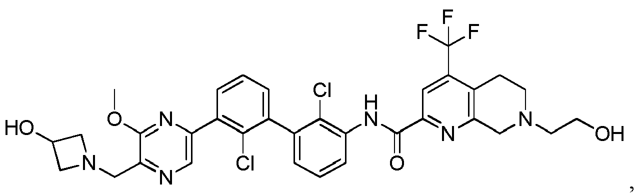

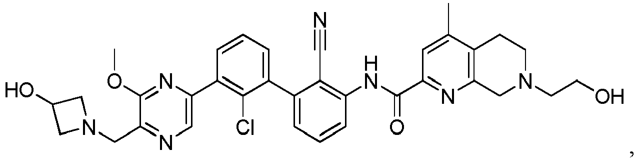

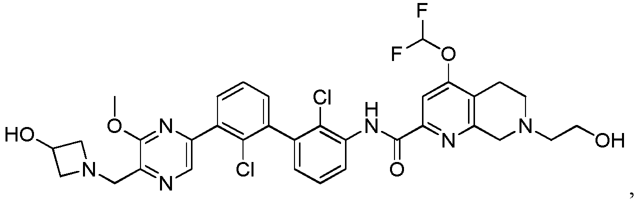

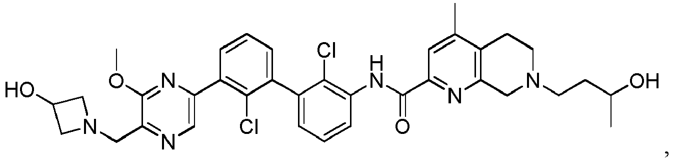

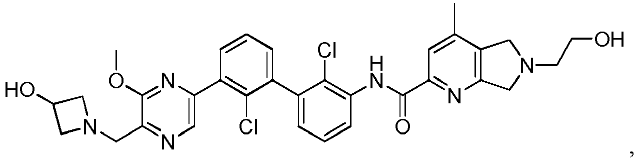

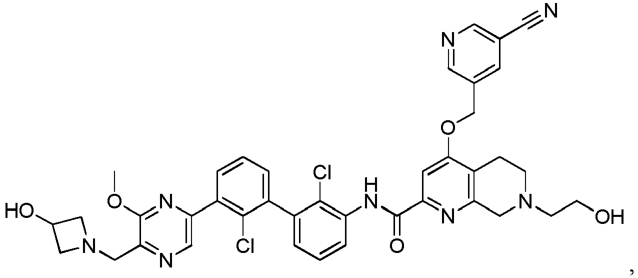

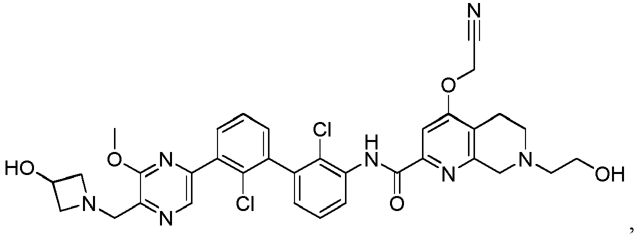

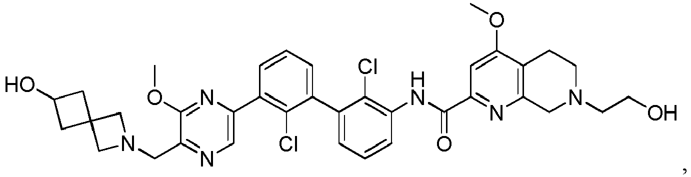

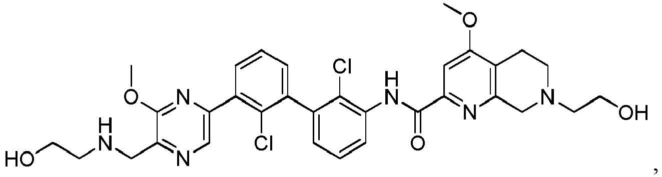

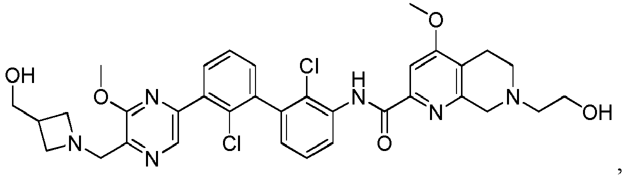

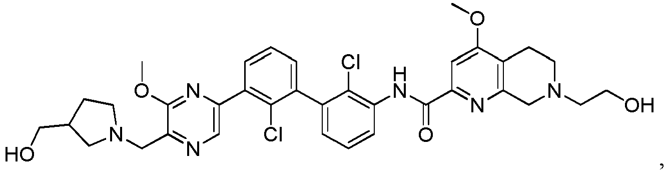

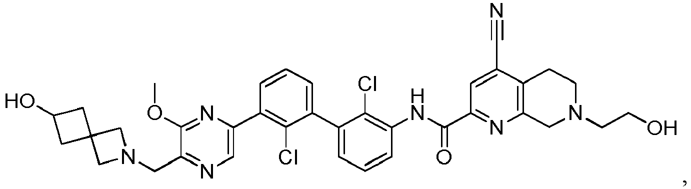

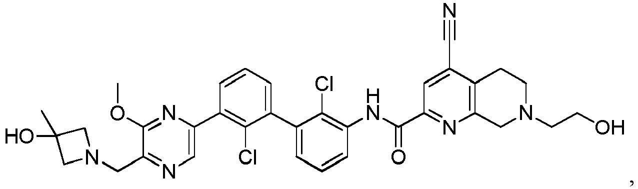

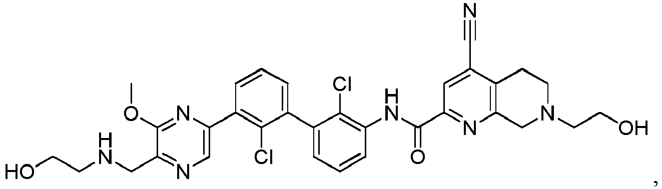

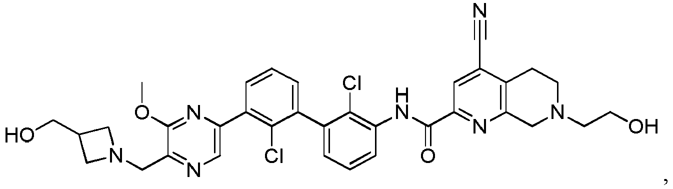

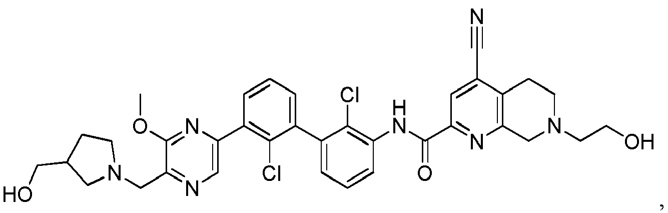

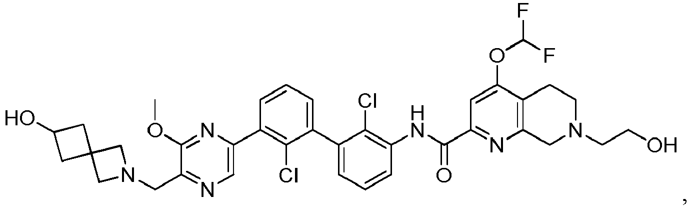

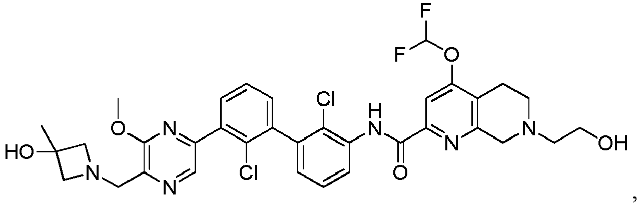

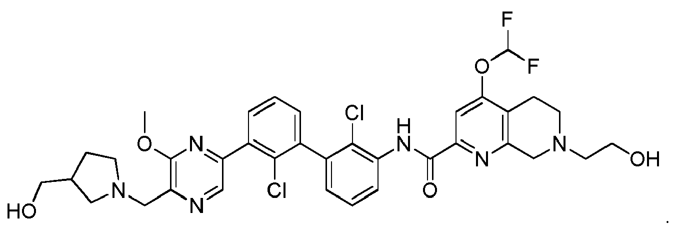

In some embodiments of the present disclosure, the above-mentioned compound, the isomer or the pharmaceutically acceptable salt thereof, which is selected from

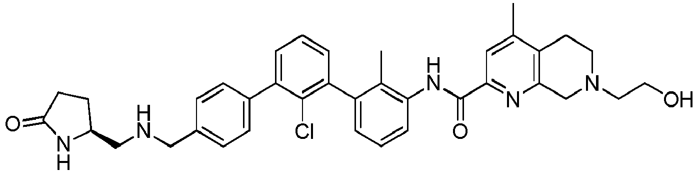

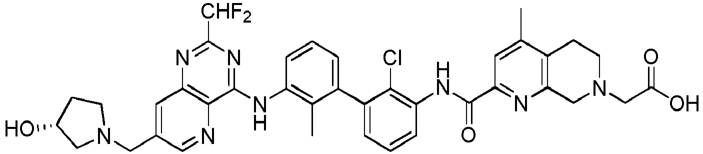

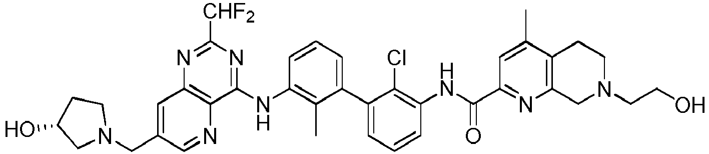

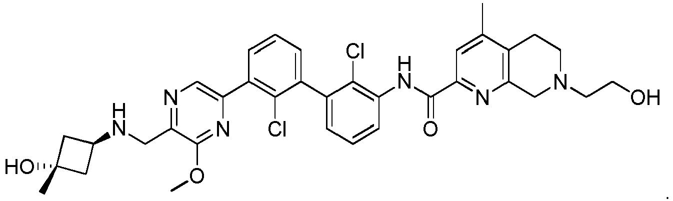

The present disclosure also provides a pharmaceutical composition comprising a therapeutically effective amount of the above-mentioned compound or the pharmaceutically acceptable salt thereof as an active ingredient and a pharmaceutically acceptable carrier.

The present disclosure also provides the use of the above-mentioned compound or the pharmaceutically acceptable salt thereof or the above-mentioned composition in the manufacture of a PD-1/PD-L1 inhibitor.

In some embodiments of the present disclosure, the above-mentioned PD-1/PD-L1 inhibitor is an anti-tumor drug.

### Definition and description

Unless otherwise specified, the following terms and phrases when used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium, or similar salts. When the compound of the present disclosure contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and salts of amino acid (such as arginine and the like), and a salt of an organic acid such as glucuronic acid and the like. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof.

Unless otherwise specified, the term "effective amount" or "therapeutically effective amount" refers to an amount that is nontoxic but achieves the desired effect. The determination of the effective amount varies from person to person, depends on the age and general condition of the recipient, and also depends on the specific active substance, and the appropriate effective amount in individual cases can be determined by those skilled in the art based on routine tests.

The compounds of the present disclosure may exist in specific geometric or stereoisomeric forms. The present disclosure contemplates all such compounds, including cis and trans isomers, (-)-and (+)-enantiomers, (*R*)-and (*S*)-enantiomers, diastereomers isomers, (D)-isomers, (L)-isomers, and racemic and other mixtures thereof, such as enantiomers or diastereomeric enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(*D*)" or "(+)" refers to dextrorotation, "(*L*)" or "(-)" refers to levorotation, and "(*DL*)" or "(±)" refers to racemic.

Unless otherwise specified, the absolute configuration of a stereogenic center is represented by a wedged solid bond ( 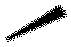 ) and a wedged dashed bond ( 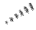 ), and the relative configuration of a stereogenic center is represented by a straight solid bond ( 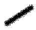 ) and a straight dashed bond ( 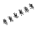 ), a wave line ( 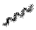 ) is used to represent a wedged dashed bond ( 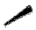 ) or a wedged dashed bond ( 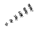 ), or the wave line ( 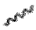 ) is used to represent a straight solid bond ( 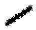 ) and a straight dashed bond ( 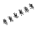 ).

Unless otherwise specified, when double bond structure, such as carbon-carbon double bond, carbon-nitrogen double bond, and nitrogen-nitrogen double bond, exists in the compound, and each of the atoms on the double bond is connected to two different substituents (including the condition where a double bond contains a nitrogen atom, the lone pair of electrons attached on the nitrogen atom is regarded as a substituent connected), if the atom on the double bond in the compound is connected to its substituent by a wave line ( 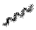 ), this refers to the (Z)-isomer, (E)-isomer or a mixture of two isomers of the compound. For example, the following formula (A) means that the compound exists as a single isomer of formula (A-1) or formula (A-2) or as a mixture of two isomers of formula (A-1) and formula (A-2); the following formula (B) means that the compound exists in the form of a single isomer of formula (B-1) or formula (B-2) or in the form of a mixture of two isomers of formula (B-1) and formula (B-2). The following formula (C) means that the compound exists as a single isomer of formula (C-1) or formula (C-2) or as two a mixture of two isomers of formula (C-1) and formula (C-2).

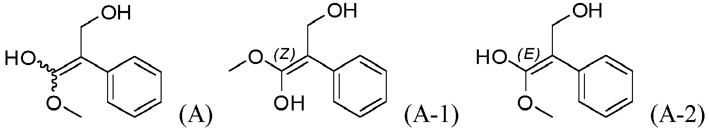

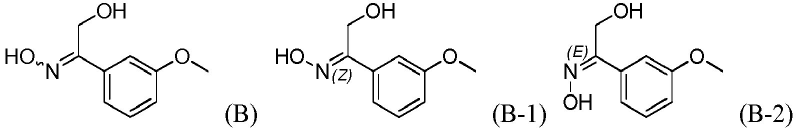

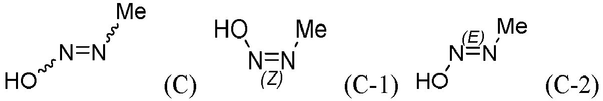

The compounds of the present disclosure may exist in specific. Unless otherwise specified, the term "tautomer" or "tautomeric form" means that at room temperature, the isomers of different functional groups are in dynamic equilibrium and can be transformed into each other quickly. If tautomers possibly exist (such as in solution), the chemical equilibrium of tautomers can be reached. For example, proton tautomer (also called prototropic tautomer) includes interconversion through proton migration, such as keto-enol isomerization and imine-enamine isomerization. Valence tautomer includes some recombination of bonding electrons for mutual transformation. A specific example of keto-enol tautomerization is the tautomerism between two tautomers of pentane-2,4-dione and 4-hydroxypent-3-en-2-one.

Unless otherwise specified, the terms "enriched in one isomer", "enriched in isomers", "enriched in one enantiomer" or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (R)- and (*S*)-isomer, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the present disclosure may contain an unnatural proportion of atomic isotope at one or more than one atom(s) that constitute the compound. For example, the compound can be radiolabeled with a radioactive isotope, such as tritium (³H), iodine-125 (¹²⁵I) or C-14 (¹⁴C). For another example, deuterated drugs can be formed by replacing hydrogen with heavy hydrogen, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, etc. All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted with the substituent, including deuterium and hydrogen variables, as long as the valence of the specific atom is normal and the substituted compound is stable. When the substituent is an oxygen (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)o-, it means that the linking group is a single bond, and -Co alkyl-A means that the structure is actually -A.

When a substituent is 0, it means that the substituent does not exist, for example, -A-(R)o means that the structure is actually -A.

When a substituent is vacant, it means that the substituent does not exist, for example, when X is vacant in A-X, the structure of A-X is actually A.

When one of the variables is selected from a single bond, it means that the two groups linked by the single bond are connected directly. For example, when L in A-L-Z represents a single bond, the structure of A-L-Z is actually A-Z.

When the bond of a substituent can be cross-linked to two or more atoms on a ring, such a substituent can be bonded to any atom on the ring, for example, a structural moiety 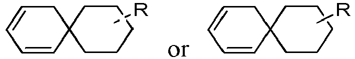 represents that the substituent R can be substituted at any position on cyclohexyl or cyclohexadiene. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring.

When the enumerative linking group does not indicate the direction for linking, the direction for linking is arbitrary, for example, the linking group L contained in 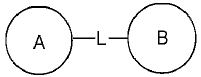 is -M-W-, then -M-W- can link ring A and ring B to form 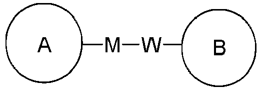 in the direction same as left-to-right reading order, and form 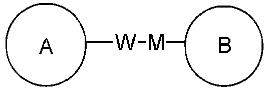 in the direction contrary to left-to-right reading order. A combination of the linking groups, substituents and/or variables thereof is allowed only when such combination can result in a stable compound.

Unless otherwise specified, when a group has one or more linkable sites, any one or more sites of the group can be linked to other groups through chemical bonds. The chemical bond between the site and other groups can be represented by a straight solid bond ( 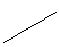 ), a straight dashed bond ( 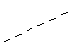 ) or a wavy line ( 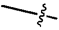 ). For example, the straight solid line bond in -OCH₃ represents that it is connected to other groups through the oxygen atom in the group; the straight dashed bond in 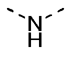 represents that it is connected to other groups through both ends of the nitrogen atom in the group; the wavy line in 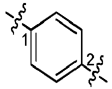 represents that it is connected to other groups through the carbon atoms in the 1 and 2 positions of the phenyl group.

Unless otherwise specified, the number of atoms in a ring is generally defined as the number of elements of a ring, e.g., "5- to 7- membered ring" refers to a "ring" composed of 5-7 joined atoms.

Unless otherwise specified, the term C₀ represents the absence of C or a single bond; e.g., C₀₋₆ alkyl represents the absence of C and C₁₋₆ alkyl, or represents a single bond and C₁₋₆ alkyl, -C₀₋₆ alkyl-C₃₋₆ cycloalkyl represents -C₃₋₆ cycloalkyl and -C₁₋₆ alkyl-C₃₋₆ cycloalkyl.

Unless otherwise specified, the term "C₁₋₆ alkyl" refers to a linear or branched saturated hydrocarbon group containing 1 to 6 carbon atoms. The C₁₋₆ alkyl includes C₁₋₅, C₁₋₄, C₁₋₃, C₁₋₂, C₂₋₆, C₂₋₄, C₆ and C₅ alkyl groups and the like. It can be monovalent (such as methyl), divalent (such as methylene) or multivalent (such as methine). Examples of C₁₋₆ alkyl include but are not limited to methyl (Me), ethyl (Et), propyl (including *n*-propyl and isopropyl), butyl (including *n*-butyl, isobutyl, *s-*butyl, and *t*-butyl), pentyl (including *n*-pentyl, isopentyl and neopentyl), hexyl, etc.

Unless otherwise specified, the term "C₁₋₃ alkyl" refers to a linear or branched saturated hydrocarbon group containing 1 to 3 carbon atoms. The C₁₋₃ alkyl group includes C₁₋₂ and C₂₋₃ alkyl groups and the like; it can be monovalent (such as methyl), divalent (such as methylene) or multivalent (such as methine). Examples of C₁₋₃ alkyl include, but are not limited to methyl (Me), ethyl (Et), propyl (including *n*-propyl and isopropyl), etc.

Unless otherwise specified, the term "C₂₋₆ alkenyl" is used to represent a straight or branched chain hydrocarbon group composed of 2 to 6 carbon atoms in which contains at least one carbon-carbon double bond, wherein the carbon-carbon double bond can be located at any position of the group. The C₂₋₆ alkenyl includes C₂₋₄, C₂₋₃, C₄, C₃ and C₂ alkenyl, etc.; it may be monovalent, divalent or multivalent. Examples of C₂₋₆ alkenyl include, but are not limited to, vinyl, propenyl, butenyl, pentenyl, hexenyl, butadienyl, pentadienyl, hexamadienyl, etc.

Unless otherwise specified, the term "C₂₋₆ alkynyl" is used to represent a straight or branched chain hydrocarbon group composed of 2 to 6 carbon atoms in which contains at least one carbon-carbon triple bond, wherein the carbon-carbon triple bond can be located at any position of the group. The C₂₋₆ alkynyl includes C₂₋₄, C₂₋₃, C₄, C₃ and C₂ alkynyl, etc. It may be monovalent, bivalent or multivalent. Examples of C₂₋₆ alkynyl include, but are not limited to, ethynyl, propynyl, butynyl, pentynyl, etc.

Unless otherwise specified, the term "halo" or "halogen" by itself or as part of another substituent refers to fluorine, chlorine, bromine or iodine atom.

Unless otherwise specified, the term "3- to 8- membered heterocycloalkyl" by itself or in combination with other terms represents a saturated cyclic group composed of 3 to 8 ring atoms, respectively, of which 1, 2, 3 or 4 ring atoms are heteroatoms independently selected from O, S, and N, and the rest are carbon atoms, wherein the nitrogen atom is optionally quaternized, and the carbon, nitrogen, and sulfur atoms are optionally oxidized (i.e., C(=O), NO and S(O)p, where p is 1 or 2). It includes monocyclic and bicyclic ring systems, wherein bicyclic ring systems include spiro, fused and bridged rings. Furthermore, with regard to the "3- to 8- membered heterocycloalkyl", a heteroatom may occupy the connection position between the heterocycloalkyl and the rest of the molecule. The 3- to 8- membered heterocycloalkyl includes 3- to 6- membered, 3- to 5- membered, 4- to 6- membered, 5- to 6- membered, 4 membered, 5 membered and 6 membered heterocycloalkyl, etc. Examples of 3- to 8- membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2 -piperidinyl and 3-piperidinyl, etc.), piperazinyl (including 1-piperazinyl and 2-piperazinyl, etc.), morpholinyl (including 3-morpholinyl and 4-morpholinyl, etc.), dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, etc.

Unless otherwise specified, the term "3- to 6- membered heterocycloalkyl" by itself or in combination with other terms represents a saturated cyclic group composed of 3 to 6 ring atoms, respectively, of which 1, 2, 3 or 4 ring atoms are heteroatoms independently selected from O, S, and N, and the rest are carbon atoms, wherein the nitrogen atom is optionally quaternized, and the carbon, nitrogen, and sulfur atoms are optionally oxidized (i.e., C(=O), NO and S(O)p, where p is 1 or 2). It includes monocyclic and bicyclic ring systems, wherein bicyclic ring systems include spiro, fused and bridged rings. Furthermore, with regard to the "3- to 6- membered heterocycloalkyl", a heteroatom may occupy the connection position between the heterocycloalkyl and the rest of the molecule. The 3- to 6- membered heterocycloalkyl includes 4- to 6- membered, 5- to 6- membered, 4 membered, 5 membered and 6 membered heterocycloalkyl ,etc. Examples of 3- to 6- membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2 -piperidinyl and 3-piperidinyl, etc.), piperazinyl (including 1-piperazinyl and 2-piperazinyl, etc.), morpholinyl (including 3-morpholinyl and 4-morpholinyl, etc.) , dioxanyl, dithianyl, isoxazolidinyl, isothiazolidinyl, 1,2-oxazinyl, 1,2-thiazinyl, hexahydropyridazinyl, homopiperazinyl, or homopiperidinyl etc.

Unless otherwise specified, the term "3- to 10- membered heterocycloalkyl" by itself or in combination with other terms respectively represents a saturated cyclic group composed of 3 to 10 ring atoms, of which 1, 2, 3 or 4 ring atoms are heteroatoms independently selected from O, S, and N, and the rest are carbon atoms, wherein the nitrogen atom is optionally quaternized, and the carbon, nitrogen, and sulfur atoms are optionally oxidized (i.e., C(=O), NO and S(O)p, where p is 1 or 2). It includes monocyclic, bicyclic and tricyclic rings, wherein bicyclic and tricyclic rings include spiro, fused and bridged rings. Furthermore, with regard to the "3- to 10- membered heterocycloalkyl", a heteroatom may occupy the connection position between the heterocycloalkyl and the rest of the molecule. The 3- to 10- membered heterocycloalkyl includes 3- to 8- membered, 3- to 6- membered, 3- to 5- membered, 4- to 6- membered, 5- to 6- membered, 4 membered, 5 membered and 6 membered heterocycloalkyl, etc. Examples of 3- to 10- membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2 -piperidinyl and 3-piperidinyl, etc.), piperazinyl (including 1-piperazinyl and 2-piperazinyl, etc.), morpholinyl (including 3-morpholinyl and 4-morpholinyl, etc.) , dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, etc.

Unless otherwise specified, the term "5- to 10- membered heterocycloalkenyl" by itself or in combination with other terms respectively represents a partially unsaturated cyclic group composed of 5 to 10 ring atoms containing at least one carbon-carbon double bond , of which 1, 2, 3 or 4 ring atoms are heteroatoms independently selected from O, S and N, and the rest are carbon atoms, wherein the nitrogen atom is optionally quaternized, and the carbon, nitrogen, and sulfur atoms are optionally oxidized (i.e., C(=O), NO and S(O)p, where p is 1 or 2). It includes monocyclic, bicyclic and tricyclic systems, wherein bicyclic and tricyclic systems include spiro, fused and bridged rings, at least one ring of the system is non-aromatic. Furthermore, with regard to the "5- to 10- membered heterocycloalkenyl", a heteroatom may occupy the connection position between the heterocycloalkenyl and the rest of the molecule. The 5- to 10- membered heterocycloalkenyl includes 5- to 8- membered, 5- to 6-membered, 4- to 5- membered, 4 membered, 5 membered and 6 membered heterocycloalkenyl, etc. Examples of 5- to 10- membered heterocycloalkenyl include, but are not limited to

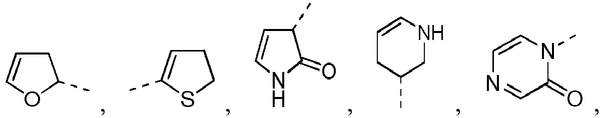

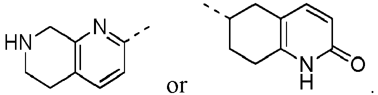

Unless otherwise specified, the terms "5- to 10- membered heteroaryl ring" and "5- to 10- membered heteroaryl" can be used interchangeably in the present disclosure. The term "5- to 10- membered heteroaryl" refers to a cyclic group consisting of 5 to 10 ring atoms, which has a conjugated π-electron system, wherein, 1, 2, 3 or 4 ring atoms are heteroatoms independently selected from O, S and N, and the rest are carbon atoms. It can be a monocyclic, fused bicyclic or fused tricyclic ring system, wherein each ring is aromatic. Wherein, the nitrogen atom is optionally quaternized, the carbon, nitrogen and sulfur heteroatoms can optionally be oxidized (i.e. C(=O), NO and S(O)ₚ, p is 1 or 2). The 5- to 10- membered heteroaryl can be attached to the rest of the molecule through a heteroatom or a carbon atom. The 5- to 10-membered heteroaryl include 5- to 8- membered, 5- to 7- membered, 5- to 6- membered, 5 and 6 membered heteroaryl, etc. Examples of the 5-10 membered heteroaryl include but are not limited to pyrrolyl (including N-pyrrolyl, 2-pyrrolyl, 3-pyrrolyl, etc.), pyrazolyl (including 2-pyrazolyl and 3-pyrrolyl, etc.), imidazolyl (including N-imidazolyl, 2-imidazolyl, 4-imidazolyl and 5-imidazolyl, etc.), oxazolyl (including 2-oxazolyl, 4-oxazolyl and 5-oxazolyl, etc.), triazolyl (1H-1,2,3-triazolyl, 2H-1,2,3-triazolyl, 1H-1,2,4-triazolyl and 4H-1, 2,4-triazolyl, etc.), tetrazolyl, isoxazolyl (3-isoxazolyl, 4-isoxazolyl and 5-isoxazolyl, etc.), thiazolyl (including 2-thiazolyl, 4-thiazolyl and 5-thiazolyl, etc.), furyl (including 2-furyl and 3-furyl, etc.), thienyl (including 2-thienyl and 3-thienyl, etc.), pyridyl (including 2 -pyridyl, 3-pyridyl and 4-pyridyl, etc.), pyrazinyl, pyrimidinyl (including 2-pyrimidinyl and 4-pyrimidinyl, etc.), benzothiazolyl (including 5-benzothiazolyl, etc.) ), purinyl, benzimidazolyl (including 2-benzimidazolyl, etc.), benzoxazolyl, indolyl (including 5-indolyl, etc.), isoquinolinyl (including 1-isoquinolinyl base and 5-isoquinolinyl, etc.), quinoxalinyl (including 2-quinoxalinyl and 5-quinoxalinyl, etc.) or quinolinyl (including 3-quinolinyl and 6-quinolinyl, etc. ).

Unless otherwise specified, Cₙ₋ₙ₊ₘ or Cₙ₋Cₙ₊ₘ includes any specific case of n to n+m carbons, for example, C₁₋₁₂ includes C₁, C₂, C₃, C₄, C₅, C₆, C₇, C₈, C₉, C₁₀, C₁₁, and C₁₂, and any range from n to n+m is also included, wherein n and m are natural numbers, for example C₁₋₁₂ includes C₁₋₃, C₁₋₆, C₁₋₉, C₃₋₆, C₃₋₉, C₃₋₁₂, C₆₋₉, C₆₋₁₂, and C₉₋₁₂, etc.; similarly, n membered to n+m membered means that the number of atoms on the ring is from n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, and any range from n to n+m is also included, for example, 3-12 membered ring includes 3-6 membered ring, 3-9 membered ring, 5-6 membered ring, 5-7 membered ring, 6-7 membered ring, 6-8 membered ring, and 6-10 membered ring, etc.; in particular, C₀ represents absence.

The term "leaving group" refers to a functional group or atom which can be replaced by another functional group or atom through a substitution reaction (such as affinity substitution reaction). For example, representative leaving groups include triflate; chlorine, bromine, and iodine; sulfonate group, such as mesylate, tosylate, p-bromobenzenesulfonate, p-toluenesulfonates and the like; acyloxy, such as acetoxy, trifluoroacetoxy and the like.

The term "protecting group" includes, but is not limited to "amino protecting group", "hydroxy protecting group" or "thio protecting group". The term "amino protecting group" refers to a protecting group suitable for blocking the side reaction on the nitrogen of an amino. Representative amino protecting groups include, but are not limited to: formyl; acyl, such as alkanoyl (e.g., acetyl, trichloroacetyl or trifluoroacetyl); alkoxycarbonyl, such as tert-butoxycarbonyl (Boc); arylmethoxycarbonyl such as benzyloxycarbonyl (Cbz) and 9-fluorenylmethoxycarbonyl (Fmoc); arylmethyl, such as benzyl (Bn), trityl (Tr), 1,1-bis-(4'-methoxyphenyl)methyl; silyl, such as trimethylsilyl (TMS) and tert-butyldimethylsilyl (TBS) and the like. The term "hydroxy protecting group" refers to a protecting group suitable for blocking the side reaction on hydroxy. Representative hydroxy protecting groups include, but are not limited to: alkyl, such as methyl, ethyl, and tert-butyl; acyl, such as alkanoyl (e.g., acetyl); arylmethyl, such as benzyl (Bn), p-methoxybenzyl (PMB), 9-fluorenylmethyl (Fm), and diphenylmethyl (benzhydryl, DPM); silyl, such as trimethylsilyl (TMS) and tert-butyl dimethyl silyl (TBS) and the like.

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure.

The structure of the compounds of the present disclosure can be confirmed by conventional methods known to those skilled in the art, and if the disclosure involves an absolute configuration of a compound, then the absolute configuration can be confirmed by means of conventional techniques in the art. For example, in the case of single crystal X-ray diffraction (SXRD), the absolute configuration can be confirmed by collecting diffraction intensity data from the cultured single crystal using a Bruker D8 venture diffractometer with CuKα radiation as the light source and scanning mode: ϕ/ω scan, and after collecting the relevant data, the crystal structure can be further analyzed by direct method (Shelxs97).

The solvent used in the present disclosure is commercially available.

Unless otherwise specified, the reagent ratio used in silica gel column chromatography and silica gel thin-layer chromatography in the present disclosure is volume ratio.

The following abbreviations are used in the present disclosure: HATU stands for O-(7-azabenzotriazol-1-yl)-N,N,N',N'-tetramethylurea hexafluorophosphate; DMSO stands for dimethyl sulfoxide; CD₃OD stands for deuterated methanol; CDCl₃ stands for deuterated chloroform; TBSO stands for tert-butyldimethylsilyloxy, Alloc stands for allyloxycarbonyl.

The compounds of the present disclosure are named according to the conventional naming principles in the art or by ChemDraw^{®} software, and the commercially available compounds use the supplier catalog names.

### Detailed description of the embodiment

The present disclosure will be specifically described below by way of embodiments carried out, but the scope of the present disclosure is not limited thereto. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, preferred implementations include but are not limited to the embodiments of the present disclosure. For those skilled in the art, it is obvious that various changes and improvements can be made to the specific embodiments of the present disclosure without departing from the spirit and scope of the present disclosure.

### Synthesis of the intermediates

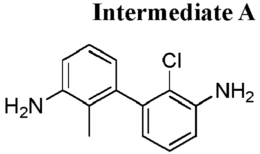

### Synthesis route:

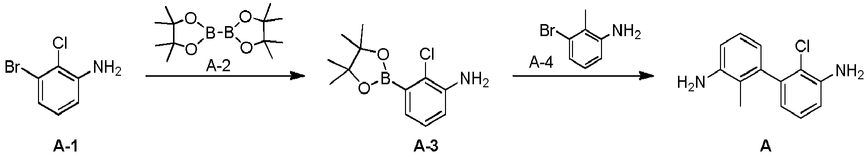

### Step 1

Compound A-1 ((10 g, 48.43 mmol)) and compound A-2 (15.99 g, 62.96 mmol) were dissolved in dioxane (100 mL), and 1,1'- bis(diphenylphosphonium)ferrocene palladium chloride (3.54 g, 4.84 mmol) and potassium acetate (11.88 g, 121.08 mmol) were added thereto. After the air was replaced with nitrogen, the reaction mixture was and stirred at 80 °C for 15 hours under nitrogen protection. After the reaction was completed, the reaction mixture was filtered and concentrated to obtain the crude product compound A-3.

MS-ESI calculated value [M+H]⁺ 254, measured value 254.

### Step 2

Compound A-3 (12.28 g, 48.44 mmol) and compound A-4 (9.01 g, 48.44 mmol, 5.97 mL) were dissolved in dioxane (100 mL), and water (6 mL) was added thereto, then 1,1'-bis(diphenylphosphonium)ferrocene palladium chloride dichloromethane (3.96 g, 4.84 mmol) and potassium carbonate (16.74 g, 121.09 mmol) were added to the reaction mixture. After the air was replaced with nitrogen, the reaction mixture was stirred at 80°C for 15 hours under nitrogen protection. After the reaction was completed, the reaction mixture was filtered and concentrated, water (200 mL) was added to the concentrated residue, followed by extraction with ethyl acetate (300 mL). The combined organic phase was dried over anhydrous sodium sulfate, then filtered, evaporated to dryness, separated and purified by silica gel column chromatography (petroleum ether: ethyl acetate=30:1 to 10:1) to obtain product intermediate A.

MS-ESI calculated value [M+H]⁺ 233, measured value 233.

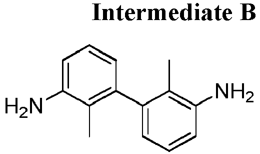

### Synthesis route:

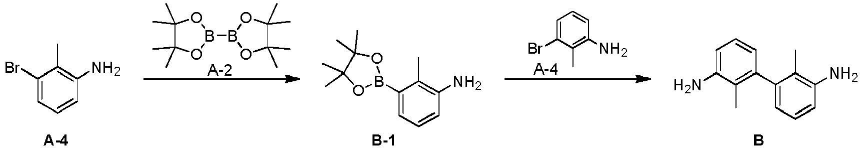

### Step 1

Compound A-4 (5 g, 26.87 mmol) and compound A-2 (7.51 g, 29.56 mmol) were dissolved in dioxane (50 mL), and 1,1'-bis( diphenylphosphonium) ferrocene palladium chloride in dichloromethane (2.19 g, 2.69 mmol) and potassium acetate (7.91 g, 80.62 mmol) were added thereto. After the air was replaced with nitrogen, the reaction mixture was stirred at 80 °C for 15 hours under nitrogen protection. After the reaction was completed, the reaction mixture was filtered and concentrated to obtain crude product B-1.

MS-ESI calculated value [M+H]⁺ 234, measured value 234.

### Step 2

Compound B-1 (6.26 g, 26.85 mmol) and compound A-4 (5.00 g, 26.85 mmol) were dissolved in dioxane (60 mL) and water (5 mL) was added thereto. [1,1'-bis(diphenylphosphino)ferrocene]dichloropalladium in dichloromethane (2.19 g, 2.69 mmol) and potassium carbonate (11.13 g, 80.56 mmol) were added to the reaction mixture. After the air was replaced with nitrogen, the reaction mixture was stirred at 80 °C for 15 hours under nitrogen protection. After the reaction was completed, the reaction mixture was filtered and concentrated, water (200 mL) was added to the concentrated residue, then the reaction mixture was extracted with ethyl acetate (300 mL). The combined organic phase was dried over anhydrous sodium sulfate, filtered, evaporated to dryness, separated and purified by silica gel column chromatography (petroleum ether: ethyl acetate = 50: 1 to 3: 1) to obtain product intermediate B.

MS-ESI calculated value [M+H]⁺ 213, measured value 213.

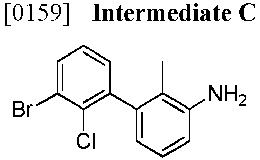

### Synthesis route:

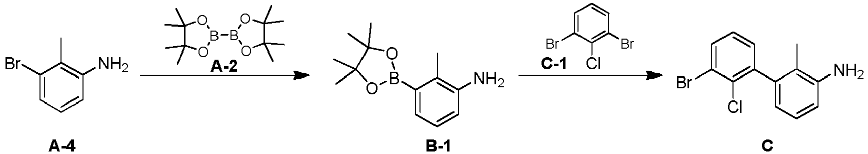

### Step 1

Compound A-4 (5 g, 26.87 mmol, 3.31 mL) and A-2 (7.09 g, 29.56 mmol) were dissolved in dioxane (50 mL), then [1,1'-bis(diphenylphosphino)ferrocene]dichloropalladium (1.97 g, 2.69 mmol), potassium acetate (7.91 g, 80.62 mmol) were added thereto. After the air in the solution was replaced with nitrogen three times, the reaction mixture was stirred at 80 °C for 12 hours under nitrogen protection. After the reaction was completed, the reaction mixture was filtered and the filtrate was evaporated to dryness to obtain the crude compound B-1.

MS-ESI calculated value [M+H]⁺ 234, measured value 234.

### Step 2

Compound B-1 (6.26 g, 26.85 mmol) and C-1 (5.81 g, 21.48 mmol) were dissolved in dioxane (50 mL) and water (5 mL), then [1,1-bis(diphenylphosphino)ferrocene]dichloropalladium (1.96 g, 2.69 mmol) and potassium carbonate (11.13 g, 80.56 mmol) were added thereto. The solution was replaced with nitrogen three times, and the reaction mixture was stirred at 80 °C for 12 hours under nitrogen protection. After the reaction was completed, the reaction mixture was diluted with 100 mL of water and extracted with 300 mL of ethyl acetate (100 mL×3). The organic phase was washed with 200 mL of brine (100 mL×2), dried over anhydrous sodium sulfate, filtered and concentrated under reduced pressure. The residue was subjected to silica gel column chromatography (petroleum ether: ethyl acetate=100:1 to 20:1) to obtain intermediate C.

MS-ESI calculated value [M+H]⁺ 296, measured value 296. ¹H NMR (DMSO-d₆, 400 MHz) δ 7.7-7.8 (m, 1H), 7.3-7.3 (m, 1H), 7.2-7.3 (m, 1H), 6.9-7.0 (m, 1H), 6.68 (dd , 1H, J=0.9, 7.9 Hz), 6.31 (dd, 1H, J=0.8, 7.4 Hz), 4.96 (s, 2H), 1.7-1.8 (m, 3H), 1.16 (s, 1H)

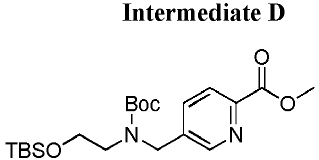

### Synthesis route:

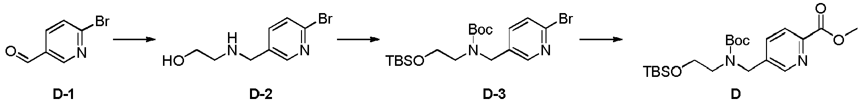

### Step 1

Compound D-1 (50 g, 269 mmol) was dissolved in methanol (500 mL), and ethanolamine (17.2 g, 282 mmol, 17.1 mL) was added to the reaction mixture and stirred for 1 hour. Then sodium cyanoborohydride (25.3 g, 403 mmol) was added and stirred at 25 °C for 12 hours. After the reaction was completed, the reaction mixture was concentrated under reduced pressure to obtain the crude compound D-2.

MS-ESI calculated value [M+H]⁺ 231 and 233, measured value 231 and 233.

### Step 2

Compound D-2 (63 g, 273 mmol) was dissolved in dichloromethane (800 mL), N,N-diisopropylethylamine (282 g, 2.18 mol, 380 mL) and di-tert-butyl dicarbonate (89.3 g, 409 mmol, 94.0 mL) were added to the reaction mixture, and the reaction mixture was stirred at 25 °C for 0.5 hours. Then tert-butyldimethylsilyl chloride (205 g, 1.36 mol, 167 mL) was added to the reaction mixture and stirred at 25 °C for 12 hours. After the reaction was completed, water (1500 mL) was slowly added to the reaction mixture, and the reaction mixture was extracted with dichloromethane (800 mL×2). The combined organic phase was dried over anhydrous sodium sulfate, filtered and concentrated under reduced pressure. The residue was separated and purified by silica gel column chromatography (petroleum ether: ethyl acetate=1:0 to 20:1) to obtain compound D-3.

MS-ESI calculated value [M+H]⁺ 445 and 447, measured value 445 and 447.

### Step 3

Compound D-3 (58.4 g, 127 mmol) was dissolved in methanol (600 mL) and N,N-dimethylformamide (60 mL), triethylamine (32.1 g, 317 mmol, 44.2 mL) and [1,1'-bis(diphenylphosphino)ferrocene]dichloropalladium (9.28 g, 12.7 mmol) was added to the reaction mixture. After the air was replaced three times, then the reaction mixture was stirred at 80°C for 12 hours under carbon monoxide gas (pressure: 50 psi). After the reaction was completed, the reaction mixture was filtered, the filtrate was concentrated in vacuum, water (1000 mL) was added to the concentrated residue and extracted with ethyl acetate (800 mL×3). The combined organic phase was dried over anhydrous sodium sulfate, filtered and concentrated under reduced pressure. The residue was separated and purified by silica gel column chromatography (petroleum ether: ethyl acetate=10:1 to 5:1) to obtain intermediate D.

MS-ESI calculated value [M+H]⁺ 425, measured value 425.

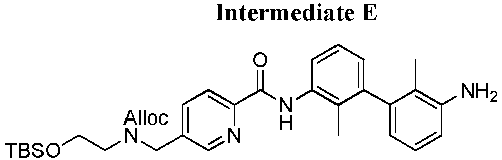

### Synthesis route:

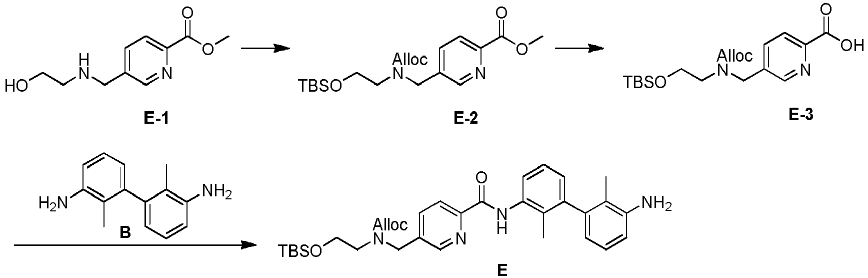

### Step 1

Compound E-1 (2.8 g, 13.32 mmol) was dissolved in dichloromethane (30 mL), diisopropylethylamine (13.77 g, 106.55 mmol) and allyl chloroformate (1.93 g, 15.98 mmol) were added thereto, and the reaction mixture was stirred at 25°C for 0.5 hours. Tert-butyldimethylsilyl chloride (6.02 g, 39.96 mmol) was added thereto, and the mixture was stirred at 25°C for 16 hours. After the reaction was completed, water (50 mL) was added to the reaction mixture, and stirred for 30 minutes, then extracted with dichloromethane (30 mL×2). The organic phase was dried over anhydrous sodium sulfate, filtered, and concentrated, and the crude product was purified by silica gel column chromatography (petroleum ether: ethyl acetate = 20:1 to 2:1) to obtain compound E-2.

MS-ESI calculated value [M+H]⁺ 409, measured value 409.

### Step 2

Compound E-2 (1.15 g, 2.81 mmol) was dissolved in a mixed solution of methanol (5 mL) and water (0.1 mL), lithium hydroxide monohydrate (153.55 mg, 3.66 mmol) was added thereto, and the reaction mixture was stirred at 25°C for 16 hours. After the reaction was completed, the pH of the reaction mixture was adjusted to 8 with hydrochloric acid (1 mol/L), and then concentrated. Compound E-3 was obtained and used directly in the next step.

MS-ESI calculated value [M+H]⁺ 395, measured value 395.

### Step 3

Compound E-3 (650 mg, 1.65 mmol) and intermediate B (559.60 mg, 2.64 mmol) were dissolved in N,N-dimethylformamide (10 mL), diisopropylethylamine (638.79 mg, 4.94 mmol) and HATU (751.72 mg, 1.98 mmol) were added thereto, the reaction mixture was stirred at 25°C for 16 hours. After the reaction was completed, the reaction mixture was concentrated. The crude product was purified by silica gel thin layer chromatography (petroleum ether: ethyl acetate=2:1) to obtain compound E.

MS-ESI calculated value [M+H]⁺ 589, measured value 589.

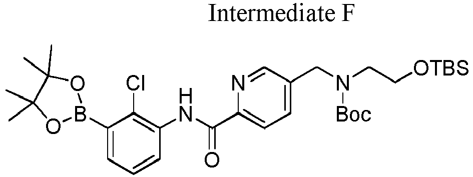

### Synthesis route:

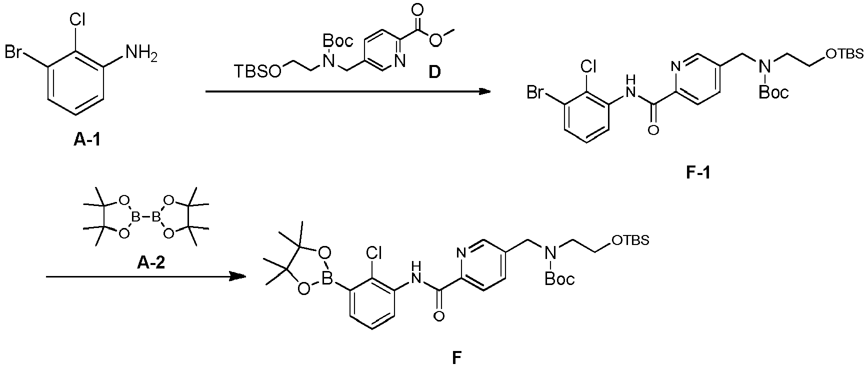

### Step 1

Compound D (1 g, 2.17 mmol) and compound A-1 (537 mg, 2.60 mmol) were dissolved in toluene (15 mL), and lithium hexamethyldisilazide (1 M in tetrahydrofuran, 4.3 mL) was added dropwise to the reaction mixture at 25 °C. The reaction mixture was stirred at 25°C for 2 hours. After the reaction was completed, saturated ammonium chloride solution (10 mL) was added thereto to quench the reaction at 25°C, the reaction mixture was diluted with water (10 mL), and then extracted with ethyl acetate (15 mL × 2). The combined organic phase was dried over anhydrous sodium sulfate, filtered and concentrated under reduced pressure. The residue was separated and purified by silica gel column chromatography (petroleum ether: ethyl acetate=50:1 to 20:1) to obtain compound F-1.

MS-ESI calculated value [M+H]⁺ 598 and 600, measured value 598 and 600.

### Step 2

Compound F-1 (100 mg, 164 mmol) and compound A-2 (58.3 mg, 230 mmol) were dissolved in dioxane (2 mL), [1,1'-bis(diphenylphosphino)ferrocene]dichloropalladium (12.0 mg, 16.4 mmol) and potassium acetate (32.2 mg, 328 mmol) were added thereto. After the air was replaced with nitrogen, the reaction mixture was stirred at 80 °C for 12 hours under nitrogen protection. The raw material F-1 was not completely consumed, and the reaction mixture was stirred for 5 hours at 90 °C under nitrogen protection after the air was replaced with nitrogen. After the reaction was completed, the reaction mixture was filtered and concentrated under reduced pressure to obtain the crude compound F.

MS-ESI calculated value [M+H]⁺ 646 and 648, measured value 646 and 648.

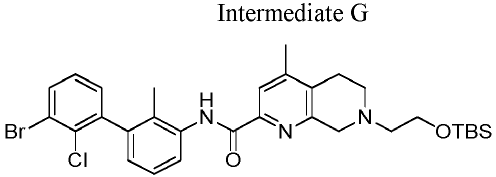

### Synthesis route:

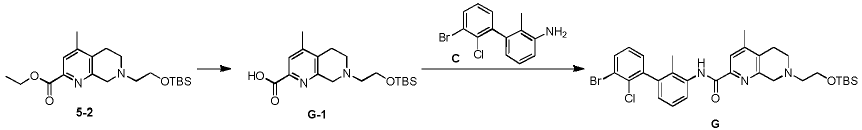

### Step 1

Lithium hydroxide monohydrate (33.25 mg, 792.44 µmol, 1.5 equiv) was dissolved in water (0.5 mL) and added dropwise to compound 5-2 (0.20 g, 528.29 µmol, 1 equiv) in methanol (2 mL), the reaction mixture was stirred at 25 °C for 1 hour. After the reaction was completed, the pH of the reaction mixture was adjusted to 7 with dilute hydrochloric acid (1M), and then concentrated under reduced pressure to obtain compound G-1.

MS-ESI calculated value [M+H]⁺ 351, measured value 351.

### Step 2

Compound G-1 (185 mg, 527.78 µmol, 1 equiv) and C (156.53 mg, 527.78 µmol, 1 equiv) were dissolved in N,N-dimethylformamide (2 mL), then N,N-diisopropylethylamine (204.63 mg, 1.58 mmol, 275.79 µl, 3 equiv), O-(7-azabenzotriazole-1-yl)-N,N, and N,N-tetramethylurea hexafluorophosphine salt (240.81 mg, 633.33 µmol, 1.2 equiv) were added to the reaction mixture, and the reaction mixture was stirred at 25°C for 2 hours. After the reaction was completed, the reaction mixture was diluted with water (20 mL), then extracted with ethyl acetate 60 mL (20 mL×3). The combined organic phase was washed with 40 mL of saturated brine (20 mL×2), dried over anhydrous sodium sulfate, filtered and concentrated under reduced pressure. The residue was purified by silica gel thin layer chromatography (petroleum ether: ethyl acetate=3:1) to obtain compound G.

MS-ESI calculated value [M+H]⁺ 630, measured value 630.

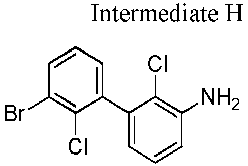

### Synthesis route:

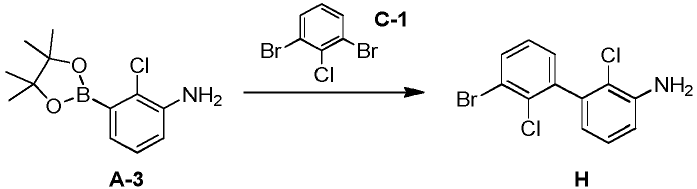

### Step 1

Compounds A-3 (600 mg, 2.37 mmol, 1 equiv) and C-1 (511.84 mg, 1.89 mmol, 0.8 equiv) were dissolved in dioxane (10 mL), [1,1-bis (diphenylphosphino)ferrocene]palladium dichloride (173.16 mg, 236.66 µmol, 0.1 equiv) and potassium carbonate (981.22 mg, 7.10 mmol, 3 equiv) were added thereto. The air in the solution was replaced with nitrogen three times, and the reaction mixture was stirred at 80°C for 12 hours under nitrogen protection. After the reaction was completed, the reaction mixture was diluted with 20 mL of water and extracted with 60 mL of ethyl acetate (20 mL × 3). The organic phase was washed with 40 mL of saturated brine (20 mL × 2), dried over anhydrous sodium sulfate, filtered and concentrated under reduced pressure. The residue was subjected to silica gel thin layer chromatography (petroleum ether: ethyl acetate=5:1) to obtain compound H.

MS-ESI calculated value [M+H]⁺ 317, measured value 317

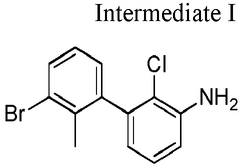

### Synthesis route:

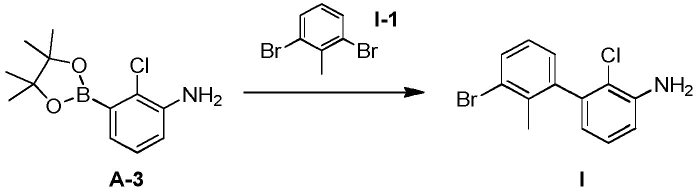

### Step 1

Intermediate A-3 (600 mg, 2.37 mmol, 1 equiv) and intermediate 1-1 (473.18 mg, 1.89 mmol, 0.8 equiv) were dissolved in dioxane (10 mL), [1,1-bis(diphenylphosphino)ferrocene]palladium dichloride (173.16 mg, 236.66 µmol, 0.1 equiv) and potassium carbonate (981.22 mg, 7.10 mmol, 3 equiv) were added thereto. The air in solution was replaced with nitrogen three times, and the reaction mixture was stirred at 80°C for 12 hours under nitrogen protection. After the reaction was completed, the reaction mixture was diluted with 20 mL of water and extracted with 60 mL of ethyl acetate (20 mL × 3). The organic phase was washed with 40 mL of saturated brine (20 mL × 2), dried over anhydrous sodium sulfate, filtered and concentrated under reduced pressure. The residue was subjected to silica gel thin layer chromatography (petroleum ether: ethyl acetate=5:1) to obtain compound I.

MS-ESI calculated value [M+H]⁺ 297, measured value 297.

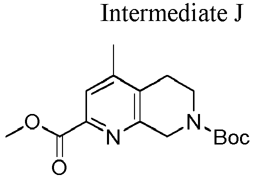

### Synthesis route:

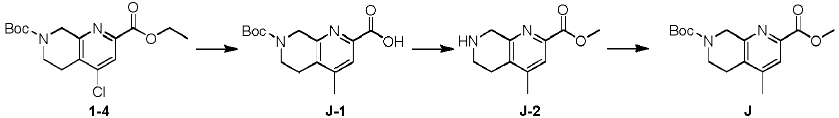

### Step 1

Compound 1-4 (40 g, 99.19 mmol, 1 equiv) and methylboronic acid (8.91 g, 148.79 mmol, 1.5 equiv) were dissolved in dioxane (300 mL) and water (20 mL), [1,1-bis(diphenylphosphino)ferrocene]palladium dichloride dichloromethane (8.10 g, 9.92 mmol, 0.1 equiv) and potassium phosphate (63.17 g, 297.58 mmol, 3 equiv) were to the reaction mixture. The reaction mixture was stirred at 100 °C for 10 hours under nitrogen protection. After the reaction was completed, the reaction mixture was filtered and concentrated. The residue was subjected to silica gel column chromatography (petroleum ether: ethyl acetate=10/1 to 0/1) to obtain compound J-1.

MS-ESI calculated value [M+H]⁺ 293, measured value 293.

### Step 2

Compound J-1 (1 g, 1.95 mmol, 1 equiv) was dissolved in methanol (20 mL), then thionyl chloride (2.30 g, 19.48 mmol, 1.41 mL, 10 equiv) was added thereto, the reaction mixture was stirred at 80 °C for 8 hours under nitrogen protection. After the reaction was completed, the reaction mixture was concentrated under reduced pressure to obtain compound J-2. MS-ESI calculated value [M+H]⁺ 207, measured value 207.

### Step 3

Compound J-2 (15 g, 72.73 mmol, 1 equiv) and di-tert-butyl dicarbonate (23.81 g, 109.10 mmol, 25.06 mL, 1.5 equiv) were dissolved in dichloromethane (10 mL), then N,N-diisopropylethylamine (28.20 g, 218.19 mmol, 38.01 mL, 3 equiv) was added thereto, the reaction was conducted at 20 °C for 1 hour. After the reaction was completed, the reaction mixture was diluted with water (300 mL), then extracted with dichloromethane 400 mL (200 mL×2). The combined organic phase was washed with saturated brine 300 mL (150 mL×2), dried over anhydrous sodium sulfate, filtered, and concentrated. The residue was purified by silica gel column chromatography (petroleum ether: ethyl acetate=20:1 to 5:1) to obtain compound J.

MS-ESI calculated value [M+H]⁺ 306, measured value 306.

¹H NMR (400 MHz, CDCl₃) δ ppm 7.81 (s, 1 H), 4.75 (s, 2 H), 3.98 (s, 3 H), 3.67 - 3.72 (m, 2 H), 2.79 (br t, 2 H), 2.31 (s, 3 H), 1.45 - 1.47 (m, 9 H)

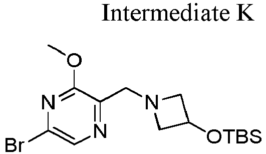

### Synthesis route:

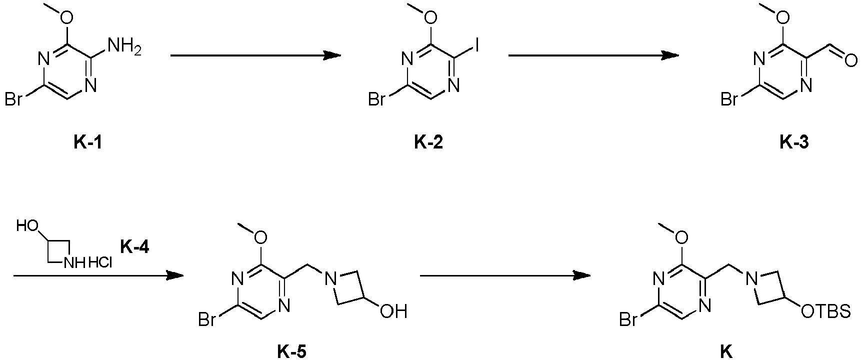

### Step 1

Compound K-1 (10 g, 49.01 mmol, 1 equiv) was added to acetonitrile (100 mL) and water (150 mL), hydroiodic acid aqueous solution (100 mL, 55% by mass) was added thereto, the reaction mixture was stirred at zero °C for 0.5 hours. Sodium nitrite (50.73 g, 735.21 mmol, 15 equiv) was dissolved in water (100 mL) and was added dropwise to the above reaction solution at zero °C. After the dropwise addition was completed, the mixture was stirred at 20 °C for 1 hour, then continued to be stirred at 50 °C for 16 hours. After the reaction was completed, the mixture was cooled to 20 °C, then the mixture was added to a sodium hydroxide aqueous solution (600 mL, 20% by mass) while the temperature was maintained at zero °C. The mixture was extracted with ethyl acetate (500 mL×2). The organic phase was dried over anhydrous sodium sulfate, filtered and concentrated. The residue was subjected to silica gel column chromatography (petroleum ether: ethyl acetate=100:1) to obtain compound K-2.

¹H NMR (400 MHz, CDCl₃) δ = 8.08 (s, 1 H), 4.05 (s, 3 H).

### Step 2

Compound K-2 (9.2 g, 29.22 mmol, 1 equiv) was dissolved in tetrahydrofuran (100 mL), isopropylmagnesium chloride lithium chloride complex (1.3 mol/L, 40.45 mL, 1.8 equiv) was added dropwise at - 60 °C. The reaction mixture was stirred for 30 minutes, then N,N-dimethylformamide (6.41 g, 87.65 mmol, 6.74 mL, 3 equiv) was added at - 40 °C, and the reaction mixture was stirred for 1 hour. After the reaction was completed, a saturated aqueous ammonium chloride solution (100 mL) was carefully added dropwise to the reaction mixture, then extracted with ethyl acetate (50 mL × 2). The organic phase was dried over anhydrous sodium sulfate, filtered and concentrated to obtain compound K-3.

MS-ESI calculated value [M+H]⁺ 217 and 219, measured value 217 and 219.

### Step 3

Compound K-3 (7 g, 32.26 mmol, 1 equiv) and compound K-4 (5.30 g, 48.38 mmol, 1.5 equiv, hydrochloride) were dissolved in methanol (100 mL), diisopropylethylamine (8.34 g, 64.51 mmol, 11.24 mL, 2 equiv) was added thereto and stirred at 25°C for 0.5 hours, then sodium cyanoborohydride (2.43 g, 38.71 mmol, 1.2 equiv) was added and stirred at 25°C for 0.5 hours. After the reaction was completed, water (200 mL) was carefully added to the reaction mixture, then extracted with ethyl acetate (100 mL × 3). The organic phase was dried over anhydrous sodium sulfate, filtered and concentrated to obtain compound K-5.

MS-ESI calculated value [M+H]⁺ 274 and 276, measured value 274 and 276.

### Step 4

Compound K-5 (9 g, 32.83 mmol, 1 equiv) was dissolved in dichloromethane (100 mL), tert-butyldimethylsilyl chloride (12.37 g, 82.08 mmol, 10.06 mL, 2.5 equiv) and imidazole (7.82 g, 114.92 mmol, 3.5 equiv) were added thereto, and the mixture was stirred at 25 °C for 16 hours. After the reaction was completed, water (100 mL) and dichloromethane (100 mL) were added to the reaction mixture and stirred for 15 minutes. The organic phase was separated and dried over anhydrous sodium sulfate, filtered and concentrated. The residue was subjected to silica gel column chromatography (petroleum ether: ethyl acetate=10:1 to 3:1) to obtain compound K.

MS-ESI calculated value [M+H]⁺ 388 and 390, measured value 388 and 390.

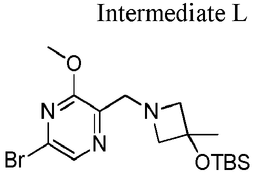

### Synthesis route:

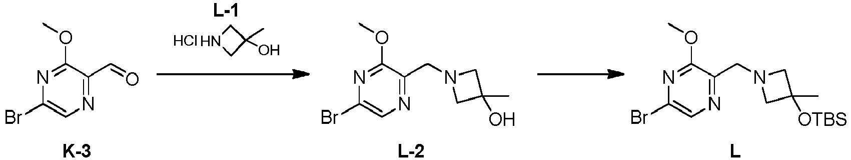

### Step 1

Compound K-3 (5.30 g, 48.38 mmol, hydrochloride) was replaced with compound L-1 (6 g, 27.65 mmol, hydrochloride), and the preparation method is as the preparation method of intermediate K. After the reaction was completed, the reaction mixture was concentrated under reduced pressure to obtain compound L-2.

MS-ESI calculated value [M+H]⁺ 288 and 290, measured value 288 and 290.

### Step 2

Compound K-5 (9 g, 32.83 mmol) was replaced with compound L-2 (5 g, 17.35 mmol, hydrochloride), and the preparation method is as the preparation method of intermediate K. After the reaction was completed, the reaction mixture was concentrated under reduced pressure to obtain compound L.

MS-ESI calculated value [M+H]⁺ 402 and 404, measured value 402 and 404.

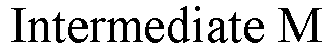

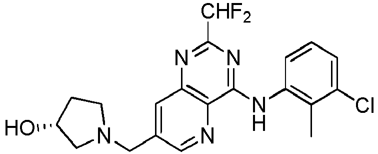

### Synthesis route:

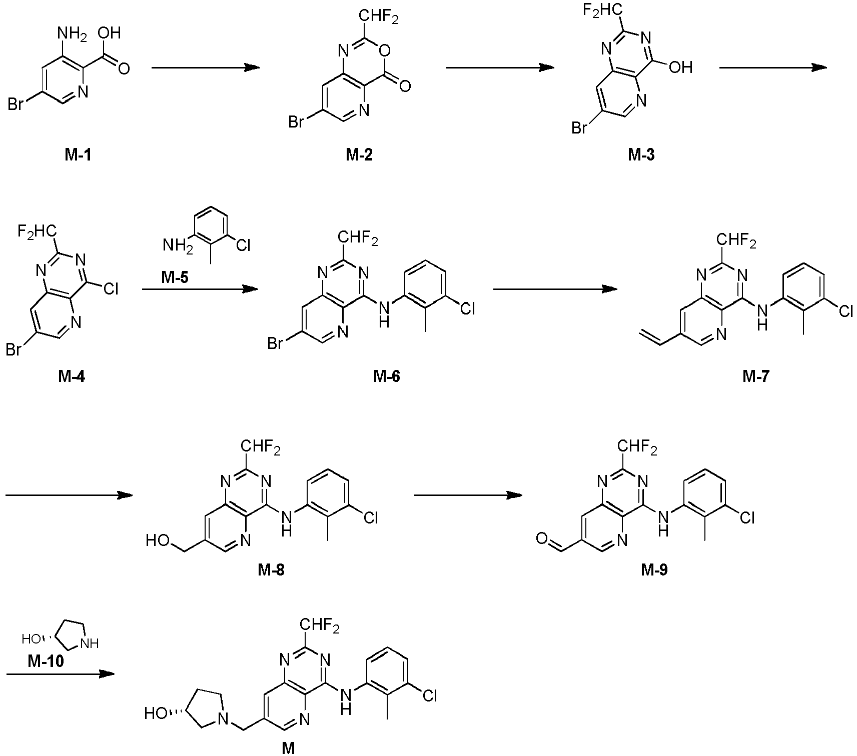

### Step 1

Compound M-1 (9.00 g, 41.5 mmol, 1 equiv) was dissolved in difluoroacetic anhydride (57.7 g, 332 mmol, 8 equiv) and stirred at 60 °C for 3 hours. After the reaction was completed, the reaction mixture was concentrated under reduced pressure to obtain compound M-2.

MS-ESI calculated value [M+H]⁺ 279, measured value 279.

### Step 2

Compound M-2 (5.00 g, 18.1 mmol, 1 equiv) was dissolved in ammonia water (45.5 g, 325 mmol, 50 mL, mass fraction 25%, 18.0 equiv), stirred at 90 °C in a stuffy tank for 3 hours. After the reaction was completed, the reaction mixture was concentrated under reduced pressure to obtain compound M-3.

MS-ESI calculated value [M+H]⁺ 278, measured value 278.

### Step 3

Compound M-3 (31.0 g, 62.3 mmol, 1 equiv) was dissolved in acetonitrile (400 mL), then N,N-diethylaniline (13.9 g, 93.5 mmol, 15.0 mL, 1.5 equiv), phosphorus oxychloride (57.4 g, 374 mmol, 34.8 mL, 6 equiv) and benzyltrimethylammonium chloride (23.2 g, 125 mmol, 21.6 mL, 2 equiv) were added thereto, stirred at 80°C 4 hours. After the reaction was completed, the reaction mixture was concentrated under reduced pressure, and the obtained residue was purified by silica gel column chromatography (petroleum ether: ethyl acetate=100:1 to 10:1) to obtain compound M-4.

MS-ESI calculated value [M+H]⁺ 295, measured value 295.

### Step 4

Compounds M-4 (10.4 g, 25.4 mmol, 1 equiv) and M-5 (3.78 g, 26.7 mmol, 3.18 mL, 1.05 equiv) were dissolved in isopropanol (100 mL), then methanesulfonic acid (2.57 g, 26.7 mmol, 1.90 mL, 1.05 equiv) was added thereto, stirred at 80 °C for 3 hours. After the reaction was completed, the reaction mixture was diluted with water (100 mL), the pH value was adjusted to 7-8 with saturated aqueous sodium bicarbonate solution, then extracted with ethyl acetate (40 mL×3). The combined the organic phases was washed with saturated brine (50 mL×2 ), dried over anhydrous sodium sulfate, concentrated under reduced pressure and filtered, and , and the residue was purified by silica gel column chromatography (petroleum ether: ethyl acetate=200:1 to 100:1) to obtain compound M-6.

MS-ESI calculated value [M+H]⁺ 401, measured value 401.

¹HNMR (400MHz, DMSO-*d*₆,) δ=10.76-10.62 (m, 1H), 9.09 (d, J=2.1 Hz, 1H), 8.67 (d, J=2.1Hz, 1H), 7.52-7.47 (m, 1H) ), 7.43 (dd, J=8.0, 1.00 Hz, 1H), 7.34-7.27 (m, 1H), 6.93-6.46 (m, 1H), 2.25 (s, 3H).

### Step 5

Compound M-6 (1.90 g, 4.75 mmol, 1 equiv), pinacol vinylboronate (1.10 g, 7.13 mmol, 1.21 mL, 1.5 equiv) was dissolved in dioxane (30 mL), water (3 mL), then potassium phosphate (2.52 g, 11.9 mmol, 2.5 equiv), [1,1'-bis(diphenylphosphino)ferrocene]dichloropalladium (348 mg, 475 µmol, 0.1 equiv) were added to the reaction mixture, stirred at 100 °C for 12 hours under nitrogen protection. After the reaction was completed, the reaction mixture was diluted with water (150 mL), then extracted with dichloromethane (100 mL×4). The combined organic phase was dried over anhydrous sodium sulfate, concentrated under reduced pressure and filtered, and the residue was purified by silica gel column chromatography (dichloromethane:ethyl acetate = 1:0 to 10:1) to obtain compound M-7.

MS-ESI calculated value [M+H]⁺ 347, measured value 347.

### Step 6

Compound M-7 (1.32 g, 3.56 mmol, 1 equiv) was dissolved in dichloromethane (100 mL), methanol (20 mL), then the reaction mixture was cooled to -78 °C, stirred for 15 minutes with ozone, then heated up to 25 °C. After purging with nitrogen until the blue color disappeared, sodium borohydride (135 mg, 3.56 mmol, 1 equiv) was added thereto, the mixture was stirred at 25 °C for 0.5 hours. After the reaction was completed, the reaction mixture was concentrated under reduced pressure to obtain compound M-8.

MS-ESI calculated value [M+H]⁺ 351, measured value 351.

### Step 7

Compound M-8 (1.20 g, 3.42 mmol, 1 equiv) was dissolved in 1,2-dichloroethane (25 mL), and manganese dioxide (5.95 g, 68.5 mmol, 20 equiv) was added to the reaction mixture, and stirred at 70°C for 2 hours. After the reaction was completed, the reaction mixture was filtered, and the filtrate was concentrated to obtain compound M-9.

MS-ESI calculated value [M+H]⁺ 349, measured value 349.

### Step 8

Compound M-9 (1.10 g, 3.15 mmol, 1 equiv) was dissolved in dichloromethane (25 mL), then M-10 (468 mg, 3.79 mmol, 445 µL, 1.2 equiv) was added to the reaction mixture, stirred at 25 °C for 1 hour, then sodium borohydride acetate (2.01 g, 9.46 mmol, 3 equiv) was added to the reaction mixture, stirred at 25 °C for 12 hours under nitrogen protection. After the reaction was completed, the reaction mixture was diluted with water (10 mL), extracted with dichloromethane (15 mL×3). The combined organic phase was dried over anhydrous sodium sulfate, filtered and concentrated under reduced pressure. The residue was purified by silica gel column chromatography (dichloromethane: methanol= 0:1 to 10:1) to obtain compound M.

MS-ESI calculated value [M+H]⁺ 420, measured value 420.

¹H HNMR (400 MHz, CDCl₃) δ = 9.28 (s, 1H), 9.00 (d, J=1.6 Hz, 1H), 8.32 (s, 1H), 8.31 (d, J=1.5 Hz, 1H), 8.28 (dd, J=6.7, 2.6 Hz, 1H), 6.86 (br s, 2H), 6.72 - 6.33 (m, 1H), 4.59 - 4.50 (m, 1H), 4.33 - 4.16 (m, 2H), 3.34 (dt, J =9.8, 7.47 Hz, 1H), 3.20 - 3.11 (m, 1H), 3.07 - 3.00 (m, 1H), 2.89 (td, J=9.4, 6.07 Hz, 1H), 2.49 (s, 3H), 2.40 - 2.24 (m, 1H), 2.09 - 1.89 (m, 1H), 1.39 - 1.16 (m, 1H).

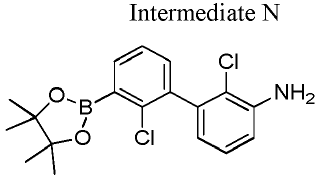

### Synthesis route:

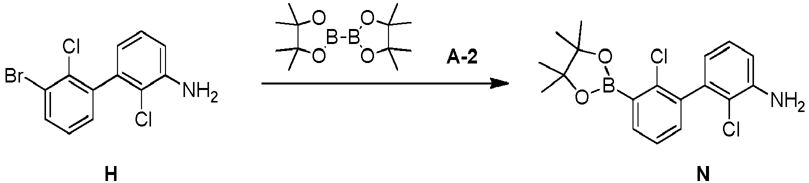

### Step 1

Compound H (8 g, 25.24 mmol, 1 equiv) and A-2 (7.05 g, 27.76 mmol, 1.1 equiv) were dissolved in dioxane (100 mL), then [1,1'-bis(diphenylphosphino)ferrocene]dichloropalladium (1.85 g, 2.52 mmol, 0.1 equiv) and potassium acetate (7.43 g, 75.72 mmol, 3 equiv) were added thereto. The air in the solution was replaced with nitrogen three times, and the reaction mixture was stirred at 90°C for 4 hours under nitrogen protection. After the reaction was completed, the reaction mixture was filtered and concentrated under reduced pressure, and the obtained residue was subjected to silica gel chromatography (petroleum ether: ethyl acetate=30:1 to 10:1) to obtain compound N.

MS-ESI calculated value [M+H]⁺ 364, measured value 364.

¹H NMR (400MHz, DMSO-d*₆*,) δ = 7.68 (dd, J = 4.3, 4.9 Hz, 1H), 7.33 - 7.28 (m, 2H), 7.11 (t, J = 7.8 Hz, 1H), 6.83 (dd, J = 1.5, 8.0 Hz, 1H), 6.64 (dd, J = 1.4, 7.5 Hz, 1H), 1.39 (s, 12H).

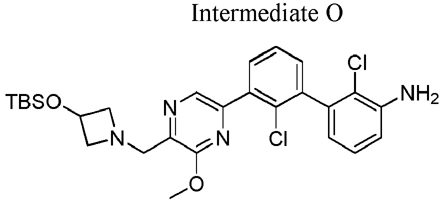

### Synthesis route:

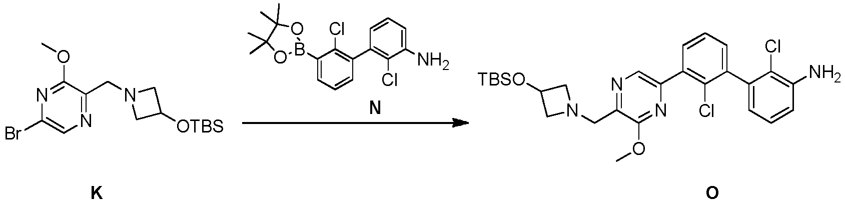

### Step 1

Compound K (7.4 g, 19.05 mmol, 1 equiv) and N (6.94 g, 19.05 mmol, 1 equiv) were dissolved in dioxane (150 mL) and water (15 mL), then [1,1 - bis(diphenylphosphino)ferrocene]palladium dichloride (1.39 g, 1.91 mmol, 0.1 equiv) and potassium carbonate (7.90 g, 57.16 mmol, 3 equiv). The air in solution was replaced with nitrogen three times, and the reaction mixture was stirred at 65°C for 4 hours under nitrogen protection. After the reaction was completed, the reaction mixture was diluted with 100 mL of water and extracted with ethyl acetate (300 mL). The organic phase was washed with saturated brine (200 mL), dried over anhydrous sodium sulfate, filtered and concentrated under reduced pressure. The residue was subjected to silica gel chromatography column (petroleum ether: ethyl acetate=50:1 to 5:1) to obtain compound O.

MS-ESI calculated value [M+H]⁺ 545, measured value 545. ¹H NMR (400 MHz, CDCl₃) δ = 8.43 (s, 1H), 7.56 (dd, J = 1.8, 7.7 Hz, 1H), 7.38 (t, J = 7.6 Hz, 1H), 7.28 (dd, J = 1.8 , 7.5 Hz, 1H), 7.15 - 7.04 (m, 1H), 6.79 (dd, J = 1.5, 8.1 Hz, 1H), 6.66 (dd, J = 1.5, 7.5 Hz, 1H), 4.47 (t, J = 6.2 Hz, 1H), 4.15 (br s, 2H), 3.99 (s, 3H), 3.81 - 3.76 (m, 2H), 3.07 - 2.95 (m, 2H), 0.90 - 0.81 (m, 10H), 0.01 ( br s, 6H).

### Example 1

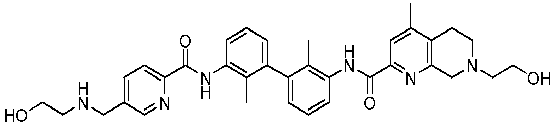

### Synthesis route:

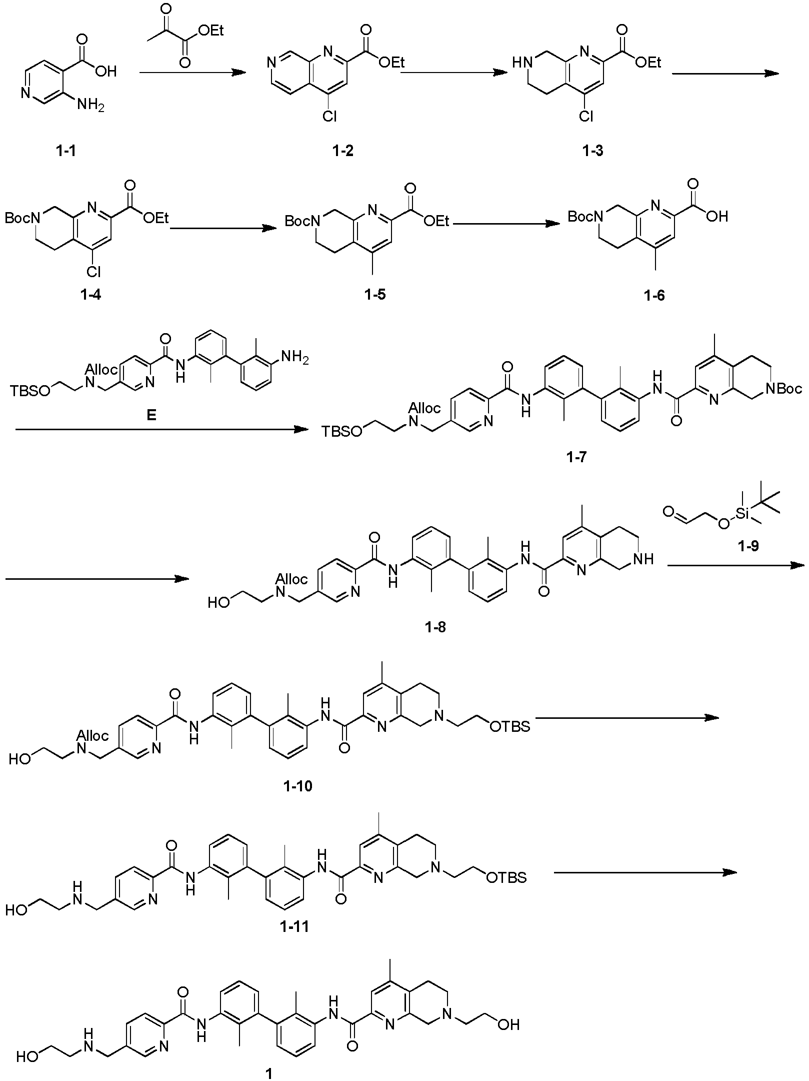

### Step 1

Compound 1-1 (20 g, 144.80 mmol) and ethyl pyruvate (84.07 g, 723.99 mmol) were mixed and stirred at 20 °C for 15 minutes. Phosphorus oxychloride (222.02 g, 1.45 mol) was then added and stirred at 100 °C for 1 hour. After the reaction was completed, the reaction mixture was poured into ice water (1 L), the pH of the reaction mixture was adjusted to 7 with sodium carbonate, and extracted with ethyl acetate (150 mL × 2). The organic phase was washed with saturated brine (100 mL × 2), and the organic layer was dried over anhydrous sodium sulfate, filtered and concentrated. The residue was purified by silica gel column chromatography (petroleum ether: ethyl acetate=15:1 to 3:1) to obtain compound 1-2. MS-ESI calculated value [M+H]⁺ 236.8, measured value 236.8.

### Step 2

Compound 1-2 (16.8 g, 70.99 mmol) and sodium cyanoborohydride (13.387 g, 212.97 mmol) were dissolved in acetic acid (100 mL), and the reaction was conducted at 20°C for 1 hour under nitrogen protection. After the reaction was completed, the reaction mixture was poured into ice water, the pH of the reaction mixture was adjusted to 7 with sodium carbonate, and then extracted with ethyl acetate (200 mL × 2). The organic phase was washed with saturated brine (100 mL × 2), and the organic layer was dried over anhydrous sodium sulfate, filtered and concentrated, and the obtained compound 1-3 was used directly in the next step without further purification.

MS-ESI calculated value [M+H]⁺ 240.8, measured value 240.8.

### Step 3

Compound 1-3 (23 g, 95.56 mmol) was dissolved in dichloromethane (200 mL), di-tert-butyl dicarbonate (31.28 g, 143.34 mmol), triethylamine (29.01 g, 286.68 mmol) were added there to, stirred at 25 °C for 0.5 hours. After the reaction was completed, water (300 mL) was added to the reaction mixture, extracted with dichloromethane (200 mL×2). The organic phase was washed with saturated brine (150 mL×2), and the organic layer was dried over anhydrous sodium sulfate, filtered and concentrated. The residue was purified by silica gel column chromatography (petroleum ether: ethyl acetate=15:1 to 5:1) to obtain compound 1-4.

MS-ESI calculated value [M+H]⁺ 340.8, measured value 340.8.

### Step 4

Compound 1-4 (30 g, 88.03 mmol), boronic acid (7.90 g, 132.04 mmol) and potassium phosphate (56.06 g, 264.08 mmol) were dissolved in dioxane (200 mL), and the reaction was conducted at 100°C for 6 hours under nitrogen protection. After the reaction was completed, water (200 mL) was added to the reaction mixture, and extracted with ethyl acetate (200 mL×2). The organic phase was washed with saturated brine (150 mL×2), and the organic layer was dried over anhydrous sodium sulfate, filtered and concentrated. The residue was purified by silica gel column chromatography (petroleum ether: ethyl acetate=10:1 to 3:1) to obtain compound 1-5.

MS-ESI calculated value [M+H]⁺ 320.9, measured value 320.9. ¹H NMR (400 MHz, CDCl₃) δ ppm 1.43 (3 H, t, J=7.09 Hz), 1.49 (9 H, s), 2.33 (3 H, s), 2.81 (2 H, br t, J= 5.71 Hz), 3.73 (2 H, t, J=5.90 Hz), 4.48 (2 H, q, J=7.11 Hz), 4.77 (2 H, s), 7.81 (1 H, s)

### Step 5

Compound 1-5 (0.350 g, 1.09 mmol, 1 equiv) was dissolved in methanol (5 mL), then water (0.2 mL) and lithium hydroxide monohydrate (91.7 mg, 2.18 mmol, 2 equiv) were added to the reaction mixture, stirred at 25 °C for 2 hours. After the reaction was completed, the reaction mixture was concentrated under reduced pressure to obtain the compound 1-6.

MS-ESI calculated value [M+H]⁺ 293, measured value 293.

### Step 6

Compound 1-6 (35.0 mg, 120 µmol, 1 equiv), E (70.5 mg, 120 µmol, 1 equiv) was dissolved in N,N-dimethylformamide (3 mL), N,N-diisopropylethylamine (46.4 mg, 359 µmol, 62.6 µl, 3 equiv), O-(7-azabenzotriazole-1-yl)-N,N,N,N-tetramethylurea hexafluorophosphonate (54.6 mg, 144 µmol, 1.2 equiv) were added to the reaction mixture under nitrogen protection, stirred at 25 °C for 12 hours. After the reaction was completed, water (15 mL) was added to dilute, extracted with ethyl acetate (10 mL×3). The combined organic phase was washed with saturated brine (20 mL×3), dried over anhydrous sodium sulfate, filtered and concentrated under reduced pressure, and the obtained residue was purified by silica gel thin-layer chromatography (petroleum ether: ethyl acetate=2:1) to obtain compound 1-7.

MS-ESI calculated value [M+H]⁺ 863, measured value 863.

### Step 7

Compound 1-7 (80.0 mg, 86.7 µmol, 1 equiv) was dissolved in ethyl acetate (1 mL), then a solution of hydrogen chloride in ethyl acetate (4 mol/L, 23.2 µL, 1.07 equiv) was added to the reaction mixture, stirred at 25 °C for 1 hour. After the reaction was completed, the reaction mixture was concentrated under reduced pressure to obtain the compound 1-8.

MS-ESI calculated value [M+H]⁺ 649, measured value 649.

### Step 8

Compounds 1-8 (40.0 mg, 61.7 µmol, 1 equiv) and 1-9 (11.8 mg, 67.8 µmol, 12.9 µl, 1.1 equiv) were dissolved in methanol (3 mL), then acetic acid (3.70 mg, 61.7 µmol, 3.53 µl, 1 equiv) was added to the reaction mixture, stirred at 25°C for 0.5 hours, then sodium cyanoborohydride (9.69 mg, 154 µmol, 2.5 equiv) was added to the reaction mixture, stirred at 25°C for 0.5 hours. After the reaction was completed, the reaction mixture was diluted with water (10 mL), then extracted with ethyl acetate (10 mL×3). The combined organic phase was washed with saturated brine (20 mL×2), dried over anhydrous sodium sulfate, filtered and concentrated under reduced pressure to obtain compound 1-10.

MS-ESI calculated value [M+H]⁺ 807, measured value 807.

### Step 9

Compound 1-10 (70.0 mg, 86.7 µmol, 1 equiv) was dissolved in tetrahydrofuran (3 mL), then tetrakistriphenylphosphine palladium (10.0 mg, 8.67 µmol, 0.1 equiv) and diethylamine (63.4 mg, 867 µmol, 89.3 µl, 10 equiv) were added to the reaction mixture, stirred at 70 °C for 12 hours. After the reaction was completed, the reaction mixture was filtered and concentrated under reduced pressure, and the obtained residue was purified by silica gel thin layer chromatography (petroleum ether: ethyl acetate=0:1) to obtain compound 1-11.

MS-ESI calculated value [M+H]⁺ 723, measured value 723.

### Step 10

Compound 1-11 (20.0 mg, 22.5 µmol, 1 equiv) was dissolved in ethyl acetate (3 mL), a solution of hydrogen chloride in dioxane (4 mol/L, 5.64 µL, 1 equiv) was added to the reaction mixture, and the reaction was conducted at 25 °C for 1 hour in microwave. After the reaction was completed, the reaction mixture was concentrated under reduced pressure, and the obtained residue was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 8%-38%, 10 minutes) to obtain the formate of compound 1.

MS-ESI calculated value [M+H]⁺ 609, measured value 609. ¹HNMR (400 MHz, CD₃OD) δ ppm 8.78 (s, 1H), 8.41 (brs, 1H), 8.29 (d, J=7.95 Hz, 1H), 8.16 - 8.11 (m, 1H), 7.94 - 7.86 (m, 3H), 7.39 - 7.28 (m, 2H), 7.06 (t, J=8.07 Hz, 2H), 4.58 (brs, 3H), 4.27 (s, 2H), 3.94 (s, 2H), 3.82 (dt, J =11.00, 5.50 Hz ,4H), 3.13- 2.99 (m ,4H), 2.96 (br ,d, J=5.50 Hz ,2H), 2.85 (t, J=5.87 Hz ,2H), 2.39 (s ,3H) , 2.09 (d, J=2.81 Hz, 6H), 1.35 - 1.25 (m, 1H).

### Example 2

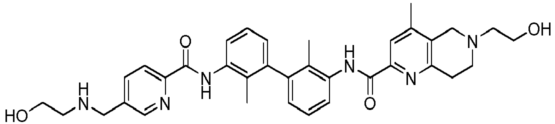

### Synthesis route:

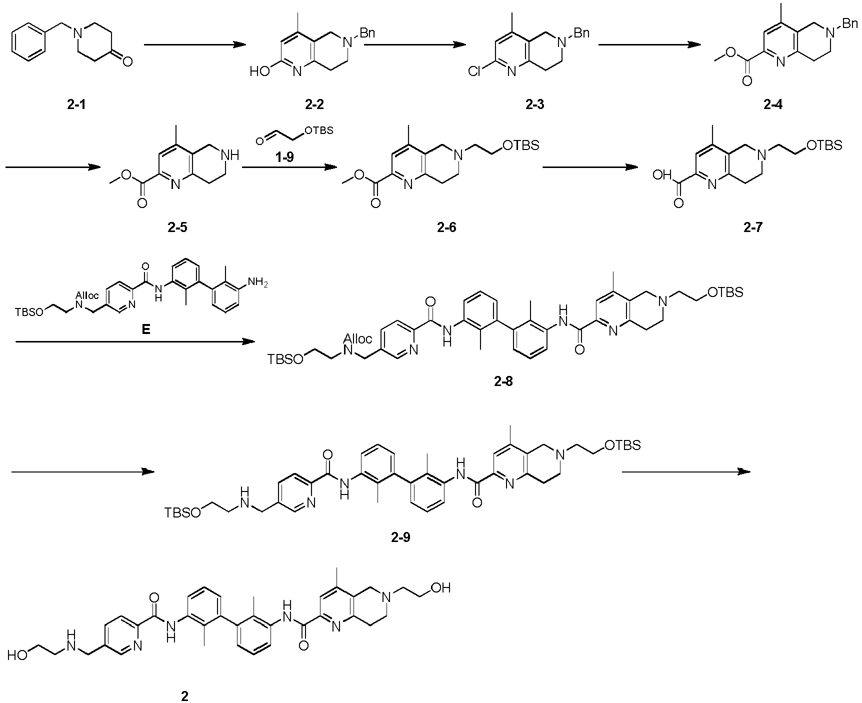

### Step 1

Compound 2-1 (10.0 g, 52.8 mmol, 9.80 mL, 1 equiv) and acetylacetamide (5.88 g, 58.1 mmol, 1.1 equiv) was dissolved in Eaton's reagent (30.4 g, 128 mmol, 20.0 mL, 2.42 equiv), stirred at 110 °C for 12 hours. After the reaction was completed, the reaction mixture was slowly poured into an aqueous solution of sodium bicarbonate (75.0 g dissolved in 500 mL of water), diluted with water (50 mL), and extracted with dichloromethane (300 mL×3). The combined organic phase was washed with saturated brine (200 mL × 2), dried over anhydrous sodium sulfate, filtered and concentrated under reduced pressure to obtain a residue. The residue was diluted with acetone (200 mL), stirred at 25 °C for 20 minutes, then filtered to obtain the compound 2-2 as the filter cake.

MS-ESI calculated value [M+H]⁺ 255, measured value 255. ¹H NMR (400 MHz, CDCl₃) δ ppm 12.57 (brs, 1H), 7.29 - 7.19 (m, 5H), 6.15 (s, 1H), 3.64 (s, 2H), 3.27 (s, 2H), 2.71 (brs d, J=5.38 Hz, 2H), 2.67 - 2.59 (m, 2H), 1.96 (s, 3H).

### Step 2

Compound 2-2 (4.50 g, 16.6 mmol, 1 equiv) was dissolved in phosphorus oxychloride (82.5 g, 538 mmol, 50 mL, 32.48 equiv), stirred at 110°C for 12 hours. After the reaction was completed, phosphorus oxychloride was distilled out of the reaction mixture under reduced pressure. The residue was diluted with water (800 mL), the pH of the reaction mixture was adjusted to 6-7 with 1 mol sodium hydroxide, and then extracted with ethyl acetate (300 mL×3). The combined organic phase was washed with saturated brine (400 mL × 2), dried over anhydrous sodium sulfate, filtered and concentrated under reduced pressure. The obtained residue was diluted with dichloromethane (100 mL), stirred at 20 °C for 0.5 hours, filtered to obtain the filter cake. The above steps were repeated again to obtain compound 2-3 as the filter cake.

MS-ESI calculated value [M+H]⁺ 273, measured value 273. ¹H NMR (400 MHz, CDCl₃) δ ppm 7.34 - 7.18 (m, 5H), 6.87 (s, 1H), 3.68 (s, 2H), 3.48 (s, 2H), 2.97 - 2.88 (m, 2H), 2.76 - 2.69 (2 m, H), 2.05 (s, 3H).

### Step 3

Compound 2-3 (4.50 g, 15.7 mmol, 1 equiv) was dissolved in methanol (30 mL), N,N-dimethylformamide (3 mL), then [1,1'-bis(diphenylphosphino)ferrocene]dichloropalladium (2.30 g, 3.14 mmol, 0.2 equiv), triethylamine (2.38 g, 23.5 mmol, 3.28 mL, 1.5 equiv) were added to the reaction mixture, stirred at 80 °C for 12 hours under carbon monoxide (50 psi). After the reaction was completed, the reaction mixture was diluted with water (60 mL), and extracted with ethyl acetate (30 mL×5). The combined organic phase was washed with saturated brine (40 mL×2),dried over anhydrous sodium sulfate, filtered and concentrated under reduced pressure. The obtained residue was purified by silica gel column chromatography (petroleum ether: ethyl acetate=10:1 to 4:1) to obtain compound 2-4.

MS-ESI calculated value [M+H]⁺ 297, measured value 297. ¹HNMR(400 MHz, CDCl₃) δ ppm 7.70(s,1H), 7.35-7.21(m,5H), 3.91(s,3H),3.69(s,2H),3.57(s,2H), 3.06(t, J=5.82 Hz ,2H), 2.89 (s ,2H), 2.82 (s ,2H), 2.76(t,J=5.88 Hz ,2H), 2.15 (s ,3H).

### Step 4

Compound 2-4 (1.00 g, 3.14 mmol, 1 equiv) was dissolved in methanol (15 mL), then wet palladium on carbon (0.100 g, 62.7 µmol, 10% purity) was added to the reaction mixture, stirred at 25 °C for 12 hours under hydrogen (15 psi). After completion of the reaction, the reaction mixture was filtered, and the filtrate was concentrated under reduced pressure to obtain compound 2-5.

MS-ESI calculated value [M+H]⁺ 207, measured value 207.

### Step 5

Compounds 2-5 (0.100 g, 481 µmol, 1 equiv) and 1-9 (92.2 mg, 529 µmol, 101 µl, 1.1 equiv) were dissolved in methanol (5 mL), then acetic acid (2.89 mg, 48.1 µmol, 2.75 µl, 0.1 equiv) was added to the reaction mixture, stirred at 25°C for 0.5 h, then sodium cyanoborohydride (75.6 mg, 1.20 mmol, 2.5 equiv) was added to the reaction mixture, stirred at 25°C for 4 hours. After the reaction was completed, the reaction mixture was diluted with water (15 mL), and extracted with ethyl acetate (10 mL×3). The combined organic phase was washed with saturated brine (20 mL×2), dried over anhydrous sodium sulfate, filtered and concentrated under reduced pressure to obtain compounds 2-6.

MS-ESI calculated value [M+H]⁺ 365, measured value 365.

### Step 6

Compound 2-6 (0.130 g, 293 µmol, 1 equiv) was dissolved in methanol (3 mL), water (0.2 mL), then lithium hydride monohydrate (18.5 mg, 439 µmol, 1.5 equiv) was added to the reaction mixture, stirred at 25 °C for 4 hours. After the reaction was completed, the reaction mixture was concentrated under reduced pressure to obtain compound 2-7.

MS-ESI calculated value [M+H]⁺ 351, measured value 351.

### Step 7

Compound 2-7 (69.8 mg, 159 µmol, 1.3 equiv), E (72.0 mg, 122 µmol, 1 equiv) were dissolved in N,N-dimethylformamide (4 mL), then N,N-diisopropylethylamine (47.4 mg, 367 µmol, 63.9 µl, 3 equiv), O-(7-azabenzotriazol-1-yl)-N,N, N,N-tetramethylurea hexafluorophosphonate (69.7 mg, 183 µmol, 1.5 equiv) were added to the reaction mixture, stirred at 25 °C for 12 hours. After the reaction was completed, the reaction mixture was diluted with water (30 mL) and extracted with ethyl acetate (15 mL×3). The combined organic phase was washed with saturated brine (20 mL×3), dried over anhydrous sodium sulfate, filtered and concentrated under reduced pressure. The obtained residue was purified by silica gel thin layer chromatography (petroleum ether: ethyl acetate=2:1) to obtain compound 2-8.

MS-ESI calculated value [M+H]⁺ 922, measured value 922.

### Step 8

Compound 2-8 (50.0 mg, 26.1 µmol, 1 equiv) was dissolved in tetrahydrofuran (3 mL), then tetrakis(triphenylphosphine)palladium (3.02 mg, 2.61 µmol, 0.1 equiv), diethylamine (19.1 mg, 261 µmol, 26.9 µl, 10 equiv) were added to the reaction mixture, stirred at 70 °C for 12 hours. After the reaction was completed, the reaction mixture was filtered and concentrated under reduced pressure. The obtained residue was purified by silica gel thin layer chromatography (petroleum ether: ethyl acetate=1:1) to obtain compound 2-9.

MS-ESI calculated value [M+H]⁺ 838, measured value 838.

### Step 9

Compound 2-9 (20.0 mg, 16.1 µmol, 1 equiv) was dissolved in ethyl acetate (2 mL), hydrogen chloride solution in ethyl acetate (4 mol/L, 4.07 µL, 1.01 equiv) were added to the reaction mixture, and the reaction was conducted at 25°C for 0.5 hours. After the reaction was completed, the reaction mixture was concentrated under reduced pressure, the obtained residue was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 5%-35%, 10 minutes) to obtain the formate of compound 2.

MS-ESI calculated value [M+H]⁺ 609, measured value 609. ¹H NMR (400 MHz, CD₃OD) δ ppm 8.79 (d, J=1.59 Hz, 1H), 8.50 (br s, 1H), 8.29 (d, J=7.95 Hz, 1H), 8.13 (dd, J=8.07, 2.08 Hz ,1H), 7.98 - 7.87 (m ,3H), 7.35 (td, J=7.83, 4.65 Hz ,2H), 7.07 (t, J=7.95 Hz ,2H), 4.23 (s ,2H), 3.89 - 3.82 (m ,4H), 3.82 - 3.77 (m ,2H), 3.19 - 3.12 (m ,2H), 3.07 - 3.00 (m ,4H), 2.88 (t, J=5.75 Hz ,2H), 2.38 (s , 3H), 2.11 (s ,6H).

### Example 3

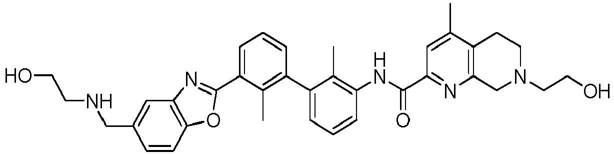

### Synthesis route:

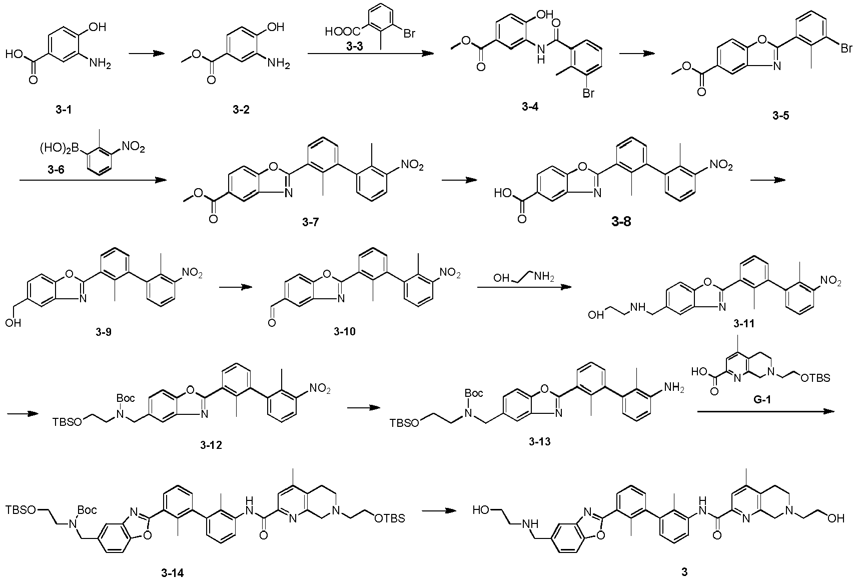

### Step 1

After acetyl chloride (15.38 g, 195.91 mmol, 13.98 mL, 3 equiv) was dissolved in methanol (300 mL) at 0 °C, compound 3-1 (10 g, 65.30 mmol, 1 equiv) was added thereto at 25 °C, the reaction mixture was stirred at 70 °C for 12 hours. After the reaction was completed, the reaction mixture was concentrated under reduced pressure to obtain the crude compound 3-2.

MS-ESI calculated value [M+OMe]⁺168, measured value 168.

### Step 2

Compound 3-3 (10.3 g, 47.90 mmol, 1 equiv) was dissolved in N,N-dimethylformamide (20 mL), and then N,N-diisopropylethylamine (18.57 g, 143.69 mmol, 25.03 mL, 3 equiv), O-(7-azabenzotriazol-1-yl)-N,N,N,N-tetramethylurea hexafluorophosphonate (21.85 g, 57.48 mmol , 1.2 equiv) and compound 3-2 (8.81 g, 52.69 mmol, 1.1 equiv) were added thereto successively. The reaction mixture was stirred at 25°C for 3 hours. After the reaction was completed, the reaction mixture was concentrated under reduced pressure, water (500 mL) was added, and extracted with ethyl acetate (300 mL × 3). The combined organic phase was dried over anhydrous sodium sulfate, filtered and concentrated under reduced pressure. Ethyl acetate (50 mL) was added to the residue, stirred at room temperature, filtered, and repeated three times to obtain the crude compound 3-4.

MS-ESI calculated value [M+H]⁺ 365, measured value 365.

### Step 3

Compound 3-4 (10 g, 27.46 mmol, 1 equiv) was dissolved in toluene (100 mL), and p-toluenesulfonic acid (9.46 g, 54.92 mmol, 2 equiv) was added thereto. The reaction mixture was stirred at 120°C under a water separator for 12 hours. After the reaction was completed, the reaction mixture was concentrated under reduced pressure, water (250 mL) was added, and extracted with ethyl acetate (80 mL × 3). The combined organic phase was dried over anhydrous sodium sulfate, filtered and concentrated under reduced pressure. The residue was subjected to silica gel column chromatography (petroleum ether: ethyl acetate=50:1) to obtain compound 3-5.

MS-ESI calculated value [M+H]⁺ 346 and 348, measured value 346 and 348.

### Step 4

Compound 3-5 (3.5 g, 10.11 mmol, 1 equiv) was dissolved in dioxane (60 mL) and water (10 mL), and then compound 3-6 (2.93 g, 11.12 mmol, 1.1 equiv), potassium carbonate (4.19 g, 30.33 mmol, 3 equiv), [1,1'-bis(diphenylphosphino)ferrocene]dichloropalladium (1.48 g, 2.02 mmol, 0.2 equiv) were added thereto successively. The reaction mixture was stirred at 80°C for 12 hours under nitrogen protection. After the reaction was completed, the reaction mixture was concentrated under reduced pressure, water (100 mL) was added, and extracted with ethyl acetate (40 mL × 3). The combined organic phase was dried over anhydrous sodium sulfate, filtered and concentrated under reduced pressure. The residue was subjected to silica gel column chromatography (petroleum ether: ethyl acetate=50:1 to 30:1) to obtain compound 3-7.

MS-ESI calculated value [M+H]⁺ 403, measured value 403. ¹H NMR (400 MHz, DMSO-d₆) δ ppm 8.37 (d, J=1.3 Hz, 1 H), 8.19 (dd, J=7.9, 1.3 Hz, 1 H), 8.08 (dd, J=8.6, 1.7 Hz , 1 H), 7.91 - 8.01 (m, 2 H), 7.53 - 7.61 (m, 3 H), 7.43 (dd, J=7.6, 1.2 Hz, 1 H), 3.90 (s, 3 H), 2.41 ( s, 3 H), 2.14 (s, 3 H).

### Step 5

Compound 3-7 (1.6 g, 3.98 mmol, 1 equiv) was dissolved in tetrahydrofuran (20 mL), methanol (20 mL) and water (3 mL), lithium hydroxide monohydrate (333.71 mg, 7.95 mmol, 2 equiv) was added thereto, the reaction mixture was stirred at 25 °C for 12 hours. After the reaction was completed, the reaction mixture was concentrated under reduced pressure, water (50 mL) and ethyl acetate (30 mL) were added thereto, and the pH of the reaction mixture was adjusted to 5 with aqueous hydrochloric acid (IN), and then extracted with ethyl acetate (30 mL × 3). The combined organic phase was dried over anhydrous sodium sulfate, filtered and concentrated under reduced pressure to obtain crude compound 3-8.

MS-ESI calculated value [M+H]⁺ 389, measured value 389.

### Step 6

Compound 3-8 (0.9 g, 2.32 mmol, 1 equiv) was dissolved in tetrahydrofuran (25 mL), 1,1-carbonyldiimidazole (563.64 mg, 3.48 mmol, 1.5 equiv) was added thereto. After the reaction mixture was stirred at 25 °C for 2 hours, water (2.5 mL) and sodium borohydride (263.00 mg, 6.95 mmol, 3 equiv) were added at 0 °C, and the reaction mixture was stirred at 0 °C for 0.1 hour. After the reaction was completed, the reaction mixture was concentrated under reduced pressure, water (50 mL) was added, and extracted with ethyl acetate (15 mL × 3). The combined organic phase was dried over anhydrous sodium sulfate, filtered and concentrated under reduced pressure to obtain the crude compound 3-9.

MS-ESI calculated value [M+H]⁺ 375, measured value 375.

### Step 7

Compound 3-9 (800 mg, 2.14 mmol, 1 equiv) was dissolved in 1,2-dichloroethane (30 mL), manganese dioxide (1.86 g, 21.37 mmol, 10 equiv) was added thereto, and the reaction mixture was stirred at 70°C for 2 hours. After the reaction was completed, the reaction mixture was filtered and concentrated under reduced pressure to obtain the crude compound 3-10.

MS-ESI calculated value [M+H]⁺ 373, measured value 373.

### Step 8

Compound 3-10 (760 mg, 2.04 mmol, 1 equiv) and ethanolamine (149.60 mg, 2.45 mmol, 148.12 µL, 1.2 equiv) were dissolved in methanol (3 mL), sodium cyanoborohydride (256.52 mg, 4.08 mmol, 2 equiv) was added, and the reaction mixture was stirred at 25 °C for 12 hours. After the reaction was completed, the reaction mixture was concentrated under reduced pressure, water (50 mL) was added, and extracted with ethyl acetate (30 mL×3). The combined organic phase was dried over anhydrous sodium sulfate, filtered and concentrated under reduced pressure. The residue was subjected to silica gel thin layer chromatography (petroleum ether: ethyl acetate=2:1) to obtain compound 3-11.

MS-ESI calculated value [M+H]⁺ 418, measured value 418.

### Step 9

Compound 3-11 (400 mg, 958.18 mmol, 1 equiv) was dissolved in dichloromethane (2 mL), triethylamine (290.88 mg, 2.87 mmol, 400.10 µL, 3 equiv) and di-tert-butyl carbonate ester (250.95 mg, 1.15 mmol, 264.16 µl, 1.2 equiv) were added thereto. The reaction mixture was stirred at 25°C for 0.5 hours, N,N-diisopropylethylamine (371.51 mg, 2.87 mmol, 500.68 µl, 3 equiv) and tertbutyldimethylsilyl chloride (173.30 mg, 1.15 mmol, 140.90 µl, 1.2 equiv) were added thereto, and stirred at 25°C for 3 hours. After the reaction was completed, the reaction mixture was concentrated under reduced pressure, water (50 mL) was added, and extracted with ethyl acetate (30 mL×3). The combined organic phase was dried over anhydrous sodium sulfate, filtered and concentrated under reduced pressure. The residue was subjected to silica gel thin layer chromatography (petroleum ether: ethyl acetate=3:1) to obtain compound 3-12.

MS-ESI calculated value [M+H]⁺ 632, measured value 632.

### Step 10

Under nitrogen atmosphere, compound 3-12 (200 mg, 316.54 mmol, 1 equiv) was dissolved in methanol (20 mL), wet palladium on carbon (30 mg, 10% purity) was added thereto, stirred under hydrogen atmosphere (15 psi) at 25 °C for 10 hours. After the reaction was completed, the reaction mixture was filtered and concentrated under reduced pressure to obtain the crude compound 3-13.

MS-ESI calculated value [M+H]⁺ 602, measured value 602.

### Step 11

Compound G-1 (10 mg, 28.53 mmol, 1 equiv) was dissolved in N,N-dimethylformamide (3 mL), and then N,N-diisopropylethylamine (11.06 mg, 85.59 mmol, 14.91 µl, 3 equiv), O-(7-azabenzotriazol-1-yl)-N,N,N,N-tetramethylurea hexafluorophosphonate (13.02 mg, 34.23 mg mol, 1.2 equiv) and compound 3-13 (20.60 mg, 34.23 mmol, 1.2 equiv) was added successively. The reaction mixture was stirred at 25°C for 1 hour. After the reaction was completed, the reaction mixture was concentrated under reduced pressure, water (30 mL) was added thereto, and extracted with ethyl acetate (10 mL × 3). The combined organic phase was dried over anhydrous sodium sulfate, filtered and concentrated under reduced pressure. The residue was subjected to silica gel thin layer chromatography (petroleum ether: ethyl acetate=2:1) to obtain compound 3-14.

MS-ESI calculated value [M+H]⁺ 934, measured value 934.

### Step 12

Compound 3-14 (30 mg, 32.11 mmol, 1 equiv) was dissolved in ethyl acetate (10 mL), a solution of hydrogen chloride in ethyl acetate (4 mol/L, 30.03 µL, 3.74 equiv) was added thereto, the reaction mixture was stirred at 25°C for 0.1 hour. After the reaction was completed, the reaction mixture concentrated under reduced pressure was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 10%-40%, 10 minutes) to obtain the formate of compound 3.

MS-ESI calculated value [M+H]⁺ 606, measured value 606. ¹H NMR (400 MHz, CD₃OD) δ ppm 8.46 (br s, 2 H), 8.14 (d, J=7.8 Hz, 1 H), 7.86 - 8.01 (m, 3 H), 7.80 (d, J=8.4 Hz , 1 H), 7.59 (dd, J=8.3, 1.6 Hz, 1 H), 7.45 - 7.53 (m, 1 H), 7.31 - 7.42 (m, 2 H), 7.06 (d, J=7.6 Hz, 1 H), 4.40 (s, 2 H), 4.03 (br d, J=5.1 Hz, 2 H), 3.85 (br d, J=5.1 Hz, 4 H), 3.16 - 3.22 (m, 2 H), 3.12 (br d, J=6.1 Hz, 2 H), 2.96 - 3.04 (m, 2 H), 2.93 (br d, J=5.6 Hz, 2 H), 2.47 (s, 3 H), 2.40 (s, 3 H), 2.08 (s, 3 H).

### Example 4

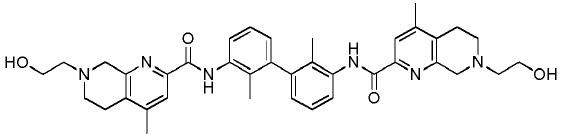

### Synthesis route:

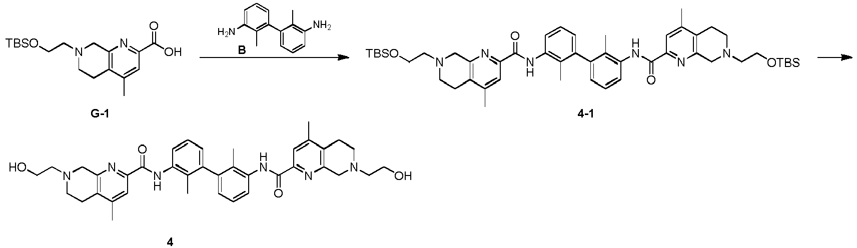

### Step 1

Compounds G-1 (29.1 mg, 82.9 µmol, 2.2 equiv), B (8 mg, 37.7 µmol, 1 equiv) were dissolved in N,N-dimethylformamide (3 mL), and then N,N-diisopropylethylamine (29.2 mg, 226 µmol, 39.4 µl, 6 equiv), O-(7-azabenzotriazole-1-yl)-N,N,N , N-tetramethylurea hexafluorophosphonate (31.5 mg, 82.9 µmol, 2.2 equiv) were added to the reaction mixture, and stirred at 25 °C for 12 hours. After the reaction was completed, the reaction mixture was diluted with water (10 mL), and extracted with ethyl acetate (10 mL×3). The combined organic phase was washed with saturated brine (15 mL×3), dried over anhydrous sodium sulfate, filtered and concentrated under reduced pressure. The obtained residue was purified by silica gel thin layer chromatography (petroleum ether: ethyl acetate=1:1) to obtain compound 4-1.

MS-ESI calculated value [M+H]⁺ 878, measured value 878.

### Step 2

Compound 4-1 (20.0 mg, 21.7 µmol, 1 equiv) was dissolved in ethyl acetate (3 mL), a solution of hydrogen chloride in ethyl acetate (4 mol, 4.07 µl, 1.01 equiv) was added to the reaction mixture, and the reaction was conducted at 25 °C for 0.5 hours. After the reaction was completed, the reaction mixture was concentrated under reduced pressure, the obtained residue was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 9%-39%, 10 minutes) to obtain the formate of compound 4.

MS-ESI calculated value [M+H]⁺ 649, measured value 649. ¹HNMR (400 MHz, CD₃OD) δ ppm 8.38 (brs, 1H), 7.95 s, 2H), 7.92 (d, J=7.34 Hz, 2H), 7.35 (t, J=7.89 Hz, 2H), 7.06 (d, J=6.72 Hz ,2H), 4.60 (brs ,4H), 4.04 (s ,4H), 3.87 (t, J=5.75 Hz ,4H), 3.18 - 3.10 (m ,4H), 3.04 - 2.98 (m ,4H) ), 2.94 (t, J=5.69 Hz, 4H), 2.42 (s, 6H), 2.11 (s, 6H).

### Example 5

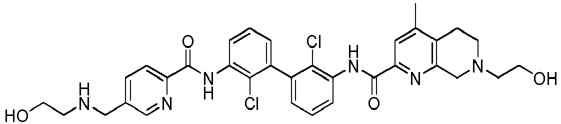

### Synthesis route:

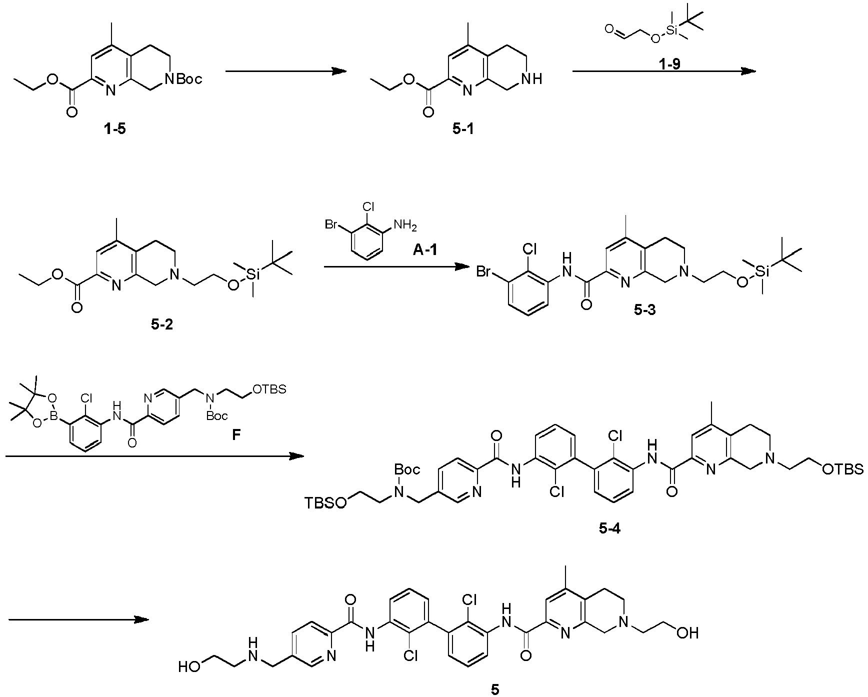

### Step 1

Compound 1-5 (1.5 g, 4.68 mmol) was dissolved in ethyl acetate (10 mL), a solution of hydrogen chloride in ethyl acetate (4 mol/L, 10 mL) was added to the reaction mixture, the reaction mixture was stirred at 25 °C for 10 minutes. After the reaction was completed, the reaction mixture was concentrated under reduced pressure to obtain the crude compound 5-1.

MS-ESI calculated value [M+H]⁺ 221, measured value 221.

### Step 2

Compound 5-1 (1.14 g, 4.44 mmol, hydrochloride) was dissolved in methanol (15 mL), and then N,N-diisopropylethylamine (574 mg, 4.44 mmol, 773 µL) and compound 1-9 (1.16 g, 6.66 mmol, 1.3 mL) were added to the reaction mixture. The reaction mixture was stirred at 25 °C for 30 min, then sodium cyanoborohydride (558 mg, 8.88 mmol) was added, and further stirred at 25 °C for 12 hours. After the reaction was completed, the reaction mixture was concentrated under reduced pressure, and water (20 mL) was added to the concentrated residue, then extracted with ethyl acetate (20 mL × 2). The combined organic phase was dried over anhydrous sodium sulfate, filtered and concentrated under reduced pressure. The residue was purified by silica gel column chromatography (petroleum ether: ethyl acetate=5:1 to 3:1) to obtain the crude compound 5-2.

MS-ESI calculated value [M+H]⁺ 379, measured value 379.

### Step 3

Compound 5-2 (200 mg, 482 mmol) and compound A-1 (129 mg, 626 mmol) were dissolved in toluene (10 mL), and lithium hexamethyldisilazide (1 mol/L, tetrahydrofuran solution, 1.5 mL) was added dropwise to the reaction mixture at 25 °C. The reaction mixture was stirred at 25 °C for 1 h. After the reaction was completed, saturated ammonium chloride solution (10 mL) was added to quench the reaction at 25 °C, the reaction mixture was diluted with water (10 mL), then extracted with ethyl acetate (15 mL × 2). The combined organic phase was dried over anhydrous sodium sulfate, filtered and concentrated under reduced pressure. The residue was separated and purified by silica gel thin layer chromatography (petroleum ether: ethyl acetate=3:1) to obtain compound 5-3.

MS-ESI calculated value [M+H]⁺ 538 and 540, measured value 538 and 540.

### Step 4

Compound F (106 mg, 164 mmol) and compound 5-3 (110 mg, 197 mmol) were dissolved in dioxane (4 mL) and water (0.8 mL), and [1,1'-bis(diphenylphosphino)ferrocene]dichloropalladium (12.0 mg, 16.4 mmol) and sodium carbonate (52.2 mg, 492 mmol) was added to the reaction mixture. After the air was replaced, the reaction mixture was stirred at 90 °C for 12 hours under nitrogen protection. After the reaction was completed, the reaction mixture was filtered and concentrated, and water (10 mL) was added to the concentrated residue, and extracted with ethyl acetate (10 mL × 2). The combined organic phase was dried over anhydrous sodium sulfate, filtered and concentrated under reduced pressure. The residue was purified by silica gel thin layer chromatography (petroleum ether: ethyl acetate=3:1) to obtain compound 5-4.

MS-ESI calculated value [M+H]⁺ 977 and 979, measured value 977 and 979.

### Step 5

Compound 5-4 (25 mg, 19.1 mmol) was dissolved in ethyl acetate (1 mL), a solution of hydrogen chloride in ethyl acetate (4 mol/L, 2 mL) was added to the reaction mixture. The reaction mixture was stirred at 25 °C for 10 minutes. After the reaction was completed, the reaction mixture was concentrated under reduced pressure. The residue was subjected to high performance liquid chromatography (chromatographic column: Waters Xbridge C18 150^{∗}50 mm^{∗}10 microns; mobile phase: mobile phase A: concentration 10 mmol per liter of amine bicarbonate in water; mobile phase B: acetonitrile; B%: 39%-69%, 10 minutes) to obtain compound 5.

MS-ESI calculated value [M+H]⁺ 649 and 651, measured value 649 and 651. ¹H NMR (400 MHz, CD₃OD) δ 8.83 (s, 1H), 8.57-8.64 (m, 2H), 8.37 (d, 1H), 8.21 (m, 1H), 8.11 (s, 1H), 7.50 (m, 2H), 7.14-7.20 (m, 2H), 4.49-4.81 (m, 2H), 4.43 (s, 2H), 3.93-4.01 (m, 2H), 3.81-3.89 (m, 2H), 3.46 ( d, 2H), 3.17-3.27 (m, 6H), 2.47 (s, 3H).

### Example 6

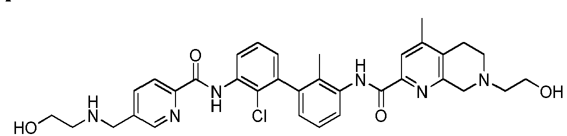

### Synthesis route:

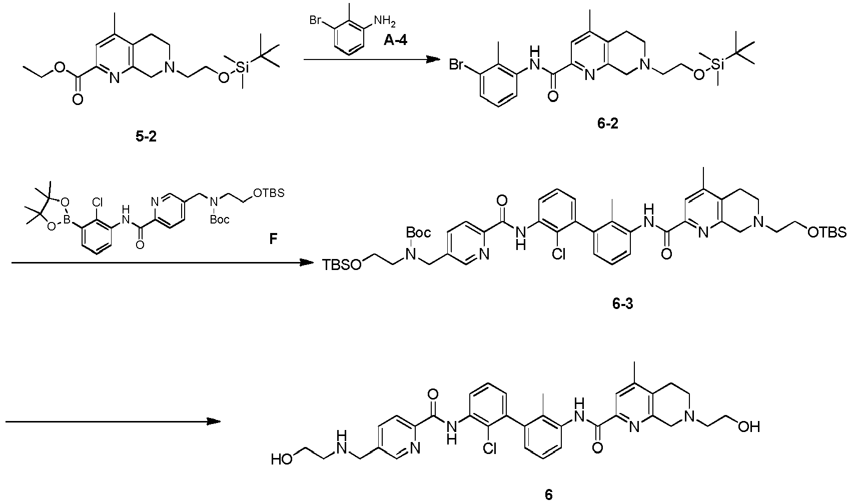

### Step 1

Compound 5-2 (205 mg, 494 mmol) and compound A-4 (119 mg, 642 mmol, 79.1 µL) were dissolved in toluene (10 mL), lithium hexamethyldisilazide (1 mol/L tetrahydrofuran solution, 1.5 mL) was added dropwise to the reaction mixture at 25 °C, the reaction mixture was stirred at 25 °C for 1 hour. After the reaction was completed, saturated ammonium chloride solution (10 mL) was added at 25°C to quench the reaction, diluted with water (10 mL), then extracted with ethyl acetate (15 mL × 2), and the combined organic phase was dried over anhydrous sodium sulfate , filtered and concentrated under reduced pressure. The residue was purified by silica gel thin layer chromatography (petroleum ether: ethyl acetate=3:1) to obtain compound 6-2.

MS-ESI calculated value [M+H]⁺ 518 and 520, measured value 518 and 520.

### Step 2

Compound F (108 mg, 167 mmol) and compound 6-2 (104 mg, 201 mmol) were dissolved in dioxane (4 mL) and water (0.8 mL), and then [1,1'-bis(diphenylphosphino)ferrocene]dichloropalladium (12.2 mg, 16.7 mmol) and sodium carbonate (53.2 mg, 501 mmol) were added to the reaction mixture. After the air was replaced, the reaction mixture was stirred at 90 °C for 12 hours under nitrogen protection. After the reaction was completed, the reaction mixture was filtered and concentrated, and water (10 mL) was added to the concentrated residue, and extracted with ethyl acetate (10 mL × 2). The combined organic phase was dried over anhydrous sodium sulfate, filtered and concentrated under reduced pressure. The residue was purified by silica gel thin layer chromatography (petroleum ether: ethyl acetate=3:1) to obtain compound 6-3.

MS-ESI calculated value [M+H]⁺ 957 and 959, measured value 957 and 959.

### Step 3

Compound 6-3 (66 mg, 48.9 mmol) was dissolved in ethyl acetate (1 mL), a solution of hydrogen chloride in ethyl acetate (4 mol/L, 2 mL) was added to the reaction mixture, and the reaction mixture was stirred at 25 °C for 10 minutes. After the reaction was completed, the reaction mixture was concentrated under reduced pressure, and the residue was subjected to high performance liquid chromatography (chromatographic column: Waters Xbridge C18 150^{∗}50 mm^{∗}10 microns; mobile phase: mobile phase A: the concentration was 10 mmol per liter of aqueous amine bicarbonate solution; mobile phase B: acetonitrile; B%: 33%-63%, 10 minutes) to obtain compound 6.

MS-ESI calculated value [M+H]⁺ 629 and 631, measured value 629 and 631. ¹H NMR (400 MHz, CD₃OD) δ 8.82 (s, 1H), 8.58 (d, 1H), 8.36 (d, 1H), 8.19 (d, 1H), 7.97 (s, 1H), 7.92 (d, 1H) ), 7.48 (m, 1H), 7.36 (m, 1H), 7.15 (d, 1H), 7.08 (d, 1H), 4.58 (s, 2H), 4.39 (s, 2H), 3.86 (m, 4H) , 3.14-3.23 (m, 4H), 3.04 (s, 4H), 2.42 (s, 3H), 2.12 (s, 3H).

### Example 7

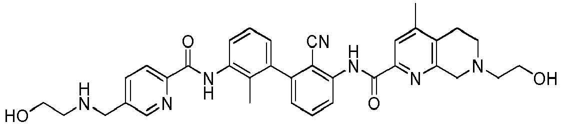

### Synthesis route:

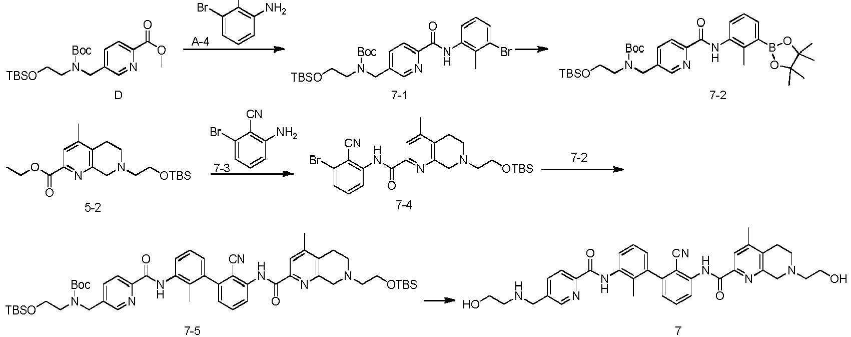

### Step 1

Compound D (10 g, 23.55 mmol, 1 equiv) and A-4 (3.94 g, 21.20 mmol, 2.61 mL, 0.9 equiv) were dissolved in toluene (100 mL), and then bis(trimethylsilicon) lithium amide 1.0M solution in tetrahydrofuran (1 M, 47.10 mL, 2 equiv) was added slowly thereto. The reaction mixture was stirred at 25°C for 2 hours under nitrogen protection. After the reaction was completed, the reaction mixture was diluted with 50 mL of saturated aqueous ammonium chloride solution and extracted with 150 mL of ethyl acetate (50 mL × 3). The obtained organic phases were combined, washed with 80 mL of saturated brine (40 mL × 2), dried over anhydrous sodium sulfate, filtered and concentrated under reduced pressure. The residue was subjected to silica gel column chromatography (petroleum ether: ethyl acetate=50:1 to 10:1) to obtain compound 7-1.

MS-ESI calculated value [M+H]⁺ 579, measured value 579.

### Step 2

Compound 7-1 (200 mg, 345.65 mmol, 1 equiv), [1,1'-bis(diphenylphosphino)ferrocene]dichloropalladium (25.29 mg, 34.57 mmol, 0.1 equiv) and potassium acetate ( 101.77 mg, 1.04 mmol, 3 equiv) were dissolved in dioxane (6 mL). After the air was replaced with nitrogen for three times, the reaction was conducted at 90 °C for 5 hours. After the reaction was completed, the reaction mixture was filtered, the filtrate was concentrated to obtain crude product 7-2.

MS-ESI calculated value [M+H]⁺ 626, measured value 626.

### Step 3

Compound 5-2 (120 mg, 316.97 mmol, 1 equiv) and compound 7-3 (93.68 mg, 475.46 mmol, 1.5 equiv) were dissolved in toluene (5 mL), and then lithium hexamethyldisilazide (1 mol/L, 633.95 µL, 2 equiv) was slowly added thereto dropwise at room temperature, stirred at room temperature for 2 hours. After the reaction was completed, the reaction mixture was diluted with ethyl acetate (20 mL), water (20 mL) was added, and the reaction mixture was extracted with ethyl acetate (20 mL×2). The combined organic phase was dried over anhydrous sodium sulfate and concentrated. The residue was subjected to silica gel column chromatography (petroleum ether: ethyl acetate=10:1 to 5:1) to obtain compound 7-4.

MS-ESI calculated value [M+H]⁺ 529 and 531, measured value 529 and 531.

### Step 4

Compound 7-2 (189.05 mg, 302.15 mmol, 1 equiv), compound 7-4 (160 mg, 302.15 mmol, 1 equiv), [1,1'-bis(diphenylphosphino)ferrocene]dichloropalladium (22.11 mg, 30.21 mmol, 0.1 equiv), sodium carbonate (83.52 mg, 787.98 mmol, 2.61 equiv) were dissolved in dioxane (8 mL) and water (2 mL). After the air was replaced by nitrogen three times, the reaction was conducted at 90 °C for 12 hours. After the reaction was completed, water (20 mL) was added, and the reaction mixture was extracted with ethyl acetate (20 mL×3). The combined organic phase was dried over anhydrous sodium sulfate and concentrated. The concentrated residue was subjected to silica gel thin layer chromatography (petroleum ether: ethyl acetate=3:1) to obtain compound 7-5.

MS-ESI calculated value [M+H]⁺ 948, measured value 948.

### Step 5

Compound 7-5 (50 mg, 52.72 mmol, 1 equiv) was dissolved in ethyl acetate (6 mL), a solution of hydrogen chloride in ethyl acetate (4 mol/L, 1.25 mL, 94.83 equiv) was added thereto, and the mixture was stirred at room temperature for 1 hour. After the reaction was completed, the reaction mixture was concentrated. The concentrated residue was subjected to high performance liquid chromatography (chromatographic column: Waters Xbridge 150^{∗}25 mm^{∗}5 microns; mobile phase: mobile phase A: 0.05% aqueous ammonia solution by volume; mobile phase B: acetonitrile; B%: 34%-54%, 10 min) to obtain compound 7.

MS-ESI calculated value [M+H]⁺ 620, measured value 620. ¹H NMR (400 MHz, CDCl₃) δ 10.96 (s, 1H), 10.18 (s, 1H), 8.72 (d, J = 8.4 Hz, 1H), 8.60 (d, J = 1.5 Hz, 1H), 8.39 (d , J = 7.9 Hz, 1H), 8.30 (d, J = 7.9 Hz, 1H), 7.96 (s, 1H), 7.91 (dd, J = 1.9, 8.0 Hz, 1H), 7.69 (t, J = 8.0 Hz , 1H), 7.47 - 7.35 (m, 1H), 7.11 (dd, J = 7.5, 14.6 Hz, 2H), 3.96 (s, 2H), 3.86 (s, 2H), 3.79 - 3.67 (m, 4H), 2.95 - 2.83 (m, 6H), 2.78 (t, J = 5.3 Hz, 2H), 2.36 (s, 3H), 2.28 (s, 3H).

### Example 8

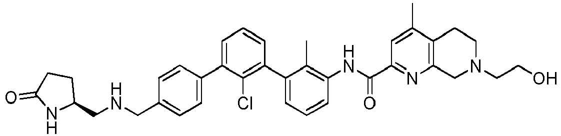

### Synthesis route:

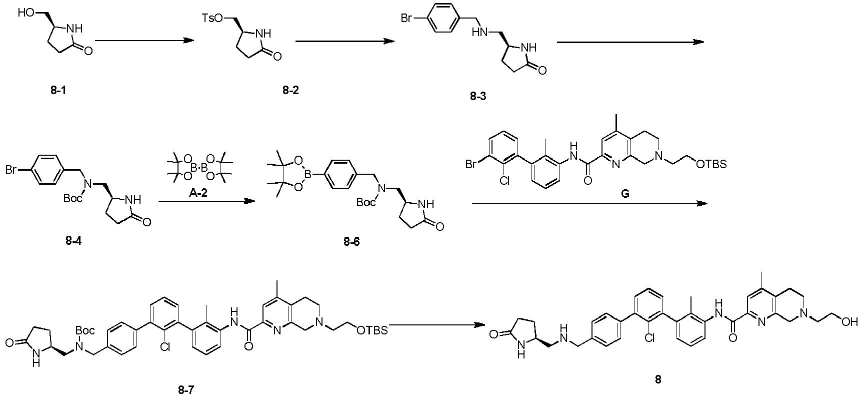

### Step 1

Compound 8-1 (4 g, 37.74 mmol, 1 equiv) and p-toluenesulfonyl chloride (7.95 g, 41.69 mmol, 1.2 equiv) were dissolved in dichloromethane (100 mL), and then triethylamine ( 7.03 g, 69.49 mmol, 9.67 mL, 2 equiv) and 4-(dimethylamino)pyridine (424.46 mg, 3.47 mmol, 0.1 equiv) were added dropwise slowly, stirred at 25°C for 20 hours. After the reaction was completed, water (100 mL) was added, the reaction mixture was extracted with ethyl acetate (50 mL×3). The combined organic phase was dried over anhydrous sodium sulfate and concentrated, and the concentrated residue was subjected to silica gel column chromatography (petroleum ether: ethyl acetate=100:0 to 5:1) to obtain compound 8-2.

MS-ESI calculated value [M+H]⁺ 270, measured value 270.

### Step 2

Compound 8-2 (5.3 g, 19.68 mmol, 1 equiv) and p-bromobenzylamine (7.32 g, 39.36 mmol, 4.98 mL, 2 equiv) were dissolved in N,N-dimethylformamide (25 mL), potassium carbonate (8.16 g, 59.04 mmol, 3 equiv) was added thereto. The air was replaced with nitrogen three times, and the reaction was conducted at 60° C for 12 hours. After the reaction was completed, ethyl acetate (50 mL) and saturated sodium carbonate solution (50 mL) were added thereto, the reaction mixture was extracted with ethyl acetate (50 mL×3). The combined organic phase was dried over anhydrous sodium sulfate and concentrated to obtain crude product 8-3.

MS-ESI calculated value [M+H]⁺ 284, measured value 283.

### Step 3

Compound 8-3 (3 g, 10.59 mmol, 1 equiv) was dissolved in dichloromethane (30 mL), di-tert-butyl carbonate (4.62 g, 21.19 mmol, 4.87 mL, 2 equiv) and triethylamine (3.22 g, 31.78 mmol, 4.42 mL, 3 equiv) was slowly added dropwise, the reaction was conducted at 25°C for 12 hours. After the reaction was completed, water (100 mL) was added, and the reaction mixture was extracted with ethyl acetate (50 mL×3). The combined organic phase was dried over anhydrous sodium sulfate and concentrated, the concentrated residue was subjected to silica gel column chromatography (petroleum ether: ethyl acetate = 100:0 to 3:1) to obtain compound 8-4.

MS-ESI calculated value [M+H]⁺ +384, measured value 384.

### Step 4

Compound 8-4 (250 mg, 652.27 µmol, 1 equiv) and A-2 (182.20 mg, 717.49 µmol, 1.1 equiv) were dissolved in dioxane (4 mL), then [1,1'-bis(diphenylphosphino)ferrocene]dichloropalladium (47.73 mg, 65.23 µmol, 0.1 equiv), potassium acetate (192.04 mg, 1.96 mmol, 3 equiv) were added thereto. The air in the solution was replaced with nitrogen three times, and the reaction mixture was stirred at 80°C for 12 hours under nitrogen protection. After the reaction was completed, the reaction mixture was filtered, and the filtrate was evaporated to dryness to obtain compound 8-6.

MS-ESI calculated value [M+H]⁺ 431, measured value 431.

### Step 5

Compound G (120 mg, 190.75 µmol, 1 equiv) and 8-6 (90.30 mg, 209.82 µmol, 1.1 equiv) were dissolved in dioxane (2 mL), then [1,1'-bis(diphenylphosphino)ferrocene]dichloropalladium (13.96 mg, 19.07 µmol, 0.1 equiv) and potassium carbonate (79.09 mg, 572.24 µmol, 3 equiv) were added thereto. The air in the solution was replaced with nitrogen three times, and the reaction mixture was stirred at 80°C for 16 hours under nitrogen protection. After the reaction was completed, the reaction mixture was diluted with 10 mL of water and extracted with 60 mL of ethyl acetate (20 mL×3). The organic phase was washed with 40 mL of saturated brine (20 mL×2), dried over anhydrous sodium sulfate, filtered and concentrated under reduced pressure. The residue was subjected to silica gel thin layer chromatography (petroleum ether: ethyl acetate=3:1) to obtain compound 8-7.

MS-ESI calculated value [M+H]⁺ 852, measured value 852.

### Step 6

Compound 8-7 (60 mg, 70.38 µmol, 1 equiv) was dissolved in methanol (1 mL), a solution of hydrogen chloride in methanol (4 mol/L, 1 mL, 56.84 equiv) was added to the reaction mixture, stirred at 25°C for 1 hour. After the reaction was completed, the reaction mixture was concentrated under reduced pressure, and the obtained residue was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 7%-37%, 10 minutes) to obtain the formate of compound 8.

MS-ESI calculated value [M+H]⁺ 638, measured value 638. ¹H NMR (400 MHz, CD₃OD) δ 8.2-8.7 (m, 1H), 7.9-8.0 (m, 2H), 7.56 (d, 4H, J=2.8 Hz), 7.5-7.5 (m, 1H), 7.3- 7.4 (m, 3H), 7.1-7.1 (m, 1H), 4.1-4.2 (m, 2H), 4.0-4.1 (m, 2H), 3.9-4.0 (m, 1H), 3.87 (t, 2H, J =5.6 Hz), 3.16 (br t, 2H, J=5.8 Hz), 3.08 (br d, 2H, J=5.5 Hz), 2.9-3.0 (m, 4H), 2.42 (s, 3H), 2.4-2.4 (m, 2H), 2.35 (s, 1H), 2.15 (s, 3H), 1.8-2.0 (m, 1H).

### Example 9

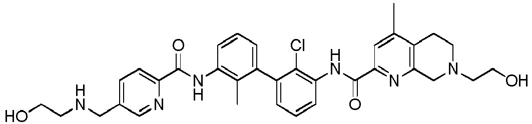

### Synthesis route:

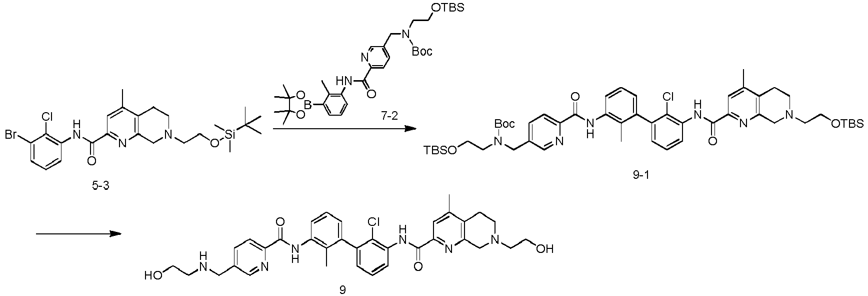

### Step 1

Compound 5-3 (50.0 mg, 92.8 µmol, 1 equiv) was dissolved in dioxane (5 mL) and water (1 mL), and then [1,1'-bis(diphenylphosphino)ferrocene]dichloropalladium (0.010 g, 13.9 µmol, 0.15 equiv), potassium carbonate (0.038 g, 278 µmol, 3 equiv), compound 7-2 (0.058 g, 92.8 µmol, 1 equiv) were added to the reaction mixture, stirred at 80 for 12 hours under nitrogen protection. After the reaction was completed, the reaction mixture was filtered and concentrated under reduced pressure. The residue was purified by silica gel thin layer chromatography (petroleum ether: ethyl acetate=3:1) to obtain compound 9-1.

MS-ESI calculated value [M+H]⁺ 957, measured value 957.

### Step 2

Compound 9-1 (10.0 mg, 10.4 µmol, 1 equiv) was dissolved in ethyl acetate (2 mL), and a solution of hydrogen chloride in ethyl acetate (4 mol/L, 4.00 µL, 1.01 equiv) was added to the reaction mixture, the reaction was conducted at 25°C for 10 minutes. After the reaction was completed, the reaction mixture was concentrated under reduced pressure, and the obtained residue was subjected to high performance liquid chromatography (chromatographic column: UniSil 3-100 C18 Ultra 150^{∗}25 mm^{∗}3 microns; mobile phase: mobile phase A: formic acid aqueous solution with volume fraction of 0.225 %; mobile phase B: acetonitrile; B%: 12%-42%, 10 minutes) to obtain the formate of compound 9.

MS-ESI calculated value [M+H]⁺ 629, measured value 629. 1H NMR (400 MHz, CD₃OD) δ 8.82 (s, 1H), 8.55 (dd, J=1.4, 8.4 Hz, 1H), 8.30 (br d, J=8.0 Hz, 2H), 8.17 (dd, J=1.9, 8.0 Hz, 1H), 7.99 (s, 1H), 7.92 (d, J=8.0 Hz, 1H), 7.46 (t, J=8.0 Hz, 1H), 7.39 - 7.31 (m, 1H), 7.14 - 7.05 ( m, 2H), 4.38 (s, 2H), 4.15 (s, 2H), 3.93 - 3.79 (m, 4H), 3.26 (br t, J=6.2 Hz, 2H), 3.22 - 3.15 (m, 2H), 3.08 - 2.99 (m, 4H), 2.41 (s, 3H), 2.12 (s, 3H).

### Example 10

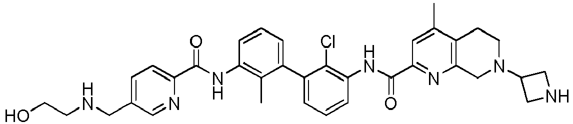

### Synthesis route:

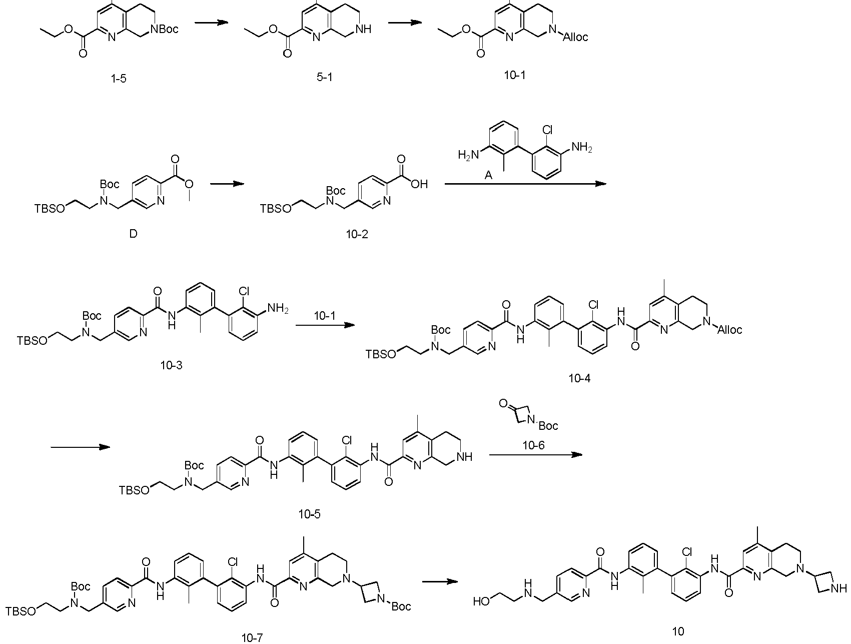

### Step 1

Compound 1-5 (500 mg, 1.56 mmol, 1 equiv) was dissolved in ethyl acetate (10 mL), a solution of hydrogen chloride solution in ethyl acetate (4 mol/L, 10 mL) was added to the reaction mixture, the reaction was conducted at 25°C for 10 minutes. After the reaction was completed, the reaction mixture was concentrated under reduced pressure to obtain compound 5-1.

### Step 2

Compound 5-1 (400 mg, 1.56 µmol, 1 equiv) was dissolved in dichloromethane (10 mL), and then N,N-diisopropylethylamine (604 mg, 4.67 mmol, 814 µl, 0.1 equiv) and allyl chloroformate (376 mg, 3.12 mmol, 329 µl, 3 equiv) were added to the reaction mixture, stirred at 25 °C for 30 minutes. After the reaction was completed, the reaction mixture was concentrated under reduced pressure, and the obtained residue was purified by silica gel thin layer chromatography (petroleum ether: ethyl acetate=2:1) to obtain compound 10-1.

MS-ESI calculated value [M+H]⁺ 305, measured value 305.

### Step 3

Compound D (500 mg, 1.18 mmol) was dissolved in a mixed solution of methanol (5 mL) and water (0.5 mL), lithium hydroxide monohydrate (54.35 mg, 1.30 mmol) was added at 0°C, stirred at 25°C for 1 hour. After the reaction was completed, the pH of the reaction mixture was adjusted to 8 with hydrochloric acid (1 mol/L), and then concentrated to obtain Compound 10-2.

MS-ESI calculated value [M+H]⁺ 411, measured value 411.

### Step 4

Compound 10-2 (500 mg, 1.22 mmol) and compound A (226.71 mg, 974.24 µmol) were dissolved in N,N-dimethylformamide (20 mL), and then diisopropylethylamine (314.78 mg, 2.44 mmol) and HATU (463.04 mg, 1.22 mmol) were added thereto, stirred at 25°C for 16 hours. After the reaction was completed, water (50 mL) was added to the reaction mixture, and extracted with ethyl acetate (50 mL). The organic phase was dried over anhydrous sodium sulfate, filtered and concentrated. The crude product was purified by silica gel column chromatography (petroleum ether: ethyl acetate=20:1 to 10:1) to obtain compound 10-3.

MS-ESI calculated value [M+H]⁺ 625, measured value 625.

### Step 5

Compound 10-3 (500 mg, 800 µmol, 1 equiv), compound 10-1 (243 mg, 800 µmol, 1 equiv) were dissolved in toluene (10.0 mL), and lithium bis(trimethylsilyl)amide (1.0M solution in tetrahydrofuran, 2.40 mL, 3 equiv) was slowly added thereto at 0°C, stirred at 25°C for 3 hours. After the reaction was completed, saturated aqueous ammonium chloride solution (10 mL) was added at room temperature to quench, then water (10 mL) was added to dilute, and the reaction mixture was extracted with ethyl acetate (25 mL×2). The combined organic phase was washed with saturated brine (50 mL×1), dried over anhydrous sodium sulfate, filtered and concentrated under reduced pressure. The obtained residue was purified by silica gel thin layer chromatography (petroleum ether: ethyl acetate=2:1) to obtain compound 10-4.

MS-ESI calculated value [M+H]⁺ 883, measured value 883.

### Step 6

Compound 10-4 (210 mg, 238 µmol, 1 equiv) was dissolved in tetrahydrofuran (5 mL), tetrakistriphenylphosphine palladium (27.5 mg, 23.8 µmol, 0.1 equiv), diethylamine (174 mg, 2.38 mmol, 245 mL, 10 equiv) was added thereto, stirred at 70 °C for 12 hours under nitrogen protection. After the reaction was completed, the reaction mixture was concentrated and the residue was purified by silica gel thin layer chromatography (petroleum ether: ethyl acetate=2:1) to obtain compound 10-5.

MS-ESI calculated value [M+H]⁺ 799, measured value 799. ¹H NMR (400MHz, CDCl₃) δ 10.85 (s, 1H), 10.15 (s, 1H), 8.69 (d, J=8.4 Hz, 1H), 8.52 (br s, 1H), 8.40 - 8.23 (m, 2H) , 7.96 (s, 1H), 7.80 (br s, 1H), 7.38 (td, J=8.0, 16.4 Hz, 2H), 7.03 (t, J=7.2 Hz, 2H), 4.64 (br d, J=7.0 Hz, 2H), 4.13 (s, 2H), 3.89 - 3.61 (m, 2H), 3.46 - 3.26 (m, 2H), 3.23 (t, J=5.8 Hz, 2H), 2.77 (br t, J=5.7 Hz, 2H), 2.35 (s, 3H), 2.18 (s, 3H), 1.55 - 1.33 (m, 9H), 0.90 (s, 9H), 0.06 (s, 6H).

### Step 7

Compound 10-5 (100 mg, 125 µmol, 1 equiv) was dissolved in methanol (5 mL), and then compound 10-6 (32.1 mg, 188 mmol, 1.2 equiv) and sodium cyanoborohydride (0.587 g, 9.35 mmol, 2 equiv) were added to the reaction mixture, stirred at 25 °C for 12 hours. After the reaction was completed, the reaction mixture was concentrated under reduced pressure to obtain compound 10-7.

MS-ESI calculated value [M+H]⁺ 955, measured value 955.

### Step 8

Compound 10-7 (100 mg, 105 µmol, 1 equiv) was dissolved in ethyl acetate (3 mL), a solution of hydrogen chloride in ethyl acetate (4 mol/L, 26.2 µL, 1.0 equiv) was added to the reaction mixture, and the reaction was conducted at 25°C for 0.5 hours. After the reaction was completed, the reaction mixture was concentrated under reduced pressure, and the obtained residue was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 10%-40%, 10 minutes) to obtain the formate of compound 10.

MS-ESI calculated value [M+H]⁺ 640, measured value 640. 1H NMR (400 MHz, CD₃OD) δ 8.76 (d, J=1.3 Hz, 1H), 8.57 (dd, J=1.5, 8.3 Hz, 1H), 8.26 (d, J=8.1 Hz, 1H), 8.11 (dd, J=2.1, 8.1 Hz, 1H), 7.99 - 7.90 (m, 2H), 7.46 (t, J=7.9 Hz, 1H), 7.36 (t, J=7.9 Hz, 1H), 7.14 - 7.04 (m, 2H) ), 4.26 - 4.14 (m, 4H), 4.13 - 4.05 (m, 2H), 3.77 (t, J=5.4 Hz, 2H), 3.73 (s, 2H), 3.64 (quin, J=6.9 Hz, 1H) , 3.00 (t, J=5.3 Hz, 2H), 2.96 - 2.89 (m, 2H), 2.82

### Example 11

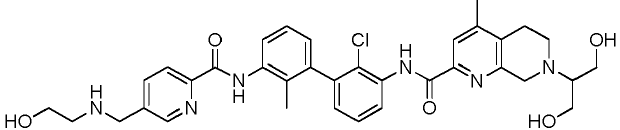

### Synthesis route:

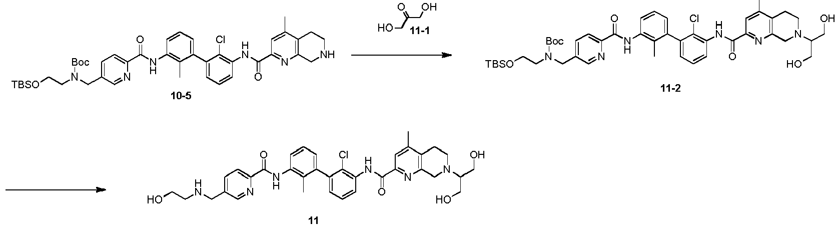

### Step 1

Compound 10-6 (32.1 mg, 188 mmol, 1.2 equiv) was replaced with compound 11-1 (9.01 mg, 100.07 mmol), and the method was the same as that in example 10. After the reaction was completed, the reaction mixture was concentrated under reduced pressure to obtain crude product 11-2.

MS-ESI calculated value [M+H]⁺ 873, measured value 873.

### Step 2

The reaction was the same as that in step 8 of example 10. After the reaction was completed, the reaction mixture was concentrated under reduced pressure, and the residue was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 11%-41%, 10 minutes) to obtain the formate of compound 11.

MS-ESI calculated value [M+H]⁺ 659, measured value 659. ¹H NMR (400 MHz, CD₃OD) δ ppm 8.74 (d, J=1.83 Hz, 1 H), 8.56 (dd, J=8.25, 1.53 Hz, 1 H), 8.50 (s, 1 H), 8.45 - 8.52 ( m, 1 H), 8.25 (d, J=8.07 Hz, 1 H), 8.09 (dd, J=8.07, 2.20 Hz, 1 H), 7.85 - 8.01 (m, 2 H), 7.45 (t, J= 7.95 Hz, 1 H), 7.35 (t, J=7.89 Hz, 1 H), 7.03 - 7.16 (m, 2 H), 4.12 (d, J=15.28 Hz, 3 H), 3.70 - 3.87 (m, 6 H), 3.66 - 3.69 (m, 1 H), 3.13 (t, J=5.87 Hz, 2 H), 2.83 - 3.04 (m, 5 H), 2.37 (s, 3 H), 2.13 (s, 3 H) ).

### Example 12

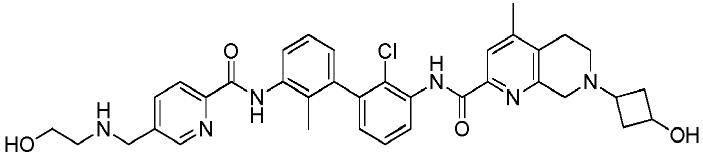

### Synthesis route:

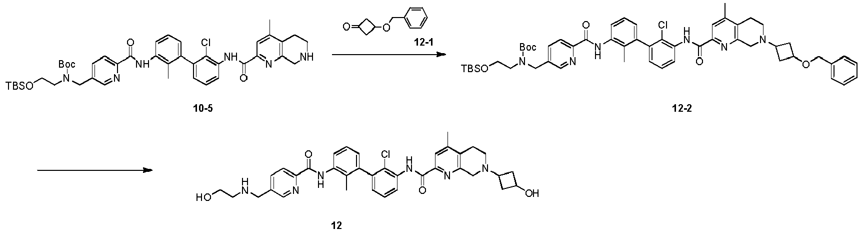

### Step 1

Compound 10-6 (32.1 mg, 188 mmol, 1.2 equiv) was replaced with compound 12-1 (33.06 mg, 187.62 mmol, 1.5 equiv), and the method was the same as that in example 10. After the reaction was completed, the reaction mixture was filtered and concentrated under reduced pressure, then separated and purified by silica gel thin layer chromatography (petroleum ether: ethyl acetate=1:1) to obtain compound 12-2.

### Step 2

Compound 12-2 (50 mg, 52.10 mmol, 1 equiv) was dissolved in dichloromethane (4 mL), and then iodotrimethylsilane (20.85 mg, 104.20 mmol, 14.18 µL, 2 equiv) was added to the reaction mixture. The reaction mixture was stirred at 25 °C for 0.25 hours. After the reaction was completed, the reaction mixture was filtered and concentrated under reduced pressure. The residue was subjected to high performance liquid chromatography (column: UniSil 3-100 C18 Ultra 150^{∗}50 mm^{∗}3 microns; mobile phase: mobile phase A: formic acid aqueous solution with volume fraction of 0.225 %; mobile phase B: acetonitrile; B%: 10% -40%, 10 min) to obtain the formate of compound 12.

MS-ESI calculated value [M+H]⁺ 655, measured value 655. ¹H NMR (400 MHz, CD₃OD) δ ppm 1.72 - 2.02 (m, 2 H) 2.13 (s, 3 H) 2.40 (s, 3 H) 2.64 (br d, J=4.89 Hz, 2 H) 2.80 - 2.90 ( m, 2 H) 2.90 - 2.99 (m, 2 H) 3.15 - 3.22 (m, 2 H) 3.75 (s, 2 H) 3.84 (s, 2 H) 3.93 - 4.10 (m, 1 H) 4.37 (s, 2 H) 7.03 - 7.17 (m, 2 H) 7.37 (t, J=7.70 Hz, 1 H) 7.46 (t, J=7.95 Hz, 1 H) 7.86 - 8.08 (m, 2 H) 8.17 (dd, J =8.07, 2.08 Hz, 1 H) 8.30 (s, 1 H) 8.32 (s, 1 H) 8.56 (dd, J=8.19, 1.47 Hz, 1 H) 8.82 (d, J=1.47 Hz, 1 H) 8.80 - 8.85 (m, 1 H)

### Example 13

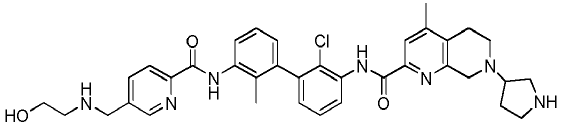

### Synthesis route:

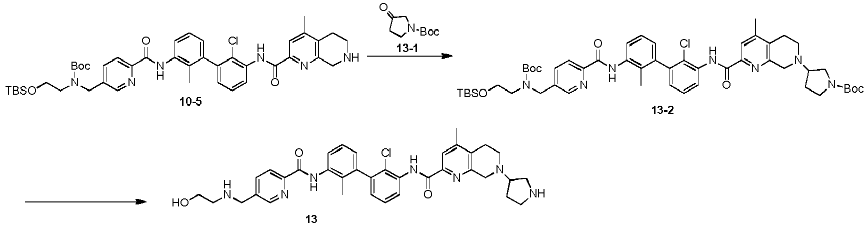

### Step 1

Compound 10-6 (32.1 mg, 188 mmol, 1.2 equiv) was replaced with compound 13-1 (13.90 mg, 75.05 mmol), and the method was the same as that in example 10. After the reaction was completed, the reaction mixture was concentrated under reduced pressure to obtain crude product 13-2.

MS-ESI calculated value [M+H]⁺ 969, measured value 969.

### Step 2

The reaction was the same as that in step 8 of example 10. After the reaction was completed, the reaction mixture was concentrated under reduced pressure, and the residue was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 8%-38%, 10 minutes) to obtain the formate of compound 13.

MS-ESI calculated value [M+H]⁺ 654, measured value 654. ¹H NMR (400 MHz, CD₃OD) δ ppm 8.70 (d, J=1.50 Hz, 1 H), 8.50 - 8.60 (m, 2 H), 8.21 (d, J=8.13 Hz, 1 H), 8.04 (dd, J=8.07, 2.06 Hz, 1 H), 7.91 - 7.98 (m, 2 H), 7.46 (t, J=7.94 Hz, 1 H), 7.35 (t, J=7.82 Hz, 1 H), 7.04 - 7.14 (m, 2 H), 3.99 (s, 2 H), 3.78 - 3.95 (m, 2 H), 3.71 (t, J=5.57 Hz, 2 H), 3.47 - 3.56 (m, 1 H), 3.38 - 3.46 (m, 1 H), 3.21 - 3.30 (m, 3 H), 2.86 - 3.03 (m, 4 H), 2.80 (t, J=5.57 Hz, 2 H), 2.39 (s, 3 H), 2.24 - 2.36 (m, 1 H), 2.03 - 2.16 (m, 4 H).

### Example 14

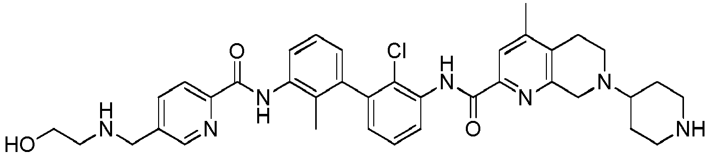

### Synthesis route:

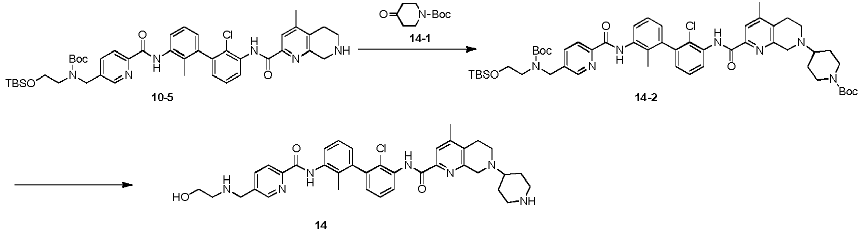

### Step 1

Compound 10-6 (32.1 mg, 188 mmol, 1.2 equiv) was replaced with compound 14-1 (14.95 mg, 75.05 mmol), and the method was the same as that in example 10. After the reaction was completed, the reaction mixture was concentrated under reduced pressure to obtain crude product 14-2.

MS-ESI calculated value [M+H]⁺ 983, measured value 983.

### Step 2

The reaction was the same as that in step 8 of example 10. After the reaction was completed, the reaction mixture was concentrated under reduced pressure, and the residue was subjected to high performance liquid chromatography (chromatographic column: Phenomenex Synergi C18 75^{∗}30^{∗}3 microns; mobile phase: mobile phase A: volume fraction 0.05% aqueous hydrochloric acid solution; mobile phase B: acetonitrile; B%: 13%-33%, 6.5 minutes) to obtain the hydrochloride of compound 14.

MS-ESI calculated value [M+H]⁺ 668, measured value 668. ¹H NMR (400 MHz, CD₃OD) δ ppm 8.86 (d, J=1.34 Hz, 1 H), 8.52 (dd, J=8.31, 1.34 Hz, 1 H), 8.28 - 8.35 (m, 1 H), 8.22 ( dd, J=8.07, 1.83 Hz, 1 H), 8.10 (s, 1 H), 7.92 (d, J=7.95 Hz, 1 H), 7.47 (t, J=7.95 Hz, 1 H), 7.37 (t, J=7.89 Hz, 1 H), 7.04 - 7.17 (m, 2 H), 4.44 (s, 2 H), 3.79 - 4.00 (m, 4 H), 3.50 - 3.71 (m, 4 H), 3.26 - 3.27 (m, 1 H), 3.10 - 3.27 (m, 4 H), 2.39 - 2.72 (m, 6 H), 2.06 - 2.32 (m, 5 H), 1.17 (t, J=7.03 Hz, 2 H).

### Example 15

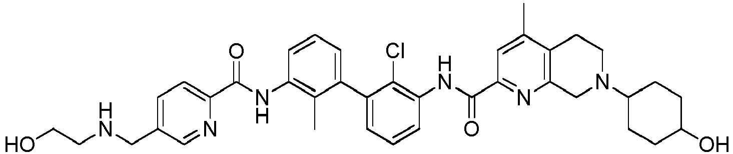

### Synthesis route:

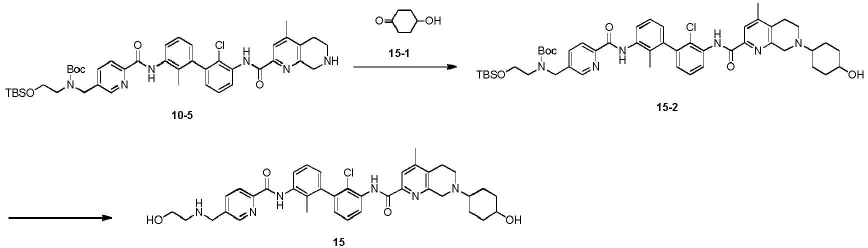

### Step 1

Compound 10-6 (32.1 mg, 188 mmol, 1.2 equiv) was replaced with compound 15-1 (5.71 mg, 50.03 mmol), the method was the same as that in example 10. After the reaction was completed, the reaction mixture was concentrated under reduced pressure to obtain crude product 15-2.

MS-ESI calculated value [M+H]⁺ 898, measured value 898.

### Step 2

The reaction was the same as that in step 8 of example 10. After the reaction was completed, the reaction mixture was concentrated under reduced pressure, and the residue was subjected to high performance liquid chromatography (chromatographic column: Phenomenex Synergi C18 75^{∗}30^{∗}3 microns; mobile phase: mobile phase A: volume fraction 0.05% aqueous hydrochloric acid solution; mobile phase B: acetonitrile; B%: 13%-33%, 6.5 minutes) to obtain the hydrochloride of compound 15.

MS-ESI calculated value [M+H]⁺ 683, measured value 683. ¹H NMR (400 MHz, CD₃OD) δ ppm 8.88 (d, J=1.47 Hz, 1 H), 8.53 (d, J=8.19 Hz, 1 H), 8.30 - 8.38 (m, 1 H), 8.20 - 8.28 ( m, 1 H), 8.10 (s, 1 H), 7.92 (d, J=8.07 Hz, 1 H), 7.48 (t, J=7.89 Hz, 1 H), 7.37 (t, J=7.83 Hz, 1 H), 7.06 - 7.19 (m, 2 H), 4.52 - 4.77 (m, 2 H), 4.46 (s, 2 H), 4.40 - 4.41 (m, 1 H), 3.80 - 4.11 (m, 4 H) , 3.43 - 3.71 (m, 3 H), 3.25 (br d, J=4.89 Hz, 2 H), 3.19 - 3.29 (m, 1 H), 2.47 (s, 3 H), 2.19 - 2.39 (m, 1 H), 2.18 - 2.20 (m, 1 H), 2.13 (s, 4 H), 2.08 (br s, 4 H), 1.63 - 1.75 (m, 2 H), 1.33 - 1.54 (m, 2 H).

### Example 16

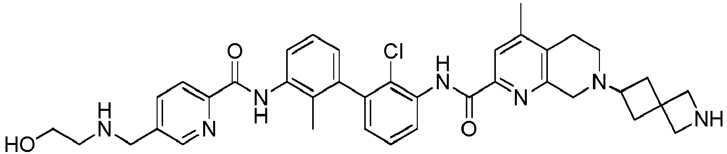

### Synthesis route:

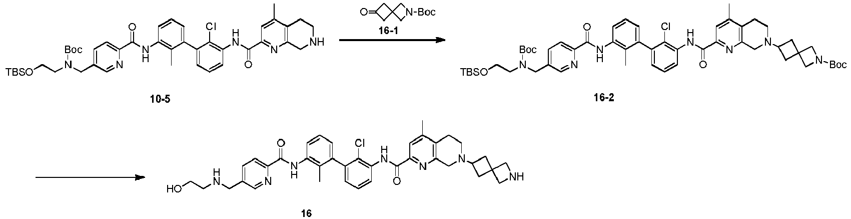

### Step 1

Compound 10-6 (32.1 mg, 188 mmol, 1.2 equiv) was replaced with compound 16-1 (39.64 mg, 187.62 mmol), the method was the same as that in example 10. After the reaction was completed, the reaction mixture was concentrated under reduced pressure to obtain crude product 16-2.

MS-ESI calculated value [M+H]⁺ 995, measured value 995.

### Step 2)

The reaction was the same as that in step 8 of example 10. After the reaction was completed, the reaction mixture was concentrated under reduced pressure, and the residue was subjected to high performance liquid chromatography (chromatographic column: Phenomenex Synergi C18 75^{∗}30^{∗}3 microns; mobile phase: mobile phase A: volume fraction 0.05% aqueous hydrochloric acid solution; mobile phase B: acetonitrile; B%: 13%-33%, 6.5 minutes) to obtain the hydrochloride of compound 16.

MS-ESI calculated value [M+H]⁺ 680, measured value 680. ¹H NMR (400 MHz, CD₃OD) δ ppm 8.87 (d, J=1.13 Hz, 1 H), 8.53 (dd, J=8.25, 1.38 Hz, 1 H), 8.33 (d, J=8.00 Hz, 1 H) , 8.23 (dd, J=8.13, 2.00 Hz, 1 H), 8.11 (s, 1 H), 7.93 (d, J=8.13 Hz, 1 H), 7.48 (t, J=7.94 Hz, 1 H), 7.38 (t, J=7.82 Hz, 1 H), 7.06 - 7.19 (m, 2 H), 4.45 (s, 3 H), 4.09 - 4.30 (m, 1 H), 3.79 - 4.03 (m, 4 H) , 3.19 - 3.30 (m, 5 H), 2.78 - 2.99 (m, 4 H), 2.48 (s, 3H), 2.13 (s, 3 H).

### Example 17

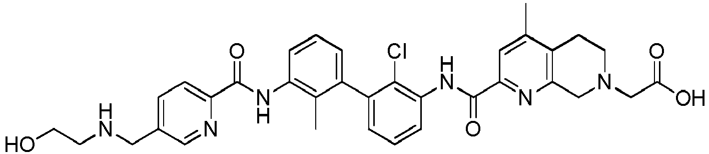

### Synthesis route:

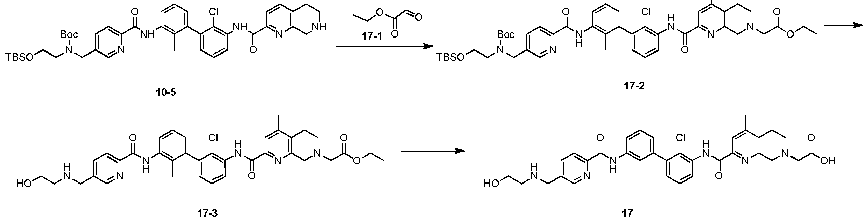

### Step 1

Compound 10-6 (32.1 mg, 188 mmol, 1.2 equiv) was replaced with compound 17-1 (9.58 mg, 93.81 mmol, 1.5 equiv), and the method was the same as that in example 10. After the reaction was completed, the reaction mixture was concentrated under reduced pressure, and separated and purified by silica gel thin layer chromatography (petroleum ether: ethyl acetate=1:1) to obtain compound 17-2.

### Step 2

Compound 17-2 (110 mg, 124.22 mmol, 1 equiv) was dissolved in methanol (4 mL), a solution of hydrogen chloride in methanol (4 mol/L, 155.27 µL, 5 equiv) was added to the reaction mixture, the reaction mixture was stirred at 25 °C for 1 hour. After the reaction was completed, the reaction mixture was filtered and concentrated under reduced pressure to obtain crude compound 17-3.

### Step 3

Compound 17-3 (50 mg, 74.50 mmol, 1 equiv) was dissolved in methanol (3 mL), and lithium hydroxide monohydrate (3.13 mg, 74.50 mmol, 1 equiv) was added to the reaction mixture. The reaction was conducted at 25 °C for 12 hours. After the reaction was completed, the reaction mixture was subjected to high performance liquid chromatography (column: UniSil 3-100 C18 Ultra 150^{∗}50 mm^{∗}3 microns; mobile phase: mobile phase A: formic acid aqueous solution with volume fraction of 0.225 %; mobile phase B: acetonitrile; B%: 12 %-42%, 10 min) to obtain the formate of compound 17.

MS-ESI calculated value [M+H]⁺ 643, measured value 643. ¹H NMR (400 MHz, CD₃OD) δ ppm 2.15 (s, 3 H) 2.46 (s, 3 H) 3.06 - 3.10 (m, 2 H) 3.12 - 3.16 (m, 2 H) 3.42 - 3.52 (m, 2 H) ) 3.65 (br d, J=15.77 Hz, 2 H) 3.76 - 3.87 (m, 2 H) 4.27 (s, 2 H) 4.38 (br s, 2 H) 7.05 - 7.19 (m, 1 H) 7.10 (s , 1 H) 7.38 (t, J=8.01 Hz, 1 H) 7.48 (t, J=7.89 Hz, 1 H) 7.97 (d, J=8.07 Hz, 1 H) 8.05 (s, 1 H) 8.15 (dd , J=8.25, 1.89 Hz, 1 H) 8.30 (d, J=8.19 Hz, 1 H) 8.46 (s, 1 H) 8.54 - 8.62 (m, 1 H) 8.80 (d, J=1.71 Hz, 1 H) ).

### Example 18

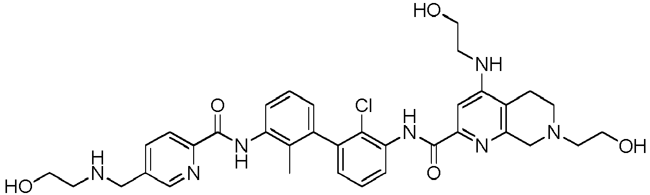

### Synthesis route:

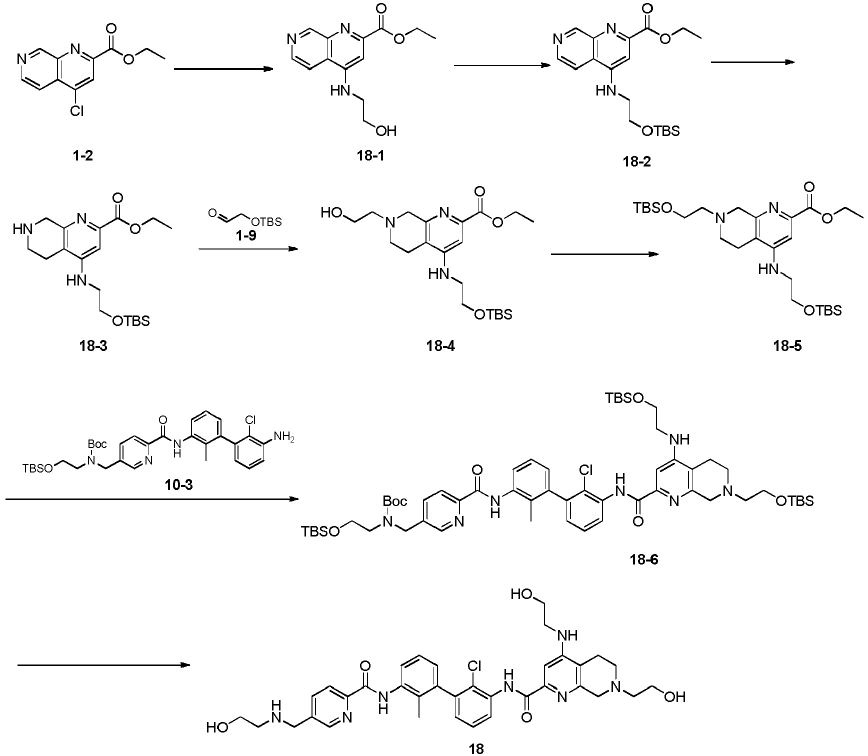

### Step 1

Compound 1-2 (100 mg, 421 mmol) was dissolved in dimethyl sulfoxide (2 mL), and then potassium fluoride (29.3 mg, 505 mmol) and ethanolamine (30.8 mg, 505 mmol, 30.5 µl) were added to the reaction mixture. The reaction mixture was stirred at 130 °C for 12 hours. After the reaction was completed, the reaction mixture was concentrated under reduced pressure to obtain the crude compound 18-1.

MS-ESI calculated value [M+H]⁺ 262, measured value 262.

### Step 2

Compound 18-1 (330 mg, 1.26 mmol) was dissolved in dichloromethane (15 mL), N,N-diisopropylethylamine (816 mg, 6.32 mmol, 1.10 mL) and tertbutyldimethylsilyl chloride (571 mg, 3.79 mmol) was added to the reaction mixture, and the reaction mixture was stirred at 25 °C for 2 hours. After the reaction was completed, water (30 mL) was added to the reaction mixture and extracted with dichloromethane (20 mL×2). The combined organic phase was dried over anhydrous sodium sulfate, filtered and concentrated under reduced pressure. The reaction mixture was separated and purified by silica gel thin layer chromatography (petroleum ether: ethyl acetate: ethanol=1:1:3) to obtain compound 18-2.

MS-ESI calculated value [M+H]⁺ 376, measured value 376.

### Step 3

Compound 18-2 (170 mg, 445 mmol) was dissolved in acetic acid (10 mL), sodium borohydride (58.9 mg, 1.56 mmol) was added to the reaction mixture at 0 °C, and the reaction mixture was stirred at 25 °C for 1 hour. After the reaction was completed, the reaction mixture was poured into ice water and the pH of the reaction mixture was adjusted to 7 with sodium carbonate, and then extracted with ethyl acetate (30 mL×3). The combined organic phase was dried over anhydrous sodium sulfate, filtered and concentrated under reduced pressure to obtain compound 18-3.

MS-ESI calculated value [M+H]⁺ 380, measured value 380.

### Step 4

Compound 18-3 (168 mg, 340.39 mmol) was dissolved in methanol (3 mL), and then compound 1-9 (101 mg, 579 mmol, 110 µL) was added to the reaction mixture. The reaction mixture was stirred at 25 °C for 0.5 hours, sodium cyanoborohydride (53.5 mg, 851 mmol) was then added to the reaction mixture and stirred at 25 °C for 1 hour. After the reaction was completed, the reaction mixture was concentrated under reduced pressure to obtain the crude compound 18-4.

MS-ESI calculated value [M+H]⁺ 424, measured value 424.

### Step 5

Compound 18-4 (250 mg, 590.15 mmol) was dissolved in dichloromethane (6 mL), and N,N-diisopropylethylamine (381 mg, 2.95 mmol, 514 µL) and tertbutyldimethylsilyl chloride (267 mg, 1.77 mmol) were added to the reaction mixture. The reaction mixture was stirred at 25 °C for 1 hour. After the reaction was completed, water (30 mL) was added to the reaction mixture and the reaction mixture was extracted with dichloromethane (30 mL × 2). The combined organic phase was dried over anhydrous sodium sulfate, filtered and concentrated under reduced pressure. The reaction mixture was subjected to silica gel thin layer chromatography (petroleum ether: ethyl acetate: ethanol=1:2:6) to obtain compound 18-5.

MS-ESI calculated value [M+H]⁺ 538, measured value 538.

### Step 6

Compound 18-5 (83 mg, 142 mmol) and compound 10-3 (134 mg, 171 mmol) were dissolved in toluene (6 mL), and lithium hexamethyldisilazide (1M solution in tetrahydrofuran, 854 µl) was added dropwise to the reaction mixture at 25 °C. The reaction mixture was stirred at 25 °C for 0.5 hours. After the reaction was completed, saturated ammonium chloride solution (10 mL) was added at 25°C to quench the reaction, the reaction mixture was diluted with water (10 mL), then extracted with ethyl acetate (15 mL × 2). The combined organic phase was dried over anhydrous sodium sulfate, filtered and concentrated under reduced pressure. The reaction mixture was subjected to silica gel thin layer chromatography (petroleum ether: ethyl acetate=3:2) to obtain compound 18-6

MS-ESI calculated value [M+H]⁺ 1116 and 1117, measured value 1116 and 1117.

### Step 7

Compound 18-6 (11.0 mg, 8.78 mmol) was dissolved in ethyl acetate (1 mL), and a solution of hydrogen chloride in ethyl acetate (4 mol/L, 1 mL) was added to the reaction mixture. The reaction mixture was stirred at 25 °C for 0.5 hours. After the reaction was completed, the reaction mixture was concentrated under reduced pressure, and the residue was subjected to high performance liquid chromatography (chromatographic column: 3_Phenomenex Luna C18 75^{∗}30 mm^{∗}3 microns; mobile phase: mobile phase A: 0.05% aqueous hydrochloric acid by volume; mobile phase B: acetonitrile; B%: 13%-33%, 6.5 minutes) to obtain the hydrochloride of compound 18.

MS-ESI calculated value [M+H]⁺ 674 and 676, measured value 674 and 676. ¹H NMR (400MHz, CD₃OD ) δ= 8.87 (s, 1H), 8.29-8.35 (m, 1H), 8.23 (d, 1H), 7.91 (d, 1H), 7.77-7.86 (m, 2H), 7.51 ( m, 1H), 7.36 (m, 1H), 7.30 (d, 1H), 7.10 (d, 1H), 4.77 (s, 4H), 4.45 (s, 2H), 4.03 (s, 2H), 3.87 (m, 4H), 3.76 (d, 2H), 3.57 (s, 2H), 3.21-3.28 (m, 2H), 3.04 (s, 2H), 2.13 (s, 3H).

### Example 19

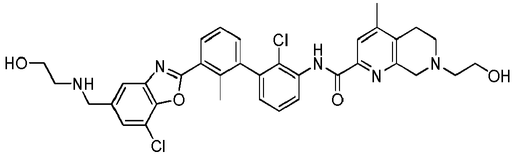

### Synthesis route:

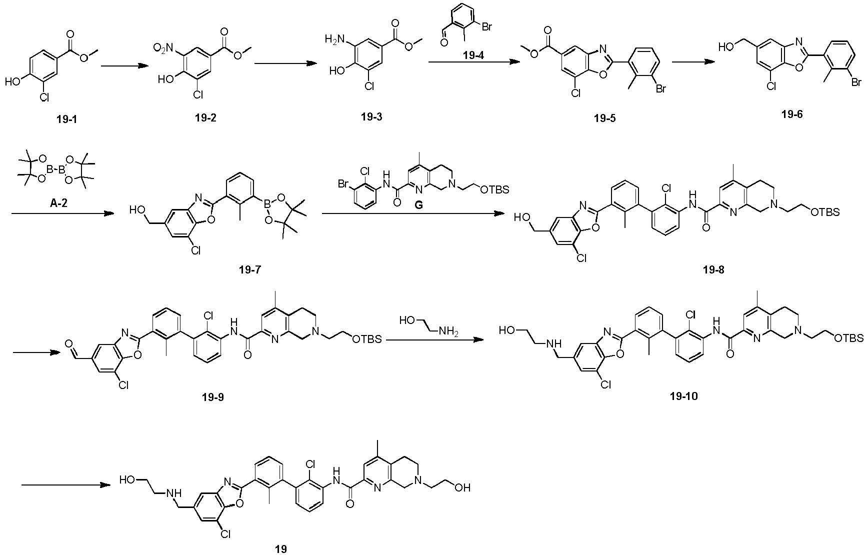

### Step 1

Compound 19-1 (5.00 g, 26.8 mmol, 1 equiv) was dissolved in acetic acid (20.0 mL), then nitric acid (6.07 g, 96.3 mmol, 4.34 mL, 3.60 equiv) was added dropwise to the reaction mixture at 0 °C, stirred at 25°C for 2 hours. After the reaction was completed, water (100 mL) was slowly added to the reaction mixture at 25°C, stirred for 30 minutes, the reaction mixture was filtered to obtain compound 19-2 as the filter cake.

### Step 2

Compound 19-2 (6.00 g, 25.9 mmol, 1 equiv) was dissolved in methanol (100 mL), and wet palladium on carbon (60.0 mg, 10% purity) was added to the reaction mixture under nitrogen. The air was replaced with hydrogen three times, and the reaction mixture was stirred at 25 °C for 12 hours under hydrogen (50 psi). After the reaction was completed, the reaction mixture was filtered and concentrated under reduced pressure to obtain compound 19-3.

### Step 3

Compound 19-3 (4.50 g, 22.3 mmol, 1 equiv) was dissolved in ethanol (10 mL), then compound 19-4 (4.00 g, 20.1 mmol, 0.9 equiv) was added to the reaction mixture, the reaction mixture was stirred at 25 °C for 1 hour, then the reaction mixture was concentrated under reduced pressure, dichloromethane (10 mL) was added thereto, then 2,3-dicyano-5,6-dichlorobenzoquinone (4.56 g, 20.1 mmol, 0.9 equiv) was added to the reaction mixture, stirred at 25°C for 0.5 hours. After the reaction was completed, the reaction mixture was diluted with water (150 mL), then extracted with ethyl acetate (150 mL×2). The combined organic phase was washed with saturated brine (200 mL×1), dried over anhydrous sodium sulfate, filtered and concentrated under reduced pressure. The residue was subjected to silica gel column chromatography (petroleum ether: ethyl acetate=100:1 to 50:1) to obtain compound 19-5.

MS-ESI calculated value [M+H]⁺ 381, measured value 381.

### Step 4

Compound 19-5 (100 mg, 263 mmol, 1 equiv) was dissolved in tetrahydrofuran (5 mL), and then lithium aluminium tetrahydride (12.0 mg, 315 µmol, 1.2 equiv) was added to the reaction mixture at 0°C, stirred at 25°C for 2 hours. After the reaction was completed, sodium sulfate decahydrate (2 g) was added to the reaction mixture, stirred for 30 minutes. The reaction mixture was filtered and concentrated under reduced pressure. The residue was purified by silica gel thin layer chromatography (petroleum ether: ethyl acetate=2:1) to obtain compound 19-6.

MS-ESI calculated value [M+H]⁺ 353, measured value 353.

### Step 5

Compound 19-6 (50.0 mg, 142 µmol, 1 equiv) was dissolved in methanol (5 mL), and then [1,1'-bis(diphenylphosphino)ferrocene]dichloropalladium (20.8 mg, 28.4 µmol, 0.2 equiv), potassium acetate (41.8 mg, 425 µmol, 3 equiv) and compound A-2 (54.0 mg, 213 µmol, 1.5 equiv) were added to the reaction mixture, stirred at 90 °C under nitrogen protection. After the reaction was completed, the mixture was filtered and concentrated under reduced pressure to obtain compound 19-7.

MS-ESI calculated value [M+H]⁺ 400, measured value 400.

### Step 6

Compound 19-7 (50.0 mg, 125 µmol, 1 equiv) was dissolved in dioxane (5 mL) and water (0.11 mL), then [1,1'-bis(diphenylphosphino)ferrocene]dichloropalladium (18.3 mg, 25.0 µmol, 0.2 equiv), potassium carbonate (51.9 mg, 375 µmol, 3 equiv) and compound (67.4 g, 125 µmol, 1 equiv) were added to the reaction mixture, stirred at 80 °C for 12 hours under nitrogen protection. After the reaction was completed, the reaction mixture was filtered and concentrated under reduced pressure. The residue was purified by silica gel thin layer chromatography (petroleum ether: ethyl acetate=3:1) to obtain compound 19-8.

MS-ESI calculated value [M+H]⁺ 731, measured value 731.

### Step 7

Compound 19-8 (20.0 mg, 27.3 µmol, 1 equiv) was dissolved in 1,2-dichloromethane (3 mL), and then manganese dioxide (23.8 mg, 273 µmol, 10 equiv) was added to the reaction mixture, stirred at 70 °C for 2 hours. After the reaction was completed, the reaction mixture was filtered and concentrated under reduced pressure to obtain compound 19-9.

MS-ESI calculated value [M+H]⁺ 729, measured value 729.

### Step 8

Compound 19-9 (20.0 mg, 27.4 µmol, 1 equiv) was dissolved in methanol (3 mL), and then ethanolamine (2.18 mg, 35.6 µmol, 2.15 µL, 1.3 equiv), sodium cyanoborohydride (3.44 mg, 54.8 µmol, 2 equiv) and acetic acid (165 µg, 2.74 µmol, 0.1 equiv) were added to the reaction mixture, stirred at 25°C for 30 minutes. After the reaction was completed, the reaction mixture was filtered and concentrated under reduced pressure to obtain compound 19-10.

MS-ESI calculated value [M+H]⁺ 388, measured value 388.

### Step 9

Compound 19-10 (20.0 mg, 25.8 µmol, 1 equiv) was dissolved in ethyl acetate (2 mL), a solution of hydrogen chloride in ethyl acetate (4 mol/L, 1 mL) was added to the reaction mixture, the reaction was conducted at 25°C for 0.5 hours. After the reaction was completed, the reaction mixture was concentrated under reduced pressure, and the obtained residue was subjected to high performance liquid chromatography (chromatographic column: UniSil 3-100 C18 Ultra 150^{∗}50 mm^{∗}3 microns; mobile phase: mobile phase A: formic acid aqueous solution with volume fraction of 0.225 %; mobile phase B: acetonitrile; B%: 20%-50%, 10 minutes) to obtain the formate of compound 19.

MS-ESI calculated value [M+H]⁺ 662, measured value 662. ¹H NMR (400MHz, CD₃OD) δ= 8.61 (dd, J=1.5, 8.2 Hz, 1H), 8.46 (m, 2H), 8.25 (d, J=7.8 Hz, 1H), 7.97 (s, 1H), 7.90 (s, 1H), 7.66 (d, J=1.2 Hz, 1H), 7.58 - 7.48 (m, 2H), 7.45 (d, J=7.6 Hz, 1H), 7.15 (dd, J=1.4, 7.6 Hz, 1H), 4.37 (s, 2H), 3.94 (s, 2H), 3.83 (q, J=5.6 Hz, 4H), 3.19 - 3.14 (m, 2H), 3.09 - 3.00 (m, 2H), 2.97 (m , J=5.8 Hz, 2H), 2.85 (t, J=5.8 Hz, 2H), 2.55 (s, 3H), 2.41 (s, 3H).

### Example 20

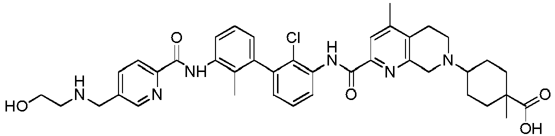

### Synthesis route:

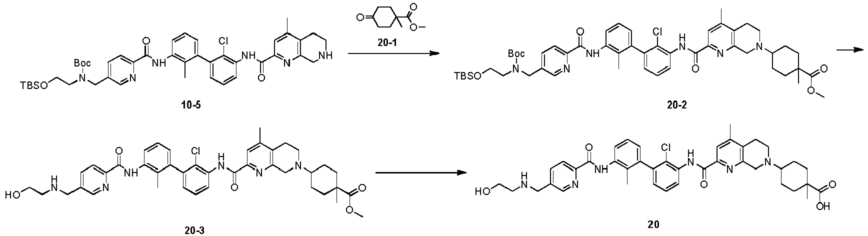

### Step 1

Compound 10-6 (32.1 mg, 188 mmol, 1.2 equiv) was replaced with compound 20-1 (31.9 mg, 188 mmol, 1.5 equiv), and the method was the same as that in example 10. After the reaction was completed, the reaction mixture was concentrated under reduced pressure to obtain compound 20-2.

MS-ESI calculated value [M+H]⁺ 954, measured value 954.

### Step 2

The reaction was the same as that in step 8 of example 10. After the reaction was completed, the reaction mixture was concentrated under reduced pressure to obtain compound 20-3.

MS-ESI calculated value [M+H]⁺ 739, measured value 739.

### Step 3

Compound 20-3 (60.0 mg, 77.3 µmol, 1 equiv, hydrochloride) was dissolved in methanol (5 mL), and lithium hydroxide monohydrate (3.25 mg, 77.3 mmol, 1 equiv) was added to the reaction mixture. The reaction was conducted at 25 °C for 1 hour. After the reaction was completed, the reaction mixture was concentrated under reduced pressure, and the obtained residue was subjected to high performance liquid chromatography (chromatographic column: UniSil 3-100 C18 Ultra 150^{∗}50 mm^{∗}3 microns; mobile phase: mobile phase A: formic acid aqueous solution with volume fraction of 0.225 %; mobile phase B: acetonitrile; B%: 12%-42%, 10 minutes) to obtain the formate of compound 20.

MS-ESI calculated value [M+H]⁺ 725, measured value 725. ¹H NMR (400MHz, CD₃OD) δ 8.82 (s, 1H), 8.54 (d, J=8.2 Hz, 1H), 8.46 - 8.24 (m, 3H), 8.18 (m, J=8.0 Hz, 1H), 7.99 ( m, 1H), 7.94 (d, J=8.2 Hz, 1H), 7.46 (t, J=7.8 Hz, 1H), 7.35 (m, J=7.8 Hz, 1H), 7.14 - 7.05 (m, 2H), 4.38 (s, 2H), 4.35 - 4.21 (m, 2H), 3.84 (t, J=5.0 Hz, 2H), 3.37 (br s, 2H), 3.18 (m, J=4.2 Hz, 2H), 3.14 - 2.94 (m, 3H), 2.42 (s, 3H), 2.32 (br d, J=13.2 Hz, 1H), 2.12 (s, 3H), 2.09 - 1.94 (m, 2H), 1.89 - 1.77 (m, 3H) ), 1.75 - 1.59 (m, 1H), 1.37 - 1.27 (m, 1H), 1.26 (s, 2H), 1.18 (s, 1H).

### Example 21

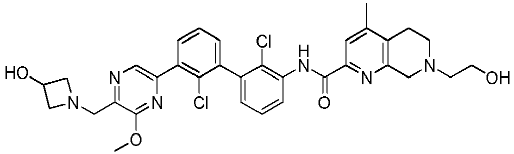

### Synthesis route:

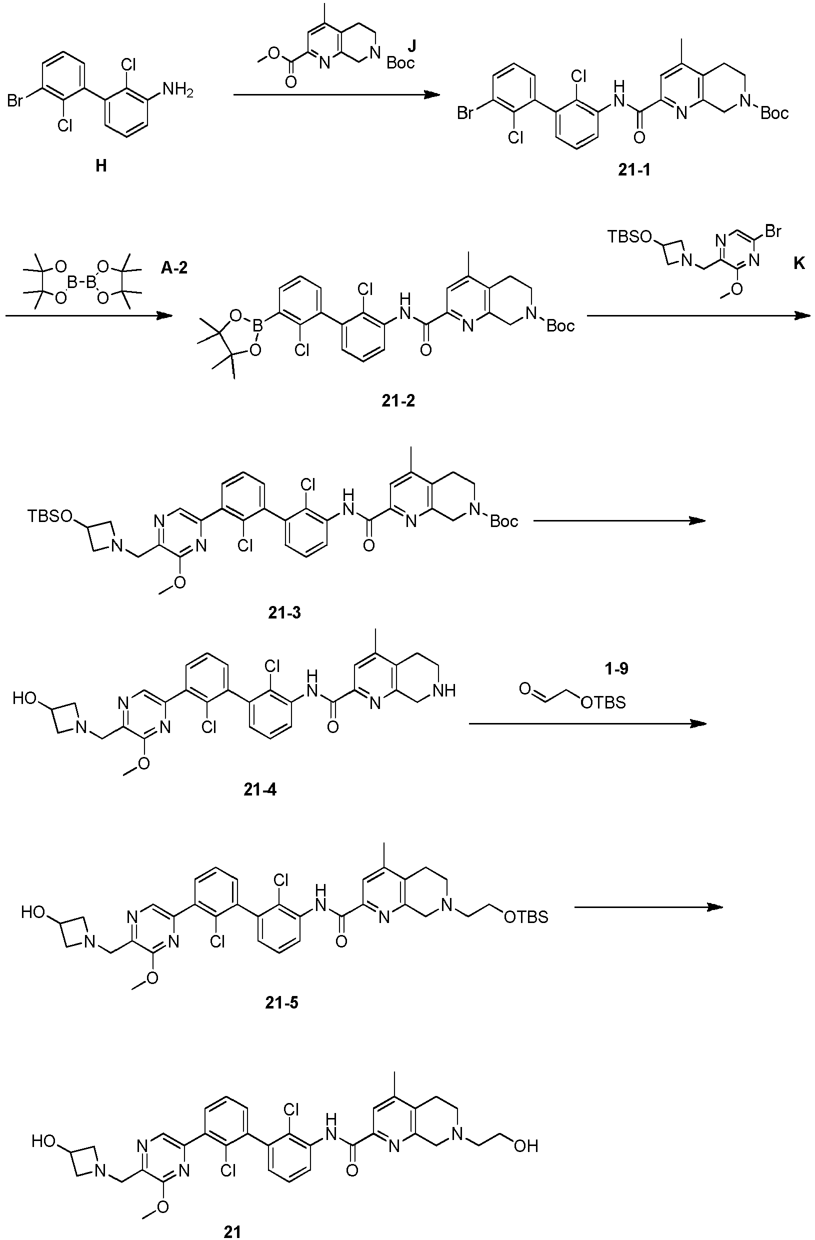

### Step 1

Intermediates H (200 mg, 630.90 µmol, 1 equiv) and J (193.28 mg, 630.90 µmol, 1 equiv) were dissolved in toluene (5 mL), a solution of lithium bis(trimethylsilyl)amide in tetrahydrofuran (1 mol/L, 1.26 mL, 2 equiv) was added thereto at 0 °C, and the reaction mixture was stirred at 15°C for 2 hours. After the reaction was completed, the reaction mixture was diluted with 20 mL of water and extracted with 60 mL of ethyl acetate (20 mL × 3). The organic phase was washed with 40 mL of saturated brine (20 mL × 2), dried over anhydrous sodium sulfate, filtered and concentrated under reduced pressure. The residue was subjected to silica gel thin-layer chromatography (petroleum ether: ethyl acetate=3:1) to obtain compound 21-1.

MS-ESI calculated value [M+H]⁺ 592, measured value 592.

### Step 2

Compound 21-1 (330 mg, 507.84 µmol, 1 equiv) and A-2 (154.75 mg, 609.41 µmol, 1.2 equiv) were dissolved in dioxane (10 mL), and then [1,1'-bis(diphenylphosphino)ferrocene]dichloropalladium (37.16 mg, 50.78 µmol, 0.1 equiv) and potassium acetate (149.52 mg, 1.52 mmol, 3 equiv) were added thereto. The air in the solution was replaced with nitrogen three times. The reaction mixture was stirred at 80°C for 12 hours under nitrogen protection. After the reaction was completed, the reaction mixture was filtered and concentrated under reduced pressure to obtain crude product 21-2.

MS-ESI calculated value [M+H]⁺ 638, measured value 638.

### Step 3

Compound 21-2 (200 mg, 313.29 µmol, 1 equiv) and Intermediate K (121.67 mg, 313.29 µmol, 1 equiv) were dissolved in dioxane (5 mL), followed by the addition of [1,1-bis (diphenylphosphine)ferrocene]palladium dichloride (22.92 mg, 31.33 µmol, 0.1 equiv) and potassium carbonate (129.90 mg, 939.87 µmol, 3 equiv). The air in the solution was replaced with nitrogen three times. The reaction mixture was stirred at 80°C for 12 hours under nitrogen protection. After the reaction was completed, the reaction mixture was diluted with 20 mL of water and extracted with 90 mL of ethyl acetate (30 mL×3). The organic phase was washed with 50 mL of saturated brine (25 mL×2), dried over anhydrous sodium sulfate, filtered and concentrated under reduced pressure. The residue was subjected to silica gel thin layer chromatography (petroleum ether: ethyl acetate=1:1) to obtain compound 21-3.

MS-ESI calculated value [M+H]⁺ 820, measured value 820.

### Step 4

Compound 21-3 (200 mg, 243.94 µmol, 1 equiv) was dissolved in ethyl acetate (5 mL), a solution of hydrogen chloride in ethyl acetate (4 mol/L, 0.5 mL, 8.20 equiv) was added thereto, and the reaction was conducted at 15 °C for 2 hours. After the reaction was completed, the reaction mixture was concentrated under reduced pressure to obtain the crude compound 21-4.

MS-ESI calculated value [M+H]⁺ 605, measured value 605.

### Step 5

Compound 21-4 (140 mg, 231.21 µmol, 1 equiv) and compound 1-9 (120.91 mg, 693.63 µmol, 132.14 µl, 3 equiv) were dissolved in methanol (3 mL), and sodium cyanoborohydride (36.32 mg, 578.02 µmol, 2.5 equiv) was added thereto. The reaction was conducted at 15 °C for 3 hours. After the reaction was completed, the reaction mixture was filtered and concentrated under reduced pressure. The residue was subjected to silica gel thin layer chromatography (petroleum ether: ethyl acetate: ethanol=4:3:1) to obtain compound 21-5.

MS-ESI calculated value [M+H]⁺ 763, measured value 763.

### Step 6

Compound 21-5 (72 mg, 94.26 µmol, 1 equiv) was dissolved in methanol (3 mL), and then potassium fluoride (54.77 mg, 942.62 µmol, 10 equiv) was added thereto. The reaction was conducted at 70 °C for 12 hours. After the reaction was completed, the reaction mixture was filtered and concentrated under reduced pressure. The obtained residue was subjected to high performance liquid chromatography (chromatographic column: Unisil 3-100 C18 Ultra 150^{∗}50mm^{∗}3µm; mobile phase: mobile phase A: formic acid aqueous solution with volume fraction of 0.225 %; mobile phase B: acetonitrile; B%: 12%-32%, 10 minutes) to obtain the formate of compound 21.

MS-ESI calculated value [M+H]⁺ 651, measured value 651. ¹H NMR (400 MHz, CD₃OD) δ =8.61 (dd, J = 1.4, 8.4 Hz, 1H), 8.52 - 8.42 (m, 2H), 7.97 (s, 1H), 7.72 (dd, J = 1.6, 7.8 Hz , 1H), 7.59 (t, J = 7.6 Hz, 1H), 7.53 - 7.41 (m, 2H), 7.17 (dd, J = 1.4, 7.6 Hz, 1H), 4.69 (br d, J = 5.9 Hz, 1H) ), 4.53 (br s, 2H), 4.45 - 4.33 (m, 2H), 4.12 (s, 3H), 3.98 - 3.87 (m, 4H), 3.84 (t, J = 5.7 Hz, 2H), 3.05 (br d, J = 5.0 Hz, 2H), 3.00 - 2.94 (m, 2H), 2.91 - 2.82 (m, 2H), 2.42 (s, 3H).

### Example 22

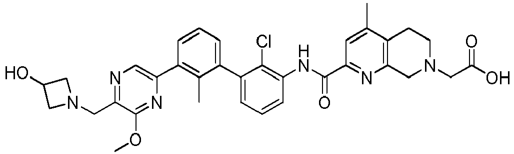

### Synthesis route:

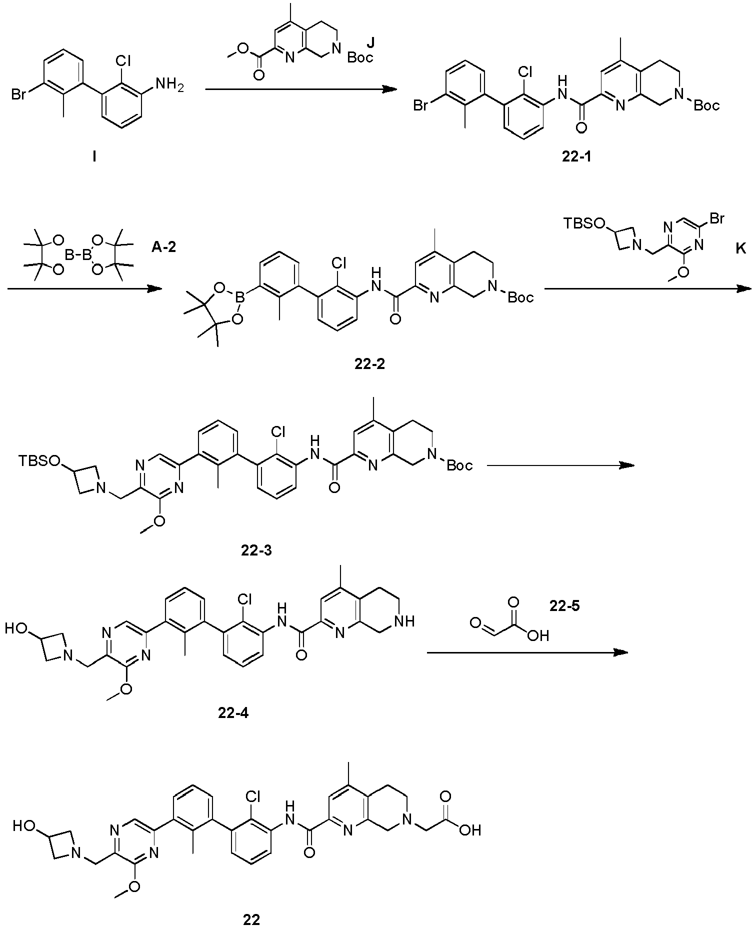

### Step 1

Intermediates I (250 mg, 842.91 µmol, 1 equiv) and J (258.23 mg, 842.91 µmol, 1 equiv) were dissolved in toluene (5 mL), and a solution of lithium bis(trimethylsilyl)amide in tetrahydrofuran (1 mol/L, 1.69 mL, 2 equiv) was added at 0 °C. The reaction mixture was stirred at 15 °C for 2 hours. After the reaction was completed, the reaction mixture was diluted with 20 mL of water and extracted with 60 mL of ethyl acetate (20 mL × 3). The organic phase was washed with 40 mL of saturated brine (20 mL × 2), dried over anhydrous sodium sulfate, filtered and concentrated under reduced pressure. The residue was subjected to silica gel thin layer chromatography (petroleum ether: ethyl acetate=3:1) to obtain compound 22-1.

MS-ESI calculated value [M+H]⁺ 572, measured value 572.

### Step 2

Compound 22-1 (400 mg, 525.48 µmol, 1 equiv) and A-2 (160.13 mg, 630.58 µmol, 1.2 equiv) were dissolved in dioxane (10 mL), and then [1,1'-bis(diphenylphosphino)ferrocene]dichloropalladium (38.45 mg, 52.55 µmol, 0.1 equiv) and potassium acetate (154.72 mg, 1.58 mmol, 3 equiv) were added thereto. The air in the solution was replaced with nitrogen three times, and the reaction mixture was stirred at 80°C for 12 hours under nitrogen protection. After the reaction was completed, the reaction mixture was filtered and concentrated under reduced pressure to obtain crude product 22-2.

MS-ESI calculated value [M+H]⁺ 618, measured value 618.

### Step 3

Intermediate 22-2 (300 mg, 485.46 µmol, 1 equiv) and intermediate K (150.83 mg, 388.37 µmol, 1 equiv) were dissolved in dioxane (5 mL), and then [1,1'-bis(diphenylphosphino)ferrocene]dichloropalladium (35.52 mg, 48.55 µmol, 0.1 equiv) and potassium carbonate (201.29 mg, 1.46 µmol, 3 equiv) were added thereto. The air in the solution was replaced three times with nitrogen, and the reaction mixture was stirred at 80°C for 12 hours under nitrogen protection. After the reaction was completed, the reaction mixture was diluted with 20 mL of water and extracted with 90 mL of ethyl acetate (30 mL × 3). The organic phase was washed with 50 mL of saturated brine (25 mL × 2), dried over anhydrous sodium sulfate, filtered and concentrated under reduced pressure. The residue was subjected to silica gel thin layer chromatography (petroleum ether: ethyl acetate=1:1) to obtain compound 22-2.

MS-ESI calculated value [M+H]⁺ 799, measured value 799.

### Step 4

Compound 22-3 (240 mg, 300.20 µmol, 1 equiv) was dissolved in dichloromethane (3 mL), then trifluoroacetic acid (9.24 g, 81.04 mmol, 6 mL, 269.94 equiv) was added thereto, and the reaction was conducted at 35 °C for 12 hours. After the reaction was completed, the reaction mixture was concentrated under reduced pressure to obtain the crude compound 22-4.

MS-ESI calculated value [M+H]⁺ 585, measured value 585.

### Step 5

Compound 22-4 (150 mg, 256.37 µmol, 1 equiv) and compound 22-5 (189.80 mg, 1.28 mmol, 142.71 µl, 5 equiv) were dissolved in methanol (5 mL) and N,N-dimethylformamide (5 mL), and stirred at 15 °C for 0.5 hours. Sodium cyanoborohydride (48.33 mg, 769.11 µmol, 3 equiv) was then added thereto, and the reaction was conducted at 15 °C for 12 hours. After the reaction was completed, the reaction mixture was filtered and concentrated under reduced pressure. The crude product was subjected to high performance liquid chromatography (chromatographic column: Phenomenex Synergi C18 150^{∗}25 mm^{∗}10 microns; mobile phase: Mobile phase A: formic acid aqueous solution with volume fraction of 0.225 %; mobile phase B: acetonitrile; B%: 18%-48%, 8.5 minutes) to obtain the formate of compound 22.

MS-ESI calculated value [M+H]⁺ 643, measured value 643. ¹H NMR (400 MHz, CD₃OD) δ=8.57 (dd, J = 1.4, 8.3 Hz, 1H), 8.40 (s, 1H), 8.34 (s, 1H), 8.03 (s, 1H), 7.56 - 7.37 (m , 3H), 7.33 - 7.22 (m, 1H), 7.12 (dd, J = 1.4, 7.6 Hz, 1H), 4.74 (s, 1H), 4.65 (s, 2H), 4.53 (br dd, J = 6.6, 11.4 Hz, 2H), 4.42 (s, 2H), 4.14 - 4.00 (m, 5H), 3.73 (s, 2H), 3.60 - 3.44 (m, 2H), 3.21 - 3.02 (m, 2H), 2.44 (s , 3H), 2.14 (s, 3H).

### Example 23

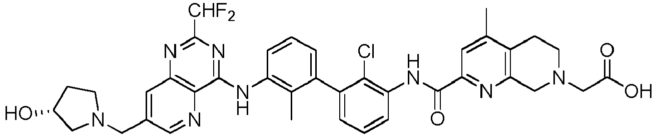

### Synthesis route:

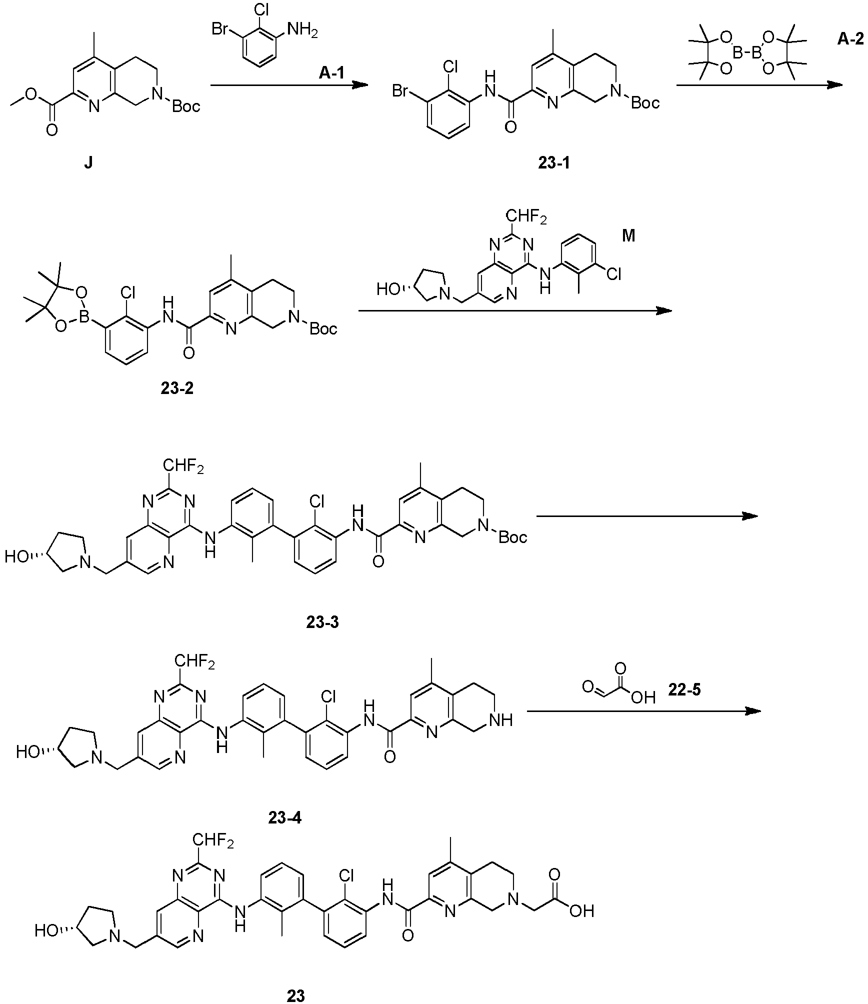

### Step 1

Compound J (1 g, 3.26 mmol, 1 equiv) and A-1 (606.55 mg, 2.94 mmol, 126.44 µl, 0.9 equiv) were dissolved in toluene (10 mL), and a solution of lithium bis(trimethylsilyl)amide in tetrahydrofuran (1 mol/L, 6.53 mL, 2 equiv) was added dropwise at 0 °C. The reaction mixture was stirred at 25°C for 2 hours. After the reaction was completed, the reaction was quenched with 10 mL of saturated aqueous ammonium chloride solution at 25 °C, then the reaction mixture was extracted with 90 mL of ethyl acetate (30 mL×3). the organic phase was washed with 60 mL of saturated brine (30 mL×2), dried over anhydrous sodium sulfate, filtered and concentrated under reduced pressure. Ethyl acetate was added to the crude product at 25°C and stirred for 30 minutes, then filtered, the obtained solid was the crude product 23-1.

MS-ESI calculated value [M+H]⁺ 481, measured value 481.

### Step 2

Compound 23-1 (500 mg, 1.04 mmol, 1 equiv) and A-2 (316.91 mg, 1.25 mmol, 1.2 equiv) were dissolved in dioxane (10 mL), and then [1,1'-bis(diphenylphosphino)ferrocene]dichloropalladium (84.93 mg, 104.00 µmol, 0.1 equiv) and potassium acetate (255.16 mg, 2.60 mmol, 2.5 equiv) were added thereto. The air in the solution was replaced with nitrogen three times, and the reaction mixture was stirred at 90°C for 3 hours under nitrogen protection. After the reaction was completed, the reaction mixture was filtered and concentrated under reduced pressure. The obtained residue was the crude compound 23-2.

MS-ESI calculated value [M+H]⁺ 528, measured value 528.

### Step 3

Compound 23-2 (410 mg, 776.74 µmol, 1 equiv) and intermediate M (163.06 mg, 388.37 µmol, 0.5 equiv) were dissolved in dioxane (10 mL), methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (70.41 mg, 77.67 µmol, 0.1 equiv) and potassium phosphate (494.63 mg, 2.33 mmol, 3 equiv) were added thereto. The air in the solution was replaced with nitrogen three times, and the reaction mixture was stirred at 80°C for12 hours under nitrogen protection. After the reaction was completed, the reaction mixture was diluted with 20 mL of water, and then extracted with 90 mL of ethyl acetate (30 mL×3). The organic phase was dried over anhydrous sodium sulfate, filtered and concentrated under reduced pressure. The obtained residue was subjected to silica gel thin layer chromatography (dichloromethane: methanol=10:1) to obtain crude product 7, then subjected to high performance liquid chromatography (chromatographic column: Shim-pack C18 150^{∗}25mm^{∗}10um; Mobile Phase: Mobile phase A: formic acid aqueous solution with volume fraction of 0.225 %; mobile phase B: acetonitrile; B%: 42%-62%, 9 minutes) to obtain compound 23-3.

MS-ESI calculated value [M+H]⁺ 785, measured value 785. ¹H NMR (400 MHz, CD₃OD) δ=9.03 (d, 1H, J=1.8 Hz), 8.5-8.7 (m, 1H), 8.28 (d, 1H, J=1.0 Hz), 7.9-8.1 (m, 2H ), 7.3-7.6 (m, 2H), 7.17 (td, 2H, J=1.5, 7.5 Hz), 6.4-6.8 (m, 1H), 4.72 (s, 2H), 4.60 (s, 2H), 4.4- 4.5 (m, 1H), 4.1-4.3 (m, 2H), 3.79 (br d, 2H, J=5.4 Hz), 3.0-3.2 (m, 3H), 2.8-3.0 (m, 4H), 2.44 (s , 3H), 2.27 (br d, 1H, J=14.0 Hz), 2.17 (s, 3H), 1.8-2.0 (m, 1H), 1.51 (s, 10H), 1.22 (s, 5H).

### Step 4

Compound 23-3 (20 mg, 25.21 µmol, 1 equiv) was dissolved in ethyl acetate (1 mL), then a solution of hydrogen chloride in ethyl acetate (4 mol/L, 396.00 µL, 62.82 equiv) was added thereto, the reaction was conducted at 25 °C for 2 hours. After the reaction was completed, the reaction mixture was concentrated under reduced pressure to obtain the crude compound 23-4.

MS-ESI calculated value [M+H]⁺ 685, measured value 685.

### Step 5

Compound 23-4 (18 mg, 24.94 µmol, 1 equiv, hydrochloride) and compound 23-5 (5.54 mg, 37.42 µmol, 4.17 µl, 50% aqueous solution, 1.5 equiv) were dissolved in methanol (1 mL), then sodium cyanoborohydride (3.14 mg, 49.89 µmol, 2 equiv) was added thereto, the reaction was conducted at 15 °C for 2 hours. After the reaction was completed, the reaction mixture was filtered and concentrated under reduced pressure. The obtained residue was subjected to high performance liquid chromatography (chromatographic column: Unisil 3-100 C18 Ultra 150^{∗}50mm^{∗}3um; Mobile Phase: Mobile phase A: formic acid aqueous solution with volume fraction of 0.225 %; mobile phase B: acetonitrile; B%: 20%-40%, 10 minutes) to obtain the formate of compound 23.

MS-ESI calculated value [M+H]⁺ 744, measured value 744. ¹H NMR (400 MHz, CD₃OD) δ=9.01 (d, 1H, J=1.4 Hz), 8.5-8.6 (m, 1H), 8.41 (s, 1H), 8.27 (s, 1H), 8.0-8.1 (m , 2H), 7.4-7.5 (m, 2H), 7.1-7.2 (m, 2H), 6.4-6.8 (m, 1H), 4.4-4.5 (m, 2H), 4.0-4.2 (m, 2H), 3.6 -3.8 (m, 2H), 3.4-3.6 (m, 2H), 3.0-3.2 (m, 4H), 2.7-2.9 (m, 2H), 2.45 (s, 3H), 2.15 (s, 3H), 1.8 -1.9 (m, 1H), 1.3-1.4 (m, 2H).

### Example 24

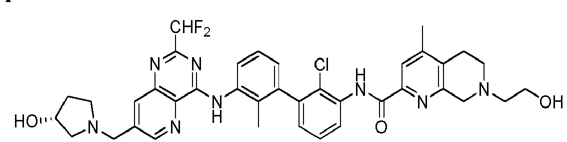

### Synthesis route:

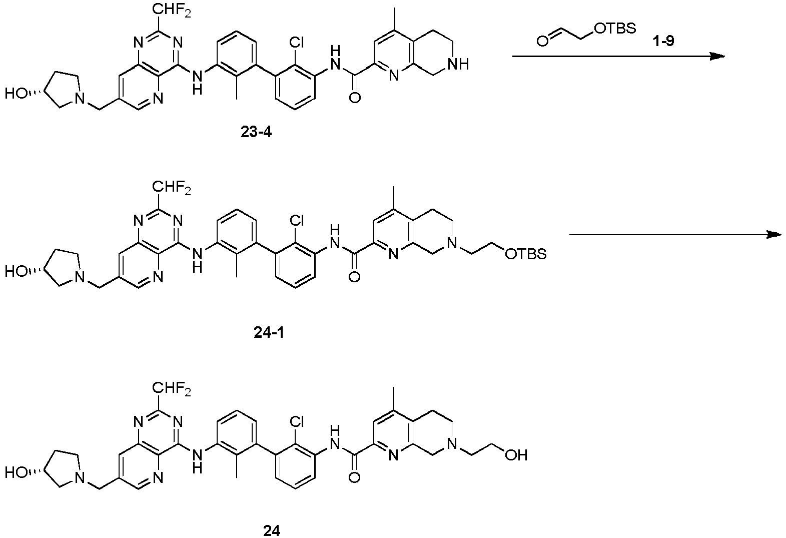

### Step 1

Compound 23-4 (38 mg, 47.83 µmol, 1 equiv, hydrochloride) was dissolved in methanol (3 mL), and diisopropylethylamine (24.72 mg, 191.32 µmol, 4 equiv) and compound 1-9 (50.02 mg, 143.49 µmol, 3 equiv) were added thereto. The mixture was stirred at 15°C for 0.5 hours, sodium cyanoborohydride (3.01 mg, 47.83 µmol, 1 equiv) was added thereto, stirred at 15°C for 3 hours. After the reaction was completed, the reaction mixture was concentrated under reduced pressure to obtain Compound 24-1.

MS-ESI calculated value [M+H]⁺ 844, measured value 844.

### Step 2

Compound 24-1 (15 mg, 17.78 µmol, 1 equiv) was dissolved in 1,4-dioxane (20 mL), and a solution of hydrogen chloride in dioxane (1 mL, 4 mol/L) was added thereto, stirred at 15 °C for 2 hours. After the reaction was completed, the reaction mixture was concentrated, and the crude product was subjected to high performance liquid chromatography (chromatographic column: Shim-pack C18 150^{∗}25 mm^{∗}10 microns; mobile phase A: water (0.225% aqueous formic acid, mobile phase B: acetonitrile; B%: 10%) -40%, 10 min) to obtain the formate of compound 24.

MS-ESI calculated value [M+H]⁺ 729, measured value 729. ¹H NMR (400 MHz, CD₃OD) δ= 9.00 (d, J=1.5 Hz, 1 H), 8.57 (dd, J=8.3, 1.4 Hz, 1 H), 8.40 (br s, 2 H), 8.25 (s , 1 H), 8.02 (d, J=7.9 Hz, 1 H), 7.97 (s, 1 H), 7.47 (t, J=7.9 Hz, 1 H), 7.40 (t, J=7.8 Hz, 1 H) ), 7.09 - 7.21 (m, 2 H), 6.39 - 6.76 (m, 1 H), 4.50 - 4.64 (m, 2 H), 4.37 - 4.49 (m, 1 H), 4.06 - 4.19 (m, 2 H) ), 3.96 - 4.06 (m, 2 H), 3.84 (t, J=5.5 Hz, 2 H), 3.09 - 3.19 (m, 2 H), 2.96 - 3.03 (m, 3 H), 2.93 (t, J =5.6 Hz, 2 H), 2.73 - 2.85 (m, 2 H), 2.41 (s, 3 H), 2.19 - 2.28 (m, 1 H), 2.14 (s, 3 H), 1.78 - 1.92 (m, 1H).

### Example 25

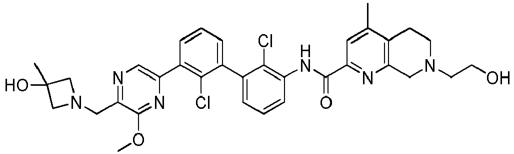

### Synthesis route:

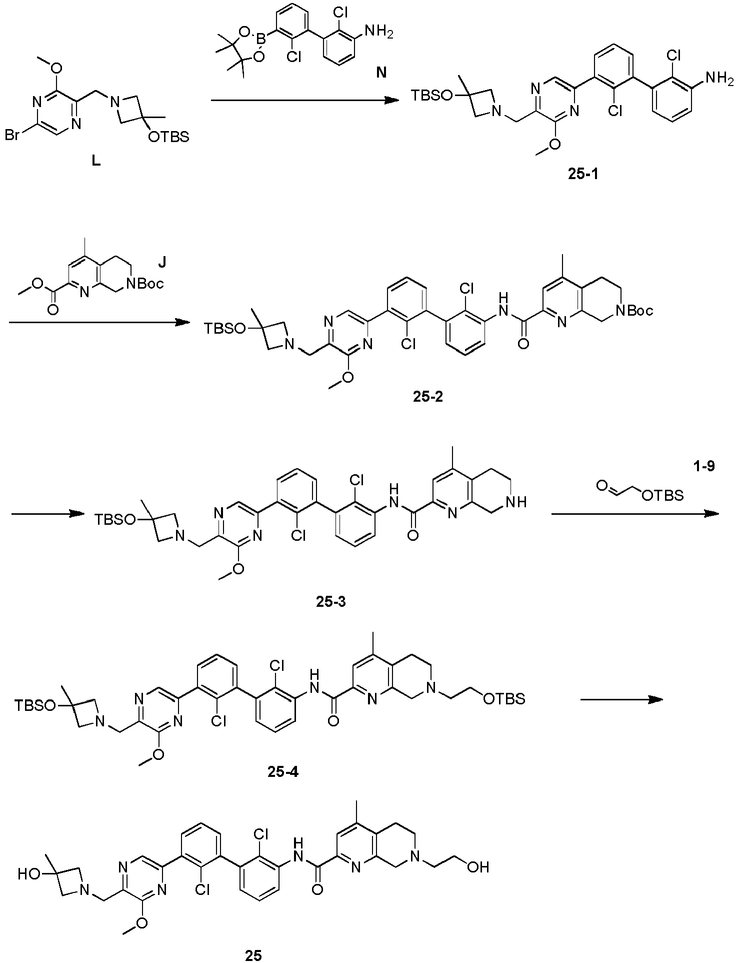

### Step 1

Compound L (100 mg, 248.51 µmol, 1 equiv) and compound N (99.52 mg, 273.36 µmol, 1.1 equiv) were dissolved in dioxane (5 mL) and water (0.5 mL), and then [1,1'-bis(diphenylphosphino)ferrocene]dichloropalladium (18.18 mg, 24.85 µmol, 0.1 equiv) and potassium carbonate (103.04 mg, 745.53 µmol, 3 equiv) were added thereto. The air in solution was replaced with nitrogen three times, and the reaction mixture was stirred at 65°C for 4 hours under nitrogen protection. After the reaction was completed, the reaction mixture was diluted with 20 mL of water and extracted with 120 mL of ethyl acetate (40 mL × 3). The organic phase was washed with 60 mL of saturated brine (30 mL × 2), dried over anhydrous sodium sulfate, filtered and concentrated under reduced pressure. The residue was subjected to silica gel thin-layer chromatography (petroleum ether: ethyl acetate=3:1) to obtain compound 25-1.

MS-ESI calculated value [M+H]⁺ 559, measured value 559. ¹H NMR (400 MHz, CD₃OD) δ= 8.31 (s, 1H), 7.53 (dd, J = 1.7, 7.7 Hz, 1H), 7.40 (t, J = 7.6 Hz, 1H), 7.26 (dd, J = 1.7 , 7.5 Hz, 1H), 7.03 (t, J = 7.8 Hz, 1H), 6.81 (dd, J = 1.5, 8.2 Hz, 1H), 6.52 (dd, J = 1.5, 7.5 Hz, 1H), 4.49 (s , 2H), 3.97 (s, 3H), 3.83 (s, 2H), 3.48 - 3.43 (m, 2H), 1.47 (s, 3H), 1.11 (s, 1H), 0.80 (s, 9H), 0.00 ( s, 6H).

### Step 2

Compound 25-1 (120 mg, 214.44 µmol, 1 equiv) and compound J (68.70 mg, 214.44 µmol, 1 equiv) were dissolved in toluene (2 mL), a solution of lithium bis(trimethylsilyl)amide in tetrahydrofuran (1 mol/L, 578.98 µL, 2.7 equiv) was added thereto at 0 °C, and the reaction mixture was stirred at 25°C for 16 hours. After the reaction was completed, the reaction mixture was quenched with 10 mL of ammonium chloride aqueous solution, and extracted with 30 mL of ethyl acetate (10 mL × 3). The organic phase was washed with 20 mL of saturated brine (10 mL × 2), dried over anhydrous sodium sulfate, filtered and concentrated under reduced pressure. The residue was subjected to silica gel thin layer chromatography (petroleum ether: ethyl acetate=2:1) to obtain compound 25-2.

MS-ESI calculated value [M+H]⁺ 833, measured value 833. ¹H NMR (400 MHz, CDCl₃) δ=10.80 (s, 1H), 8.73 (dd, J = 1.4, 8.3 Hz, 1H), 8.55 (s, 1H), 7.99 (s, 1H), 7.65 (dd, J = 1.7, 7.7 Hz, 1H), 7.53 - 7.47 (m, 1H), 7.43 (t, J = 7.9 Hz, 1H), 7.39 (dd, J = 1.7, 7.6 Hz, 1H), 7.11 (dd, J = 1.4, 7.6 Hz, 1H), 4.70 (br s, 2H), 4.43 - 4.21 (m, 2H), 4.08 (s, 3H), 3.76 (br t, J = 5.6 Hz, 2H), 2.83 (br t, J = 5.6 Hz, 2H), 2.38 (s, 3H), 1.74 (br s, 3H), 1.65 (br d, J = 7.2 Hz, 2H), 1.50 (s, 9H), 1.36 - 1.14 (m, 2H) ), 0.97 - 0.77 (m, 9H), 0.19 - 0.05 (m, 6H).

### Step 3

Compound 25-2 (130 mg, 155.89 µmol, 1 equiv) was dissolved in dichloromethane (2 mL), then trifluoroacetic acid (3.08 g, 27.01 mmol, 2.00 mL, 173.28 equiv) was added thereto, the reaction was conducted at 25 °C for 2 hours. After the reaction was completed, the reaction mixture was concentrated under reduced pressure to obtain the crude compound 25-3. MS-ESI calculated value [M+H]⁺ 733, measured value 733.

### Step 4

Compound 25-3 (120 mg, 141.54 µmol, 1 equiv, trifluoroacetate) was dissolved in methanol (3 mL) N,N-diisopropylethylamine (45.73 mg, 353.85 µmol, 61.63 µl, 2.5 equiv) and compound 1-9 (123.36 mg, 707.69 µmol, 134.82 µl, 5 equiv) were added thereto. After the reaction mixture was stirred at 25°C for 0.5 h, sodium cyanoborohydride (26.68 mg, 424.62 µmol, 3 equiv) was added thereto, the reaction was conducted at 25 °C for 15.5 hours. After the reaction was completed, the reaction mixture was filtered and concentrated under reduced pressure. The residue was subjected to silica gel thin layer chromatography (petroleum ether: ethyl acetate=2:1) to obtain compound 25-4.

MS-ESI calculated value [M+H]⁺ 891, measured value 891.

### Step 5

Compound 25-4 (110 mg, 123.30 µmol, 1 equiv) was dissolved in ethyl acetate (2 mL), a solution of hydrogen chloride in ethyl acetate (4 mol/L, 2 mL, 64.88 equiv) was added thereto, and the reaction was conducted at 25 °C for 1 hour. After the reaction was completed, the reaction mixture was concentrated under reduced pressure, and the obtained residue was subjected to high performance liquid chromatography (chromatographic column: Phenomenex luna C18 150^{∗}25mm^{*}10µm; mobile phase: mobile phase A: formic acid aqueous solution with volume fraction of 0.225 %; mobile phase B: acetonitrile; B%: 10%-40%, 11 minutes) to obtain the formate of compound 25.

MS-ESI calculated value [M+H]⁺ 663, measured value 663. ¹H NMR (400 MHz, CD₃OD) δ= 8.59 (dd, J = 1.5, 8.3 Hz, 1H), 8.47 (s, 2H), 7.94 (s, 1H), 7.70 (dd, J = 1.7, 7.7 Hz, 1H ), 7.56 (t, J = 7.7 Hz, 1H), 7.51 - 7.41 (m, 2H), 7.15 (dd, J = 1.5, 7.6 Hz, 1H), 4.47 (s, 2H), 4.10 (s, 3H) , 4.07 (s, 2H), 3.95 - 3.87 (m, 4H), 3.81 (t, J = 5.8 Hz, 2H), 3.00 (br d, J = 5.3 Hz, 2H), 2.95 (br d, J = 5.5 Hz, 2H), 2.83 (t, J = 5.7 Hz, 2H), 2.39 (s, 3H), 1.55 (s, 3H).

### Example 26

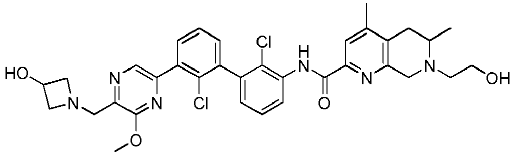

### Synthesis route:

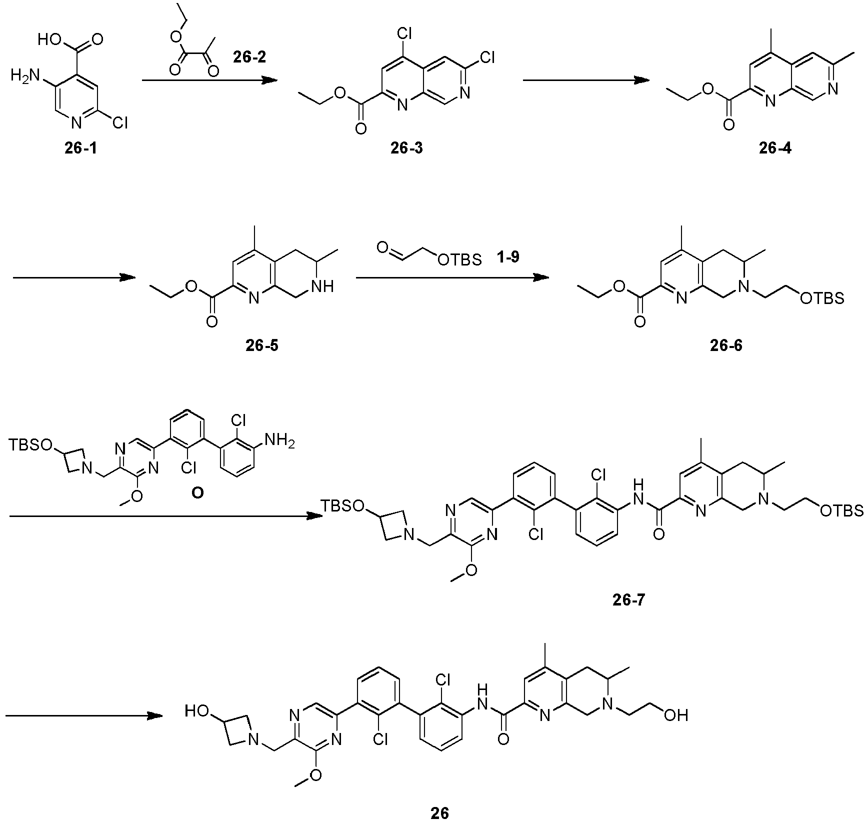

### Step 1

Intermediates 26-1 (10.0 g, 58.0 mmol, 1 equiv), 26-2 (10.1 g, 86.9 mmol, 9.61 mL, 1.5 equiv) were dissolved in toluene (100 mL). The reaction mixture was stirred at 25°C for 15 minutes, then phosphorus oxychloride (26.7 g, 174 mmol, 16.2 mL, 3 equiv) was slowly added to the reaction mixture, and stirred at 100 °C for 1 hour. After the reaction was completed, the reaction mixture was slowly added to water (300 mL) for dilution, then the pH of the reaction mixture was adjusted to 7 with saturated aqueous sodium carbonate solution, and then extracted with ethyl acetate (600 mL). The combined organic phase was diluted with saturated brine (450 mL), dried over anhydrous sodium sulfate, and filtered. The filtrate was concentrated under reduced pressure, and the residue was separated and purified by silica gel column chromatography (petroleum ether: ethyl acetate= 100:1-20:1) to obtain compound 26-3.

MS-ESI calculated value [M+H]⁺ 272, measured value 272. ¹HNMR (400 MHz, CDCl₃) δ =9.54 (d, J=0.74 Hz, 1 H), 8.45 (s, 1 H), 8.19 - 8.10 (m, 1 H), 4.58 (q, J=7.10 Hz, 2 H), 1.51 (t, J=7.16 Hz, 3 H).

### Step 2

Intermediate 26-3 (1.10 g, 4.06 mmol, 1 equiv) and methylboronic acid (850 mg, 14.2 mmol, 3.5 equiv) were dissolved in dioxane (20 mL) and water (0.05 mL), and then potassium phosphate (2.58 g, 12.2 mmol, 3 equiv) and [1,1'-bis(diphenylphosphino)ferrocene]dichloropalladium (331 mg, 406 µmol, 0.1 equivalent) were added to the reaction mixture. The air in the solution was replaced with nitrogen three times, and the reaction mixture was stirred at 90°C for 12 hours under nitrogen protection. After the reaction was completed, the reaction mixture was filtered, and the filtrate was concentrated under reduced pressure, and the obtained residue was separated and purified by silica gel column chromatography (petroleum ether: ethyl acetate=10:1-2:1) to obtain compound 26-4.

MS-ESI calculated value [M+H]⁺ 231, measured value 231.

### Step 3

Intermediate 26-4 (240 mg, 1.04 mmol, 1 equiv) was dissolved in acetic acid (3 mL), then sodium cyanoborohydride (197 mg, 3.13 mmol, 3 equiv) was added to the reaction mixture, stirred at 25 °C for 2 hours. After the reaction was completed, the reaction mixture was concentrated under reduced pressure to remove the solvent, and the residue was diluted with water (10 mL), then the pH of the reaction mixture was adjusted to 6-7 with saturated aqueous sodium bicarbonate solution, and then extracted with dichloromethane (45 mL). The combined organic phase was washed with saturated brine (20 mL), dried over anhydrous sodium sulfate, and filtered. The filtrate was concentrated under reduced pressure to obtain compound 26-5.

MS-ESI calculated value [M+H]⁺ 235, measured value 235.

### Step 4

Intermediates 26-5 (240 mg, 1.02 mmol, 1 equiv) and 1-9 (536 mg, 3.07 mmol, 585 µl, 3 equiv) were dissolved in dichloromethane (4 mL), stirred at 25°C for 0.5 hours. Sodium triacetoxyborohydride (651 mg, 3.07 mmol, 3 equiv) was added to the reaction mixture, stirred at 25°C for 1.5 hours. After the reaction was completed, water (10 mL) was added, and the reaction mixture was extracted with dichloromethane (15 mL). The organic phase was washed with saturated brine (20 mL), dried over anhydrous sodium sulfate, filtered and concentrated. The residue was subjected to silica gel thin layer chromatography (petroleum ether: ethyl acetate=1:1) to obtain compound 26-6.

MS-ESI calculated value [M+H]⁺ 393, measured value 393. ¹HNMR (400 MHz, CDCl₃) δ = 7.85 - 7.64 (m, 1 H), 4.46 (q, J=7.10Hz, 2 H), 3.90- 4.07 (m, 1 H), 4.08 - 3.89 (m, 1 H) ), 3.88 - 3.74 (m, 2 H), 3.20 - 3.12 (m, 1 H), 2.92 - 2.69 (m, 3 H), 2.53 (dd, J=17.18, 6.42 Hz, 1 H), 2.28 (s , 3 H), 1.67 (br s, 2 H), 1.43 (t, J=7.16 Hz, 3 H), 1.18 (d, J=6.48 Hz, 3 H), 0.90 (s, 9 H), 0.07 ( s, 6H).

### Step 5

Intermediate 26-6 (120 mg, 306 µmol, 1 equiv), intermediate O (167 mg, 306 µmol, 1 equiv) were dissolved in toluene (3 mL), then lithium bis(trimethylsilyl)amide (1 mol per liter, 917 µl, 3 equiv) was added to the reaction at 0°C, stirred at 25° C for 2 hours. After the reaction was completed, saturated aqueous ammonium chloride solution (5 mL) was added to the reaction mixture, then the reaction mixture was extracted with ethyl acetate (15 mL). The organic phase was washed with saturated brine (20 mL), dried over anhydrous sodium sulfate, filtered and concentrated. The residue was subjected to silica gel thin layer chromatography (petroleum ether: ethyl acetate=2:1) to obtain compound 26-7.

MS-ESI calculated value [M+H]⁺ 893, measured value 893.

### Step 6

Intermediate 26-7 (150 mg, 157 µmol, 1 equiv) was dissolved in dichloromethane (2 mL), then a solution of hydrogen chloride in ethyl acetate (4 mol/L, 1 mL, 25.5 equiv) was added to the reaction mixture, stirred at 25°C for 0.5 hours. After the reaction was completed, the reaction mixture was concentrated, and the crude product was subjected to high performance liquid chromatography (chromatographic column: Phenomenex Synergi C18 150^{∗}25 mm^{∗}10 microns; mobile phase: mobile phase A: formic acid aqueous solution with volume fraction of 0.225 %; mobile phase B: acetonitrile; B%: 8%-38%, 10 minutes) to obtain the formate of compound 26.

MS-ESI calculated value [M+H]⁺ 664, measured value 664. ¹HNMR (400 MHz, CD₃OD) δ = 8.60 (dd, J=8.30, 1.48 Hz, 1 H), 8.50 (s, 1 H), 8.44 (s, 1 H), 8.00 (s, 1 H), 7.73 ( dd, J=7.76, 1.66 Hz, 1 H), 7.59 (t, J=7.64 Hz, 1 H), 7.54 - 7.44 (m, 2 H), 7.20 - 7.13 (m, 1 H), 4.71 (q, J=6.14 Hz, 1 H), 4.60 (s, 2 H), 4.51 - 4.37 (m, 2 H), 4.32 - 4.16 (m, 2 H), 4.13 (s, 3 H), 4.00 (br dd, J=11.38, 5.62 Hz, 2 H), 3.84 (t, J=5.76 Hz, 2 H), 3.54 - 3.43 (m, 1 H), 3.33 (dt, J=3.28, 1.60 Hz, 6 H), 3.11 (dd, J=17.74, 5.00 Hz, 1 H), 3.04- 2.90 (m, 2 H), 2.78 (dd, J=17.74, 6.86 Hz, 1 H), 2.43 (s, 3 H), 1.31 (d , J=6.60 Hz, 3 H).

### Example 27

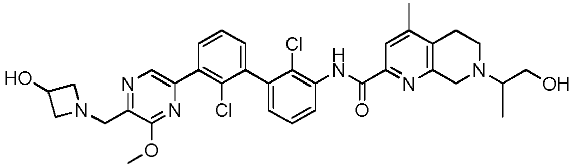

### Synthesis route:

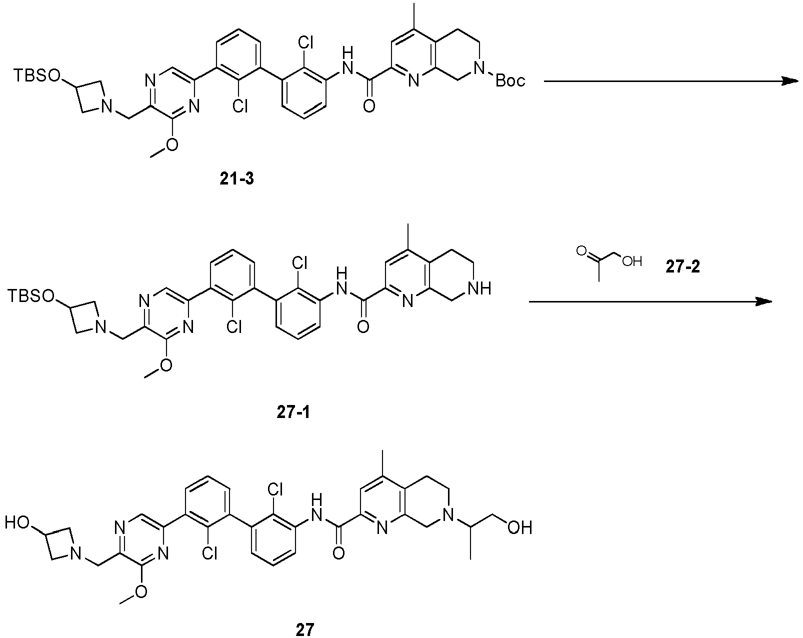

### Step 1

Compound 21-3 (0.500 g, 610 mmol) was dissolved in dichloromethane (3.5 mL), trifluoroacetic acid (0.65 mL) was added to the reaction mixture, stirred at 15 °C for 0.5 hours. The reaction mixture was slowly poured into saturated sodium bicarbonate solution (30 mL), the aqueous phase was extracted with dichloromethane (10 mL × 3). The organic phases were combined. The combined organic phase was dried over anhydrous sodium sulfate, filtered, and concentrated under reduced pressure to obtain the crude product compound 27-1.

MS-ESI calculated value [M+H]⁺ 719 and 721, measured value 719 and 721.

### Step 2

Compound 27-1 (trifluoroacetate, 100 mg, 119 mmol) was dissolved in dichloromethane (3 mL), diisopropylethylamine (31.0 mg, 240 mmol, 41.8 µL) was added thereto. After the reaction mixture was stirred for 0.5 hours, hydroxypropanone (17.8 mg, 240 µmol, 16.5 µL) was added thereto. After the reaction mixture was stirred for 1 hour, sodium triacetoxyborohydride (76.3 mg, 360 µmol) was added dropwise to the reaction system, the reaction was conducted at 15 °C for 0.5 hours. The reaction mixture was slowly poured into water (20 mL), and the aqueous phase was extracted with dichloromethane (10 mL×3). The organic phases were combined. The combined organic phase was dried over anhydrous sodium sulfate, filtered, and concentrated under reduced pressure. The crude product was subjected to high performance liquid chromatography (chromatographic column: Phenomenex Gemini-NX C18 75^{∗}30 mm^{∗}3 microns; mobile phase A: 10 mmol/L ammonium bicarbonate aqueous solution, mobile phase B: acetonitrile; B%: 33%-53 %, 6 minutes) to obtain compound 27.

MS-ESI calculated value [M+H]⁺ 663 and 665, measured value 663 and 665. ¹HNMR (400 MHz, DMSO-*d*₆) δ=10.69 (s, 1H), 8.48 (dd, J=8.0, 1.6 Hz, 1H), 8.38 (s, 1H), 7.84 (s, 1H), 7.71-7.69 (m, 1H), 7.56-7.54 (m, 1H), 7.51-7.44 (m, 2H), 7.19-7.17 (m, 1H), 5.24 (d, J=6.8 Hz, 1H), 4.37-4.34 (m, 1H), 4.15-4.10 (m, 1H), 3.92 (s, 3H), 3.79 (s, 1H), 3.68 (s, 2H), 3.54-3.50 (m, 3H), 3.38-3.34 (m, 1H) , 3.25 (s, 1H), 2.91-2.88 (m, 2H), 2.81-2.74 (m, 4H) 2.64-2.61 (m, 1H), 2.30-2.28 (m, 3H).

### Example 28

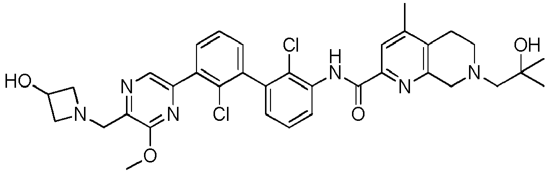

### Synthesis route:

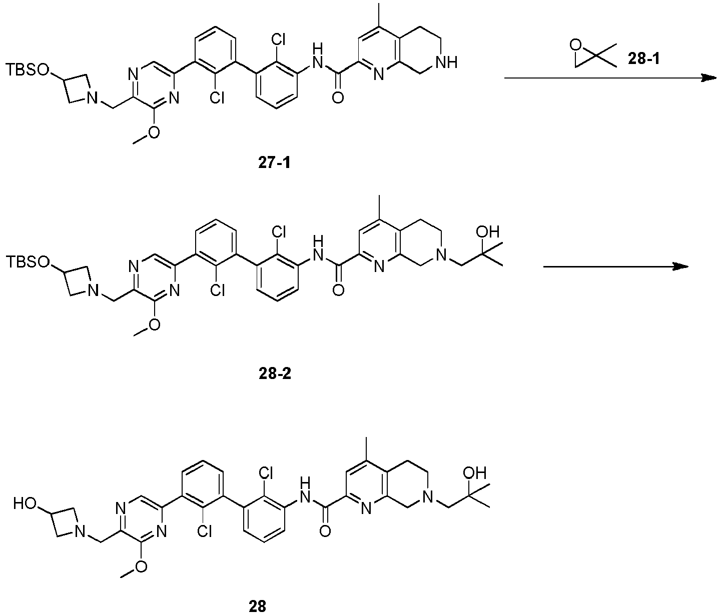

### Step 1

Compound 27-1 (50 mg, 69.47 mmol, 1 equiv) and lithium perchlorate (14.78 mg, 138.93 mmol, 2 equiv) were dissolved in ethanol (4 mL), and compound 28-1 (10.02 mg, 138.93 mmol, 2 equiv) was added thereto, stirred at 80 °C for 6 hours. After the reaction was completed, the reaction mixture was directly concentrated to obtain the crude product 28-2 without further purification.

MS-ESI calculated value [M+H]⁺ 791, measured value 791.

### Step 2

Compound 28-2 (50 mg, 63.14 umol, 1 equiv) was dissolved in a solution of hydrogen chloride in ethyl acetate (1 mol per liter, 1 mL, 15.84 equiv), stirred at 15°C for 1 hour. After the reaction was completed, the reaction mixture was directly concentrated. The crude product was subjected to high performance liquid chromatography (chromatographic column: Waters Xbridge BEH C18 100^{∗}25 mm^{∗}5 microns; mobile phase A: 10 mmol/L ammonium bicarbonate aqueous solution, mobile phase B: acetonitrile; B%: 50%-80%, 10 min) to obtain compound 28.

MS-ESI calculated value [M+H]⁺ 677, measured value 677. ¹HNMR (400 MHz, DMSO-d6) δ=10.72 (s, 1H), 8.54 (d, J=8.4 Hz, 1H), 8.42 (s, 1H), 7.89 (s, 1H), 7.73 (d, J= 7.2 Hz, 1H), 7.60-7.58 (m, 1H), 7.53-7.50 (m, 2H), 7.23-7.21 (m, 1H), 5.29-5.27 (m, 1H), 4.21-4.17 (m, 2H) , 3.83 (s, 3H), 3.71 (s, 2H), 3.58-3.56 (m, 2H), 3.35-3.32 (m, 2H), 2.95-2.91 (m, 3H), 2.81-2.80 (m, 2H) , 2.43 (s, 3H), 2.35 (s, 3H), 1.12 (s, 6H).

### Example 29

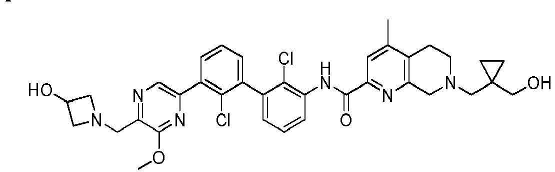

### Synthesis route:

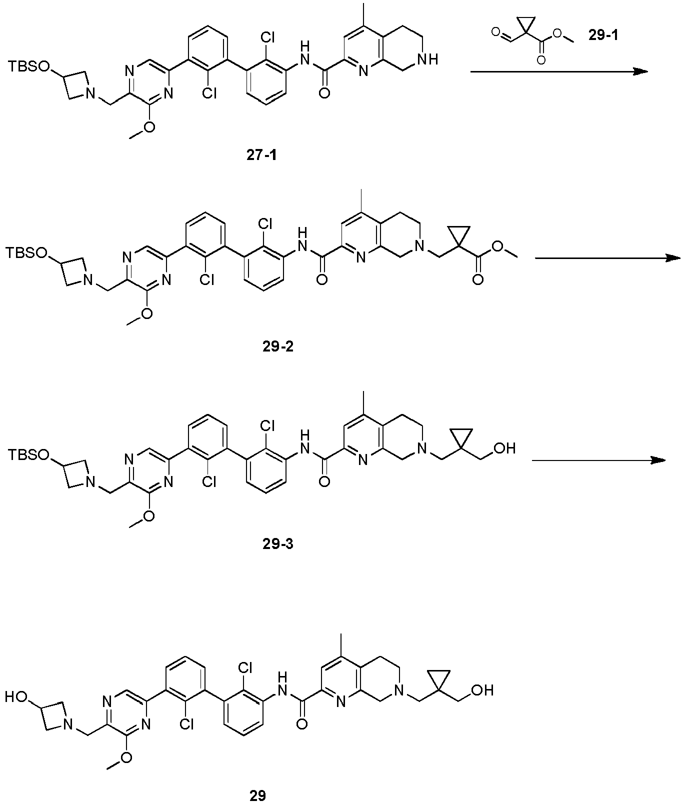

### Step 1

Compound 27-1 (200 mg, 278 mmol) was dissolved in dichloromethane (5 mL), and then compound 29-1 (71.2 mg, 556 mmol) and acetic acid (16.69 mg, 278 mmol, 15.9 µl) were added thereto. After the reaction mixture was stirred for 1 hour, sodium triacetoxyborohydride (177 mg, 834 mmol) was added dropwise to the reaction system, stirred at 15 °C for 3 hours. The reaction mixture was slowly poured into half-saturated sodium bicarbonate solution (20 mL), the aqueous phase was extracted with dichloromethane (10 mL×3). The organic phases were combined, and the combined organic phase was dried over anhydrous sodium sulfate, filtered, and concentrated under reduced pressure to obtain Crude compound 29-2. MS-ESI calculated value [M+H]⁺ 831 and 833, measured value 831 and 833.

### Step 2

Compound 29-2 (150 mg, 180 mmol) was dissolved in tetrahydrofuran (5 mL), lithium aluminum hydride (6.84 mg, 180 mmol) was added at 0 °C, stirred at 15 °C for 1 hour. Saturated potassium sodium tartrate solution (100 µL) was slowly added to the reaction mixture, dried over anhydrous sodium sulfate, filtered, and concentrated under reduced pressure to obtain the crude compound 29-3.

### Step 3

Compound 29-3 (150 mg, 186 mmol) was dissolved in a solution of hydrogen chloride in ethyl acetate (1 mol per liter, 5 mL), stirred at 15 °C for 0.1 hour. The reaction mixture was directly concentrated under reduced pressure and subjected to high performance liquid chromatography (chromatographic column: Waters Xbridge Prep OBD C18 150^{∗}40 mm^{∗}10 microns; mobile phase A: 10 mmol/L ammonium bicarbonate aqueous solution, mobile phase B: acetonitrile; B%: 45%-65%, 8 min) to obtain compound 29.

MS-ESI calculated value [M+H]⁺ 689 and 691, measured value 689 and 691. ¹H NMR (400 MHz, DMSO-*d*₆) δ=10.74 (s, 1H), 8.51 (t, J=8.0 Hz, 1H), 8.41 (s, 1H), 7.89 (s, 1H), 7.76-7.71 (m , 1H), 7.62-7.58 (m, 1H), 7.56-7.48 (m, 2H), 7.24-7.20 (m, 1H), 5.28 (d, J=6.4 Hz, 1H), 4.43 (s, 1H), 4.19-4.14 (m, 1H), 3.99-3.85 (m, 4H), 3.72-3.64 (m, 4H), 3.58-3.49 (m, 2H), 2.95-2.91 (m, 2H), 2.81 (s, 4H), 2.72-2.63 (m, 1H), 2.35 (s, 3H), 2.33-2.28 (m, 1H).

### Example 30

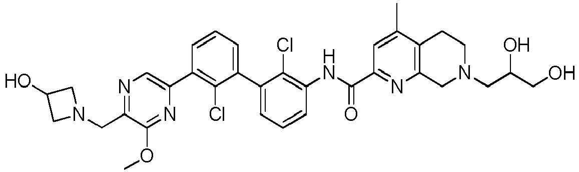

### Synthesis route:

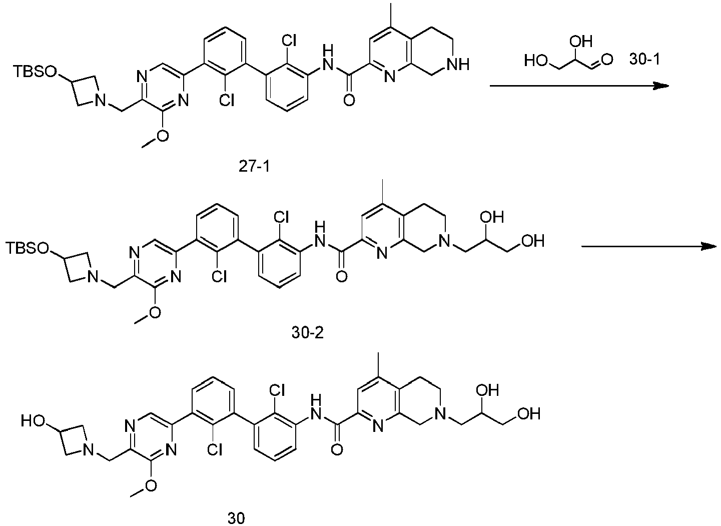

### Step 1

Compound 27-1 (350 mg, 419.77 µmol, trifluoroacetate) and compound 30-1 (75.62 mg, 839.53 µmol) were dissolved in methanol (5 mL), then sodium cyanoborohydride ( 65.95 mg, 1.05 mmol) was added thereto, stirred at 20 °C for 1 hour. After the reaction was completed, the reaction mixture was filtered and directly concentrated under reduced pressure. Compound 30-2 was obtained.

MS-ESI calculated value [M+H]⁺ 796, measured value 796.

### Step 2

Intermediate 30-2 (380 mg, 478.68 µmol, 1 equiv) was dissolved in ethyl acetate (1.5 mL), a solution of hydrogen chloride in ethyl acetate (4 mol/L, 598.35 µL) was added thereto, stirred at 25 °C for 0.5 hours. After the reaction was completed, the reaction mixture was filtered and concentrated under reduced pressure. The crude product was subjected to high performance liquid chromatography (chromatographic column: Phenomenex Gemini-NX 80^{∗}40 mm^{∗}3 microns; mobile phase A: 0.05% ammonia water by weight + 10 mmol/L ammonium bicarbonate aqueous solution, mobile phase B: acetonitrile; B %: 31%-61%, 8 minutes) to obtain compound 30.

MS-ESI calculated value [M+H]⁺ 679, measured value 679. ¹HNMR (400MHz, CD₃OD) δ=8.63 - 8.56 (m, 1H), 8.41 (s, 1H), 7.89 (s, 1H), 7.68 (dd, J=1.5, 7.8 Hz, 1H), 7.54 (t, J =7.7 Hz, 1H), 7.49 - 7.38 (m, 2H), 7.13 (dd, J=1.5, 7.5 Hz, 1H), 4.46 (quin, J=6.3 Hz, 1H), 4.06 (s, 3H), 4.03 (s, 2H), 3.96 - 3.91 (m, 3H), 3.88 - 3.79 (m, 2H), 3.60 - 3.49 (m, 2H), 3.36 - 3.32 (m, 2H), 2.98 - 2.85 (m, 4H) , 2.75 - 2.59 (m, 2H), 2.36 (s, 3H).

### Example 31

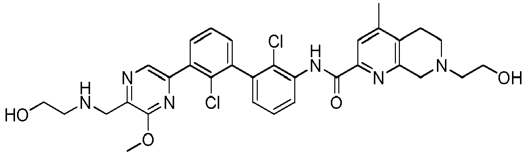

### Synthesis route:

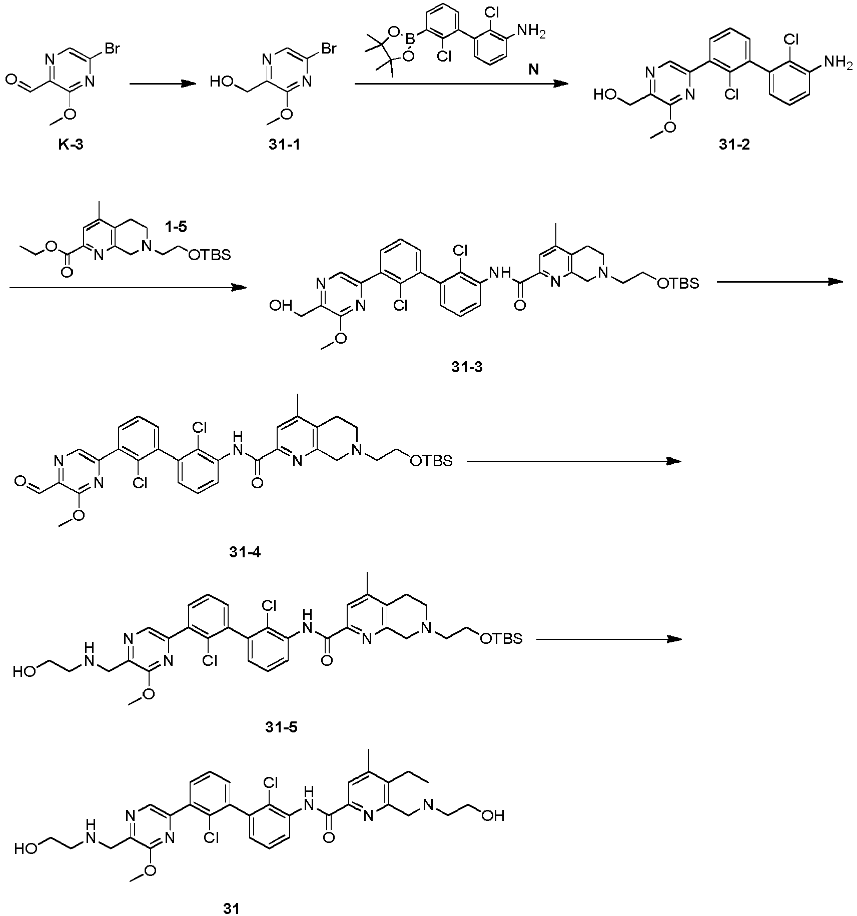

### Step 1

Compound K-3 (1.5 g, 6.91 mmol, 1 equiv) was dissolved in methanol (15 mL), then sodium borohydride (784.47 mg, 20.74 mmol, 3 equiv) was added at 0°C, and stirred at 25°C for 2 hours. After the reaction was completed, the reaction was quenched with 20 mL of saturated aqueous ammonium chloride solution. After most of methanol was concentrated under reduced pressure, the reaction mixture was diluted with water (20 mL), then extracted with ethyl acetate (90 mL). The organic phase was washed with saturated brine (60 mL), dried over anhydrous sodium sulfate, filtered and concentrated under reduced pressure. The residue was subjected to silica gel column chromatography (petroleum ether: ethyl acetate=30:1 to 10:1) to obtain compound 31-1. ¹H NMR (400 MHz, DMSO-d₆) δ = 8.34 (s, 1H), 5.24 (t, J= 6.0 Hz, 1H), 4.52 (d, *J* = 6.0 Hz, 2H), 3.94 (s, 3H)

### Step 2

Compound 31-1 (1.4 g, 6.39 mmol, 1 equiv) and N (2.33 g, 6.39 mmol, 1 equiv) were dissolved in dioxane (15 mL) and water (2 mL), then [1,1'-bis(diphenylphosphino)ferrocene]dichloropalladium (467.68 mg, 639.17 µmol, 0.1 equiv) and potassium carbonate (2.65 g, 19.17 mmol, 3 equiv) were added thereto. The air in the reaction mixture was replaced with nitrogen three times, and the reaction mixture was stirred at 65°C for 4 hours under nitrogen protection. After the reaction was completed, the reaction mixture was diluted with water (40 mL) and extracted with ethyl acetate (150 mL). The organic phase was washed with saturated brine (60 mL), dried over anhydrous sodium sulfate, filtered and concentrated under reduced pressure. The residue was subjected to silica gel column chromatography (petroleum ether: ethyl acetate=30:1 to 3:1) to obtain compound 31-2.

MS-ESI calculated value [M+H]⁺ 376, measured value 376.

### Step 3

Compounds 31-2 (1 g, 2.66 mmol, 1 equiv) and 1-5 (1.11 g, 2.92 mmol, 1.1 equiv) were dissolved in toluene (20 mL), then a solution of lithium bis(trimethylsilyl)amide in tetrahydrofuran (1 mol/L, 7.97 mL, 3 equiv) was added there to at 0 °C, and the reaction mixture was stirred at 25°C for 2 hours. After the reaction was completed, the reaction mixture was quenched with 20 mL of ammonium chloride aqueous solution, diluted with water (20 mL), and extracted with ethyl acetate (90 mL). The organic phase was washed with saturated brine (40 mL), dried over anhydrous sodium sulfate, filtered and concentrated under reduced pressure. The residue was subjected to silica gel column chromatography (petroleum ether: ethyl acetate=10:1 to 3:1) to obtain compound 31-3.

MS-ESI calculated value [M+H]⁺ 708, measured value 708.

### Step 4

Intermediate 31-3 (100 mg, 142 µmol, 1 equiv) was dissolved in dichloromethane (3 mL), and Dess-Martin Periodinane (90.0 mg, 212 µmol, 1.5 equiv) was slowly added to the reaction, stirred at 25°C for 2 hours. After the reaction was completed, the reaction mixture was slowly added to water (3 mL) to dilute, then diluted with saturated aqueous sodium thiosulfate solution (5 mL), then the pH of the reaction mixture was adjusted to 7-8 with saturated aqueous sodium bicarbonate solution, and then extracted with dichloromethane (15 mL). The combined organic phase was washed with saturated brine (20 mL) and saturated aqueous sodium thiosulfate solution (20 mL). The organic phase was dried over anhydrous sodium sulfate, filtered, and the filtrate was concentrated under reduced pressure to obtain the compound 31-4.

MS-ESI calculated value [M+H]⁺ 706, measured value 706.

### Step 5

Intermediate 31-4 (100 mg, 142 µmol, 1 equiv) and ethanolamine (86.4 mg, 1.42 mmol, 85.6 µl, 10 equiv) was dissolved in dichloromethane (3 mL), then sodium triacetoxyborohydride (90.0 mg, 425 µmol, 3 equiv) was added to the reaction mixture, stirred at 25 °C for 2 hours. After the reaction was completed, water (10 mL) was added to the reaction mixture, the reaction mixture was extracted with dichloromethane (30 mL). The organic phase was washed with saturated brine (40 mL), dried over anhydrous sodium sulfate, filtered and concentrated. The residue was subjected to silica gel thin layer chromatography (petroleum ether: ethyl acetate: ethanol=1:3:1) to obtain compound 31-5.

MS-ESI calculated value [M+H]⁺ 751, measured value 751.

### Step 6

Intermediate 31-5 (20.0 mg, 26.6 µmol, 1 equiv) was dissolved in dichloromethane (2 mL), a solution of hydrogen chloride in ethyl acetate (4 mol/L, 0.25 mL, 37.6 equiv) was added to the reaction mixture, stirred for 0.25 h at 25 °C. After the reaction was completed, the reaction mixture was concentrated, and the crude product was subjected to high performance liquid chromatography (chromatographic column: Phenomenex Synergi C18 150^{∗}25 mm^{∗}10 microns; mobile phase: mobile phase A: formic acid aqueous solution with volume fraction of 0.225 %; mobile phase B: acetonitrile; B%: 6%-36%, minutes) to obtain compound 31.

MS-ESI calculated value [M+H]⁺ 637, measured value 637. ¹HNMR (400 MHz, CD₃OD) δ = 8.61 (d, J=8.08 Hz, 1 H), 8.52 - 8.44 (m, 1 H), 7.95 (s, 1 H), 7.73 (d, J=7.46 Hz, 1 H), 7.63 - 7.55 (m, 1 H), 7.49 (q, J=7.38 Hz, 2 H), 7.24 - 7.10 (m, 1 H), 4.70 - 4.52 (m, 3 H), 4.42 - 4.26 ( m, 2 H), 4.20 - 4.03 (m, 3 H), 3.95- 3.70 (m, 6 H), 3.15 (br s, 2 H), 2.96 (br dd, J=8.56, 3.91 Hz, 4 H) , 2.80 (t, J=5.81 Hz, 2 H), 2.48 - 2.37 (m, 3 H).

### Example 32

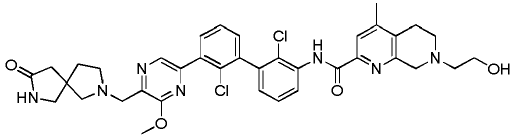

### Synthesis route:

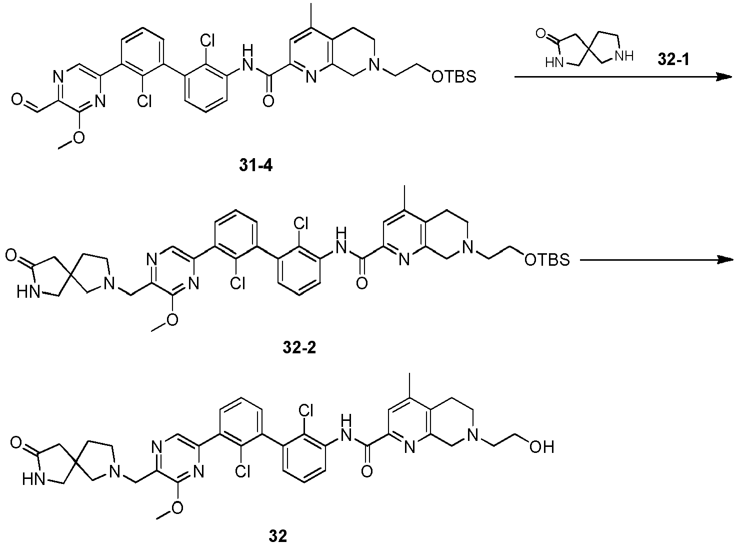

### Step 1

Compound 32-1 (49.99 mg, 282.99 µmol) was dissolved in dichloromethane (2 mL), and then N,N-diisopropylethylamine (36.57 mg, 282.99 µmol) and intermediate 31-4 (100 mg, 141.50 µmol) were added thereto. After the reaction mixture was stirred for half an hour, sodium cyanoborohydride (89.97 mg, 424.49 µmol) was added, the reaction was conducted at room temperature for 2 hours. After the reaction was completed, the reaction mixture was diluted with dichloromethane (20 mL), washed with saturated brine (20 mL). The organic phase was dried over anhydrous sodium sulfate, filtered and concentrated. The concentrated residue was subjected to silica gel thin layer chromatography (dichloromethane: methanol=10:1) to obtain intermediate 32-2.

MS-ESI calculated value [M+H]⁺ 830, measured value 830.

### Step 2

Intermediate 32-2 (65 mg, 78.23 µmol) was dissolved in ethyl acetate (4 mL), a solution of hydrogen chloride in ethyl acetate (4 mol/L, 2 mL) was added thereto, stirred at room temperature for 0.5 hours. After the reaction was completed, the reaction mixture was concentrated. The crude product was subjected to high performance liquid chromatography (column: Unisil 3-100 C18 Ultra 150^{∗}50 mm^{∗}3 microns; mobile phase: mobile phase A: formic acid aqueous solution with volume fraction of 0.225 %; mobile phase B: acetonitrile; B%: 15%- 35%, 10 minutes) to obtain the formate of compound 32.

MS-ESI calculated value [M+H]⁺ 716, measured value 716. ¹H NMR (400 MHz, CD₃OD) δ = 8.63 - 8.57 (m, 1 H), 8.52 (s, 1 H), 8.41 - 8.35 (m, 1 H), 7.99 (s, 1 H), 7.76 - 7.71 (m, 1 H), 7.59 (t, J = 7.6 Hz, 1 H), 7.52 - 7.44 (m, 2 H), 7.20 - 7.15 (m, 1 H), 4.42 (s, 2 H), 4.14 - 4.08 (m, 5 H), 3.88 (t, J = 5.6 Hz, 2 H), 3.53 - 3.48 (m, 1 H), 3.45 - 3.34 (m, 5 H), 3.24 - 3.17 (m, 2 H), 3.04 - 2.97 (m, 4 H), 2.60 - 2.52 (m, 1 H), 2.49 - 2.41 (m, 4 H), 2.22 - 2.12 (m, 2 H).

### Example 33

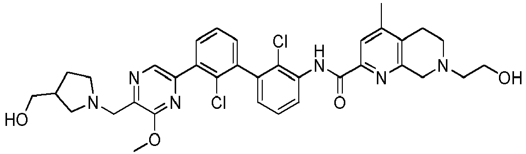

### Synthesis route:

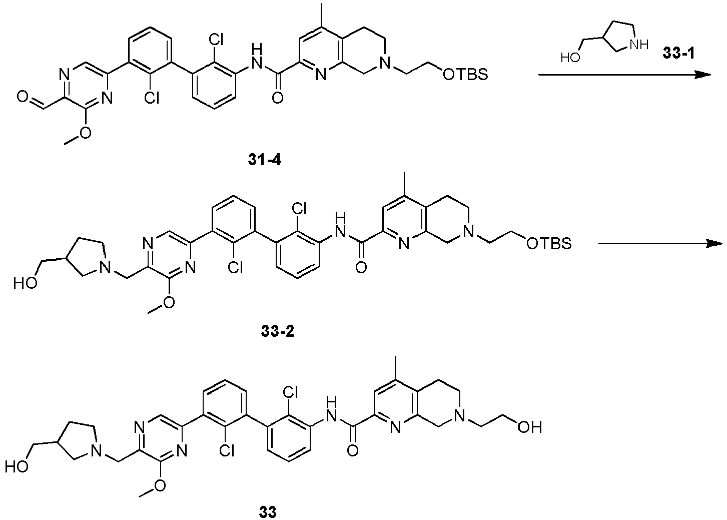

### Step 1

Compound 32-1 (49.99 mg, 282.99 µmol) was replaced with compound 33-1 (58.41 mg, 424.50 µmol), and the method was the same as that in example 32. After the reaction was completed, the reaction mixture was diluted with dichloromethane (20 mL), and washed with saturated brine (20 mL). The organic phase was dried over anhydrous sodium sulfate, filtered and concentrated. The concentrated residue was subjected to silica gel thin layer chromatography (dichloromethane: methanol=8:1) to obtain intermediate 33-2.

MS-ESI calculated value [M+H]⁺ 791, measured value 791.

### Step 2

Intermediate 33-2 (71 mg, 89.66 µmol) was dissolved in dichloromethane (4 mL), a solution of hydrogen chloride in dioxane (4 mol/L, 2 mL) was added, stirred at room temperature for 1 hour. After the reaction was completed, the reaction mixture was concentrated. The crude product was subjected to high performance liquid chromatography (chromatographic column: Phenomenex luna C18 150^{∗}25 mm^{∗}10 microns; mobile phase: mobile phase A: formic acid aqueous solution with volume fraction of 0.225 %; mobile phase B: acetonitrile; B%: 8%-38%, 10 minutes) to obtain compound 33.

MS-ESI calculated value [M+H]⁺ 677, measured value 677. ¹H NMR (400 MHz, CD₃OD) δ = 8.64 - 8.59 (m, 1 H), 8.54 (s, 1 H), 7.94 (s, 1 H), 7.77 - 7.71 (m, 1 H), 7.59 (t, J = 7.6 Hz, 1 H), 7.52 - 7.46 (m, 2 H), 7.21 - 7.14 (m, 1 H), 4.48 (s, 2 H), 4.16 - 4.10 (s, 3 H), 3.90 (s , 2 H), 3.83 (t, J = 6.0 Hz, 2 H), 3.68 - 3.57 (m, 2 H), 3.55 - 3.47 (m, 1 H), 3.44 - 3.36 (m, 2 H), 3.25 - 3.15 (m, 1 H), 3.02 - 2.92 (m, 4 H), 2.82 (t, J = 5.6 Hz, 2 H), 2.71 - 2.60 (m, 1 H), 2.43 - 2.39 (m, 3 H) , 2.29 - 2.16 (m, 1 H), 1.95 - 1.83 (m, 1 H).

### Example 34

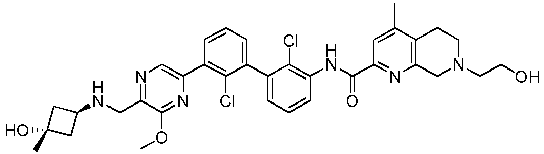

### Synthesis route:

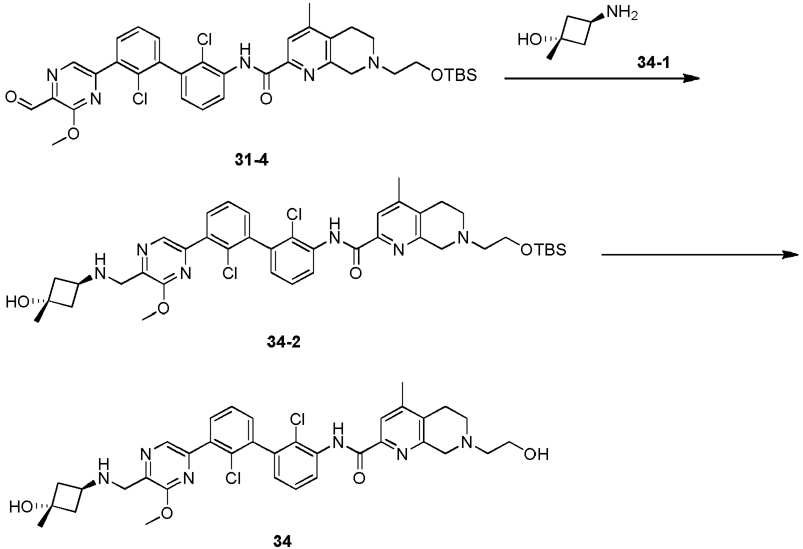

### Step 1

Intermediate 31-4 (80 mg, 113.20 µmol, 1 equiv) was dissolved in dichloromethane (2 mL), then compound 34-1 (46.73 mg, 339.59 µmol, 3 equiv, hydrochloride) and acetic acid (20.39 mg, 339.59 µmοl, 19.42 µl, 3 equiv) was added thereto, stirred at 25°C for 0.5 hours. Sodium triacetoxyborohydride (71.97 mg, 339.59 µmol, 3 equiv) was then added thereto, and stirred at 25°C for 15.5 hours. After the reaction was completed, the reaction mixture was diluted with 5 mL of water, and extracted with ethyl acetate (9 mL). The organic phase was washed with brine (10 mL), then dried over anhydrous sodium sulfate, filtered and concentrated under reduced pressure, and the obtained residue was subjected to silica gel thin layer chromatography (petroleum ether: ethyl acetate: ethanol=4:3:1) to obtain compound 34-2.

MS-ESI calculated value [M+H]⁺ 791, measured value 791.

### Step 2

Compound 32-2 (65 mg, 78.23 µmol) was replaced with compound 34-2 (60 mg, 73.50 µmol, 1 equiv), and the method was the same as that in example 32. After the reaction was completed, the reaction mixture was concentrated, and the crude product was subjected to high performance liquid chromatography (chromatographic column: Phenomenex luna C18 150^{∗}25 mm^{∗}10 microns; mobile phase: mobile phase A: formic acid aqueous solution with volume fraction of 0.225 %; mobile phase B: acetonitrile; B%: 13%-43%, 10 minutes) to obtain the formate of compound 34.

MS-ESI calculated value [M+H]⁺ 677, measured value 677. ¹H NMR (400 MHz, CD₃OD) δ = 8.61 (dd, J = 1.5, 8.3 Hz, 1H), 8.53 (s, 1H), 7.97 (s, 1H), 7.73 (dd, J = 1.7, 7.7 Hz , 1H), 7.59 (t, J = 7.6 Hz, 1H), 7.54 - 7.43 (m, 2H), 7.18 (dd, J = 1.5, 7.6 Hz, 1H), 4.29 (s, 2H), 4.14 (s, 3H), 4.00 (br t, J = 8.1 Hz, 1H), 3.93 (s, 2H), 3.83 (t, J = 5.7 Hz, 2H), 3.02 (br d, J = 5.1 Hz, 2H), 2.97 ( br d, J = 5.4 Hz, 2H), 2.84 (t, J = 5.8 Hz, 2H), 2.48 - 2.36 (m, 5H), 2.31 - 2.17 (m, 2H), 1.42 (s, 3H).

### Example 35

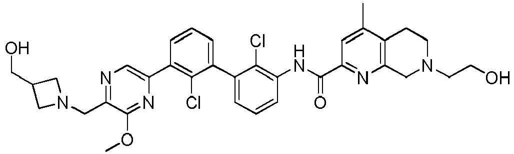

### Synthesis route:

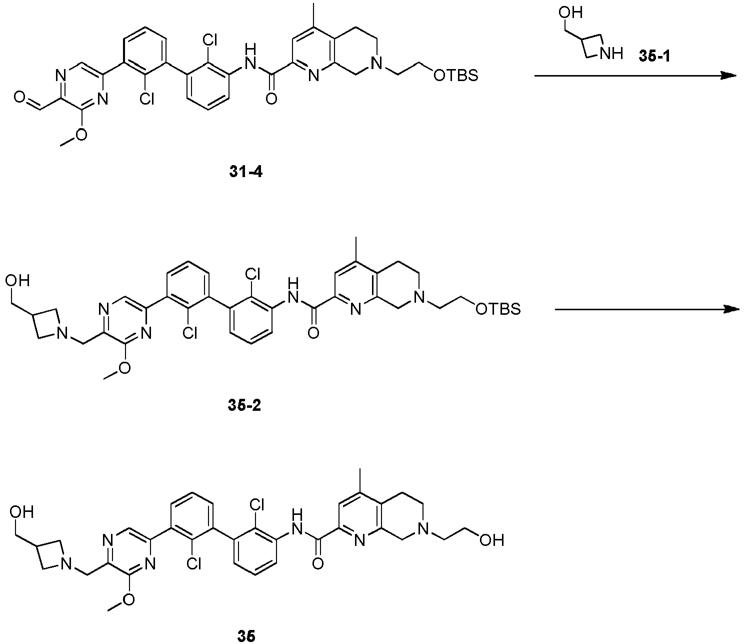

### Step 1

Ethanolamine (86.4 mg, 1.42 mmol, 85.6 µL, 10 equiv) was replaced with compound 35-1 (37.0 mg, 299 µmοl, 2.11 equiv, hydrochloride), and the method was the same as that in example 31. After the reaction was completed, the concentrated residue was separated and purified by silica gel thin layer chromatography (petroleum ether: ethyl acetate: ethanol=1:3:1) to obtain compound 35-2.

MS-ESI calculated value [M+H]⁺ 777, measured value 777.

### Step 2

Compound 31-5 (20.0 mg, 26.6 µmol, 1 equiv) was replaced with compound 35-2 (15 mg, 19.3 µmol, 1 equiv), and the method was the same as that in example 31. After the reaction was completed, the reaction mixture was concentrated, and the crude product was subjected to high performance liquid chromatography (chromatographic column: Unisil 3-100 C18 Ultra 150^{∗}50 mm^{∗}3 microns; mobile phase: mobile phase A: formic acid aqueous solution with volume fraction of 0.225 %; mobile phase B: acetonitrile; B%: 15%-35%, 10 minutes) to obtain compound 35.

MS-ESI calculated value [M+H]⁺ 663, measured value 663. ¹HNMR (400 MHz, CD₃OD) δ = 8.61 (dd, J=8.26, 1.38 Hz, 1 H), 8.51 (s, 1 H), 8.02 - 7.96 (m, 1 H), 7.73 (dd, J=7.70, 1.56 Hz, 1 H), 7.59 (t, J=7.64 Hz, 1 H), 7.53 - 7.45 (m, 2 H), 7.18 (dd, J=7.58, 1.44 Hz, 1 H), 4.69 (s, 2 H), 4.61 (br d, J=1.38 Hz, 4 H), 4.40 (br t, J=9.76 Hz, 2 H), 4.27 - 4.19 (m, 2 H), 4.14 (s, 3 H), 4.05 (s, 2 H), 3.86 (t, J=5.68 Hz, 2 H), 3.72 (d, J=4.14 Hz, 2 H), 3.14 (q, J=5.88 Hz, 3 H), 3.03 - 2.98 ( m, 2 H), 2.95 (t, J=5.68 Hz, 2 H), 2.43 (s, 3 H).

### Example 36

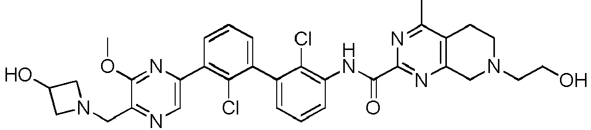

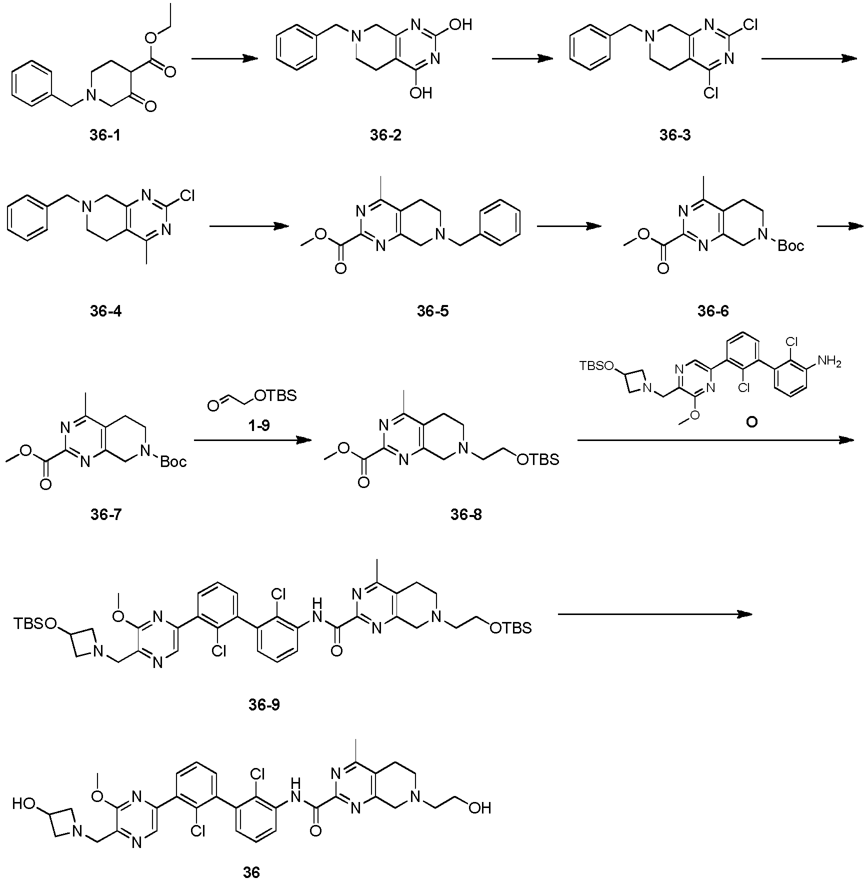

### Step 1

Compound 36-1 (20 g, 76.54 mmol, 1 equiv) was dissolved in ethanol (350 mL), and then sodium methoxide (45.36 g, 17.48 mmol, 10.97 equiv) and urea (20 g, 333.03 mmol, 4.35 equiv) were added to the reaction mixture. The reaction mixture was placed at 75 °C and the reaction was conducted for 12 hours. After the reaction was completed, 4 mol/L of hydrochloric acid was added to adjust the pH to 6 at 0 °C, stirred at room temperature for 1 hour, filtered and concentrated under reduced pressure to obtain compound 36-2.

MS-ESI calculated value [M+H]⁺ 257, measured value 258.

### Step 2

Compound 36-2 (8 g, 31.09 mmol, 1 equiv) was dissolved in phosphorus oxychloride (30 mL), and then N,N-diisopropylethylamine (12.06 g, 93.28 mmol, 3 equiv) was added to the reaction mixture. The reaction mixture was placed at 110 °C and the reaction was conducted for 12 hours. After the reaction was completed, the reaction mixture was distilled under reduced pressure, and the reaction residue was slowly added to stirring water at room temperature to quench, the pH of the reaction mixture was neutralized with 2 mol/L sodium hydroxide to 7-8, and then extracted with 300 mL of dichloromethane. The organic phase was washed with saturated brine (150 mL), dried over anhydrous sodium sulfate, filtered and concentrated under reduced pressure. The residue was subjected to silica gel column chromatography (petroleum ether: ethyl acetate=100:1-10:1) to obtain compound 36-3.

MS-ESI calculated value [M+H]⁺ 293, measured value 294.

### Step 3

Compound 36-3 (1.3 g, 4.42 mmol, 1 equiv) was dissolved in dioxane (10 mL), methylboronic acid (396.79 mg, 6.63 mmol, 1.5 equiv), and then 1,1'-bis(diphenylphosphino)ferrocene-palladium(II)dichloride dichloromethane complex (360.88 mg, 441.91 µmol, 0.1 equiv) and potassium carbonate (1.22 g, 8.84 mmol, 3 equiv) and water (0.1 mL) were added to the reaction mixture. The reaction mixture was placed at 90 °C and the reaction was conducted for 12 hours. After the reaction was completed, the reaction was diluted with water (10 mL), and extracted with 20 mL of ethyl acetate. The organic phase was washed with 10 mL of saturated brine, dried over anhydrous sodium sulfate, filtered and concentrated under reduced pressure, and the obtained residue was subjected to silica gel column chromatography (petroleum ether: ethyl acetate=100:1-10:1) to obtain compound 36-4.

MS-ESI calculated value [M+H]⁺ 273, measured value 274.

### Step 4

Compound 36-4 (750 mg, 2.74 mmol, 1 equiv) was dissolved in methanol (15 mL) and N,N-dimethylformamide (5 mL), and then 1,1'-bis(diphenylphosphino)ferrocene-palladium(II)dichloride dichloromethane complex (200.46 mg, 273.96 µmol, 0.1 equiv) and triethylamine (831.66 mg, 8.22 mmol, 1.14 mL, 3 equiv) were added thereto. After the air in the reaction device was replaced with argon three times, the argon in the device was replaced with carbon monoxide three times, then the reaction was conducted at 80 °C for 16 hours under carbon monoxide (50 psi). After the reaction was completed, the reaction mixture was diluted with 30 mL and 10 mL of water, and extracted with 120 mL of ethyl acetate. The organic phase was washed with 80 mL of saturated brine, dried over anhydrous sodium sulfate, filtered and concentrated under reduced pressure. The residue was subjected to silica gel column chromatography (petroleum ether: ethyl acetate=30:1 to 1:1) to obtain compound 36-5.

MS-ESI calculated value [M+H]⁺ 298, measured value 298. ¹H NMR (400 MHz, CDCl₃) δ = 7.40 - 7.27 (m, 5H), 4.04 (s, 3H), 3.75 (d, *J* = 4.6 Hz, 4H), 2.87 (s, 4H), 2.56 (s, 3H).

### Step 5

Compound 36-5 (300 mg, 1.01 mmol, 1 equiv) was dissolved in ethanol (20 mL), then palladium on carbon (50 mg, 1.01 mmol, 10% purity, 1 equiv) and di-tert-butyl dicarbonate (264.23 mg, 1.21 mmol, 278.14 µl, 1.2 equiv) were added thereto. The reaction mixture was replaced with hydrogen for several times, then the reaction was conducted at 25°C for 4 hours under hydrogen environment (15psi). After the reaction was completed, the reaction mixture was filtered and concentrated under reduced pressure. The residue was subjected to silica gel column chromatography (petroleum ether: ethyl acetate=10:1 to 1:1) to obtain compound 36-6.

MS-ESI calculated value [M+H]⁺ 308, measured value 308.

### Step 6

Compound 36-6 (300 mg, 976.10 µmol, 1 equiv) was dissolved in ethyl acetate (3 mL), then a solution of hydrogen chloride in ethyl acetate (4 mol/L, 1.5 mL, 6.15 equiv) was added thereto, and the reaction was conducted at 25 °C for 1 hour. After the reaction was completed, the reaction mixture was concentrated under reduced pressure to obtain the crude compound 36-7.

MS-ESI calculated value [M+H]⁺ 208, measured value 208.

### Step 7

Compound 36-7 (200 mg, 965.12 µmol, 1 equiv) was dissolved in dichloromethane (5 mL), and then N,N-diisopropylethylamine (249.47 mg, 1.93 mmol, 336.21 µl), 2 equiv) and compound 1-9 (504.70 mg, 2.90 mmol, 551.58 µl, 3 equiv) were added thereto. After the reaction mixture was stirred at 25°C for 0.5 hours, sodium triacetoxyborohydride (613.64 mg, 2.90 mmol, 3 equiv) was added thereto, and the reaction was conducted at 25 °C for 1.5 hours. After the reaction was completed, the reaction mixture was quenched with 10 mL of saturated sodium carbonate at 25°C, diluted with 20 mL of water, and extracted with 60 mL of dichloromethane. The organic phase was washed with 40 mL of saturated brine, then dried with anhydrous sodium sulfate, filtered and concentrated. The residue was subjected to silica gel column chromatography (petroleum ether: ethyl acetate=20:1 to 2:1) to obtain compound 36-8.

MS-ESI calculated value [M+H]⁺ 366, measured value 366

### Step 8

Compound 36-8 (120 mg, 328.28 µmol, 1 equiv) and O (179.10 mg, 328.28 µmοl, 1 equiv) were dissolved in toluene (5 mL), a solution of lithium bis(trimethylsilyl)amide in tetrahydrofuran (1 mol/L, 820.70 µL, 2.5 equiv) was added thereto at 0°C, the reaction mixture was stirred at 25°C for 1 hour. After the reaction was completed, the reaction mixture was quenched with 10 mL of ammonium chloride aqueous solution, and extracted with 60 mL of ethyl acetate. The organic phase was washed with 40 mL of saturated brine, dried over anhydrous sodium sulfate, filtered and concentrated under reduced pressure. The residue was subjected to silica gel thin layer chromatography (petroleum ether: ethyl acetate=1:1) to obtain compound 36-9.

MS-ESI calculated value [M+H]⁺ 880, measured value 880.

### Step 9

Compound 36-9 (160 mg, 182.01 µmol, 1 equiv) was dissolved in ethyl acetate (1 mL), a solution of hydrogen chloride in ethyl acetate (4 mol/L, 1 mL, 21.98 equiv) was added thereto, the reaction was conducted at 25 °C for 1 hour. After the reaction was completed, the reaction mixture was filtered and concentrated under reduced pressure. The obtained residue was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 7%-37%, 10 minutes) to obtain the formate of compound 36.

MS-ESI calculated value [M+H]⁺ 650, measured value 650. ¹H NMR (400 MHz, CD₃OD) δ = 8.63 (dd, J = 1.4, 8.3 Hz, 1H), 8.42 (br s, 1H), 7.73 (dd, J = 1.6, 7.8 Hz, 1H), 7.59 (t, J = 7.7 Hz, 1H), 7.52 (t, J = 8.0 Hz, 1H), 7.48 (dd, J = 1.7, 7.6 Hz, 1H), 7.21 (dd, J = 1.5, 7.7 Hz, 1H), 4.72 - 4.68 (m, 1H), 4.59 (s, 2H), 4.51 - 4.39 (m, 2H), 4.13 (s, 3H), 3.99 (br dd, J = 5.5, 11.1 Hz, 2H), 3.90 (s, 2H) ), 3.82 (t, J = 5.7 Hz, 2H), 2.98 (br dd, J = 4.1, 8.8 Hz, 4H), 2.82 (t, J = 5.8 Hz, 2H), 2.61 (s, 3H).

### Example 37

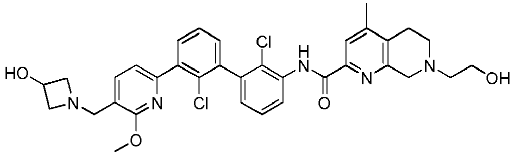

### Synthesis route:

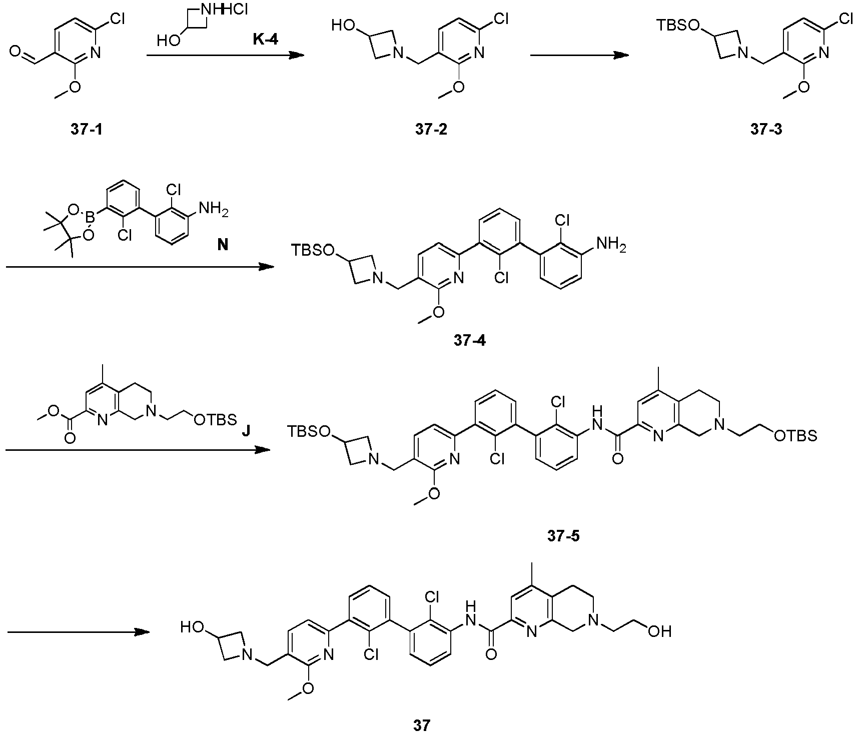

### Step 1

Compound 37-1 (3 g, 17.48 mmol, 1 equiv) was dissolved in dichloromethane (30 mL), and then diisopropylethylamine (2.26 g, 17.48 mmol, 1 equiv), K-4 (5.75 g, 52.45 mmol, 3 equiv) and sodium triacetoxyborohydride (11.12 g, 52.45 mmol, 3 equiv) were added thereto. The reaction mixture was placed at 25 °C and the reaction was conducted for 2 hours. After the reaction was completed, the reaction mixture was extracted with ethyl acetate (15 mL × 2). The combined organic phase was washed with saturated brine (25 mL), dried over anhydrous sodium sulfate. The residue was filtered and concentrated under reduced pressure to obtain compound 37-2.

MS-ESI calculated value [M+H]⁺ 228, measured value 229.

### Step 2

Compound 37-2 (1.6 g, 7 mmol, 1 equiv) was dissolved in dichloromethane (20 mL), and then imidazole (1.43 g, 20.99 mmol, 3 equiv) and t-butyldimethylchlorosilane (2.11 g, 13.99 mmol, 2 equiv) were added to the reaction mixture. The reaction mixture was placed at 25 °C and the reaction was conducted for 2 hours. After the reaction was completed, the reaction mixture was diluted with water (15 mL), and extracted with ethyl acetate (25 mL×2), and the combined organic phase was washed with saturated brine (25 mL), dried over anhydrous sodium sulfate, filtered and concentrated under reduced pressure. The residue was purified by silica gel column chromatography (petroleum ether: ethyl acetate=100:1-10:1) to obtain compound 37-3.

MS-ESI calculated value [M+H]⁺ 342, measured value 343.

### Step 3

Compound 37-3 (500 mg, 1.46 mmol, 1 equiv) was dissolved in dioxane (10 mL), and then Intermediate N (796.23 mg, 2.19 mmol, 1.5 equiv) and [1,1'-bis(diphenylphosphino)ferrocene]dichloropalladium (160.03 mg, 218.7 µmol, 0.15 equiv), potassium carbonate (604.53 mg, 4.37 mmol, 3 equiv) and water (1 mL) were added to the reaction mixture. The reaction mixture was placed at 75 °C and the reaction was conducted for 12 hours. After the reaction was completed, the reaction mixture was diluted with water (25 mL), and extracted with ethyl acetate (15 mL × 2). The combined organic phase was washed with saturated brine (25 mL), dried over anhydrous sodium sulfate, filtered and concentrated under reduced pressure. The residue was separated and purified by silica gel thin-layer chromatography (petroleum ether: ethyl acetate=3:1) to obtain compound 37-4.

MS-ESI calculated value [M+H]⁺ 543, measured value 544.

### Step 4

Compound 37-4 (150 mg, 275.44 µmol, 1 equiv) was dissolved in toluene (5 mL), and then Intermediate J (125.13 mg, 330.53 µmol, 1.2 equiv) and a solution of lithium bis(trimethylsilyl)amide in tetrahydrofuran (1 mol/L, 826.32 µL, 3 equiv) were added to the reaction mixture. The reaction mixture was placed at 25 °C and the reaction was conducted for 2 hours. After the reaction was completed, the reaction mixture was quenched with saturated ammonium chloride (5 mL), and extracted with ethyl acetate (10 mL × 2). The combined organic phase was washed with saturated brine (15 mL), dried over anhydrous sodium sulfate, filtered and concentrated under reduced pressure. The residue was subjected to silica gel thin layer chromatography (petroleum ether: ethyl acetate=1: 1) to obtain compound 37-5.

MS-ESI calculated value [M+H]⁺ 875, measured value 846.

### Step 5

Compound 37-5 (210 mg, 239.43 µmol, 1 equiv) was dissolved in ethyl acetate (3 mL), a solution of hydrogen chloride in ethyl acetate (4 mol/L, 8.55 µL) was added to the reaction mixture, the reaction mixture was placed at 25 °C and the reaction was conducted for 2 hours. After the reaction was completed, the reaction mixture was concentrated under reduced pressure. The residue was subjected to high performance liquid chromatography (chromatographic column: Phenomenex Synergi C18 150^{∗}25^{∗}10 microns; mobile phase: mobile phase A: formic acid aqueous solution with volume fraction of 0.225 %; mobile phase B: acetonitrile; B%: 15%-35%, 10 minutes) to obtain the formate of compound 37.

MS-ESI calculated value [M+H]⁺ 647, measured value 648. ¹H NMR (400 MHz, CD₃OD) δ= 8.57 - 8.63 (m, 1 H) 8.45 (br s, 1 H) 7.98 (s, 1 H) 7.87 (d, J=7.46 Hz, 1 H) 7.69 (dd, J=7.70, 1.47 Hz, 1 H) 7.45 - 7.58 (m, 2 H) 7.35 - 7.44 (m, 2 H) 7.17 (dd, J=7.58, 1.10 Hz, 1 H) 4.65 (br t, J=6.30 Hz, 1 H) 4.36 - 4.36 (m, 1 H) 4.38 (s, 1 H) 4.28 - 4.35 (m, 2 H) 4.10 (s, 3 H) 4.02 (s, 2 H) 3.83 - 3.95 (m, 4 H) 3.08 - 3.15 (m, 2 H) 2.97 - 3.03 (m, 2 H) 2.93 (t, J=5.69 Hz, 2 H) 2.42 (s, 3 H).

### Example 38

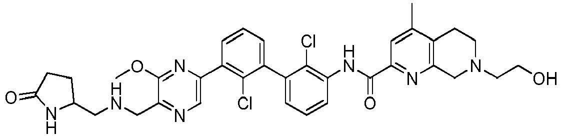

### Synthesis route:

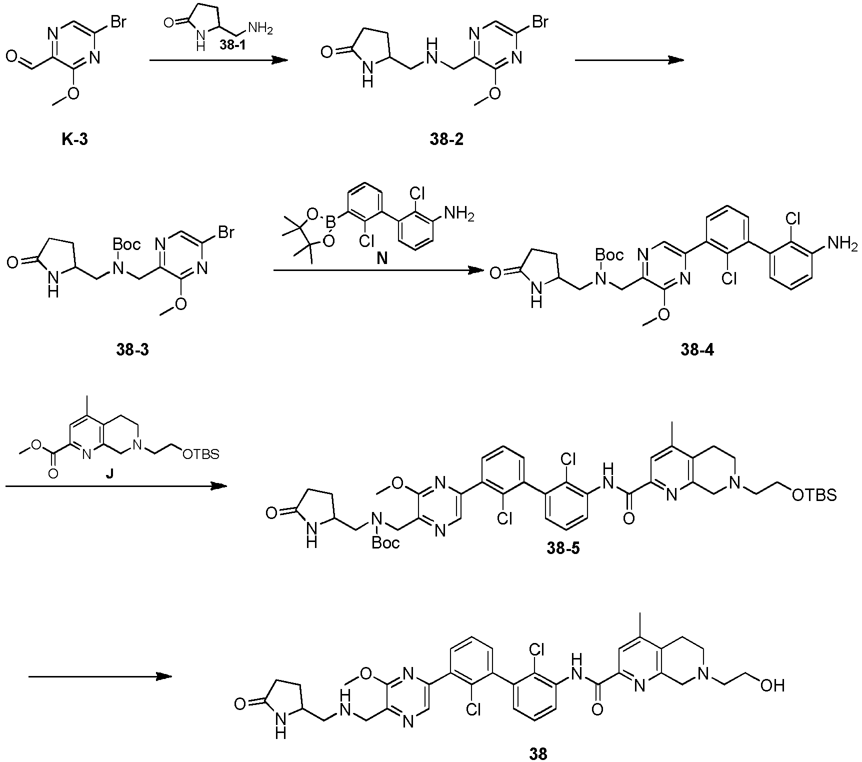

### Step 1

Intermediate K-3 (2.00 g, 9.22 mmol, 1 equiv) was dissolved in dichloromethane (30 mL), then 38-1 (1.31 g, 911.5 mmol, 1.25 equiv) was added to the reaction mixture, stirred at 25 °C for 0.5 hours. Sodium triacetoxyborohydride (5.86 g, 27.7 mmol, 3 equiv) was then added to the reaction mixture, stirred at 25 °C for 16 hours. After the reaction was completed, the reaction mixture was diluted with dichloromethane (80 mL), and the organic phase was washed with saturated aqueous sodium bicarbonate solution (80 mL), and concentrated under reduced pressure to obtain compound 38-2.

MS-ESI calculated value [M+H]⁺ 315, measured value 315.

### Step 2

Intermediate 38-2 (2.50 g, 7.93 mmol, 1 equiv) was dissolved in dichloromethane (50 mL), triethylamine (1.61 g, 15.9 mmol, 2.21 mL, 2 equiv), di-tert-butyl dicarbonate (2.25 g, 10.3 mmol, 2.37 mL, 1.3 equiv) was added to the reaction mixture, stirred at 25 °C for 16 hours. After the reaction was completed, the residue concentrated under reduced pressure was subjected to high performance liquid chromatography (chromatographic column: Phenomenex luna C18 250^{∗}50 mm^{∗}10 microns; mobile phase: mobile phase A: 10 % of ammonium bicarbonate aqueous solution by weight; mobile phase B: acetonitrile; B%: 25%-55%, 25 minutes) to obtain compound 38-3.

MS-ESI calculated value [M+H]⁺ 315,361, measured value 315,361.

### Step 3

Intermediate 38-3 (360 mg, 867 µmol, 1 equiv) was dissolved in dioxane (5 mL) and water (1 mL), and then N (473 mg, 1.30 mmol, 1.5 equiv), potassium carbonate (359 mg, 2.60 mmol, 3 equiv) and [1,1'-bis(diphenylphosphino)ferrocene]dichloropalladium (70.1 mg, 86.7 µmol, 0.1 equiv) were added to the reaction mixture, stirred at 65°C for 4 hours. After the reaction was completed, the reaction mixture was filtered and concentrated under reduced pressure. The residue was separated and purified by silica gel column chromatography (petroleum ether: ethyl acetate=10:1-1:1) to obtain compound 38-4.

MS-ESI calculated value [M+H]⁺ 573, measured value 573. ¹HNMR (400 MHz, CDCl₃) δ = 8.47 - 8.34 (m, 1 H), 7.71 - 7.55 (m, 1 H), 7.52 (dd, J=7.7, 1.72 Hz, 1 H), 7.41 - 7.34 (m, 1 H), 7.28 (br d, J=6.98 Hz, 1 H), 7.12 - 7.04 (m, 1 H), 6.77 (dd, J=8.0, 1.28 Hz, 1 H), 6.66 - 6.58 (m, 1 H), 4.68 (br dd, J=16.9, 11.44 Hz, 1 H), 4.36 - 4.21 (m, 1 H), 4.08 - 4.00 (m, 1 H), 4.03 - 4.00 (m, 1 H), 3.97 (d, J=1.48Hz, 3 H), 3.86 - 3.72 (m, 1 H), 3.88 - 3.71 (m, 1 H), 3.60 (br s, 1 H), 3.71 - 3.52 (m, 1 H) , 3.50 - 3.40 (m, 1 H), 3.33 - 2.96 (m, 1 H), 2.35 - 2.22 (m, 2 H), 2.22 - 2.10 (m, 1 H), 1.97 (s, 2 H), 1.44 - 1.37 (m, 3 H), 1.16 - 1.09 (m, 6 H).

### Step 4

Intermediate 38-4 (150 mg, 262 µmol, 1 equiv), J (105 mg, 288 µmol, 1.1 equiv) was dissolved in toluene (3 mL), then lithium bis(trimethylsilyl)amide (1 mol/L, 655 µL, 2.5 equiv) was added to the reaction mixture, stirred at 25°C for 2 hours. After the reaction was completed, water (20 mL) and saturated aqueous ammonium chloride solution (10 mL) were added to the reaction mixture, and extracted with ethyl acetate (60 mL). The organic phase was washed with saturated brine (60 mL), dried over anhydrous sodium sulfate, filtered and concentrated to obtain compound 38-5.

MS-ESI calculated value [M+H]⁺ 905, measured value 905. ¹HNMR (400 MHz, CDCl₃) δ =10.89 - 10.74 (m, 1 H), 8.74 (d, J=7.82 Hz, 1 H), 8.49 (br s, 1 H), 8.02 - 7.95 (m, 1 H) , 7.70 - 7.59 (m, 1 H), 7.53 - 7.34 (m, 3 H), 7.11 (ddd, J=7.61, 3.21, 1.54 Hz, 1 H), 4.88 - 4.68 (m, 1 H), 4.45 - 4.30 (m, 1 H), 4.17 - 4.03 (m, 4 H), 3.93 (br s, 4 H), 3.82 - 3.62 (m, 1 H), 3.62 - 3.46 (m, 1 H), 3.08 - 2.96 (m, 2 H), 2.95 - 2.82 (m, 4 H), 2.47 - 2.33 (m, 5 H), 2.30 - 2.17 (m, 1 H), 1.57 (s, 1 H), 1.50 (br s, 3 H), 1.28 - 1.14 (m, 6 H), 0.92 (s, 9 H), 0.10 (s, 6 H).

### Step 5

Intermediate 38-5 (50.0 mg, 55.3 µmol, 1 equiv) was dissolved in dichloromethane (1 mL), a solution of hydrogen chloride in ethyl acetate (4 mol/L, 1 mL, 72.4 equiv) was added to the reaction mixture, stirred at 25°C for 1 hour. After the reaction was completed, the reaction mixture was concentrated, and the crude product was subjected to high performance liquid chromatography (chromatographic column: Phenomenex luna C18 150^{∗}25 mm^{∗}10 microns; mobile phase: mobile phase A: formic acid aqueous solution with volume fraction of 0.225 %; mobile phase B: acetonitrile; B%: 11%-41%, 11 minutes) to obtain compound 38.

MS-ESI calculated value [M+H]⁺ 690, measured value 690. ¹HNMR (400 MHz, CD₃OD) δ = 8.61 (dd, J=8.25, 1.16 Hz, 1 H), 8.48 (s, 1 H), 8.42 (s, 1 H), 7.98 - 7.94 (m, 1 H), 7.76 - 7.69 (m, 1 H), 7.58 (t, J=7.64 Hz, 1 H), 7.53 - 7.42 (m, 2 H), 7.18 (dd, J=7.64, 1.42 Hz, 1 H), 4.74 - 4.56 (m, 1 H), 4.16 (d, J=1.60 Hz, 1 H), 4.12 - 4.10 (m, 3 H), 3.99 - 3.91 (m, 3 H), 3.90 - 3.74 (m, 3 H) , 3.11 - 3.03 (m, 2 H), 3.02- 2.90 (m, 4 H), 2.89 - 2.83 (m, 2 H), 2.42 (s, 3 H), 2.37 (br d, J=3.30 Hz, 2 H), 1.95 - 1.80 (m, 1 H).

### Example 39

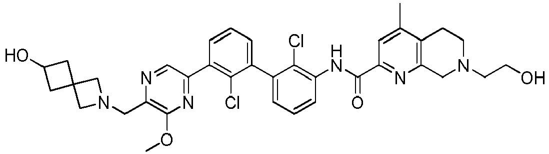

### Synthesis route:

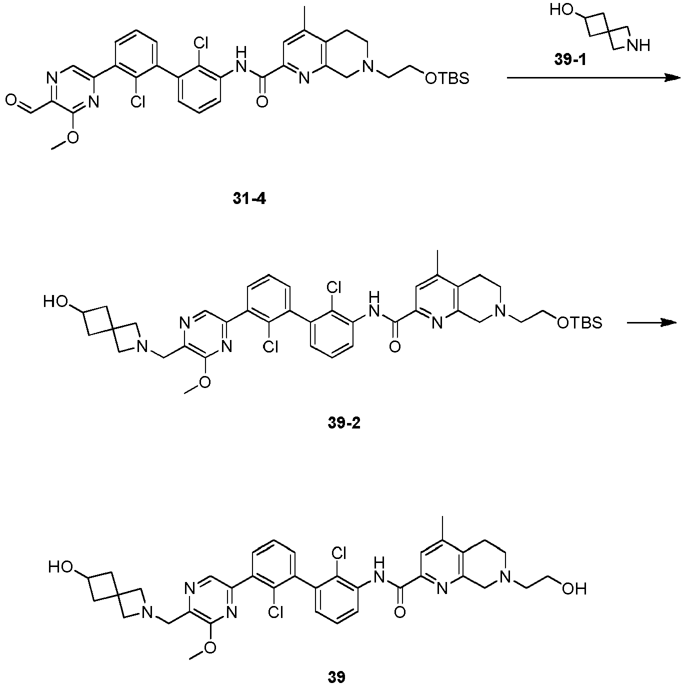

### Step 1

Ethanolamine (86.4 mg, 1.42 mmol, 85.6 µL, 10 equiv) was replaced with compound 39-1 (hydrochloride, 32.02 mg, 214.03 µmol, 1.51 equiv), and the method was the same as that in example 31. After the reaction was completed, the reaction mixture was filtered and concentrated, and the crude product was purified by silica gel thin-layer chromatography (petroleum ether: ethyl acetate: ethanol=4:3:1) to obtain compound 39-2.

MS-ESI calculated value [M+H]⁺ 803, measured value 803.

### Step 2

Compound 31-5 (20.0 mg, 26.6 µmol, 1 equiv) was replaced with compound 39-2 (40 mg, 49.76 µmol, 1 equiv), and the method was the same as that in example 31. After the reaction was completed, the reaction mixture was filtered and concentrated, and the crude product was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 10%-40%, 10 minutes) to obtain the formate of compound 39.

MS-ESI calculated value [M+H]⁺ 689, measured value 689. ¹H NMR (400 MHz, CD₃OD) δ =8.59 (dd, 1 H), 8.47 (s, 2 H), 7.93 (s, 1 H), 7.70 (dd, 1 H), 7.56 (t, 1 H), 7.39 - 7.49 (m, 2 H), 7.14 (dd, 1 H), 4.52 (s, 2 H), 4.21 (s, 2 H), 4.18 (s, 2 H), 4.13 - 4.17 (m, 1 H) ), 4.09 (s, 3 H), 3.91 (s, 2 H), 3.81 (t, 2 H), 2.98 - 3.04 (m, 2 H), 2.90 - 2.96 (m, 2 H), 2.83 (t, 2H), 2.65 (ddd, 2H), 2.38 (s, 3H), 2.13 - 2.23 (m, 2H).

### Example 40

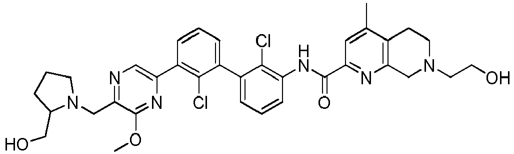

### Synthesis route:

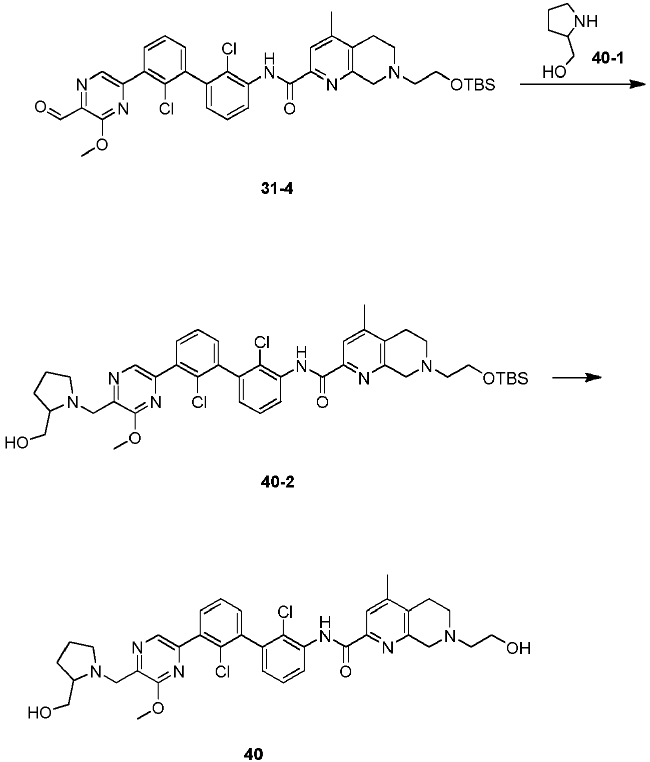

### Step 1

Ethanolamine (86.4 mg, 1.42 mmol, 85.6 µL, 10 equiv) was replaced with compound 40-1 (hydrochloride, 97.35 mg, 707.48 µmol, 5 equiv), and the method was the same as that in example 31. After the reaction was completed, the reaction mixture was filtered and concentrated, and the crude product was purified by silica gel thin-layer chromatography (dichloromethane: methanol=10:1) to obtain compound 40-2.

MS-ESI calculated value [M+H]⁺ 791, measured value 791.

### Step 2

Compound 31-5 (20.0 mg, 26.6 µmol, 1 equiv) was replaced with compound 40-2 (50 mg, 49.88 µmol, 1 equiv), and the method was the same as that in example 31. After the reaction was completed, the reaction mixture was filtered and concentrated, and the crude product was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 14%-44%, 10 minutes) to obtain the formate of compound 40.

MS-ESI calculated value [M+H]⁺ 677, measured value 677. ¹H NMR (400 MHz, CD₃OD) δ = 8.58 (dd, 1 H), 8.41 - 8.53 (m, 2 H), 7.91 (s, 1 H), 7.72 (dd, 1 H), 7.56 (t, 1 H) ), 7.39 - 7.50 (m, 2 H), 7.14 (dd, 1 H), 4.65 (br d, 1 H), 4.27 (br d, 1 H), 4.09 (s, 3 H), 3.88 (s, 2 H), 3.70 - 3.85 (m, 4 H), 3.51 (br dd, 2 H), 3.03 - 3.11 (m, 1 H), 2.96 - 3.02 (m, 2 H), 2.92 (br d, 2 H) ), 2.81 (t, 2 H), 2.37 (s, 3 H), 2.11 - 2.21 (m, 1 H), 1.92 - 2.03 (m, 2 H), 1.87 (dt, 1 H).

### Example 41

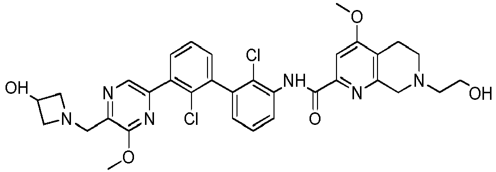

### Synthesis route:

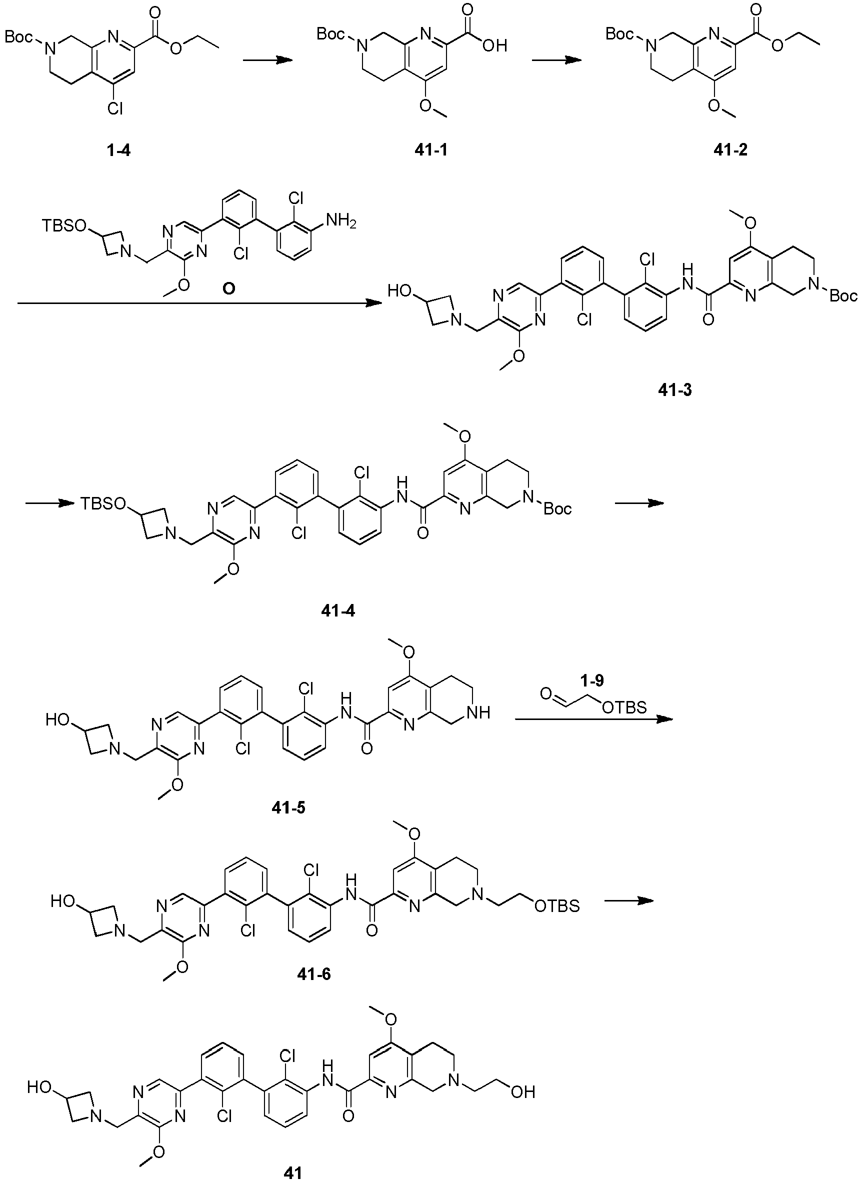

### Step 1

Compound 1-4 (500 mg, 1.38 mmol, 1 equiv) was dissolved in methanol (10 mL), then sodium methoxide (744.99 mg, 13.79 mmol, 10 equiv) was added to the reaction mixture, stirred at 70 °C for 10 hours. The reaction mixture was poured into saturated aqueous ammonium chloride (10 mL), and extracted with 30 mL of ethyl acetate (10 mL × 3). The combined organic phase was washed with saturated brine 30 mL (10 mL × 3), dried over anhydrous sodium sulfate, filtered and concentrated under reduced pressure to obtain compound 41-1.

MS-ESI calculated value [M+H]⁺ 308, measured value 308.

### Step 2

Compound 41-1 (400 mg, 1.30 mmol, 1 equiv) was dissolved in N,N-dimethylformamide (5 mL), ethyl iodide (1.01 g, 6.49 mmol, 5 mL,equiv) and potassium carbonate (359.59 mg, 2.59 mmol, 2 equiv) were to the reaction mixture, stirred at 60 °C for 10 hours. After the reaction was completed, the reaction mixture was diluted with water (10 mL), and extracted with 40 mL of ethyl acetate (20 mL × 2). The combined organic phase was washed with 40 mL of saturated brine (20 mL × 2), dried over anhydrous sodium sulfate, filtered, and concentrated. The crude product was purified by silica gel column chromatography (petroleum ether: ethyl acetate=10:1 to 3:1) to obtain compound 41-2.

MS-ESI calculated value [M+H]⁺ 337, measured value 337. ¹H NMR (400 MHz, CDCl₃) δ = 7.53 (s, 1 H), 4.80 (s, 2 H), 4.51 (q, 2 H), 3.97 (s, 3 H), 3.68 (t, 2 H), 2.79 (br t, 2 H), 1.67 (br s, 9 H), 1.43 - 1.48 (m, 3 H).

### Step 3

Compound 41-2 (180 mg, 503.00 µmol, 1.1 equiv) and Compound O (249.48 mg, 457.27 µmol, 1 equiv) were dissolved in toluene (5 mL), a solution of lithium bis(trimethylsilyl)amide in tetrahydrofuran (1 mol/L, 1.23 mL, 2.7 equiv) was added to the reaction mixture at 0 °C, stirred at 20°C for 2 hours. The reaction mixture was poured into saturated aqueous ammonium chloride (10 mL), and extracted with 30 mL of ethyl acetate (10 mL × 3). The combined organic phase was washed with saturated brine 30 mL (10 mL × 3), dried over anhydrous sodium sulfate, filtered and concentrated under reduced pressure to obtain compound 41-3.

MS-ESI calculated value [M+H]⁺ 721, measured value 721.

### Step 4

Compound 41-3 (500 mg, 692.88 µmol, 1 equiv) was dissolved in dichloromethane (5 mL), then imidazole (235.85 mg, 3.46 mmol, 5 equiv) and t-butyldimethylchlorosilane (522.16 mg, 3.46 mmol, 5 equiv) was added to the reaction mixture, stirred at 20°C for 0.5 hours. After the reaction was completed, the reaction mixture was diluted with water (10 mL) and extracted with 30 mL of ethyl acetate (10 mL × 3). The combined organic phase was washed with 30 mL of saturated brine (10 mL × 3), dried over anhydrous sodium sulfate, filtered, and concentrated. The residue was purified by silica gel thin layer chromatography (dichloromethane: methanol=10:1) to obtain compound 41-4.

MS-ESI calculated value [M+H]⁺ 835, measured value 835.

### Step 5

Compound 41-4 (150 mg, 179.45 µmol, 1 equiv) was dissolved in hydrogen chloride in ethyl acetate (4 mol/L, 5 mL), stirred at 25 °C for 0.15 hour. After the reaction was completed, the reaction mixture was concentrated under reduced pressure to obtain compound 41-5.

MS-ESI calculated value [M+H]⁺ 621, measured value 621.

### Step 6

Compound 41-5 (150 mg, 227.97 µmοl, 1 equiv, hydrochloride) was dissolved in methanol (2 mL), and then N,N-diisopropylethylamine (29.46 mg, 227.97 µmol, 39.71 µl, 1 equiv), compound 1-9 (79.48 mg, 55.95 µmol, 86.86 µl, 2 equiv) and sodium cyanoborohydride (35.81 mg, 569.93 µmol, 2.5 equiv) were added thereto. The reaction was conducted at 25°C for 2 hours. After the reaction was completed, the reaction mixture was filtered and concentrated under reduced pressure. The residue was subjected to silica gel thin layer chromatography (petroleum ether: ethyl acetate: ethanol=12:3:1) to obtain compound 41-6.

MS-ESI calculated value [M+H]⁺ 779, measured value 779.

### Step 7

Compound 41-6 (80 mg, 96.43 µmol, 1 equiv) was dissolved in ethyl acetate (2 mL), then hydrogen chloride in ethyl acetate (4 mol/L, 120.54 µL, 1 equiv) was added to the reaction mixture, the reaction was conducted at 25 °C for 0.25 hourS. After the reaction was completed, the reaction mixture was concentrated under reduced pressure, and the obtained residue was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 10%-40%, 10 minutes) to obtain the formate of compound 41.

MS-ESI calculated value [M+H]⁺ 665, measured value 665. ¹H NMR (400 MHz,CD₃OD) δ= 8.60 (dd, 1 H), 8.46 (s, 2 H), 7.68 - 7.73 (m, 2 H), 7.56 (t, 1 H), 7.42 - 7.50 (m, 2 H), 7.15 (dd, 1 H), 4.63 (t, 1 H), 4.46 (s, 2 H), 4.27 - 4.37 (m, 2 H), 4.09 (s, 3 H), 4.02 (s, 3 H), 3.88 (s, 2 H), 3.80 (br t, 4 H), 3.54 - 3.63 (m, 1 H), 2.95 (br d, 2 H), 2.85 - 2.90 (m, 2 H), 2.82 (t, 2H).

### Example 42

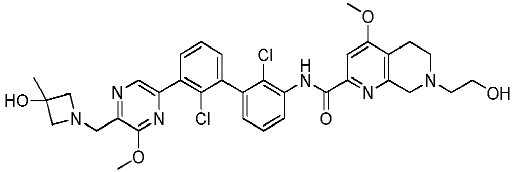

### Synthesis route:

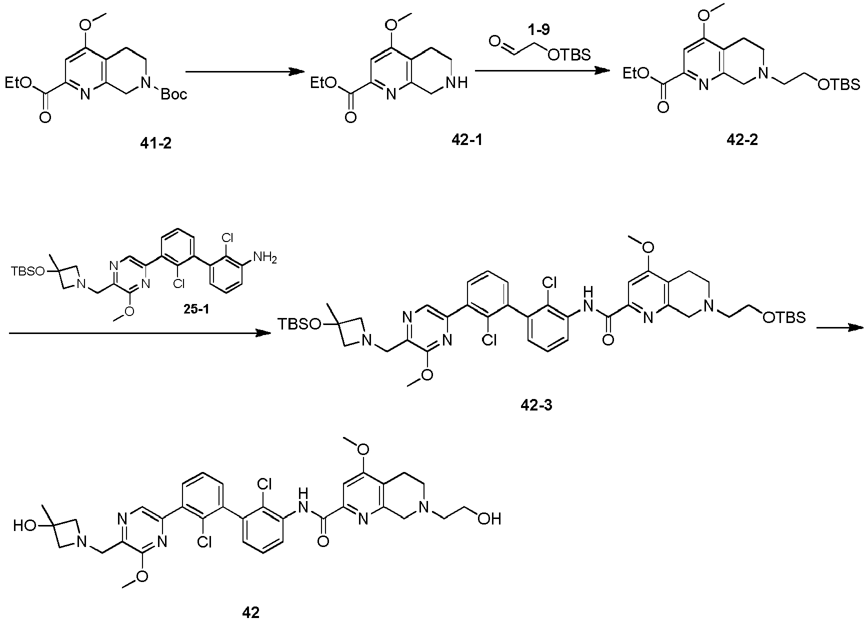

### Step 1

Compound 41-2 (300 mg, 891.84 µmol, 1 equiv) was dissolved in ethyl acetate (3 mL), then hydrogen chloride in ethyl acetate (4 mol/L, 1.11 mL, 5 equiv) was added thereto dropwise, stirred at 20 °C for 0.5 hours. After the reaction was completed, the reaction mixture was concentrated under reduced pressure to obtain the hydrochloride of compound 42-1.

MS-ESI calculated value [M+H]⁺ 236, measured value 236.

### Step 2

Compound 42-1 (hydrochloride, 250 mg, 916.67 µmol, 1 equiv) and compound 1-9 (319.57 mg, 1.83 mmol, 349.26 µl, 2 equiv) were dissolved in dichloromethane (3 mL), and then N,N-diisopropylethylamine (118.47 mg, 916.67 µmοl, 159.67 µl, 1 equiv) and sodium triacetoxyborohydride (582.84 mg, 2.75 mmol, 3 equiv) were added thereto. The reaction was conducted at 20 °C for 2 hours. After the reaction was completed, the reaction mixture was filtered and concentrated under reduced pressure. The residue was subjected to silica gel thin-layer chromatography (petroleum ether: ethyl acetate=3:1) to obtain compound 42-2. MS-ESI calculated value [M+H]⁺ 395, measured value 395.

### Step 3

Compound 42-2 (150 mg, 364.95 µmol, 1 equiv) and compound 25-1 (183.80 mg, 328.45 µmol, 0.9 equiv) were dissolved in toluene (5 mL), then a solution of lithium bis(trimethylsilyl)amide in tetrahydrofuran (1 mol/L, 0.912 mL, 2.5 equiv) was added to the reaction mixture at 0°C, stirred at 20°C for 1 hour. The reaction mixture was poured into saturated aqueous ammonium chloride (50 mL), and extracted with 100 mL of ethyl acetate (50 mL×2). The combined organic phase was washed with 100 mL of saturated brine (50 mL×2), dried over anhydrous sodium sulfate, filtered and concentrated under reduced pressure. The residue was subjected to silica gel thin-layer chromatography (petroleum ether: ethyl acetate=2:1) to obtain compound 42-3. MS-ESI calculated value [M+H]⁺ 395, measured value 395.

### Step 4

Compound 42-3 (200 mg, 204.82 µM, 1 equiv) was dissolved in ethyl acetate (5 mL), hydrogen chloride in ethyl acetate (4 mol/L, 256.03 µL, 5 equiv) was added thereto, stirred at 20°C for 1 hour. After the reaction was completed, the reaction mixture was filtered and concentrated, and the crude product was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 15%-35%, 10 minutes) to obtain the formate of compound 42.

MS-ESI calculated value [M+H]⁺ 679, measured value 679. ¹H NMR (400 MHz, CD₃OD) δ = 8.60 (dd, 1 H), 8.48 (s, 1 H), 8.42 (br s, 1 H), 7.73 (s, 1 H), 7.71 (dd, 1 H) , 7.57 (t, 1 H), 7.43 - 7.51 (m, 2 H), 7.16 (dd, 1 H), 4.55 (s, 2 H), 4.15 (d, 2 H), 4.11 (s, 3 H) , 4.04 (s, 3 H), 3.98 (br d, 2 H), 3.91 (s, 2 H), 3.82 (t, 2 H), 2.99 (br d, 2 H), 2.81 - 2.91 (m, 4 H), 1.56 (s, 3 H).

### Example 43

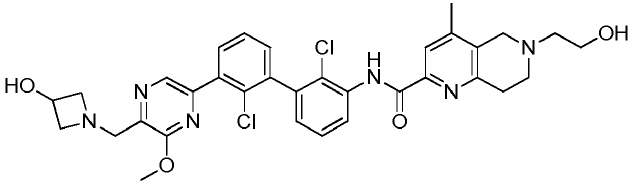

### Synthesis route:

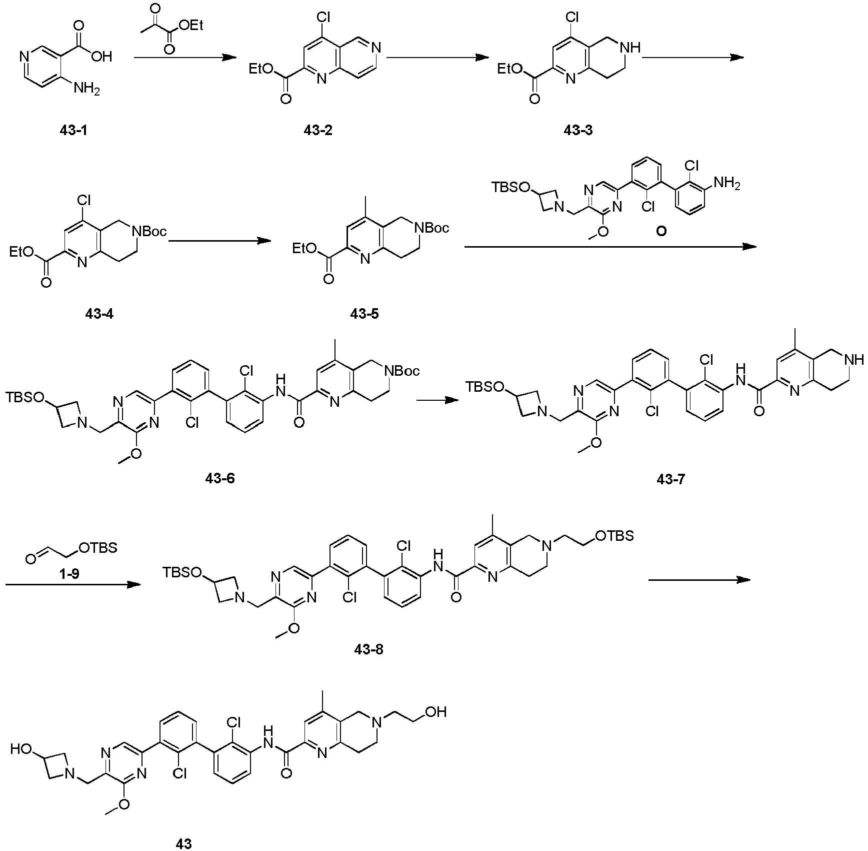

### Step 1

Compound 43-1 (5.00 g, 36.20 mmol, 1 equiv) and ethyl pyruvate (21.02 g, 181.00 mmol, 20.02 mL, 5 equiv) were mixed, stirred at 20 °C for 15 minutes, and then phosphorus oxychloride (55.50 g, 361.99 mmol, 33.64 mL, 10 equiv) was added dropwise to the reaction mixture. The reaction was conducted at 100 °C for 1 hour. After the reaction was completed, the reaction mixture was poured into water (1000 mL), the pH of the reaction mixture was adjusted to 7 with sodium carbonate, and then extracted with 600 mL of ethyl acetate (200 mL × 3). The combined organic phase was washed with 450 mL of saturated brine (150 mL×3), then dried over anhydrous sodium sulfate, filtered and concentrated under reduced pressure. The residue was purified by silica gel column chromatography (petroleum ether: ethyl acetate=10:1 to 2:1) to obtain compound 43-2.

MS-ESI calculated value [M+H]⁺ 236, measured value 236. ¹H NMR (400 MHz,CDCl₃) δ= 9.76 (s, 1 H), 8.94 (d, 1 H), 8.34 (s, 1 H), 8.15 (d, 1 H), 4.59 (q, 2 H), 4.06 - 4.40 (m, 6 H), 1.50 - 1.53 (m, 3 H)

### Step 2

Compound 43-2 (1 g, 3.48 mmol, 1 equiv) was dissolved in acetic acid (10 mL), sodium cyanoborohydride (546.70 mg, 8.70 mmol, 2.5 equiv) was added thereto, stirred at 20°C for 0.5 hours. After the reaction was completed, ice water was added to the reaction mixture, the pH of the reaction mixture was adjusted to 7 with sodium carbonate, and then extracted with 40 mL of ethyl acetate (20 mL × 2). The combined organic phase was washed with 200 mL of saturated brine (100 mL × 2), then dried over anhydrous sodium sulfate, filtered and concentrated under reduced pressure to obtain the crude product of compound 43-3.

MS-ESI calculated value [M+H]⁺ 239, measured value 239.

### Step 3

Compound 43-3 (1 g, 4.15 mmol, 1 equiv) was dissolved in dichloromethane (10 mL), and triethylamine (1.26 g, 12.46 mmol, 1.73 mL, 3 equiv) and di-tert-butyl dicarbonate (1.36 g, 6.23 mmol, 1.43 mL, 1.5 equiv) were added dropwise to the reaction mixture, stirred at 20°C for 0.5 hours. After the reaction was completed, the reaction mixture was extracted with 20 mL of dichloromethane (10 mL × 2). The combined organic phase was washed with 20 mL of saturated brine (10 mL × 2), dried over anhydrous sodium sulfate, filtered and concentrated under reduced pressure. The residue was purified by silica gel column chromatography (petroleum ether: ethyl acetate=10:1 to 5:1) to obtain compound 43-4.

MS-ESI calculated value [M+H]⁺ 327, measured value 327.

### Step 4

Compound 43-4 (100 mg, 293.43 µmοl, 1 equiv) and methylboronic acid (26.35 mg, 440.147 µmol, 1.5 equiv), potassium phosphate (186.86 mg, 880.28 µmοl, 3 equiv) were dissolved in dioxane (100 mL), then [1,1-bis(diphenylphosphino)ferrocene]dichloropalladium dichloromethane (23.96 mg, 29.34 µmol, 0.1 equiv) was added thereto, the reaction was conducted at 100 °C for 10 hours under nitrogen protection. After the reaction was completed, the reaction mixture was filtered and concentrated under reduced pressure. The residue was purified by silica gel thin layer chromatography (petroleum ether: ethyl acetate=2:1) to obtain compound 43-5.

MS-ESI calculated value [M+H]⁺ 321, measured value 321.

### Step 5

Compound 43-5 (200 mg, 624.25 µmol, 1 equiv) and compound O (340.58 mg, 624.25 µmol, 1 equiv) were dissolved in toluene (2 mL), then a solution of lithium bis(trimethylsilyl)amide in tetrahydrofuran (1 mol/L, 1.56 mL, 2.5 equiv) was added to the reaction mixture, stirred at 0 °C for 2 hours under nitrogen protection. The reaction mixture was poured into saturated aqueous ammonium chloride (20 mL), and extracted with 40 mL of ethyl acetate (20 mL×2). The combined organic phase was washed with saturated brine 40 mL (20 mL×2), then dried over anhydrous sodium sulfate, filtered and concentrated under reduced pressure. The residue was subjected to silica gel thin-layer chromatography (dichloromethane: methanol=20:1) to obtain compound 43-6.

MS-ESI calculated value [M+H]⁺ 819, measured value 819.

### Step 6

Compound 43-6 (140 mg, 48.79 µmol, 1 equiv) was dissolved in dichloromethane (2 mL) and trifluoroacetic acid (1 mL) and stirred at 25°C for 0.5 hours. After the reaction was completed, the reaction mixture was filtered and concentrated under reduced pressure to obtain the trifluoroacetate of compound 43-7.

MS-ESI calculated value [M+H]⁺ 719, measured value 719.

### Step 7

Compound 43-7 (trifluoroacetate, 40 mg, 47.97 µmol, 1 equiv) and compound 1-9 (41.81 mg, 239.87 µmol, 45.70 µl, 5 equiv) were dissolved in methanol (2 mL), and then sodium cyanoborohydride (7.54 mg, 119.93 µmοl, 2.5 equiv) was added thereto. The reaction was conducted at 20 °C for 2 hours. After the reaction was completed, the reaction mixture was filtered and concentrated under reduced pressure to obtain the crude product of compound 43-8.

MS-ESI calculated value [M+H]⁺ 763, measured value 763.

### Step 8

Compound 43-8 (40 mg, 52.37 µmol, 1 equiv) was dissolved in ethyl acetate (2 mL), a solution of hydrogen chloride in ethyl acetate (4 mol/L, 65.46 µL, 5 equiv) was added to the reaction mixture, the reaction was conducted at 20°C for 1 hour. After the reaction was completed, the reaction mixture was filtered and concentrated under reduced pressure. The obtained residue was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 13%-33%, 10 minutes) to obtain the formate of compound 43.

MS-ESI calculated value [M+H]⁺ 649, measured value 649. ¹H NMR (400 MHz, DMSO-*d*₆+D₂O) δ ppm 8.47 (dd, 1 H), 8.39 (s, 1 H), 8.32 (s, 2 H), 7.87(s, 1 H), 7.72-7.74 ( m, 1 H), 7.60-7.62 (m, 1 H), 7.55-7.58 (m, 1 H), 7.49-7.53 (m, 1 H), 7.19-7.21 (m, 1H), 4.20-4.26 (m , 2 H), 4.13-4.19 (m, 1H), 4.10 (s, 3H), 3.94-3.96 (m, 2 H), 3.71-3.80(m, 4 H), 3.58(br d, 2 H), 2.94-2.96 (m, 2 H), 2.87-2.88 (m, 2 H), 2.81-2.84 (m, 2 H)

### Example 44

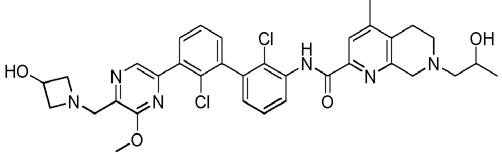

### Synthesis route:

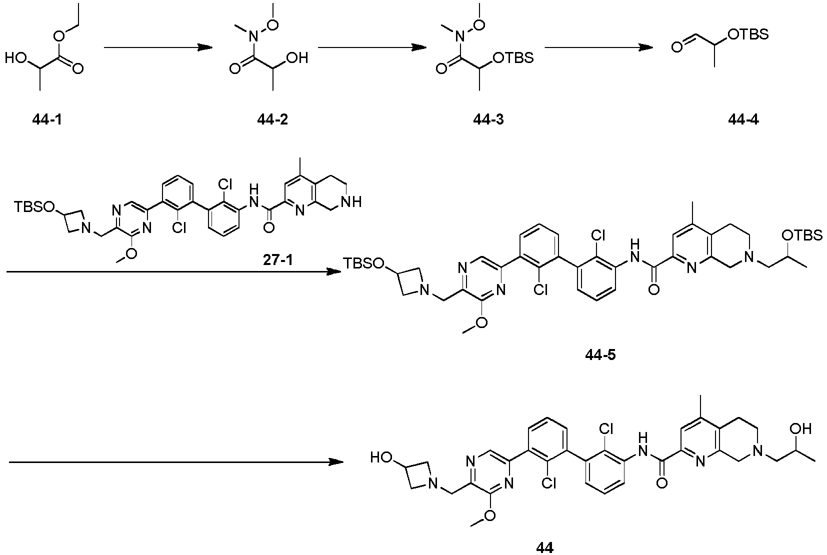

### Step 1

Compound 44-1 (4.00 g, 33.86 mmol, 1 equiv) and O,N-dimethylhydroxylamine hydrochloride (9.07 g, 67.72 mmol, 2 equiv, hydrochloride) were dissolved in tetrahydrofuran (50 mL), and then isopropylmagnesium chloride (2 mol/L, 59.26 mL, 3.5 equiv) was added dropwise to the reaction mixture. The reaction was conducted at -20 °C for 1 hour under nitrogen protection. After the reaction was completed, the reaction mixture was poured into saturated aqueous ammonium chloride (30 mL), and extracted with ethyl acetate 60 mL (20 mL×2). The combined organic phase was washed with saturated brine 60 mL (30 mL×2), dried over anhydrous sodium sulfate, filtered and concentrated under reduced pressure to obtain compound 44-2.

¹H NMR (400 MHz, CDCl₃) δ =4.49 (q, 1 H), 3.72 (s, 3 H), 3.25 (s, 3 H), 2.83 (m, 3 H), 1.37 (d, 3 H)

### Step 2

Compound 44-2 (1 g, 7.51 mmol, 1 equiv) was dissolved in dichloromethane (2 mL), and then imidazole (1.02 g, 15.02 mmol, 2 equiv) and t-butyldimethylchlorosilane (1.70 g, 11.27 mmol, 1.38 mL, 1.5 equiv) were added thereto, stirred at 20°C for 1 hour. After the reaction was completed, water (30 mL) was added to the reaction mixture, the reaction mixture was extracted with dichloromethane 60 mL (30 mL × 2). The combined organic phase was washed with 40 mL of saturated brine (20 mL × 2), dried over sodium sulfate, filtered and concentrated under reduced pressure to obtain the crude product of compound 44-3.

¹H NMR (400 MHz, CDCl₃) δ = 4.68-4.70 (m, 1 H), 3.71 (s, 3 H), 3.22 (s, 3 H), 1.36-1.38 (br s, 1 H), 0.91 (s, 9 H), 0.09-0.11 (m, 6 H)

### Step 3

Compound 44-3 (500 mg, 2.02 mmol, 1 equiv) was dissolved in tetrahydrofuran (5 mL), and then diisobutyl aluminium hydride (1 mol/L, 4.04 mL, 2 equiv) was added dropwise thereto. The reaction was conducted at -78°C for 1 hour under nitrogen protection. After the reaction was completed, the reaction mixture was poured into saturated aqueous ammonium chloride (20 mL) solution, and extracted with 40 mL of ethyl acetate (20 mL×2). The combined organic phase was washed with 40 mL of saturated brine (20 mL×2), dried over anhydrous sodium sulfate, filtered and concentrated under reduced pressure to obtain compound 44-4.

¹H NMR (400 MHz, CDCl₃) δ = 9.62 (s, 1 H), 4.07-4.13 (m, 1 H), 1.28-1.29 (m, 3 H) , 0.92 (s, 9 H), 0.10-0.11(m, 6 H)

### Step 4

Compound 44-4 (22.59 mg, 119.93 µmol, 2 equiv) and compound 27-1 (trifluoroacetate, 50 mg, 59.97 µmol, 1 equiv) were dissolved in methanol (2 mL), and then sodium cyanoborohydride (9.42 mg, 149.92 mmol, 2.5 equiv) was added thereto. The reaction was conducted at 25 °C for 1 hour. After the reaction was completed, the reaction mixture was filtered and concentrated under reduced pressure to obtain the crude product of compound 44-5.

MS-ESI calculated value [M+H]⁺ 891, measured value 891.

### Step 5

Compound 44-5 (30 mg, 33.63 µmol, 1 equiv) was dissolved in ethyl acetate (2 mL), and then a solution of hydrogen chloride in ethyl acetate (4 mol/L, 42.04 µL, 5 equiv) was added to the reaction mixture. The reaction was conducted at 20°C for 1 hour. After the reaction was completed, the reaction mixture was concentrated under reduced pressure, and the obtained residue was subjected to high performance liquid chromatography (chromatographic column: Phenomenex Gemini-NX C18 75^{∗}30^{∗}3 microns; mobile phase: mobile phase A: formic acid aqueous solution with volume fraction of 0.225 %; mobile phase B: acetonitrile; B%: 10%-40%, 5 minutes) to obtain the formate of compound 44.

MS-ESI calculated value [M+H]⁺ 663, measured value 663. ¹H NMR (400 MHz,CD₃OD) δ =8.60 (d, 1 H), 8.59 (s, 1 H), 8.48 (s, 1 H), 7.96 (s, 1 H), 7.70 - 7.72 (m, 1 H) ), 7.57 (d, 1 H), 7.47- 7.48 (m, 1 H), 7.15- 7.17 (m, 1 H) 4.44 - 4.70 (m, 1 H), 4.44 (s, 2 H), 4.40 - 4.43 (m, 2 H), 4.11 (s, 3 H), 3.96-3.99 (m, 3 H), 2.95-2.98 (m, 2 H), 2.69 - 2.70 (m, 2 H), 2.67-2.68(m , 2 H), 2.40 (s, 2 H), 1.20-1.22 (m, 3 H)

### Example 45

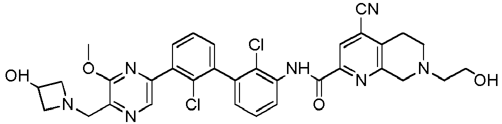

### Synthesis route:

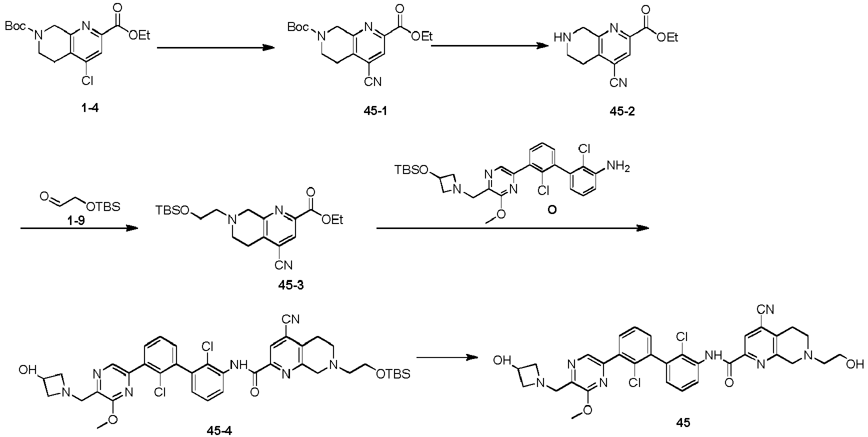

### Step 1

Compound 1-4 (1 g, 2.76 mmol, 1 equiv) was dissolved in N,N-dimethylacetamide (10 mL), and then zinc cyanide (649.76 mg, 5.52 mmol, 2 equiv) and methanesulfonato(2-dicyclohexylphosphino-3,6-dimethoxy-2',4',6'-tri-i-propyl-1,1'-biphenyl)(2'-amino-1,1'-biphenyl-2-yl)palladium(II) (500.06 mg, 551.64 µmol, 0.2 equiv) were added to the reaction mixture, stirred at 120 °C for 10 hours under nitrogen protection. After the reaction was completed, the reaction mixture was diluted with water (10 mL) and extracted with 30 mL of ethyl acetate (10 mL×3). The combined organic phase was washed with 30 mL of saturated brine (10 mL×3), dried over anhydrous sodium sulfate, filtered and concentrated. The obtained residue was subjected to high performance liquid chromatography (chromatographic column: column: Phenomenex luna C18 250^{∗}50 mm^{∗}10 microns; mobile phase: mobile phase A: formic acid aqueous solution with volume fraction of 0.225 %; mobile phase B: acetonitrile; B%: 25%-55%, 30 minutes) to obtain compound 45-1.

MS-ESI calculated value [M+H]⁺ 332, measured value 332. ¹H NMR (400 MHz, DMSO-*d*₆) δ ppm 8.23 (s, 1 H), 4.64 (br s, 2 H), 3.66 - 3.72 (m, 2 H), 3.02 (br s, 2 H), 1.44 (s, 9 H)

### Step 2

Compound 45-1 (150 mg, 452.67 µmol, 1 equiv) was dissolved in dichloromethane (5 mL) and trifluoroacetic acid (1 mL), stirred at 20° C for 1 hour. After the reaction was completed, the reaction mixture was concentrated under reduced pressure to obtain the trifluoroacetate of compound 45-2.

MS-ESI calculated value [M+H]⁺ 232, measured value 232.

### Step 3

Compound 45-2 (100 mg, 432.43 µmol, 1 equiv, trifluoroacetate) and compound 1-9 (150.76 mg, 864.86 µmol, 164.76 µl, 2 equiv) were dissolved in methanol (5 mL), and then N,N-diisopropylethylamine (55.89 mg, 432.43 µmol, 75.32 µl, 1 equiv) and sodium cyanoborohydride (67.94 mg, 1.08 mmol, 2.5 equiv) were added thereto. The reaction was conducted at 20 °C for 1 hour. After the reaction was completed, the reaction mixture was poured into saturated aqueous ammonium chloride solution (30 mL), and extracted with 60 mL of ethyl acetate (30 mL×2). The combined organic phase was washed with saturated brine 60 mL (30 mL×2), dried over anhydrous sodium sulfate, filtered and concentrated under reduced pressure to obtain compound 45-3.

MS-ESI calculated value [M+H]⁺ 389, measured value 389.

### Step 4

Compound 45-3 (80 mg, 184.82 µmol, 1.1 equiv) and compound O (91.67 mg, 168.02 µmol, 1 equiv) were dissolved in toluene (5 mL), then a solution of lithium bis(trimethylsilyl)amide in tetrahydrofuran (1 mol/L, 0.456 mL, 2.7 equiv) was added to the reaction mixture at 0°C, stirred at 20°C for 2 hours. The reaction mixture was poured into saturated aqueous ammonium chloride solution (10 mL), and extracted with 30 mL of ethyl acetate (10 mL×3). The combined organic phase was washed with saturated brine 30 mL (10 mL×3), then dried over anhydrous sodium sulfate, filtered and concentrated under reduced pressure. The residue was subjected to silica gel thin-layer chromatography (petroleum ether: ethyl acetate=2:1) to obtain compound 45-4.

MS-ESI calculated value [M+H]⁺ 888, measured value 888.

### Step 5

Compound 45-4 (75 mg, 81.83 µmol, 1 equiv) was dissolved in trifluoroacetic acid (1.5 mL) and dichloromethane (1.5 mL), and the reaction was conducted at 20°C for 2 hours. After the reaction was completed, the reaction mixture was concentrated under reduced pressure, and the obtained residue was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 10%-40%, 10 minutes) to obtain compound 45.

MS-ESI calculated value [M+H]⁺ 660, measured value 660. ¹H NMR (400 MHz, CD₃OD) δ=8.55 (d, 1 H), 8.48 (s, 1 H), 8.33 (s, 1 H), 7.70 (d, 1 H), 7.56 (td, 1 H), 7.41 - 7.51 (m, 2 H), 7.18 (d, 1 H), 4.66 - 4.73 (m, 1 H), 4.61 (s, 2 H), 4.46 (br dd, 2 H), 4.10 (d, 3 H), 4.01 (br dd, 2 H), 3.95 (s, 2 H), 3.79(t, 2 H), 3.13 - 3.21 (m, 2 H), 2.95 - 3.03 (m, 2 H), 2.81 (t, 2H)

### Example 46

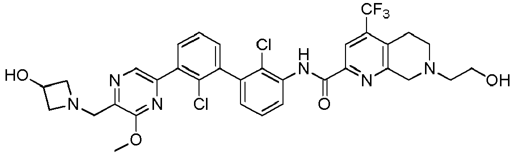

### Synthesis route:

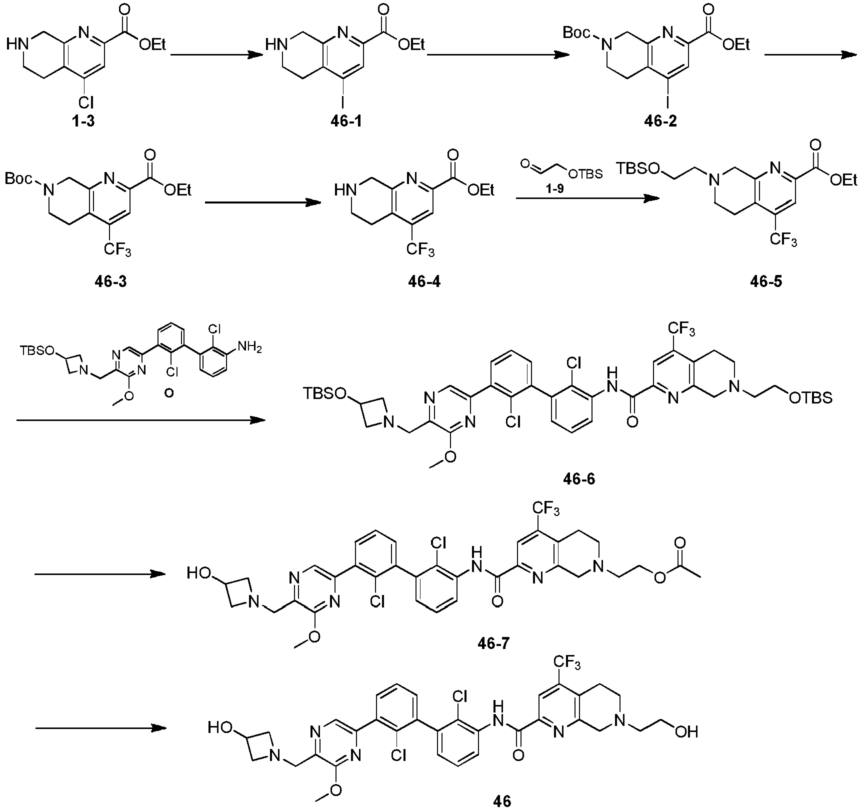

### Step 1

Intermediate 1-3 (1.8 g, hydrochloride, 6.49 mmol) was dissolved in acetonitrile (40 mL), and then sodium iodide (6.81 g, 45.46 mmol) was added thereto. The reaction mixture was heated to 80 °C and the reaction was conducted for 16 hours. After the reaction was completed, the reaction mixture was diluted with ethyl acetate (120 mL) and washed with saturated sodium bicarbonate solution (120 mL). The organic phase was dried over sodium sulfate, filtered, and concentrated to obtain intermediate 46-1.

MS-ESI calculated value [M+H]⁺ 333, measured value 333.

### Step 2

Intermediate 46-1 (2.0 g, 6.02 mmol) and triethylamine (913.98 mg, 9.03 mmol) were dissolved in dichloromethane (40 mL), and then di-tert-butyl dicarbonate (1.58 g, 7.23 mmol) was added thereto. The reaction was conducted at room temperature for 1 hour. After the reaction was completed, the reaction mixture was concentrated, and the residue was purified by silica gel column (petroleum ether: ethyl acetate=10:1) to obtain intermediate 46-2.

MS-ESI calculated value [M+H]⁺ 433, measured value 433.

### Step 3

Intermediate 46-2 (1 g, 2.31 mmol) was dissolved in N,N dimethylformamide (25 mL), and then cuprous iodide (881.20 mg, 4.63 mmol) and methyl 2,2-difluoro-2-(fluorosulfonyl)acetate (888.90 mg, 4.63 mmol) were added thereto. The reaction mixture was heated to 75 °C and the reaction was conducted for 8 hours under nitrogen protection. After the reaction was completed, the reaction mixture was diluted with ethyl acetate (80 mL) and filtered. The filtrate was washed with saturated brine (80 mL × 2), dried, filtered and concentrated. The crude product was purified by silica gel column (petroleum ether: ethyl acetate=10:1) to obtain intermediate 46-3.

MS-ESI calculated value [M+H]⁺ 375, measured value 375.

### Step 4

Intermediate 46-3 (805 mg, 2.15 mmol) was dissolved in ethyl acetate (8 mL), a solution of hydrogen chloride in ethyl acetate (4 mol/L, 8 mL) was added thereto, stirred at room temperature for 1 hour. After the reaction was completed, the reaction mixture was concentrated to obtain the hydrochloride of intermediate 46-4.

### Step 5

Intermediate 46-4 (660 mg, hydrochloride, 2.13 mmol) was dissolved in methanol (10 mL), and then N,N-diisopropylethylamine (311.04 mg, 2.41 mmol) and compound 1-9 (1.05 g, 6.02 mmol) were added thereto. After the reaction mixture was stirred for half an hour, sodium cyanoborohydride (453.72 mg, 7.22 mmol) was added thereto, and the reaction was conducted at room temperature for 16 hours. After the reaction was completed, the reaction mixture was diluted with dichloromethane (50 mL), washed with saturated brine (50 mL). The organic phase was dried over anhydrous sodium sulfate, filtered and concentrated. The crude product was purified by silica gel column (petroleum ether: ethyl acetate=10:1) to obtain intermediate 46-5.

MS-ESI calculated value [M+H]⁺ 433, measured value 433.

### Step 6

Intermediate 46-5 (120 mg, 277.42 µmol) and intermediate O (151.36 mg, 277.42 µmol) were dissolved in toluene (3 mL), then lithium bis(trimethylsilyl)amide (1 mol/L, 832.27 µl) was added thereto at 0°C under nitrogen protection, the reaction was conducted at room temperature for 1 hour. After the reaction was completed, the reaction mixture was quenched with saturated ammonium chloride solution (40 mL), and extracted with ethyl acetate (40 mL). The organic phase was dried over anhydrous sodium sulfate, filtered and concentrated. The crude product was separated and purified by silica gel thin layer chromatography (petroleum ether: ethyl acetate=5:2) to obtain intermediate 46-6.

MS-ESI calculated value [M+H]⁺ 933, measured value 933.

### Step 7

Intermediate 46-6 (135 mg, 144.84 µmol) was dissolved in ethyl acetate (3 mL), a solution of hydrogen chloride in ethyl acetate (4 mol/L, 3 mL) was added thereto, and the mixture was stirred at room temperature for 1 hour. After the reaction was completed, the reaction mixture was quenched with saturated sodium bicarbonate solution (40 mL), and extracted with ethyl acetate (40 mL), dried and concentrated. The crude product was separated and purified by silica gel thin layer chromatography (dichloromethane: methanol=10:1) to obtain intermediate 46-7.

MS-ESI calculated value [M+H]⁺ 745, measured value 745.

### Step 8

Intermediate 46-7 (52 mg, 69.74 µmol) was dissolved in methanol (2 mL), then potassium carbonate (19.28 mg, 139.49 µmol) was added thereto, the reaction was conducted at room temperature for 2 hours. After the reaction was completed, the reaction mixture was concentrated. The crude product was subjected to high performance liquid chromatography (column: Unisil 3-100 C18 Ultra 150^{∗}50 mm^{∗}3 microns, mobile phase: mobile phase A: formic acid aqueous solution with volume fraction of 0.225 %; mobile phase B: acetonitrile; B%: 18%- 38%, 10 minutes) to obtain the formate of compound 46.

MS-ESI calculated value [M+H]⁺ 703, measured value 703. ¹H NMR (400 MHz, CD₃OD) δ = 8.62 - 8.57 (m, 1 H), 8.50 (s, 1 H), 8.48 - 8.44 (m, 1 H), 8.35 - 8.31 (m, 1 H), 7.73 ( dd, J = 1.7, 7.7 Hz, 1 H), 7.59 (t, J = 7.6 Hz, 1 H), 7.54 - 7.45 (m, 2 H), 7.25 - 7.16 (m, 1 H), 4.72 - 4.66 (m, 1 H), 4.56 (s, 2 H), 4.46 - 4.38 (m, 2 H), 4.12 (s, 3 H), 4.01 (s, 2 H), 3.98 - 3.92 (m, 2 H), 3.85 - 3.79 (m, 2 H), 3.25 - 3.15 (m, 2 H), 3.01 - 2.94 (m, 2 H), 2.85 - 2.79 (m, 2 H)

### Example 47

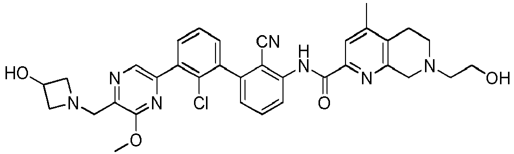

### Synthesis route:

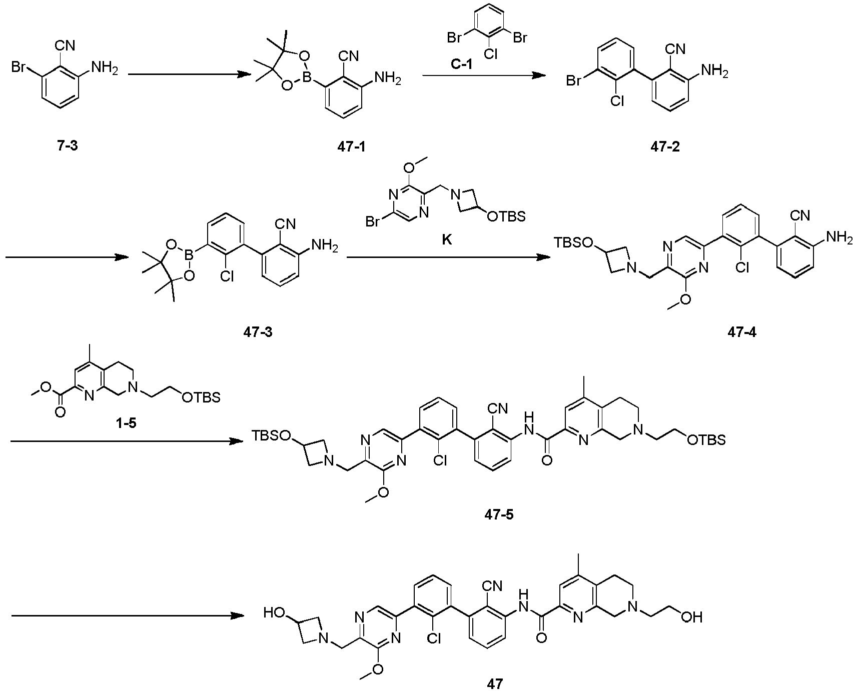

### Step 1

Compound 7-3 (5.00 g, 25.38 mmol, 1 equiv), bis(pinacolato)diboron (9.67 g, 38.06 mmol, 1.5 equiv) and potassium acetate (7.47 g, 76.13 mmol, 3 equiv) were dissolved in dioxane (50 mL), then [1,1'-bis(diphenylphosphino)ferrocene]dichloropalladium (1.86 g, 2.54 mmol, 0.1 equiv) was added to the reaction mixture, and the reaction was conducted at 70 °C for 10 hours under nitrogen protection. After the reaction was completed, the filtrate was filtered and concentrated under reduced pressure. The residue was purified by silica gel column chromatography (petroleum ether: ethyl acetate=10:1-3:1) to obtain intermediate 47-1.

MS-ESI calculated value [M+H]⁺ 244, measured value 244. ¹H NMR (400 MHz, CDCl₃) δ=7.30 (dd, 1 H), 7.17 (dd, 1 H), 6.83 (dd, 1 H), 4.49 (br s, 2 H), 1.37 (s, 12 H)

### Step 2

Intermediate 47-1 (2 g, 8.19 mmol, 1 equiv), C1 (2.22 g, 8.19 mmol, 1 equiv) were dissolved in dioxane (20 mL), water (2 mL), then potassium carbonate (3.40 g, 24.58 mmol, 3 equiv) and [1,1'-bis(diphenylphosphino)ferrocene]dichloropalladium (599.52 mg, 819.35 µmol, 0.1 equiv) were added to the reaction mixture, and the reaction was conducted at 80 °C for 4 hours under nitrogen protection. After the reaction was completed, the filtrate was filtered and concentrated under reduced pressure. The residue was purified by silica gel column chromatography (petroleum ether: ethyl acetate=20:1-5:1) to obtain intermediate 47-2.

MS-ESI calculated value [M+H]⁺ 308, measured value 308. ¹H NMR (400 MHz, CDCl₃) δ =7.71 (dd, 1 H), 7.38 (dd, 1 H), 7.27 - 7.31 (m, 1 H), 7.16 - 7.25 (m, 1 H), 6.79 (dd, 1 H), 6.68 (dd, 1 H)

### Step 3

Intermediate 47-2 (1.5 g, 4.39 mmol, 1 equiv), bis(pinacolato)diboron (1.67 g, 6.58 mmol, 1.5 equiv), potassium acetate (1.29 g, 13.17 mmol, 3 equiv) ) was dissolved in dioxane (20 mL), then [1,1'-bis(diphenylphosphino)ferrocene]dichloropalladium (321.16 mg, 438.92 µmol, 0.1 equiv) was added to the reaction mixture, and the reaction was conducted at 70 °C for 4 hours under nitrogen protection. After the reaction was completed, the filtrate was filtered and concentrated under reduced pressure to obtain the crude product of Intermediate 47-3.

MS-ESI calculated value [M+H]⁺ 355, measured value 355.

### Step 4

Intermediates 47-3 (700 mg, 1.97 mmol, 1 equiv) and 1-5 (766.59 mg, 1.97 mmol, 1 equiv) were dissolved in dioxane (10 mL) and water (1 mL), then potassium carbonate (818.39 mg, 5.92 mmol, 3 equiv) and [1,1'-bis(diphenylphosphino)ferrocene]dichloropalladium (144.43 mg, 197.38 µmol, 0.1 equiv) were added to the reaction mixture, and the reaction was conducted at 65 °C for 4 hours under nitrogen protection. After the reaction was completed, the reaction mixture was filtered and concentrated. The crude product was purified by silica gel column chromatography (petroleum ether: ethyl acetate=20:1-3:1) to obtain intermediate 47-4.

MS-ESI calculated value [M+H]⁺ 538, measured value 538. ¹H NMR (400 MHz, CDCl₃) δ = 8.47 (s, 1 H), 7.63 (dd, 1 H), 7.43 - 7.49 (m, 1 H), 7.37 - 7.42 (m, 2 H), 6.78 (ddd, 2 H), 4.50 - 4.59 (m, 3 H), 4.03 (s, 3 H), 3.80 - 3.89 (m, 4 H), 3.02 - 3.12 (m, 2 H), 1.25 (s, 6 H), 0.89 (s, 9H)

### Step 5

Compound 47-4 (200 mg, 354.39 µmol, 1 equiv) and compound 1-5 (157 mg, 389.82 µmol, 1.1 equiv) were dissolved in toluene (5 mL), then a solution of lithium bis(trimethylsilyl)amide in tetrahydrofuran (1 mol/L, 1.06 mL, 3 equiv) was added to the reaction mixture at 0°C, stirred at 0°C for 1 hour. After the reaction was completed, the reaction mixture was poured into saturated aqueous ammonium chloride (10 mL), and extracted with 30 mL of ethyl acetate (10 mL×3). The combined organic phase was washed with saturated brine 30 mL (10 mL×3), dried with anhydrous sodium sulfate, filtered and concentrated under reduced pressure. The crude product was purified by silica gel column chromatography (petroleum ether: ethyl acetate=10:1-2:1) to obtain intermediate 47-5.

MS-ESI calculated value [M+H]⁺ 721, measured value 721.

### Step 6

Compound 47-5 (110 mg, 126.63 µmol, 1 equiv) was dissolved in ethyl acetate (5 mL), a solution of hydrogen chloride in ethyl acetate (4 mol/L, 158.29 µL, 1 equiv) was added to the reaction mixture, the reaction was conducted at 20 °C for 0.1 hour. After the reaction was completed, the reaction mixture was concentrated under reduced pressure, and the obtained residue was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 8%-38%, 10 minutes) to obtain the formate of compound 47.

MS-ESI calculated value [M+H]⁺ 640, measured value 640. ¹H NMR (400 MHz,CD₃OD) δ = 8.55 (d, 1 H), 8.50 (s, 1 H), 8.48 (s, 1 H), 7.95 (s, 1 H), 7.75 - 7.85 (m, 2 H)), 7.53- 7.63 (m, 2 H), 7.31 (d, 1 H), 4.56 - 4.61 (m, 1 H), 4.36 (s, 2 H), 4.18 - 4.27 (m, 2 H), 4.09 ( s, 3 H), 3.89 (s, 2 H), 3.81 (t, 2 H), 3.68 - 3.74 (m, 2 H), 2.96 (br dd, 4 H), 2.80 (t, 2 H), 2.39 (s, 3H)

### Example 48

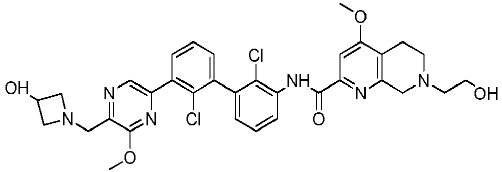

### Synthesis route:

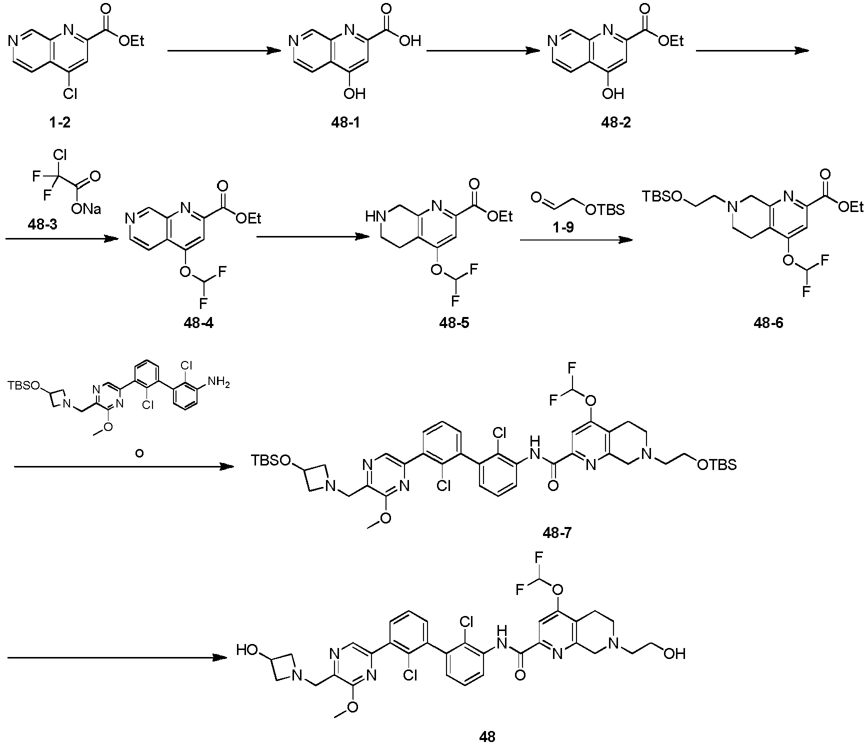

### Step 1

Compound 1-2 (1 g, 4.23 mmol, 1 equiv) was dissolved in tetrahydrofuran (10 mL) and water (2 mL), then sodium hydroxide (845.05 mg, 21.13 mmol, 5 equiv) was added to the reaction mixture, stirred at 70°C for 10 hours. The reaction mixture was filtered and concentrated under reduced pressure to obtain the crude product of compound 48-1.

MS-ESI calculated value [M+H]⁺ 191, measured value 191.

### Step 2

Compound 48-1 (0.9 g, 4.73 mmol, 1 equiv) was dissolved in ethanol (10 mL), and then concentrated sulfuric acid (2.32 g, 23.66 mmol, 1.26 mL, 5 equiv) was added to the reaction mixture, stirred at 70°C for 2 hours. After the reaction was completed, the filtrate was filtered and concentrated under reduced pressure. The residue was purified by silica gel column chromatography (petroleum ether: ethyl acetate=20:1~1:1) to obtain compound 48-2.

MS-ESI calculated value [M+H]⁺ 219, measured value 219.

### Step 3

Compound 48-2 (130 mg, 583.85 µmol, 1 equiv), compound 48-3 (267.04 mg, 1.75 mmol, 3 equiv) and cesium carbonate (570.68 mg, 1.75 mmol, 3 equiv) were dissolved in N, N-dimethylformamide (2 mL), stirred at 80°C for 0.5 hours. After the reaction was completed, the filtrate was filtered and concentrated under reduced pressure. The residue was purified by silica gel thin layer chromatography (petroleum ether: ethyl acetate=2:1) to obtain compound 48-4.

MS-ESI calculated value [M+H]⁺ 268, measured value 268.

### Step 4

Compound 48-4 (90 mg, 325.48 µmol, 1 equiv) was dissolved in glacial acetic acid (2 mL), then sodium cyanoborohydride (40.91 mg, 650.97 µmol, 2 equiv) was added to the reaction mixture, stirred at 20°C for 0.5 hours. The reaction mixture was concentrated under reduced pressure to obtain the crude product of compound 48-5.

MS-ESI calculated value [M+H]⁺ 272, measured value 272.

### Step 5

Compound 48-5 (80 mg, 293.85 µmol, 1 equiv) was dissolved in dichloromethane (2 mL), and then compound 7 (102.44 mg, 587.70 µmol, 111.96 µl, 2 equiv) and sodium triacetoxyborohydride (186.84 mg, 881.55 µmol, 3 equiv) were added to the reaction mixture. The reaction was conducted at 20°C for 1 hour. After the reaction was completed, the reaction mixture was filtered and concentrated under reduced pressure. The residue was subjected to silica gel thin layer chromatography (petroleum ether: ethyl acetate=3:1) to obtain compound 48-6.

MS-ESI calculated value [M+H]⁺ 431, measured value 431.

### Step 6

Compound 48-6 (50 mg, 110.32 µmol, 1 equiv) and compound O (54.17 mg, 99.29 µmol, 0.9 equiv) were dissolved in toluene (2 mL), then a solution of lithium bis(trimethylsilyl)amide in tetrahydrofuran (1 mol/L, 0.297 mL, 2.7 equiv) was added to the reaction mixture at 0°C, stirred at 0°C for 1 hour. The reaction mixture was poured into saturated aqueous ammonium chloride solution (10 mL), and extracted with 30 mL of ethyl acetate (10 mL×3). The combined organic phase was washed with saturated brine 30 mL (10 mL×3), then dried over anhydrous sodium sulfate, filtered and concentrated under reduced pressure. The residue was subjected to silica gel thin-layer chromatography (petroleum ether: ethyl acetate=2:1) to obtain compound 48-7.

MS-ESI calculated value [M+H]⁺ 931, measured value 931.

### Step 7

Compound 48-7 (60 mg, 56.93 µmol, 1 equiv) was dissolved in ethyl acetate (1 mL), and then a solution of hydrogen chloride in ethyl acetate (4 mol/L, 71.16 µL, 2 equiv) was added to the reaction mixture, stirred at 25°C for 0.5 hours. After the reaction was completed, the reaction mixture was concentrated under reduced pressure, and the obtained residue was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 12%-42%, 10 minutes) to obtain the formate of compound 48.

MS-ESI calculated value [M+H]⁺ 701, measured value 701. ¹H NMR (400 MHz,CD₃OD) δ= 8.58 (d, 1 H), 8.52 (s, 1 H), 8.43 (s, 1 H), 7.85 (s, 1 H), 7.66 - 7.72 (m, 1 H)), 7.55 (t, 1 H), 7.43 - 7.50 (m, 2 H), 7.07 - 7.27 (m, 2 H), 4.49 (t, 1 H), 4.13 (s, 2 H), 4.07 (s, 3 H), 4.03 (br d, 2 H), 3.87 (s, 2 H), 3.78 - 3.81 (m, 2 H), 3.44 (br dd, 2 H), 2.93 (s, 4 H), 2.78 (t, 2H)

### Example 49

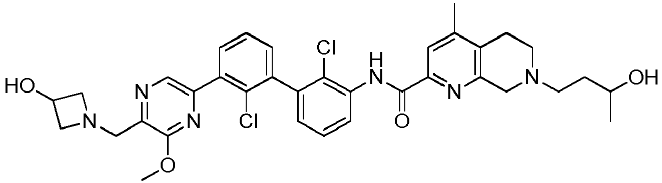

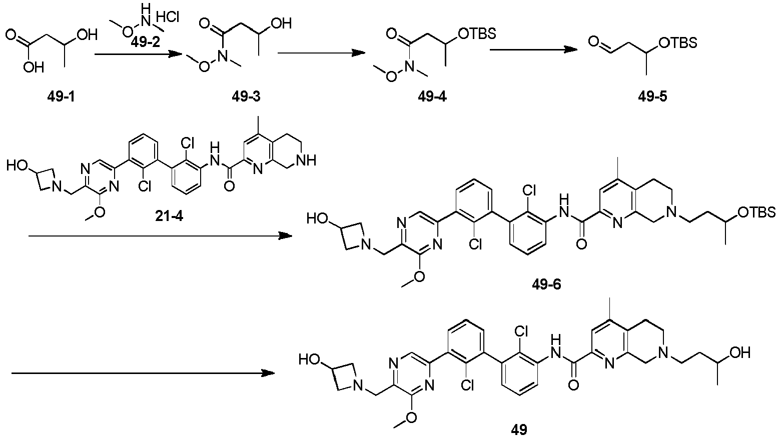

### Step 1

Compound 49-1 (3 g, 28.82 mmol, 2.65 mL, 1 equiv) was dissolved in N,N-dimethylformamide (20 mL), and then 49-2 (3.37 g, 34.58 mmol, 1.2 equiv, hydrochloride) and N,N-diisopropylethylamine (18.62 g, 144.09 mmol, 25.10 mL, 5 equiv) and O-(7-azabenzotriazole-1-yl)-N,N,N,N-tetramethylurea hexafluorophosphonate (13.15 g, 34.58 mmol, 1.2 equiv) were added thereto. The reaction was conducted at 25 °C for 2 hours. After the reaction was completed, the reaction mixture was diluted with water (100 mL), and extracted with 150 mL of ethyl acetate (50 mL×3). The combined organic phase was washed with saturated brine 120 mL (60 mL×2), dried over sodium sulfate, filtered and concentrated under reduced pressure to obtain the crude product of compound 49-3.

¹H NMR (400 MHz, CDCl₃) δ = 3.67 - 3.59 (m, 3H), 3.59 - 3.41 (m, 1H), 2.04 - 1.93 (m, 1H), 1.97 (s, 1H), 1.40 - 1.35 (m, 3H), 1.35 - 1.30 (m, 3H)

### Step 2

Compound 49-3 (4 g, 27.18 mmol, 1 equiv) was dissolved in dichloromethane (50 mL), and then imidazole (3.70 g, 54.36 mmol, 2 equiv) and t-butyldimethylchlorosilane (6.14 g, 40.77 mmol, 5.00 mL, 1.5 equiv) were added thereto, stirred at 25 °C for 2 hours. After the reaction was completed, water (20 mL) was added to the reaction mixture, and extracted with 120 mL of dichloromethane (40 mL × 3). The combined organic phase was washed with 40 mL of saturated brine (20 mL × 2), dried over sodium sulfate, filtered and concentrated under reduced pressure to obtain compound 49-4.

¹H NMR (400 MHz, CDCl₃) δ = 4.30 - 4.21 (m, 1H), 3.79 - 3.63 (m, 3H), 3.17 (s, 3H), 2.82 (s, 3H), 2.50 - 2.41 (m, 1H), 2.39 - 2.29 (m, 1H), 0.89 - 0.78 (m, 9H), 0.14 -0.02 (m, 6H)

### Step 3

Compound 49-4 (500 mg, 1.91 mmol, 1 equiv) was dissolved in tetrahydrofuran (10 mL), and then diisobutyl aluminium hydride (1 mol/L, 2.49 mL) was added dropwise to the reaction mixture at -78 °C, 1.3 equiv). The reaction was conducted at -78°C for 1 hour under nitrogen protection. After the reaction was completed, the reaction mixture was poured into saturated aqueous ammonium chloride (5 mL), diluted with water (20 mL), and extracted with 60 mL of ethyl acetate (20 mL × 3). The combined organic phase was washed with 40 mL of saturated brine (20 mL × 2), dried over anhydrous sodium sulfate, filtered and concentrated under reduced pressure to obtain the crude product of compound 49-5.

¹H NMR (400 MHz, CDCl₃) δ = 9.80 - 9.52 (m, 1H), 3.63 - 3.54 (m, 1H), 2.70 (s, 3H), 1.26 - 1.21 (m, 1H), 1.10 - 1.07 (m, 1H), 0.83 - 0.77 (m, 9H), 0.05 -0.08 (m, 6H)

### Step 4

Compound 49-5 (140.64 mg, 694.89 µmol, 5 equiv) and compound 21-4 (trifluoroacetate, 100 mg, 138.98 µmol, 1 equiv) were dissolved in methanol (2 mL), and then sodium cyanoborohydride (17.47 mg, 277.96 mmol, 2 equiv) were added thereto. The reaction was conducted at 25 °C for 12 hours. After the reaction was completed, the reaction mixture was diluted with water (10 mL), and extracted with 60 mL of ethyl acetate (20 mL × 3). The combined organic phase was washed with 40 mL of saturated brine (20 mL × 2), dried over anhydrous sodium sulfate, filtered and concentrated under reduced pressure. The obtained residue was subjected to silica gel thin-layer chromatography (dichloromethane: methanol=10:1) to obtain compound 49-6.

MS-ESI calculated value [M+H]⁺ 791, measured value 791.

### Step 5

Compound 49-6 (20 mg, 25.26 µmol, 1 equiv) was dissolved in methanol (1 mL), and then a solution of hydrogen chloride in ethyl acetate (4 mol/L, 0.5 mL, 79.19 equiv) was added thereto. The reaction was conducted at 25 °C for 1 hour. After the reaction was completed, the reaction mixture was filtered and concentrated under reduced pressure. The obtained residue was subjected to high performance liquid chromatography (chromatographic column: Phenomenex luna C18 150^{∗}25mm^{∗}10um; mobile phase: mobile phase A: formic acid aqueous solution with volume fraction of 0.225 %; mobile phase B: acetonitrile; B%: 11%-41%, 10 minutes) to obtain compound 49.

MS-ESI calculated value [M+H]⁺ 677, measured value 677 ¹H NMR (400 MHz, CD₃OD) δ = 8.59 (dd, *J =* 1.4, 8.3 Hz, 1H), 8.53 (s, 1H), 8.49 (s, 1H), 8.00 (s, 1H), 7.72 (dd, *J* = 1.7, 7.7 Hz, 1H), 7.58 (t, *J* = 7.7 Hz, 1H), 7.52 - 7.44 (m, 2H), 7.17 (dd, *J* = 1.5, 7.7 Hz, 1H), 4.74 (td, *J* = 6.1, 12.2 Hz, 1H), 4.68 (s, 2H), 4.53 (br dd, *J* = 6.8, 11.4 Hz, 2H), 4.13 (br d, *J* = 4.1 Hz, 2H), 4.12 (s, 3H), 4.10 - 4.05 (m, 2H), 3.90 (br d, *J* = 6.1 Hz, 1H), 3.28 - 3.18 (m, 2H), 3.16 - 3.08 (m, 1H), 3.04 (br t, *J* = 6.3 Hz, 2H), 3.01 - 2.93 (m, 1H), 2.43 (s, 3H), 1.85 (br d, *J* = 7.2 Hz, 2H), 1.23 (d, *J* = 6.3 Hz, 3H)

### Example 50

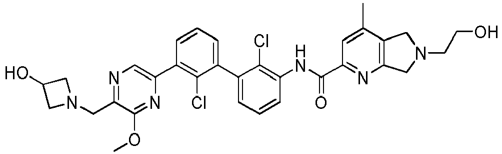

### Synthesis route:

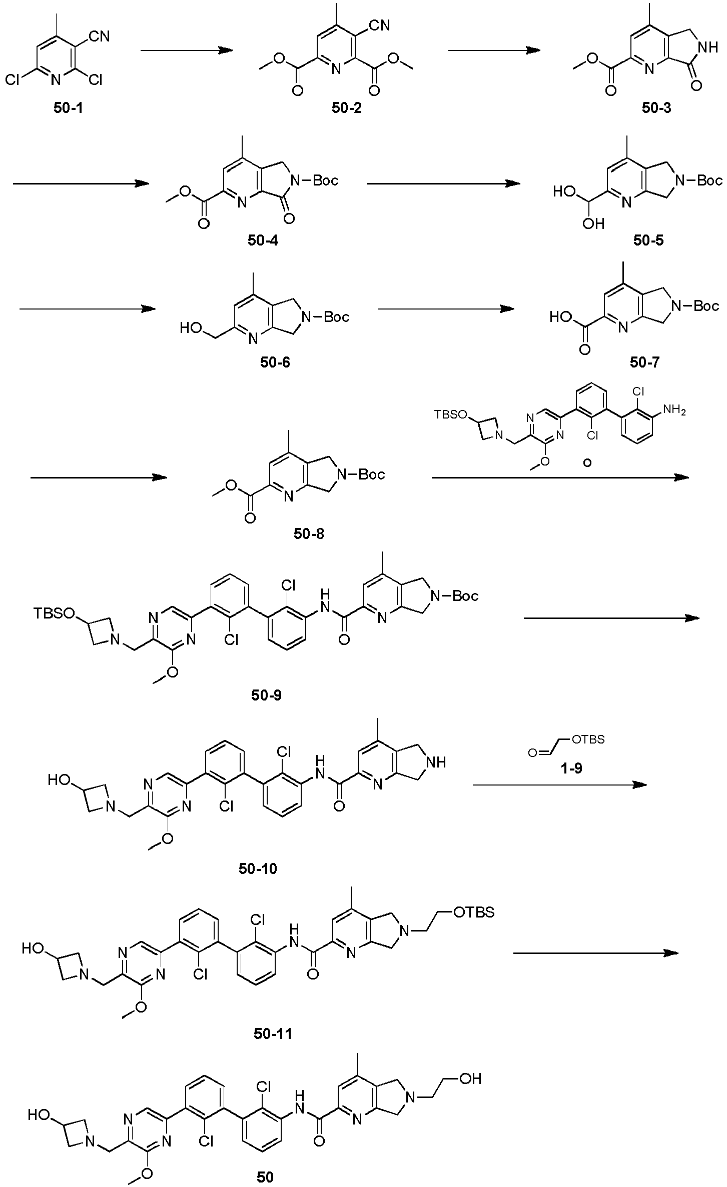

### Step 1

Intermediate 50-1 (6 g, 32.18 mmol) was dissolved in methanol (100 mL) and N,N dimethylformamide (12 mL), and then triethylamine (9.74 g, 96.24 mmol) and [1,1'-bis(diphenylphosphino)ferrocene]dichloropalladium (1.17g, 1.60mmol) were added thereto. The reaction mixture was heated to 80 °C under carbon monoxide (pressure 50 psi) and the reaction was conducted for 12 hours. After the reaction was completed, the reaction mixture was combined with another batch, water (200 mL) was added. The combined reaction mixture was extracted with ethyl acetate (50 mL×3). The combined organic phase was washed with saturated brine (70 mL×3), dried over anhydrous sodium sulfate, filtered and concentrated. Methyl tert-butyl ether: dichloromethane: methanol = 10:1:1 (50 mL) were added to the crude product, stirred at room temperature, then filtered and dried to obtain intermediate 50-2.

MS-ESI calculated value [M+H]⁺ 235, measured value 235. ¹H NMR (400 MHz, CDCl₃) δ = 8.20 (s, 1 H), 4.02 (s, 3 H), 3.98 (s, 3 H)ₒ

### Step 2

Intermediate 50-2 (7.5 g, 31.92 mmol) was dissolved in methanol (200 mL), and then Raney nickel (6.30 g, 73.49 mmol) was added thereto under nitrogen protection. The reaction mixture was heated to 50 °C under hydrogen atmosphere (50 psi) and the reaction was conducted for 12 hours. After the reaction was completed, the reaction mixture was filtered and concentrated. Methanol (30 mL) was added to the crude product, stirred at room temperature, filtered, and the filter cake was dried to obtain intermediate 50-3.

MS-ESI calculated value [M+H]⁺ 207, measured value 207. ¹H NMR (400 MHz, DMSO-*d₆*) δ = 9.11 (s, 1 H), 8.07 (s, 1 H), 4.47 (s, 2 H) , 3.92 (s, 3 H), 2.45 (s, 3 H)o

### Step 3

Intermediate 50-3 (3.6 g, 17.46 mmol) was dissolved in dichloromethane (40 mL), and then di-tert-butyl dicarbonate (4.19, 19.20 mmol) and 4-dimethylaminopyridine (319.94 mg, 2.62 mmol) were added thereto. The reaction was conducted at room temperature for 3 hours. After the reaction was completed, the reaction mixture was concentrated and purified by silica gel column chromatography (petroleum ether: ethyl acetate=2:1) to obtain intermediate 50-4.

MS-ESI calculated value [M+H]⁺ 307, measured value 307.

### Step 4

Intermediate 50-4 (580 mg, 1.89 mmol) was dissolved in tetrahydrofuran (10 mL), and then diisobutyl aluminium hydride (1 mol/L, 5.68 mL) was added at - 5 °C. The reaction was conducted at - 5 °C for 2 hours, then the reaction was conducted at room temperature for 16 hours. Monitoring that the reaction was not completed, diisobutyl aluminium hydride (1 mol/L, 5.68 mL) was added thereto at -5 °C, and the reaction was conducted at room temperature for 16 hours. After the reaction was completed, the reaction mixture was quenched with sodium sulfate decahydrate (10 g), filtered, and the filtrate was concentrated to obtain the crude product of intermediate 50-5.

MS-ESI calculated value [M+H]⁺ 281, measured value 281.

### Step 5

Intermediate 50-5 (300 mg, 1.07 mmol) was dissolved in acetic acid (10 mL), and thensodium cyanoborohydride (201.76, 3.21 mmol) was added thereto under nitrogen protection at 0 °C. The reaction was conducted at room temperature for 16 hours. After the reaction was completed, the reaction mixture was diluted with ethyl acetate (80 mL), washed with saturated aqueous sodium bicarbonate solution (80 mL×2), and the organic phase was dried over anhydrous sodium sulfate, filtered, and concentrated to obtain the crude product of intermediate 50-6.

MS-ESI calculated value [M+H]⁺ 265, measured value 265.

### Step 6

Intermediate 50-6 (250 mg, 945.83 µmol) was dissolved in acetonitrile (8 mL), and then aqueous potassium dihydrogen phosphate (136.18 mg in 2.4 mL water) and 2,2,6,6-tetramethylpiperidinooxy (29.75 mg, 189.17 µmol) were added thereto. After the reaction mixture was stirred for half an hour, sodium chlorite (256.63 mg, 2.84 mmol) and sodium hypochlorite aqueous solution (116.38 µl, 10%) were added thereto, and the reaction was conducted at room temperature for 19 hours. After the reaction was completed, the reaction mixture was diluted with ethyl acetate (50 mL) and washed with aqueous sodium hydroxide solution (1 mol/L, 50 mL). The pH of aqueous phase was adjusted to 4 with dilute hydrochloric acid (1 mol/L), and the reaction mixture was extracted with dichloromethane: methanol = 20:1 (40 mL). The organic phase was dried over anhydrous sodium sulfate, filtered, and concentrated to obtain the crude product of intermediate 50-7.

MS-ESI calculated value [M+H]⁺ 279, measured value 279.

### Step 7

Intermediate 50-7 (410 mg, 503.05 (µmol) was dissolved in dichloromethane (8 mL) and methanol (4 mL), and then (trimethylsilyl)diazomethane (2 mol/L, 503.05 (µl) was added thereto at 0°C under nitrogen protection. The reaction was conducted at room temperature for 2 hours. After the reaction was completed, dichloromethane (80 mL) was diluted, and the reaction mixture was washed with brine (80 mL). The organic phase was dried over anhydrous sodium sulfate, filtered and concentrated. The crude product was subjected to silica gel thin layer chromatography (petroleum ether: ethyl acetate=2:1) to obtain the crude product of intermediate 50-8.

MS-ESI calculated value [M+H]⁺ 293, measured value 293.

### Step 8

Intermediate 50-8 (62 mg, 212.09 µmol) and intermediate O (115.71 mg, 212.09 µmol) were dissolved in toluene (3 mL), then lithium bis(trimethylsilyl)amide (1 mol/L, 636.27 (µl) was added at 0 °C under nitrogen protection, and the reaction was conducted at room temperature for 2 hours. Monitoring that the reaction was not completed, lithium bis(trimethylsilyl)amide (1 mol/L, 212.09 (µl) was added thereto at 0 °C under nitrogen protection, and the reaction was conducted at room temperature for 2 hours. The reaction mixture was quenched with saturated ammonium chloride solution (8 mL) and extracted with ethyl acetate (8 mL). The organic phase was dried over anhydrous sodium sulfate, filtered and concentrated. The crude product was subjected to silica gel thin layer chromatography (dichloromethane: methanol = 20: 1) and high performance liquid chromatography (chromatographic column: Phenomenex luna C18 150^{∗}25 mm^{∗}10 microns; mobile phase: mobile phase A: formic acid aqueous solution with volume fraction of 0.225 %; mobile phase B: acetonitrile; B%: 43%-73%, 11 minutes) to obtain intermediate 50-9.

MS-ESI calculated value [M+H]⁺ 805, measured value 805. ¹H NMR (400 MHz, CDCl₃) δ=10.62 - 10.72 (m, 1 H), 8.61 - 8.72 (m, 1 H), 8.50 (s, 1 H), 7.97 (d, J = 15.9 Hz, 1 H) , 7.53 - 7.66 (m, 1 H), 7.32 - 7.45 (m, 3 H), 7.05 (d, J = 7.9 Hz, 1 H), 4.54 - 4.84 (m, 7 H), 4.32 - 4.48 (m, 2 H), 4.02 - 4.08 (s, 3 H), 3.75 - 3.76 (m, 2 H), 2.30 - 2.39 (s, 3 H), 1.48 (s, 9 H), 0.81 (s, 9 H), 0.01 (s, 6H).

### Step 9

Intermediate 50-9 (33 mg, 40.95 µmol) was dissolved in ethyl acetate (2 mL), a solution of hydrogen chloride in ethyl acetate (4 mol/L, 2 mL) was added thereto, stirred at room temperature for 1 hour. After the reaction was completed, the reaction mixture was concentrated to obtain the hydrochloride of intermediate 50-10.

MS-ESI calculated value [M+H]⁺ 591, measured value 591.

### Step 10

Intermediate 50-10 (25 mg, hydrochloride, 39.81 mmol) was dissolved in methanol (2 mL), and then N,N-diisopropylethylamine (5.15 mg, 39.81 mmol) and intermediate 1-9 (17.35 mg, 99.53 µmol) were added thereto. After the reaction mixture was stirred for half an hour, sodium cyanoborohydride (7.51 mg, 119.44 µmol) was added thereto, and the reaction was conducted at room temperature for 16 hours. After the reaction was completed, the reaction mixture was diluted with ethyl acetate (30 mL) and washed with saturated brine (30 mL). The organic phase was dried over anhydrous sodium sulfate, filtered, and concentrated to obtain the crude product of intermediate 50-11.

MS-ESI calculated value [M+H]⁺ 749, measured value 749.

### Step 11

Intermediate 50-11 (29 mg, 38.68 µmol) was dissolved in ethyl acetate (2 mL), then a solution of hydrogen chloride in ethyl acetate (2 mL, 4 mol/L) was added thereto, and the reaction was conducted at room temperature for 1 hour. After the reaction was completed, the reaction mixture was concentrated. The crude product was subjected to high performance liquid chromatography (chromatographic column: Shim-pack C18 150^{∗}25^{∗}10 microns; mobile phase: mobile phase A: formic acid aqueous solution with volume fraction of 0.225 %; mobile phase B: acetonitrile; B%: 9%-39%, 10 minutes) to obtain the formate of compound 50.

MS-ESI calculated value [M+H]⁺ 635, measured value 635. ¹H NMR (400 MHz, CD₃OD) δ= 8.58 - 8.63 (m, 1 H), 8.51 (s, 1 H), 7.96 - 8.00 (m, 1 H), 7.68 - 7.75 (m, 1 H), 7.55 - 7.61 (m, 1 H), 7.45 - 7.53 (m, 2 H), 7.15 - 7.20 (m, 1 H), 4.68 (m, 1 H), 4.52 (s, 2 H), 4.35 - 4.42 ( m, 2 H), 4.11 - 4.19 (m, 7 H), 3.88 - 3.95 (m, 2 H), 3.77 - 3.84 (m, 2 H), 3.02 (t, J = 5.8 Hz, 2 H), 2.44 (s, 3H)

### Example 51

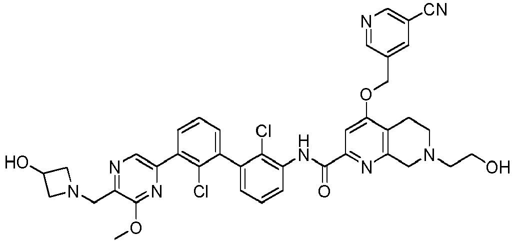

### Synthesis route:

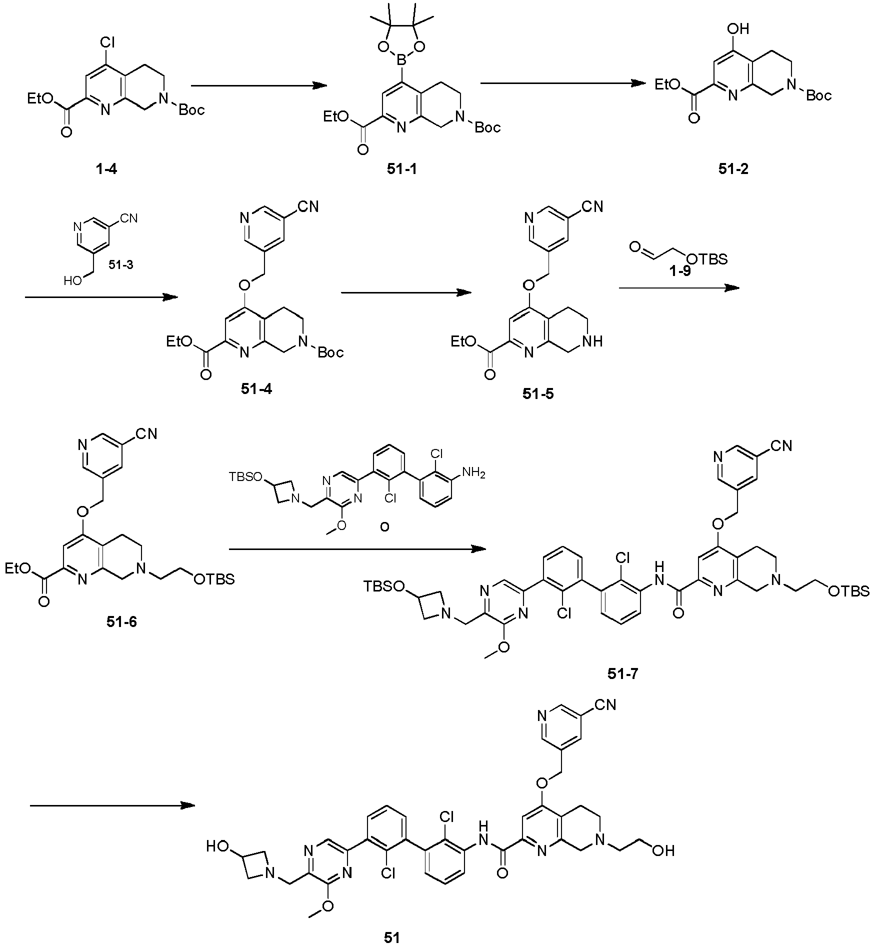

### Step 1

Intermediate 1-4 (1 g, 2.93 mmol) was dissolved in dioxane (20 mL), bis(pinacolato)diboron (968.66 mg, 3.81 mmol), and then potassium acetate (719.94 mg, 7.34 mmol) and [1,1'-bis(diphenylphosphino)ferrocene]dichloropalladium dichloromethane complex (239.62 mg, 293.43 (µmol) were added thereto. The reaction mixture was heated to 90 °C and the reaction was conducted for 14 hours under nitrogen protection. After the reaction was completed, the reaction mixture was filtered, and the filtrate was diluted with ethyl acetate (60 mL) and washed with saturated brine (60 mL). The organic phase was dried over sodium sulfate, filtered and concentrated. The crude product was purified by silica gel column chromatography (petroleum ether: ethyl acetate=15:1) to obtain intermediate 51-1.

¹H NMR (400 MHz, CDCl₃) δ = 8.07 (s, 1 H), 4.61 (s, 2 H), 4.37 - 4.33 (m, 2 H), 3.65 - 3.55 (m, 2 H), 3.10 - 3.06 (m, 2 H), 1.44 (s, 9 H), 1.35 - 1.31 (m, 3 H), 1.17 (s, 12 H).

### Step 2

Intermediate 51-1 (300 mg, 693.94 µmol) was dissolved in tetrahydrofuran (3 mL) and water (1.5 mL), and then sodium perborate tetrahydrate (427.08 mg, 2.78 mmol) was added thereto. The reaction was conducted at room temperature for 1 hour. After the reaction was completed, the pH of reaction mixture was adjusted to 6 with 1 mol/L dilute hydrochloric acid, and then extracted with ethyl acetate (20 mL). The organic phase was dried over sodium sulfate, filtered and concentrated. The crude product was subjected to silica gel thin layer chromatography (ethyl acetate) to obtain intermediate 51-2.

MS-ESI calculated value [M+H]⁺ 323, measured value 323.

### Step 3

Intermediate 51-2 (70 mg, 217.15 µmol) was dissolved in tetrahydrofuran (4 mL), and then 51-3 (32.04 mg, 238.87 µmol), triphenylphosphine (113.91 mg, 434.30 (µmol) and diisopropyl azodiformate (87.82 mg, 434.30 µmol) were added thereto at 0 °C under nitrogen protection. The reaction was conducted at room temperature for 16 hours. Remains of raw materials and the formed product were monitored through LCMS. Triphenylphosphine (56.96 mg, 217.15 (µmol) and diisopropyl azodiformate (43.91 mg, 217.15 µmol) were added thereto at 0 °C, and the reaction was conducted at room temperature for 2 hours. The reaction mixture was heated to 55 °C and the reaction was conducted for 2 hours. The reaction mixture was concentrated and subjected to silica gel thin layer chromatography (petroleum ether: ethyl acetate=1:2) to obtain intermediate 51-4.

MS-ESI calculated value [M+H]⁺ 439, measured value 439. ¹H NMR (400 MHz, CDCl₃) δ = 8.94 - 8.93 (m, 2 H), 8.09 (s, 1 H), 5.30 (s, 2 H), 4.81 (s, 2 H), 4.56 - 4.48 (m, 2 H), 3.74 - 3.71 (m, 2 H), 2.88 - 2.85 (m, 2 H), 1.51-1.45 (m, 12 H).

### Step 4

Intermediate 51-4 (100 mg) was dissolved in ethyl acetate (4 mL), then a solution of hydrogen chloride in ethyl acetate (4 mol/L, 1 mL) was added thereto, and stirred at room temperature for 1 hour. After the reaction was completed, the reaction mixture was concentrated to obtain the hydrochloride of intermediate 51-5.

MS-ESI calculated value [M+H]⁺ 339, measured value 339.

### Step 5

Intermediate 51-5 (85 mg, hydrochloride) was dissolved in methanol (3 mL), and then N,N-diisopropylethylamine (29.31 mg, 226.78 µmol) and intermediate 1-9 (98.82 mg, 566.94 µmol) were added thereto. After the reaction mixture was stirred for half an hour, sodium cyanoborohydride (42.75 mg, 680.33 µmol) was added thereto, and the reaction was conducted at room temperature for 16 hours. After the reaction was completed, the reaction mixture was concentrated, and the crude product was subjected to silica gel thin layer chromatography (petroleum ether: ethyl acetate=1:2) to obtain intermediate 51-6.

MS-ESI calculated value [M+H]⁺ 497, measured value 497.

### Step 6

Intermediate 51-6 (87 mg) and intermediate O (86.01 mg, 157.65 µmol) were dissolved in toluene (2 mL), and then a solution of lithium bis(trimethylsilyl)amide in tetrahydrofuran (1 mol/L, 525.50 (µl) was added thereto at 0 °C under nitrogen protection. The reaction was conducted at room temperature for 1 hour. After the reaction was completed, the reaction mixture was quenched with saturated ammonium chloride solution (20 mL), and extracted with ethyl acetate (20 mL). The organic phase was dried over anhydrous sodium sulfate, filtered and concentrated. The crude product was separated and purified by silica gel thin layer chromatography (petroleum ether: ethyl acetate=1:2) to obtain intermediate 51-7.

MS-ESI calculated value [M+H]⁺ 997, measured value 997.

### Step 7

Intermediate 51-7 (59 mg) was dissolved in ethyl acetate (3 mL), then a solution of hydrogen chloride in ethyl acetate (4 mol/L, 0.8 mL) was added thereto, and stirred at room temperature for 1.5 hours. After the reaction was completed, the reaction mixture was concentrated, diluted with ethyl acetate (20 mL), and washed with saturated sodium bicarbonate solution (20 mL). The organic phase was dried over sodium sulfate, filtered and concentrated. The crude product was subject to preparative plate chromatography (dichloromethane: methanol = 10:1) and high performance liquid chromatography (chromatographic column: column: Unisil 3-100 C18 Ultra 150^{∗}50mm^{∗}3 microns; mobile phase: mobile phase A: formic acid aqueous solution with volume fraction of 0.225%; mobile phase B: acetonitrile; B%: 18%-38%, 10 minutes) to obtain the formate of compound 51.

MS-ESI calculated value [M+H]⁺ 767, measured value 767. ¹H NMR (400 MHz, CD₃OD) δ = 9.02 - 8.98 (m, 1 H), 8.95 (d, J = 1.9 Hz, 1 H), 8.64 - 8.58 (m, 1 H), 8.52 - 8.48 (m, 1 H), 8.44 (br s, 1 H), 8.38 (t, J = 2.0 Hz, 1 H), 7.87 - 7.82 (m, 1 H), 7.75 - 7.69 (m, 1 H), 7.62 - 7.55 (m , 1 H), 7.52 - 7.46 (m, 2 H), 7.21 - 7.15 (m, 1 H), 5.51 (s, 2 H), 4.88-4.69 (m, 1 H), 4.58 (s, 2 H) , 4.47 - 4.41 (m, 2 H), 4.13 (s, 3 H), 4.01 - 3.91 (m, 4 H), 3.84 (t, J = 5.8 Hz, 2 H), 3.05 - 2.94 (m, 4 H)), 2.87 (t, J = 5.8 Hz, 2 H).

### Example 52

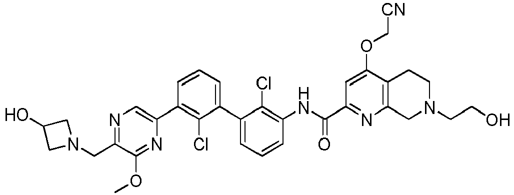

### Synthesis route:

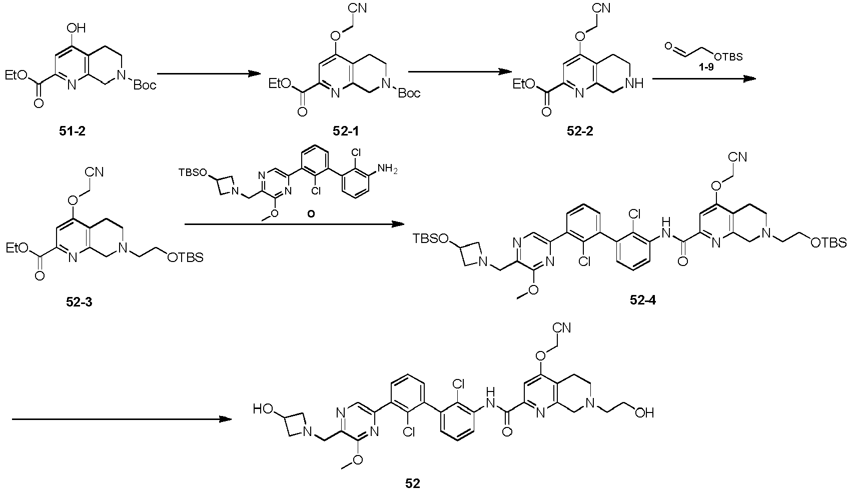

### Step 1

Intermediate 51-2 (350 mg, 1.09 mmol) was dissolved in acetone (10 mL), and then bromoacetonitrile (230.01 mg, 1.92 mmol), cesium carbonate (389.14 mg, 1.19 mmol) were added thereto. The reaction was conducted at 25 °C for 18 hours. After the reaction was completed, the reaction mixture was diluted with ethyl acetate (140 mL) and extracted with water. The organic phase was dried over sodium sulfate, filtered and concentrated to obtain the crude product of intermediate 52-1.

MS-ESI calculated value [M+H]⁺ 362, measured value 362. ¹H NMR (400 MHz, CDCl₃) δ = 7.57 (s, 1 H), 4.95(s, 2 H), 4.79 (s, 2 H), 4.53 (q, *J* =7.11 Hz, 2 H), 3.71 (t, *J* =5.77 Hz, 2 H), 2.80 - 2.87 (m, 2 H), 1.50 (s, 9 H) , 1.46 (t, *J* =7.09 Hz, 3 H)

### Step 2

Intermediate 52-1 (196 mg, 542.35 (µmol) was dissolved in dichloromethane (8 mL), and then trifluoroacetic acid (3.92 mL) was added thereto. The reaction was conducted at room temperature for 1 hour under nitrogen protection. After the reaction was completed, the reaction mixture was concentrated to obtain the crude product of intermediate 52-2.

MS-ESI calculated value [M+H]⁺ 262, measured value 262.

### Step 3

Intermediate 52-2 (200 mg, 532.91 µmol) was dissolved in methanol (12 mL), and then N,N-diisopropylethylamine (68.87 mg, 532.91 µmol) and intermediate 1-9 (232.23 mg, 1.33 mmol) were added thereto. After the reaction mixture was stirred for half an hour, sodium cyanoborohydride (100.47 mg, 1.60 mmol) was added thereto, and the reaction was conducted at 25 °C for 16 hours. After the reaction was completed, the reaction mixture was diluted with ethyl acetate (40 × 2 mL) and washed with water (40 mL). The organic phase was dried over anhydrous sodium sulfate, filtered and concentrated. The residue was subjected to silica gel thin layer chromatography (petroleum ether: ethyl acetate=1:1.2) to obtain intermediate 52-3.

MS-ESI calculated value [M+H]⁺ 420, measured value 420. ¹H NMR (400 MHz, CDCl₃) δ = 7.51 (s, 1 H), 4.91 (s, 2 H), 4.45 - 4.55 (m, 2 H), 3.80 - 3.92 (m, 4 H), 2.86 (br dd , J=4.8, 4.65 Hz, 4 H), 2.76 (t, J=6.4 Hz, 2 H), 1.41 - 1.47 (m, 3 H), 0.91 (s, 9 H), 0.07 - 0.10 (m, 6 H).

### Step 4

Intermediate 52-3 (100 mg, 238.33 µmol) and intermediate O (117.02 mg, 214.50 µmol) were dissolved in tetrahydrofuran (5 mL), and then a solution of lithium bis(trimethylsilyl)amide in tetrahydrofuran (1 mol/L, 714.99 µL) was added thereto. The reaction was conducted at 0 °C for 1 hour. After the reaction was completed, the reaction mixture was quenched with saturated ammonium chloride solution (20 mL), and extracted with ethyl acetate (20 mL). The organic phase was dried over anhydrous sodium sulfate, filtered and concentrated. The crude product was separated and purified by silica gel thin layer chromatography (petroleum ether: ethyl acetate=2:3) to obtain intermediate 52-4.

MS-ESI calculated value [M+H]⁺ 918, measured value 918.

### Step 5

Intermediate 52-4 (61 mg, 66.37 µmol) was dissolved in dichloromethane (3 mL), and then trifluoroacetic acid (4 mL) was added thereto. The reaction was conducted at 25 °C for 12 hours. Remains of raw materials and the formed product were monitored. After trifluoroacetic acid (2 mL) was added, the reaction was conducted at 25 °C for 16 hours. After the reaction was completed, the reaction mixture was concentrated. The crude product was subjected to high performance liquid chromatography (chromatographic column: Phenomenex Gemini-NX C18 75^{∗}30 mm^{∗}3 microns; mobile phase: mobile phase A: formic acid aqueous solution with volume fraction of 0.225 %; mobile phase B: acetonitrile; B%: 10%-40%, 7 minutes) to obtain compound 52.

MS-ESI calculated value [M+H]⁺ 690, measured value 690. ¹H NMR (400 MHz, CD₃OD) δ = 8.62 (dd, J=8.26, 1.25 Hz, 1 H), 8.53 (br s, 1 H), 8.47 (s, 1 H), 7.84 (s, 1 H), 7.72 (dd, J =7.69, 1.56 Hz, 1 H), 7.58 (t, J =7.69 Hz, 1 H), 7.45 - 7.53 (m, 2 H), 7.16 - 7.22 (m, 1 H), 5.29 ( s, 2 H), 4.58 (br s, 1 H), 4.25 - 4.38 (m, 2 H), 4.15 - 4.22 (m, 2 H), 4.11 (s, 3 H), 3.88 (s, 2 H) , 3.82 (t, J =5.6 Hz, 2 H), 3.59 - 3.71 (m, 2 H), 2.90 - 2.97 (m, 4 H), 2.81 (t, J =5.82 Hz, 2 H)

### Example 53

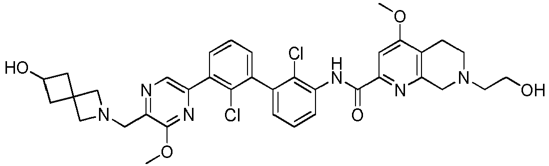

### Synthesis route:

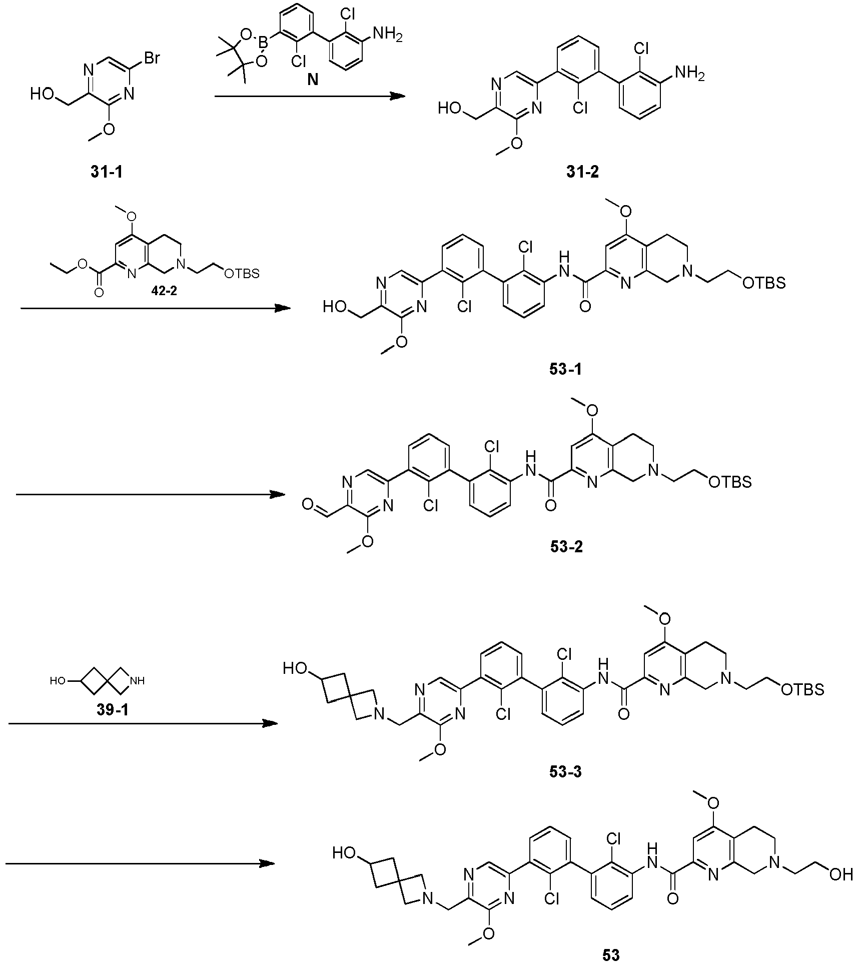

### Step 1

Intermediate 31-1 (3.30 g, 15.1 mmol, 1 equiv) and intermediate N (5.49 g, 15.1 mmol, 1 equiv) were dissolved in dioxane (60 mL) and water (6 mL), and then potassium carbonate (6.25 g, 45.2 mmol, 3 equiv) and [1,1'-bis(diphenylphosphino)ferrocene]dichloropalladium (1.10 g, 1.51 mmol, 0.1 equiv) were added to the reaction mixture. The air in the solution was replaced with nitrogen three times, and the reaction mixture was stirred at 65°C for 4 hours under nitrogen protection. After the reaction was completed, the reaction mixture was diluted with water (100 mL), and extracted with ethyl acetate (50 mL×3). The organic phase was washed with saturated brine (60 mL×2), dried over anhydrous sodium sulfate, filtered, the filtrate was concentrated under reduced pressure. The residue was separated and purified by silica gel column chromatography (petroleum ether: ethyl acetate=20:1-4:1) to obtain compound 31-2.

MS-ESI calculated value [M+H]⁺ 376, measured value 376.

### Step 2

Intermediate 31-2 (200 mg, 506.87 µmol, 1 equiv) and intermediate 42-2 (171.63 mg, 456.18 µmol, 0.9 equiv) were dissolved in tetrahydrofuran (4 mL), and then lithium bis(trimethylsilyl)amide (1 mol/L, 1.77 mL, 3.5 equiv) was added thereto at 0 °C under nitrogen protection. The reaction was conducted at 0 °C for 1 hour. After the reaction was completed, the reaction mixture was quenched with saturated ammonium chloride solution (20 mL), and extracted with ethyl acetate (20 mL). The organic phase was dried over anhydrous sodium sulfate, filtered and concentrated. The crude product was separated and purified by silica gel thin layer chromatography (petroleum ether: ethyl acetate=1:2) to obtain intermediate 53-1.

MS-ESI calculated value [M+H]⁺ 724, measured value 724.

### Step 3

Intermediate 53-1 (130 mg, 186.27 µmol) was dissolved in 1,1-dichloroethane (2 mL), and then manganese dioxide (161.94 mg, 1.86 mmol) was added thereto. The reaction mixture was heated to 90 °C and the reaction was conducted for 2 hours under nitrogen protection. After the reaction was completed, the reaction mixture was filtered and concentrated to obtain Intermediate 53-2.

MS-ESI calculated value [M+H]⁺ 724, measured value 724.

### Step 4

Intermediate 53-2 (130 mg, 179.87 (µmol) was dissolved in dichloromethane (3 mL), and then intermediate 39-1 (40.37 mg, 269.81 µmol, hydrochloride), diisopropylethylamine (427.08 mg, 2.78 mmol) and sodium triacetoxyborohydride (114.37 mg, 539.61 µmol) were added thereto. The reaction was conducted at room temperature for 0.5 hours. After the reaction was completed, the reaction was quenched with water (10 mL), diluted with dichloromethane (10 mL), and extracted with dichloromethane (10 mL×2). The organic phase was washed with saturated brine (5 mL×2), dried over anhydrous sodium sulfate, filtered, and concentrated. The crude product was subjected to high performance liquid chromatography (chromatographic column: Xtimate C18 150^{∗}25 mm^{∗}10 microns; mobile phase: mobile phase A: formic acid aqueous solution with volume fraction of 0.225 %; mobile phase B: acetonitrile; B%: 9%-39%, 10 minutes) to obtain intermediate 53-3.

MS-ESI calculated value [M+H]⁺ 821, measured value 821.

### Step 5

A solution of hydrogen chloride in ethyl acetate (4 mol/L, 1 mL) was added to intermediate 53-3 (20 mg, 24.39 µmol) dissolved in dichloromethane (2 mL), and the reaction mixture was stirred at room temperature for 0.25 hour. After the reaction was completed, the reaction mixture was concentrated. The crude product was subjected to high performance liquid chromatography (chromatographic column: Xtimate C18 150^{∗}25mm^{∗}10um; mobile phase: mobile phase A: formic acid aqueous solution with volume fraction of 0.225 %; mobile phase B: acetonitrile; B%: 9%-39%, 10 minutes) to obtain compound 53.

MS-ESI calculated value [M+H]⁺ 705, measured value 705. ¹HNMR (400 MHz, MeOD) δ = 8.62 (dd, J = 1.3, 8.2 Hz, 1H), 8.47 - 8.41 (m, 1H), 7.76 - 7.67 (m, 2H), 7.57 (t, J = 7.6 Hz, 1H), 7.53 - 7.43 (m, 2H), 7.17 (dd, J = 1.4, 7.7 Hz, 1H), 4.15 (s, 2H), 4.12 (br d, J = 6.9 Hz, 1H), 4.09 (s, 3H), 4.04 (s, 3H), 3.87 - 3.79 (m, 6H), 3.77 (s, 2H), 2.88 (br dd, J = 4.8, 13.7 Hz, 3H), 2.88 - 2.83 (m, 1H), 2.79 (t, J = 5.8 Hz, 2H), 2.58 (ddd, J = 2.9, 7.0, 9.9 Hz, 2H), 2.11 (ddd, J = 2.9, 7.3, 10.0 Hz, 2H)

### Example 54

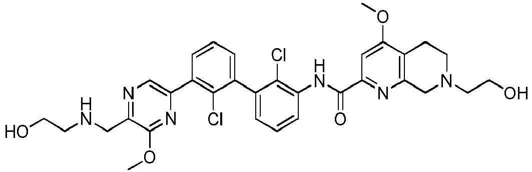

### Synthesis route:

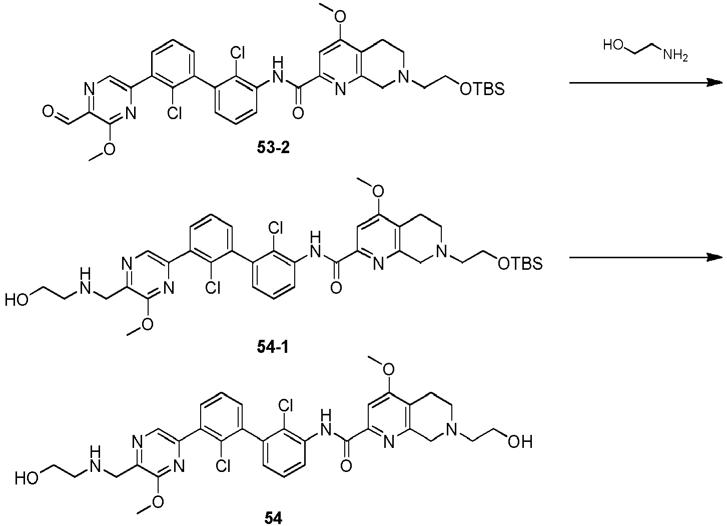

### Step 1

Intermediate 39-1 (40.37 mg, 269.81 µmol, hydrochloride) was replaced with ethanolamine (15.21 mg, 249.06 µmol), and the method was the same as that in example53. The reaction was conducted at room temperature for 2 hours. After the reaction was completed, the reaction mixture was quenched with water (10 mL), diluted with dichloromethane (5 mL), and extracted with dichloromethane (10 mL×3). The organic phase was washed with saturated brine (20 mL×2), dried over anhydrous sodium sulfate, filtered, and concentrated. The crude product was purified by silica gel thin layer chromatography (dichloromethane: methanol=8:1) to obtain the crude product of intermediate 54-1.

MS-ESI calculated value [M+H]⁺ 767, measured value 767.

### Step 2

Compound intermediate 53-3 (20 mg, 24.39 (µmol) was replaced with 54-1 (75 mg, 97.68 µmol), and the method was the same as that in example 53. After the reaction was completed, the reaction mixture was concentrated. The crude product was subjected to high performance liquid chromatography (chromatographic column: Xtimate C18 150^{∗}25mm^{∗}10um; mobile phase: mobile phase A: formic acid aqueous solution with volume fraction of 0.225 %; mobile phase B: acetonitrile; B%: 8%-38%, 10 minutes) to obtain compound 54.

MS-ESI calculated value [M+H]⁺ 653, measured value 653. ¹H NMR (400 MHz, CD₃OD) δ = 8.62 (dd, J = 1.3, 8.2 Hz, 1H), 8.47 - 8.41 (m, 1H), 7.76 - 7.67 (m, 2H), 7.57 (t, J = 7.6 Hz) , 1H), 7.53 - 7.43 (m, 2H), 7.17 (dd, J = 1.4, 7.7 Hz, 1H), 4.15 (s, 2H), 4.12 (br d, J = 6.9 Hz, 1H), 4.09 (s , 3H), 4.04 (s, 3H), 3.87 - 3.79 (m, 6H), 3.77 (s, 2H), 2.88 (br dd, J = 4.8, 13.7 Hz, 3H), 2.88 - 2.83 (m, 1H) , 2.79 (t, J = 5.8 Hz, 2H), 2.58 (ddd, J = 2.9, 7.0, 9.9 Hz, 2H), 2.11 (ddd, J = 2.9, 7.3, 10.0 Hz, 2H)

### Example 55

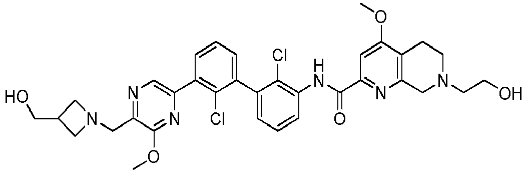

### Synthesis route:

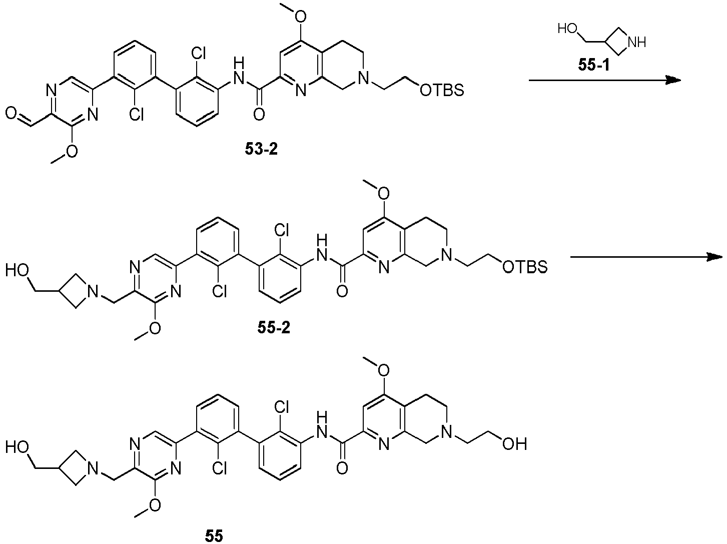

### Step 1

Intermediate 39-1 (40.37 mg, 269.81 µmol, hydrochloride) was replaced with intermediate 55-1 (30.78 mg, 249.06 µmol, hydrochloride), and the method was the same as that in example 53. The reaction was conducted at room temperature for 2 hours. After the reaction was completed, the reaction mixture was quenched with water (10 mL), diluted with dichloromethane (5 mL), and extracted with dichloromethane (10 mL×3). The organic phase was washed with saturated brine (20 mL×2), dried over anhydrous sodium sulfate, filtered, and concentrated. The crude product was purified by silica gel thin layer chromatography (dichloromethane: methanol=8:1) to obtain the crude product of intermediate 55-2.

MS-ESI calculated value [M+H]⁺ 793, measured value 793.

### Step 2

Intermediate 53-3 (20 mg, 24.39 µmol) was replaced with intermediate 55-2 (100 mg, 125.97 (µmol), and the method was the same as that in example 53. After the reaction was completed, the reaction mixture was concentrated. The crude product was subjected to high performance liquid chromatography (chromatographic column: Xtimate C18 150^{∗}25 mm^{∗}10 microns; mobile phase: mobile phase A: formic acid aqueous solution with volume fraction of 0.225 %; mobile phase B: acetonitrile; B%: 8%-38%, 10 minutes) to obtain compound 55.

MS-ESI calculated value [M+H]⁺ 680, measured value 680. ¹H NMR (400 MHz, CD₃OD) δ = 8.62 (dd, J = 1.4, 8.3 Hz, 1H), 8.46 - 8.41 (m, 1H), 7.74 - 7.68 (m, 2H), 7.57 (t, J = 7.6 Hz) , 1H), 7.52 - 7.42 (m, 2H), 7.17 (dd, J = 1.3, 7.6 Hz, 1H), 4.21 (s, 2H), 4.09 (s, 3H), 4.04 (s, 3H), 3.92 (t, J = 8.8 Hz, 2H), 3.84 - 3.77 (m, 4H), 3.72 - 3.63 (m, 4H), 2.88 (br dd, J = 4.8, 13.3 Hz, 5H), 2.78 (t, J = 5.8 Hz, 2H)

### Example 56

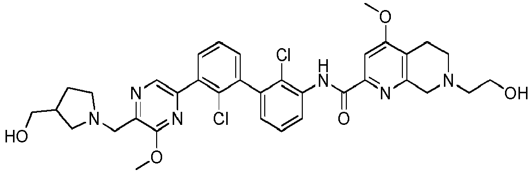

### Synthesis route:

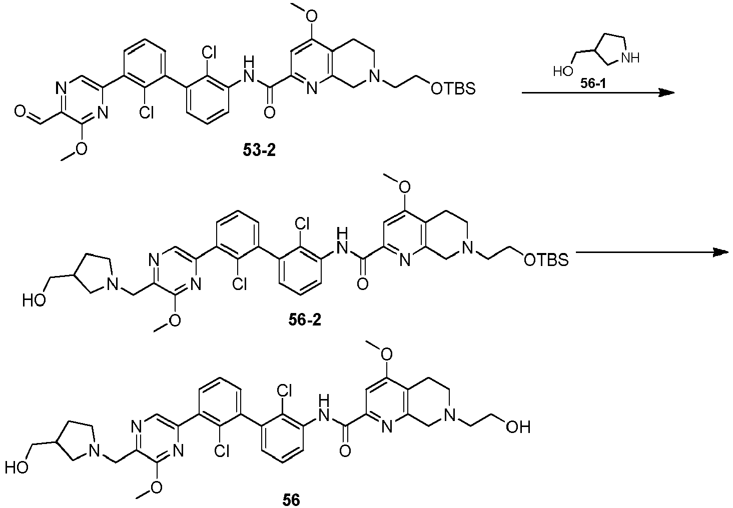

### Step 1

Intermediate 39-1 (40.37 mg, 269.81 µmol, hydrochloride) was replaced with intermediate 56-1 (34.27 mg, 249.06 µmol, hydrochloride), and the method was the same as that in example 53. After the reaction was completed, the reaction mixture was diluted with dichloromethane (20 × 2 mL) and washed with water (20 mL). The organic phase was dried over anhydrous sodium sulfate, filtered and concentrated. The residue was subjected to silica gel thin layer chromatography (dichloromethane: methanol=10:1) to obtain intermediate 56-2.

MS-ESI calculated value [M+H]⁺ 807, measured value 807.

### Step 2

Intermediate 53-3 (20 mg, 24.39 µmol) was replaced by Intermediate 56-2 (64 mg, 79.22 µmol), and the method was the same as that in example 53. After the reaction was completed, the reaction mixture was concentrated. The crude product was subjected to high performance liquid chromatography (chromatographic column: Phenomenex Gemini-NX C18 75^{∗}30 mm^{∗}3 microns; mobile phase: mobile phase A: formic acid aqueous solution with volume fraction of 0.225 %; mobile phase B: acetonitrile; B%: 10%-40 %, 7 minutes) to obtain compound 56.

MS-ESI calculated value [M+H]⁺ 693, measured value 693. ¹H NMR (400 MHz, CD₃OD) δ = 8.57 - 8.65 (m, 1 H), 8.49 (s, 1 H), 7.70 - 7.77 (m, 2 H), 7.58 (t, J=7.63 Hz, 1 H) , 7.41 - 7.53 (m, 2 H), 7.15 - 7.22 (m, 1 H), 4.19 (s, 2 H), 4.11 (s, 3 H), 4.04 (s, 3 H), 3.77 - 3.86 (m , 4 H), 3.50 - 3.63 (m, 2 H), 3.23 (br t, J=9.13 Hz, 1 H), 3.01 - 3.15 (m, 2 H), 2.83 - 2.96 (m, 5 H), 2.76 - 2.82 (m, 2 H), 2.48 - 2.60 (m, 1 H), 2.05 - 2.17 (m, 1 H), 1.66 - 1.78 (m, 1 H).

### Example 57

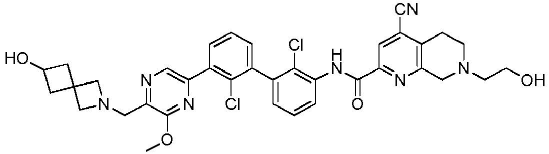

### Synthesis route:

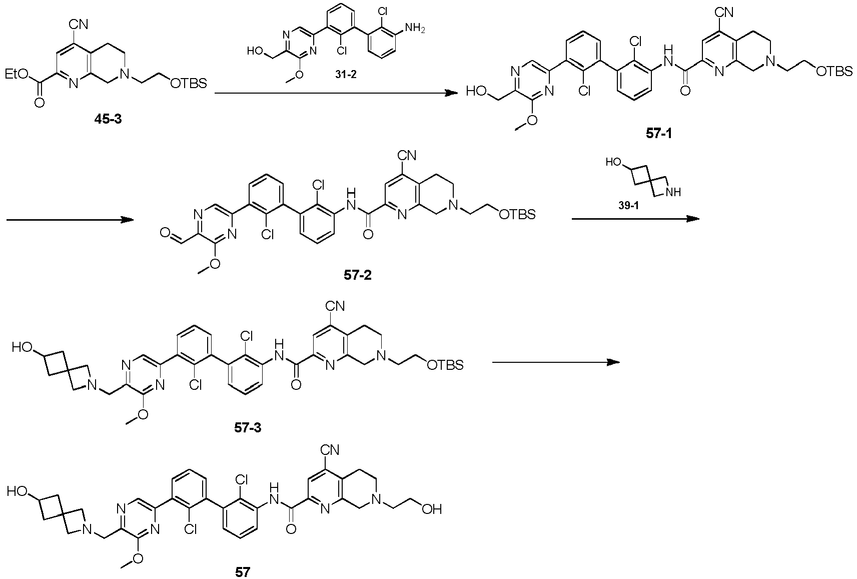

### Step 1

Intermediates 45-3 (200 mg, 513 µmol, 1 equiv) and 31-2 (186 mg, 462 µmol, 0.9 equiv) were dissolved in tetrahydrofuran (5 mL), then a solution of lithium bis(trimethylsilyl)amide in n-hexane (1 mol/L, 1.8 mL, 3.5 equiv) was added slowly to the reaction mixture at 0°C, and stirred at 30°C for 1 hour. After the reaction was completed, the reaction mixture was slowly added to water (10 mL) for dilution, and then extracted with dichloromethane (10 mL×3). The combined organic phase was washed with saturated brine (15 mL×3), dried over anhydrous sodium sulfate, filtered, and the filtrate was concentrated under reduced pressure. The residue was subjected to silica gel thin-layer chromatography (petroleum ether: ethyl acetate=2:1) to obtain compound 57-1.

MS-ESI calculated value [M+H]⁺ 719, measured value 719.

### Step 2

Intermediate 57-1 (170 mg, 218 µmol, 1 equiv) was dissolved in 1,2 dichloroethane (3 mL), then manganese dioxide (114 mg, 1.31 mmol, 6 equiv) was slowly added to the reaction mixture, and stirred at 90°C for 12 hours. After the reaction was completed, the reaction mixture was filtered, and the filtrate was concentrated to obtain compound 57-2.

MS-ESI calculated value [M+H]⁺ 717, measured value 717.

### Step 3

Intermediates 57-2 (150 mg, 209 µmol, 1 equiv) and 39-1 (46.9 mg, 313 µmol, 85.6 µl, 1.5 equiv, hydrochloride) were dissolved in dichloromethane (3 mL), and then N,N-diisopropylethylamine (81.0 mg, 627 µmol, 109 µl, 3 equiv) and sodium triacetoxyborohydride (133 mg, 627 µmol, 3 equiv) were added to the reaction mixture, stirred at 30 °C for 12 hours. After the reaction was completed, water (5 mL) was added, the reaction mixture was extracted with dichloromethane (8 mL×3). The organic phase was washed with saturated brine (10 mL×2), dried over anhydrous sodium sulfate, filtered, and concentrated. The concentrated residue was subjected to silica gel thin-layer chromatography (petroleum ether: ethyl acetate: ethanol=4:3:1) to obtain compound 57-3.

MS-ESI calculated value [M+H]⁺ 814, measured value 814.

### Step 4

Intermediate 57-3 (100 mg, 123 µmol, 1 equiv) was dissolved in dichloromethane (3 mL), and then a solution of hydrogen chloride in ethyl acetate (4 mol/L, 1.25 mL, 40.7 equiv) was added to the reaction mixture, stirred at 30°C for 1 hour. After the reaction was completed, the reaction mixture was concentrated, and the crude product was subjected to high performance liquid chromatography (chromatographic column: Unisil 3-100 C18 Ultra 150^{∗}50 mm^{∗}3 microns; mobile phase: mobile phase A: formic acid aqueous solution with volume fraction of 0.225 %; mobile phase B: acetonitrile; B%: 15%-35%, 10 minutes) to obtain compound 57.

MS-ESI calculated value [M+H]⁺ 637, measured value 637. ¹HNMR (400 MHz, CD₃OD) δ =8.58 (dd, J=8.3, 1.54 Hz, 1 H), 8.48 (s, 1 H), 8.36 (s, 1 H), 7.72 (dd, J=7.7, 1.66 Hz , 1 H), 7.59 (t, J=7.64 Hz, 1 H), 7.55 - 7.46 (m, 2 H), 7.21 (dd, J=7.7, 1.48 Hz, 1 H), 4.45 (s, 2 H) , 4.21 - 4.04 (m, 8 H), 3.96 (s, 2 H), 3.81 (t, J=5.69 Hz, 2 H), 3.21 (br t, J=5.62 Hz, 2 H), 3.01 (t, J=5.87 Hz, 2 H), 2.82 (t, J=5.69 Hz, 2 H), 2.65 (ddd, J=9.93, 7.00, 3.00 Hz, 2 H), 2.22 - 2.13 (m, 2 H)

### Example 58

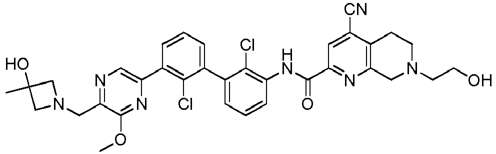

### Synthesis route:

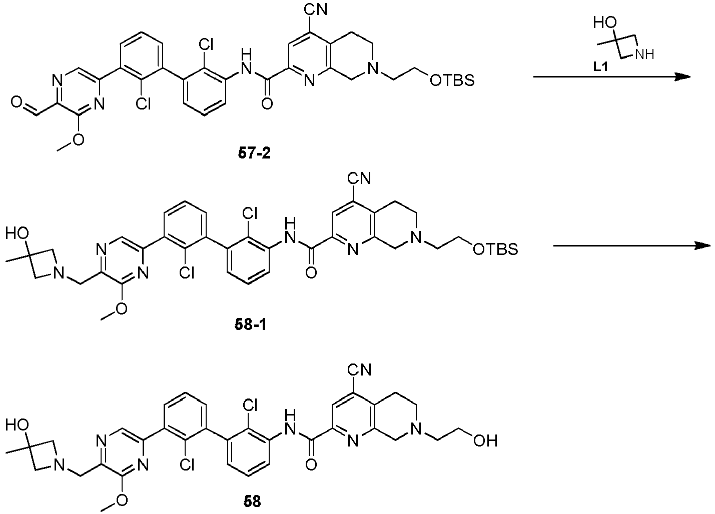

### Step 1

Intermediate 39-1 (40.37 mg, 269.81 µmol, hydrochloride) was replaced with intermediate L1 (18.1 mg, 146 µmol, hydrochloride), and the method was the same as that in example 57. After the reaction was completed, water (5 mL) was added, and the reaction mixture was extracted with dichloromethane (8 mL×3). The organic phase was washed with saturated brine (10 mL×2), dried over anhydrous sodium sulfate, filtered, and concentrated. The concentrated residue was separated and purified by silica gel thin-layer chromatography (petroleum ether: ethyl acetate: ethanol=4:3:1) to obtain compound 58-1.

MS-ESI calculated value [M+H]⁺ 788, measured value 788.

### Step 2

Intermediate 58-1 (33.0 mg, 39.0 µmol, 1 equiv) was dissolved in dichloromethane (0.5 mL), and then trifluoroacetic acid (770 mg, 6.75 mmol, 500 µL, 173 equiv) was added to the reaction mixture, stirred at 30°C for 12 hours. After the reaction was completed, the reaction mixture was concentrated, and the crude product was subjected to high performance liquid chromatography (chromatographic column: Unisil 3-100 C18 Ultra 150^{∗}50 mm^{∗}3 microns; mobile phase: mobile phase A: formic acid aqueous solution with volume fraction of 0.225 %; mobile phase B: acetonitrile; B%: 15%-35%, 10 minutes) to obtain compound 58.

MS-ESI calculated value [M+H]⁺ 674, measured value 674. ¹HNMR (400 MHz, CD₃OD) δ =8.58 (dd, J=8.3, 1.48Hz, 1 H), 8.47 (s, 1 H), 8.36 (s, 1 H), 7.75-7.70(m, 1 H), 7.58 (t, J=7.64 Hz, 1 H), 7.55 - 7.44 (m, 2 H), 7.21 (dd, J=7.6, 1.54 Hz, 1 H), 4.32 (s, 2 H), 4.10 (s, 3 H), 3.96 (s, 2 H), 3.92 (br d, J=9.54 Hz, 2 H), 3.81 (t, J=5.70 Hz, 2 H), 3.72 (br d, J=8.94 Hz, 2 H), 3.24 - 3.17 (m, 2 H), 3.04 - 2.98 (m, 2 H), 2.82 (t, J=5.70 Hz, 2 H), 1.55 (s, 3 H)

### Example 59

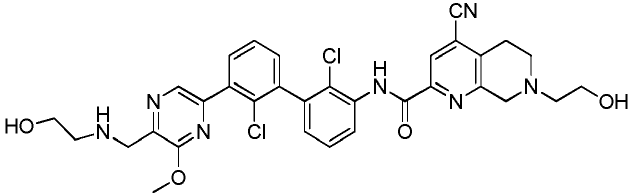

### Synthesis route:

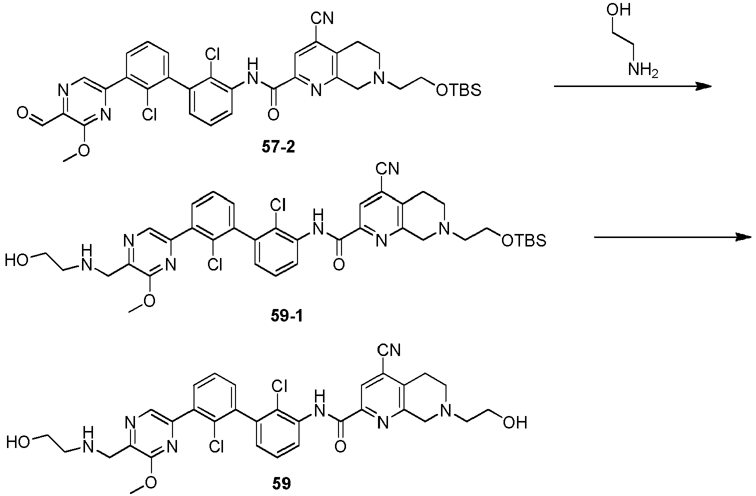

### Step 1

Intermediate 39-1 (40.37 mg, 269.81 µmol, hydrochloride) was replaced with ethanolamine (8.94 mg, 146 µmol, 8.85 µl), and the method was the same as that in example 57. After the reaction was completed, water (5 mL) was added, and the reaction mixture was extracted with dichloromethane (8 mL×3). The organic phase was washed with saturated brine (10 mL×2), dried over anhydrous sodium sulfate, filtered, and concentrated. The concentrated residue was separated and purified by silica gel thin-layer chromatography (dichloromethane: methanol = 10: 1) to obtain compound 59-1.

MS-ESI calculated value [M+H]⁺ 762, measured value 762.

### Step 2

Intermediate 58-1 (20 mg, 24.39 µmol) was replaced with intermediate 59-1 (50.0 mg, 59.9 µmol, 1 equiv), and the method was the same as that in example 58. After the reaction was completed, the reaction mixture was concentrated, and the crude product was subjected to high performance liquid chromatography (chromatographic column: Unisil 3-100 C18 Ultra 150^{∗}50 mm^{∗}3 microns; mobile phase: mobile phase A: formic acid aqueous solution with volume fraction of 0.225 %; mobile phase B: acetonitrile; B%: 15%-35%, 10 minutes) to obtain compound 59.

MS-ESI calculated value [M+H]⁺ 648, measured value 648. ¹HNMR (400 MHz, CD₃OD) δ =8.58 (dd, J=8.3, 1.54 Hz, 1 H), 8.53 (s, 1 H), 8.36 (s, 1 H), 7.74 (dd, J=7.7, 1.60 Hz , 1 H), 7.60 (t, J=7.70 Hz, 1 H), 7.55 - 7.44 (m, 2 H), 7.24 - 7.18 (m, 1 H), 4.47 - 4.37 (m, 2 H), 4.14 ( s, 3 H), 3.96 (s, 2 H), 3.91 - 3.85 (m, 2 H), 3.81 (t, J=5.70 Hz, 2 H), 3.28 - 3.23 (m, 2 H), 3.21 (t, J=5.70 Hz, 2 H), 3.05 - 2.98 (m, 2 H), 2.82 (t, J=5.62 Hz, 2 H)

### Example 60

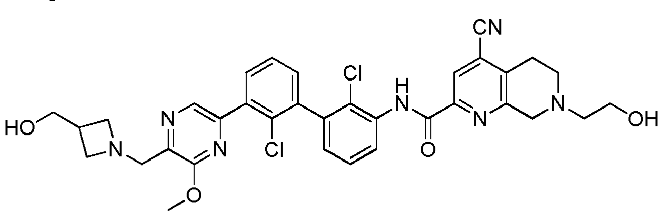

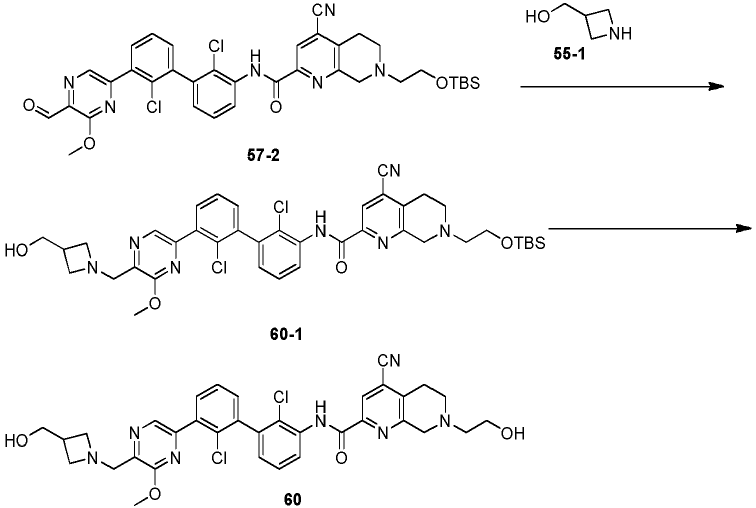

### Step 1

Intermediate 39-1 (40.37 mg, 269.81 µmol, hydrochloride) was replaced with intermediate 55-1 (18.08 mg, 146.30 µmol, 1.5 equiv, hydrochloride), and the method was the same as that in example 57. After the reaction was completed, the reaction mixture was diluted with water (5 mL), and extracted with ethyl acetate (3 mL×3). The combined organic phase was washed with saturated brine (5 mL×2), dried over anhydrous sodium sulfate, and filtered and concentrated under reduced pressure. The residue was subjected to silica gel thin-layer chromatography (petroleum ether: ethyl acetate: ethanol=4:3:1) to obtain compound 60-1.

MS-ESI calculated value [M+H]⁺ 788, measured value 788.

### Step 2

Intermediate 58-1 (20 mg, 24.39 µmol) was replaced with intermediate 60-1 (50.0 mg, 59.9 µmol, 1 equiv), and the method was the same as that in example 58. After the reaction was completed, the reaction mixture was filtered and concentrated under reduced pressure. The obtained residue was subjected to high performance liquid chromatography (chromatographic column: Phenomenex Synergi C18 150^{∗}25mm^{∗}10um; mobile phase: mobile phase A: formic acid aqueous solution with volume fraction of 0.225 %; mobile phase B: acetonitrile; B%: 6%-39%, 11 minutes) to obtain compound 60.

MS-ESI calculated value [M+H]⁺ 674, measured value 674 ¹H NMR (400 MHz, CD₃OD) δ = 8.57 (dd, J = 1.5, 8.3 Hz, 1H), 8.51 (s, 1H), 8.38 (s, 1H), 7.73 (dd, J = 1.6, 7.7 Hz, 1H)), 7.60 (t, J = 7.6 Hz, 1H), 7.55 - 7.43 (m, 2H), 7.21 (dd, J = 1.5, 7.7 Hz, 1H), 4.61 (br s, 4H), 4.45 - 4.28 (m , 2H), 4.14 (s, 3H), 4.03 (s, 2H), 3.83 (t, J = 5.6 Hz, 2H), 3.72 (br s, 2H), 3.26 - 3.21 (m, 2H), 3.17 - 3.11 (m, 1H), 3.10 - 3.04 (m, 2H), 2.89 (t, J = 5.6 Hz, 2H)

### Example 61

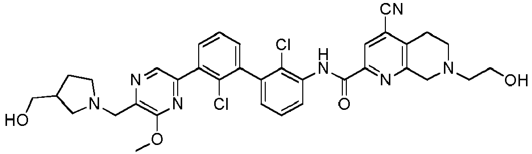

### Synthesis route:

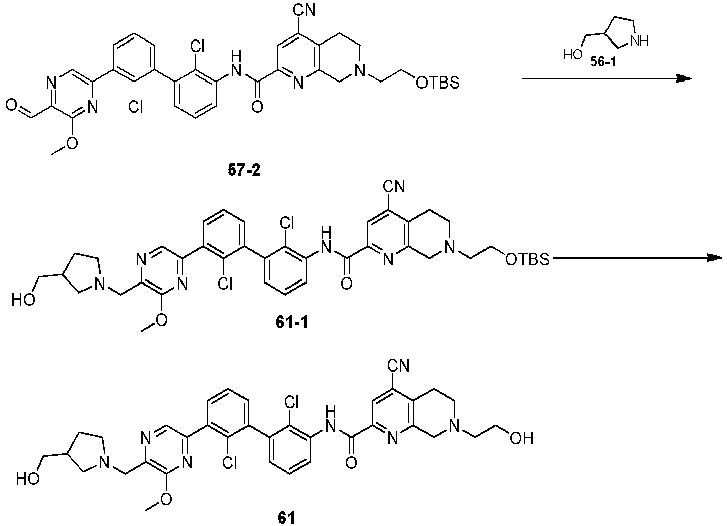

### Step 1

Intermediate 39-1 (40.37 mg, 269.81 µmol, hydrochloride) was replaced with intermediate 56-1 (28.76 mg, 209.00 µmol, hydrochloride), and the method was the same as that in example 57. After the reaction was completed, the reaction mixture was diluted with dichloromethane (20 mL), and washed with saturated brine (20 mL). The organic phase was dried over anhydrous sodium sulfate, filtered and concentrated. The residue was subjected to silica gel thin layer chromatography (dichloromethane: methanol=10: 1) to obtain intermediate 61-1 (32 mg, 38.14% yield).

MS-ESI calculated value [M+H]⁺ 802, measured value 802.

### Step 2

Intermediate 58-1 (20 mg, 24.39 µmol) was replaced with intermediate 61-1 (32 mg, 39.86 µmol), and the method was the same as that in example 58. After the reaction was completed, the reaction mixture was concentrated. The crude product was subjected to high performance liquid chromatography (chromatographic column: column: Unisil 3-100 C18 Ultra 150^{∗}50mm^{∗}3µm; mobile phase: mobile phase A: formic acid aqueous solution with volume fraction of 0.225 %; mobile phase B: acetonitrile; B%: 15%-35%, 10 minutes) to obtain compound 61.

MS-ESI calculated value [M+H]⁺ 688, measured value 688. ¹H NMR (400 MHz, CD₃OD) δ = 8.56 - 8.61 (m, 1 H), 8.52 (s, 1 H), 8.36 (s, 1 H), 7.70 - 7.77 (m, 1 H), 7.56 - 7.63 (m, 1 H), 7.45 - 7.54 (m, 2 H), 7.18 - 7.23 (m, 1 H), 4.40 (s, 2 H), 4.12 (s, 3 H), 3.96 (s, 2 H), 3.77 - 3.85 (m, 2 H), 3.55 - 3.67 (m, 2 H), 3.38 - 3.46 (m, 1 H), 3.27 - 3.32 (m, 2 H), 3.18 - 3.23 (m, 2 H), 3.05 - 3.16 (m, 1 H), 2.97 - 3.03 (m, 2 H), 2.79 - 2.85 (m, 2 H), 2.54 - 2.68 (m, 1 H), 2.11 - 2.26 (m, 1 H), 1.77 - 1.90 (m, 1 H).

### Example 62

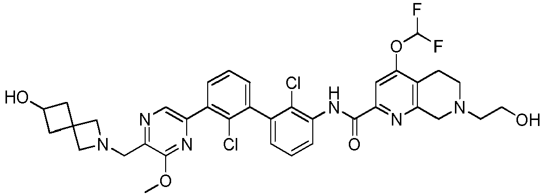

### Synthesis route:

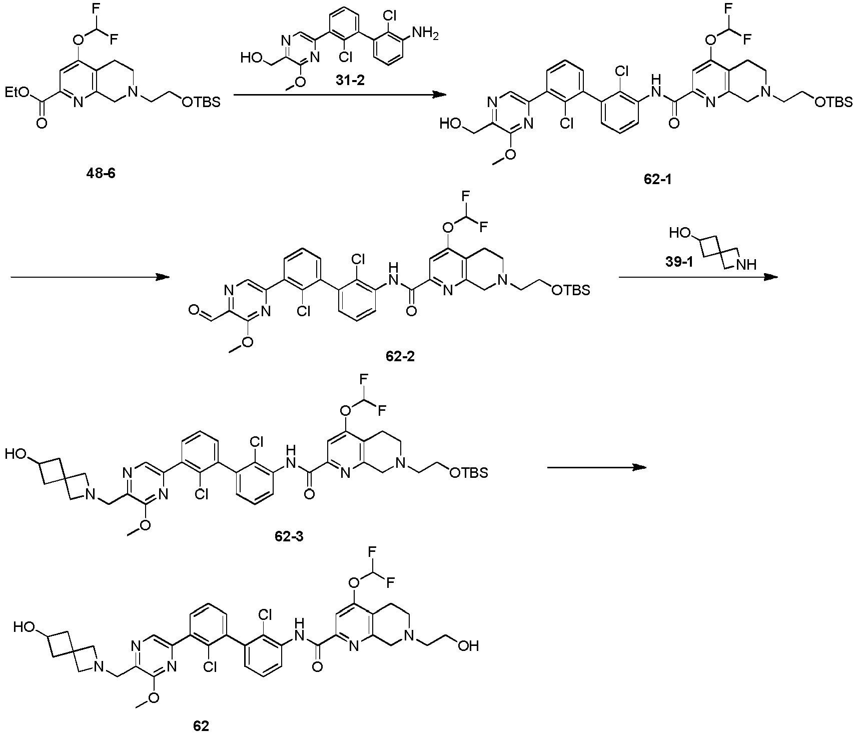

### Step 1

Compound 48-6 (1.2 g, 2.79 mmol, 1 equiv) and compound 31-2 (838.88 mg, 2.23 mmol, 0.8 equiv) were dissolved in tetrahydrofuran (15 mL), and then a solution of lithium bis(trimethylsilyl)amide in tetrahydrofuran (1 mol/L, 9.75 mL, 3.5 equiv) was added to the reaction mixture at 0 °C, stirred at 25°C for 1 hour. After the reaction was completed, the reaction mixture was poured into saturated aqueous ammonium chloride solution (50 mL) at 0 °C, then diluted with water (30 mL), and extracted with ethyl acetate (50 mL×3). The combined organic phase was washed with saturated brine (20 mL × 2), dried over anhydrous sodium sulfate, filtered and concentrated under reduced pressure. The residue was subjected to silica gel column chromatography (petroleum ether: ethyl acetate = 20: 1 to 5: 1) to obtain compound 62- 1.

MS-ESI calculated value [M+H]⁺ 760, measured value 760.

### Step 2

Compound 62-1 (450 mg, 591.54 µmol, 1 equiv) was dissolved in dichloroethane (10 mL), and then manganese dioxide (257.13 mg, 2.96 mmol, 5 equiv) was added thereto. The reaction was conducted at 80 °C for 12 hours. After the reaction was completed, the reaction mixture was filtered and concentrated under reduced pressure to obtain the crude product of compound 62-2.

MS-ESI calculated value [M+H]⁺ 758, measured value 758.

### Step 3

Compound 62-2 (150 mg, 197.70 µmol, 1 equiv) was dissolved in dichloromethane (2 mL), and then compound 39-1 (44.37 mg, 296.56 µmol, 1.5 equiv, hydrochloride) and N,N-diisopropylethylamine (38.33 mg, 296.56 µmol, 51.65 µl, 1.5 equiv) were added thereto. The reaction mixture was stirred at 25 °C for 0.5 hours, then sodium triacetoxyborohydride (125.70 mg, 593.11 µmol, 3 equiv) was added, and the reaction was conducted at 25°C for 1.5 hours. After the reaction was completed, the reaction mixture was diluted with water (5 mL), and extracted with ethyl acetate (3 mL×3). The combined organic phase was washed with saturated brine (5 mL×2), dried over anhydrous sodium sulfate, filtered and concentrated under reduced pressure. The residue was subjected to silica gel thin layer chromatography (petroleum ether: ethyl acetate: ethanol=4:3:1) to obtain compound 62-3.

MS-ESI calculated value [M+H]⁺ 855, measured value 855.

### Step 4

Compound 62-3 (150 mg, 175.26 µmol, 1 equiv) was dissolved in dichloromethane (1.5 mL), and then a solution of hydrogen chloride in ethyl acetate (4 mol/L, 0.5 mL, 11.41 equiv) was added thereto. The reaction was conducted at 25 °C for 0.5 hours. After the reaction was completed, the reaction mixture was filtered and concentrated under reduced pressure. The residue was subjected to high performance liquid chromatography (chromatographic column: Unisil 3-100 C18 Ultra 150^{∗}50mm^{∗}3um; mobile phase: mobile phase A: formic acid aqueous solution with volume fraction of 0.225 %; mobile phase B: acetonitrile; B%: 18%-38%, 10 minutes) to obtain the formate of compound 62.

MS-ESI calculated value [M+H]⁺ 741, measured value 741. ¹H NMR (400 MHz, CD₃OD) δ = 8.60 (dd, J = 1.5, 8.3 Hz, 1H), 8.48 (s, 2H), 7.88 (s, 1H), 7.72 (dd, J = 1.7, 7.7 Hz, 1H), 7.59 (t, J = 7.7 Hz, 1H), 7.53 - 7.45 (m, 2H), 7.32 - 7.06 (m, 2H), 4.50 (s, 2H), 4.24 - 4.16 (m, 3H), 4.15 ( s, 2H), 4.12 (s, 3H), 3.90 (s, 2H), 3.82 (t, J = 5.8 Hz, 2H), 2.96 (s, 4H), 2.81 (t, J = 5.8 Hz, 2H), 2.72 - 2.55 (m, 2H), 2.28 - 2.10 (m, 2H)

### Example 63

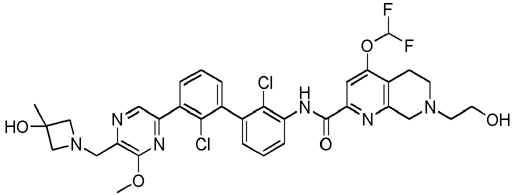

### Synthesis route:

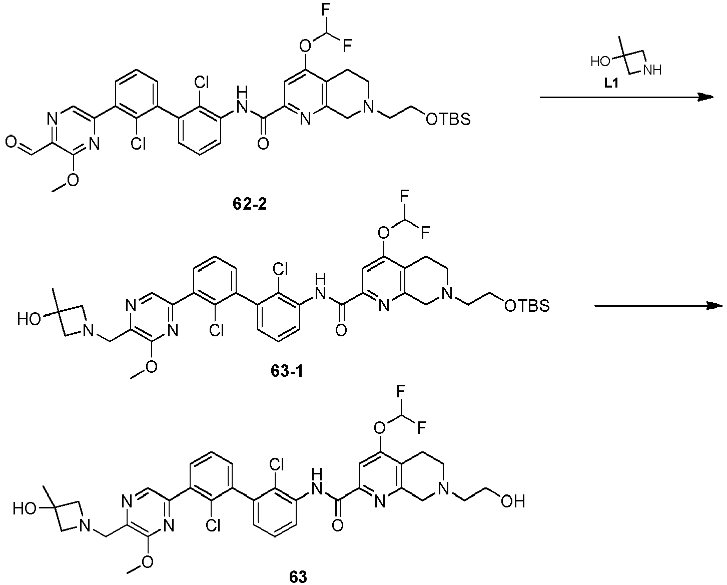

### Step 1

Compound intermediate 39-1 (40.37 mg, 269.81 µmol, hydrochloride) was replaced with intermediate L1 (68.41 mg, 553.57 µmol, hydrochloride), and the method was the same as that in example 53. After the reaction was completed, the reaction mixture was diluted with dichloromethane (20 mL), and washed with saturated brine (20 mL). The organic phase was dried over anhydrous sodium sulfate, filtered and concentrated. The residue was subjected to silica gel thin layer chromatography (dichloromethane: methanol=10:1) to obtain intermediate 63-1.

MS-ESI calculated value [M+H]⁺ 829, measured value 829.

### Step 2

Intermediate 53-3 (20 mg, 24.39 µmol) was replaced with intermediate 63-1 (71 mg, 85.56 µmol), and the method was the same as that in example 53. After the reaction was completed, the reaction mixture was concentrated. The crude product was subjected to high performance liquid chromatography (chromatographic column: column: Unisil 3-100 C18 Ultra 150^{∗}50mm^{∗}3µm; mobile phase: mobile phase A: formic acid aqueous solution with volume fraction of 0.225 %; mobile phase B: acetonitrile; B%: 15%-35%, 10 minutes) to obtain the formate of compound 63.

MS-ESI calculated value [M+H]⁺ 715, measured value 715. ¹H NMR (400 MHz, CD₃OD) δ = 8.58 - 8.61 (m, 1 H), 8.50 (s, 1 H), 7.88 (s, 1 H), 7.71-7.72 (m, 1 H), 7.56-7.59 (m, 1 H), 7.46 - 7.50 (m, 2 H), 7.19 -7.28 (m, 2 H), 4.59 (s, 2 H), 4.17 - 4.24 (m, 2 H), 4.12 (s, 3 H) ), 4.01 - 4.07 (m, 2 H), 3.93 (s, 2 H), 3.78 - 3.85 (m, 2 H), 2.92 - 3.02 (m, 4 H), 2.84 (t, J = 5.8 Hz, 2 H), 1.58 (s, 3 H)

### Example 64

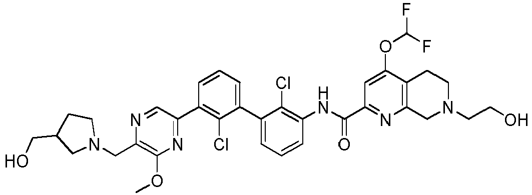

### Synthesis route:

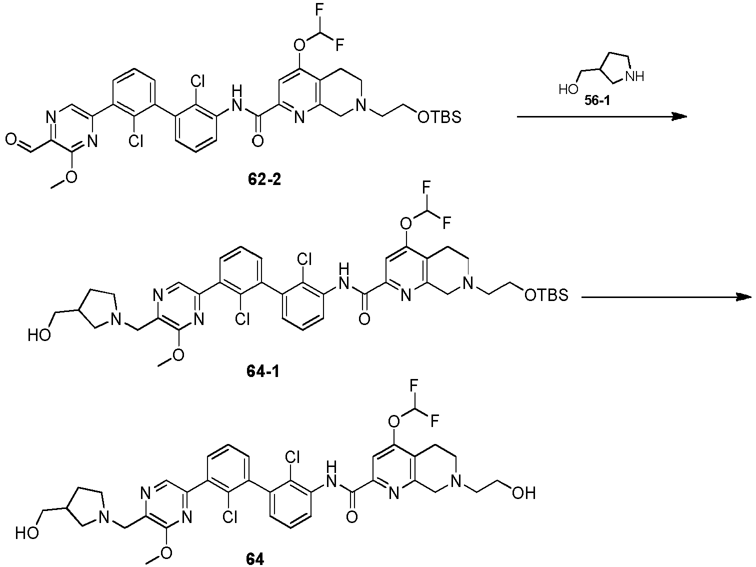

### Step 1

Intermediate 39-1 (40.37 mg, 269.81 µmol, hydrochloride) was replaced with intermediate 56-1 (34.27 mg, 249.04 µmol), and the method was the same as that in example 53. After the reaction was completed, the reaction mixture was diluted with dichloromethane (25 × 2 mL), and washed with water (25 mL). The organic phase was dried over anhydrous sodium sulfate, filtered and concentrated. The residue was subjected to silica gel thin layer chromatography (dichloromethane: methanol=10:1) to obtain intermediate 64-1.

MS-ESI calculated value [M+H]⁺ 843, measured value 843.

### Step 2

Intermediate 53-3 (20 mg, 24.39 µmol) was replaced with intermediate 64-1 (80 mg, 94.80 µmol), and the method was the same as that in example 53. After the reaction was completed, the reaction mixture was concentrated. The crude product was subjected to high performance liquid chromatography (chromatographic column: Phenomenex Gemini-NX C18 75^{∗}30 mm^{∗}3 microns; mobile phase: mobile phase A: formic acid aqueous solution with volume fraction of 0.225 %; mobile phase B: acetonitrile; B%: 10%-40 %, 7 minutes) to obtain compound 64.

MS-ESI calculated value [M+H]⁺ 729, measured value 729. ¹H NMR (400 MHz, CD₃OD) δ = 8.57 - 8.63 (m, 1 H), 8.41 - 8.49 (m, 1 H), 7.84 - 7.90 (m, 1 H), 7.84 - 7.90 (m, 1 H), 7.70 - 7.75 (m, 1 H), 7.54 - 7.61 (m, 1 H), 7.42 - 7.53 (m, 2 H), 7.05 - 7.30 (m, 2 H), 4.09 (s, 3 H), 4.02 ( s, 2 H), 3.89 (s, 2 H), 3.81 (t, J =5.75 Hz, 2 H), 3.47 - 3.60 (m, 2 H), 3.04 - 3.11 (m, 1 H), 2.90 - 3.00 (m, 5 H), 2.83 - 2.90 (m, 1 H), 2.80 (t, J=5.75 Hz, 2 H), 2.63 - 2.70 (m, 1 H), 2.41 - 2.53 (m, 1 H), 1.99 - 2.11 (m, 1 H), 1.57 - 1.68 (m, 1 H)

### Experimental example 1: PD-1/PD-L1 Homogenouse Time-Resolved Fluorescence (HTRF) binding experiment

### Experimental principle:

Small molecule compounds can competitively inhibit the binding of PD-1 and PD-L1 by binding to PD-L1. When the PD-1 molecule as the donor is very close to the PD-L1 molecule as the acceptor, the donor molecule will transfer the energy to the receptor molecule, which in turn causes the receptor molecule to emit fluorescence. The ability of small molecules to prevent the binding of PD-L1 to PD-1 can be tested by detecting the intensity of fluorescence. A homogeneous time-resolved fluorescence (HTRF) binding experiment is used to detect the ability of the compounds of the present disclosure to inhibit the binding of PD-1/PD-L1.

### Experimental Materials:

PD1/PD-L1 TR-FRET detection kit was purchased from BPS Biosciences. Nivo multi-label analyzer (PerkinElmer).

### Experimental method:

PD1-Eu, Dye-labeled acceptor, PD-L1-biotin and compounds to be tested were diluted with the buffer in the kit. The compounds to be tested were subjected to 5-fold dilution to eight concentration gradients with a row gun. That is, from 40 µM to 0.5 nM, the DMSO concentration was 4%, and a double-well experiment was set up. 5 µL of inhibitor with each concentration gradient were added to the microwell plate, and 5 µL buffer containing 4% DMSO and 5 µL PD-L1-biotin (PD-L1 -biotin) (60 nM) were added into well with the maximum signal (Max) and the well with the minimum signal (Min), only 5 µL of buffer was added to the well with the minimum signal (Min), and the incubation was conducted at 25°C for 20 minutes. After the incubation was completed, 5 µL of diluted PD1-Eu (10 nM) and 5 µL of diluted Dye-labeled acceptor were added to each well. The reaction system was placed at 25 °C for 90 minutes. The TR-FRET signal was read by a multi-label analyzer after the reaction was completed.

### Data analysis:

Using the equation (sample-Min)/(Max-Min)× 100% to convert the raw data into inhibition rate, and the IC₅₀ value can be obtained by curve fitting with four parameters (obtained from log(inhibitor) vs. response -- Variable slope mode in GraphPad Prism). Table 1 provides the inhibitory activity of the compounds of the examples of the present disclosure on PD1/PD-L1 binding.

**Table 1: Test results of IC₅₀ values of the compounds of the examples of the present disclosure for binding to PD-1/PD-L1**

| **Test compounds** | **IC₅₀(nM)** |
|---|---|
| Formate of compound 1 | 2.08 |
| Formate of compound 2 | 2.10 |
| Compound 6 | 4.65 |
| Formate of compound 7 | 3.22 |
| Formate of compound 11 | 4.54 |
| Hydrochloride of compound 15 | 8.86 |
| Formate of compound 17 | 1.49 |
| Hydrochloride of compound 18 | 3.16 |
| Formate of compound 20 | 3.58 |
| Formate of compound 21 | 7.14 |
| Formate of compound 23 | 3.68 |

Experimental conclusion: The compounds of the present disclosure have a significant inhibitory effect on the binding of PD-1/PD-L1.

### Experimental example 2: Using MDA-MR-231 cells to detect the effect of compounds on the expression level of PD-L1

### Experimental principle:

The use of a triple-negative breast cancer cell line (MDA-MB-231) is an indirect method to assess PD-L1 endocytosis. PD-L1 molecules on the cell surface can be degraded by lysosomal and proteasome pathways, and small molecule inhibitors are added to induce PD-L1 endocytosis. After co-incubating small molecules with MDA-MB-231 cells for 24 hours, flow cytometry (Fluorescence-activated Cell Sorting, FACS) is used to detect the content of PD-L1 on the cell surface, which can indirectly reflect the effect of small molecule-induced PD-L1 endocytosis. Flow cytometry (FACS) is used to detect the effect of the compounds of the present disclosure on the expression level of PD-L1 in MDA-MR-231 cells.

### Experimental Materials:

Phosphate buffer (DPBS), 1640 medium, penicillin-streptomycin, fetal bovine serum, non-essential amino acids, 2-hydroxy-1-ethanethiol (2-ME), human interferon γ, LIVE/DEAD staining solution, staining buffer, fixation buffer, 0.25% trypsin, EDTA, anti-human PD-L1, anti-human PD-L1 isotype.

1640 complete medium preparation: 50 mL of fetal bovine serum, 5 mL of non-essential amino acids, 5 mL of penicillin-streptomycin and 0.5 mL of 2-hydroxy-1-ethanethiol were added to 439.5 mL of 1640 medium, and evenly mixed.

10mM EDTA preparation: 1mL of 0.5M EDTA was added to 49mL of DPBS, and evenly mixed.

### Experimental procedures

1) MDA-MB-231 cell counting and plating: The culture flask was taken out, the medium was removed and the culture flask was rinsed once with DPBS. After the culture flask was rinsed, 3 mL of 0.25% trypsin was added to the culture flask and the culture flask was placed in a 37°C incubator for 1.5 min. The culture flask was taken out, 9 mL of 1640 complete medium was added to stop the reaction, the cells was transfered to a 50 mL centrifuge tube, and centrifuged at 1000 rpm for 5 min at 37 °C. An appropriate volume of medium was added to re-suspend the cells according to the number of cells, and the number of cells was counted with a cell counter. The cell concentration was adjusted to 5 × 10⁵ cells/mL with medium. Planking: A volume of 200 µL of cell suspension was added to each well of a 96-well plate, ensure that the number of cells in each well was 1 × 10⁵. The cells were incubated overnight in an incubator.
2) Drug incubation: 100-fold dilution of compounds were prepared, and the drug was subjected to a 5-fold gradient dilution. 2 µL of each 100-fold dilution of compounds were added to each well. The cells were incubated in an incubator at 37°C for 24 hours. 3) PD-L1 cell staining and FACS detection: the culture plate was taken out and the supernatant medium was discarded. The cells were washed once with 200 µL 1XPBS. 100 µL of EDTA (final concentration of 10 mM) was added to the cell suspension, and the cell suspension was treated at 37°C for 10 min. Then after the cell suspension was centrifuged at 1500 rpm for 5 min, the cell suspension was washed once with 200 µL of staining solution. Staining: anti-human PD-L1 (2 µL per well) and LIVE/DEAD staining solution (1:1000) were diluted in staining solution, 50 µL of the staining solution was added to each well, and the staining was conducted at 4°C for 30 min. Then the solution was washed twice with 200 µL of staining solution. Fixation: 100 µL of fixing solution was added to each well, the fixation was conducted at 4°C for 15 min. The solution was then washed twice with 200 µL of staining solution, and 150 µL of solution was added to re-suspend the cells, followed by FACS detection.

Table 2 provides the effects of the compounds of the examples of the present disclosure on the expression level of PD-L1 in MDA-MR-231 cells.

**Table 2: Test results of the effects of the compounds of the examples of the present disclosure on the expression level of PD-L1 in MDA-MR-231 cells**

| **Test compounds** | **IC₅₀(nM)** | **Test compounds** | **IC₅₀(nM)** |
|---|---|---|---|
| Formate of compound 1 | 62.54 | Compound 33 | 6.99 |
| Compound 5 | 32.68 | Formate of compound 34 | 21.94 |
| Compound 6 | 93.2 | Compound 35 | 5.18 |
| Formate of compound 9 | 41.08 | Formate of compound 36 | 95.77 |
| Formate of compound 11 | 25.07 | Formate of compound 37 | 28.08 |
| Hydrochloride of compound 14 | 96.87 | Compound 38 | 8.86 |
| Hydrochloride of compound 15 | 29.28 | Formate of compound 39 | 5.52 |
| Formate of compound 17 | 0.75 | Formate of compound 40 | 22.52 |
| Hydrochloride of compound 18 | 70.6 | Formate of compound 41 | 5.27 |
| Formate of compound 21 | 5.73 | Formate of compound 42 | 9.48 |
| Formate of compound 22 | 79.67 | Compound 45 | 7.81 |
| Formate of compound 23 | 0.22 | Formate of compound 46 | 22.62 |
| Formate of compound 25 | 9.97 | Formate of compound 47 | 37.21 |
| Compound 27 | 14.55 | Formate of compound 48 | 8.93 |
| Compound 30 | 10.51 | Compound 49 | 33.79 |
| Compound 31 | 8.58 | Formate of compound 51 | 25.83 |
| Formate of compound 32 | 5.53 | / | / |

Experimental conclusion: The compounds of the present disclosure have a significant inhibitory effect on the expression level of PD-L1 in MDA-MR-231 cells.

### Experimental example 3: NFAT activity test

### Experimental principle:

The engineered T cell expresses PD-1 molecules and T cell receptor (TCR) on the surface, which can activate the NFAT signaling pathway of T cells after co-culturing with engineered antigen presenting cells (APCs). Expression of PD-L1 molecules on APCs can effectively attenuate the NFAT signaling pathway in T cells. PD-L1 inhibitors can be used effectively block the PD-1/PD-L1 regulatory mechanism, thereby reversing the weakened NFAT signaling pathway. After being pretreated with APC, the small molecule is co-cultured with T cells, then the expression of luciferase was detected to indirectly reflect the activation of the NFAT pathway in T cells.

### Experimental Materials:

The PD1/PD-L1 NFAT detection kit was purchased from BPS Biosciences. Birght-Glo reagent was purchased from Promega. Nivo multi-label analyzer (PerkinElmer).

### Experimental method:

The TCR Activitor/PD-L1 CHO cells with a growth confluence of 80% were plated into the plate at 35,000 cells per well and placed in a 37°C cell culture incubator overnight; the compounds to be tested were diluted 5-fold to the 8th concentration, from 20 µM to 0.25 nM, and the DMSO concentration was 2%, a double-well experiment was set up. The TCR Activitor/PD-L1 CHO cell supernatant was discarded, 50 µl of compound working solution was added to each well, incubated at 37°C for 30 minutes; after the incubation was completed, 50 µL of PD-1/NFAT Reporter-Jurkat cell suspension with a concentration of 4^{∗}10⁵ cells/mL was added to each well, incubated at 37°C for 5 hours. After the incubation was completed, 100 µL Bright-Glo was added to each well, and after mixing, the chemiluminescence signal was detected by a Nivo multi-label analyzer.

### Data analysis:

Using the equation (sample-Min)/(Max-Min)× 100% to convert the raw data into inhibition rate, and the IC₅₀ value can be obtained by curve fitting with four parameters (obtained from log(inhibitor) vs. response -- Variable slope mode in GraphPad Prism). Table 3 provides the inhibitory activity of the compounds of the examples of the present disclosure on PD1/PD-L1 binding.

**Table 3 Test results of the inhibitory activity of the compounds of the examples of the present disclosure on PD1/PD-L1 binding**

| **Test compounds** | **EC₅₀(nM)** | **Maximum effect (induction fold)** | **Test compounds** | **EC₅₀(nM)** | **Maximum effect (induction fold)** |
|---|---|---|---|---|---|
| Formate of compound 1 | 34.9 | 3.71 | Compound 28 | 34.77 | 3.63 |
| Compound 5 | 16.2 | 3.68 | Formate of compound 41 | 29.20 | 3.50 |
| Compound 6 | 14.3 | 3.54 | Formate of compound 42 | 23.41 | 4.39 |
| Compound 7 | 38.4 | 3.38 | Formate of compound 43 | 241.7 | 5.16 |
| Formate of compound 9 | 32.6 | 3.77 | Formate of compound 44 | 206.5 | 5.15 |
| Formate of compound 11 | 42.54 | 3.81 | Compound 45 | 45.38 | 4.37 |
| Hydrochloride of compound 15 | 33.93 | 3.80 | Formate of compound 46 | 570.8 | 5.56 |
| Formate of compound 17 | 25.15 | 4.23 | Formate of compound 47 | 51.98 | 4.01 |
| Formate of compound 18 | 79.74 | 3.77 | Formate of compound 48 | 21.25 | 3.80 |
| Formate of compound 20 | 16.9 | 3.43 | Compound 49 | 174.8 | 5.30 |
| Formate of compound 21 | 13.87 | 3.96 | Formate of compound 50 | 45.77 | 4.55 |
| Formate of compound 22 | 58.78 | 3.79 | Formate of compound 51 | 47.93 | 4.28 |
| Formate of compound 23 | 18.4 | 4.84 | Compound 52 | 51.47 | 4.74 |
| Formate of compound 25 | 29.23 | 3.39 | Compound 53 | 13.68 | 3.81 |
| Compound 27 | 28.91 | 3.48 | Compound 54 | 38.5 | 3.91 |
| Compound 29 | 51.99 | 3.52 | Compound 55 | 25.19 | 4.56 |
| Compound 30 | 26.56 | 4.80 | Compound 56 | 10.06 | 2.75 |
| Compound 31 | 22.60 | 4.12 | Compound 57 | 40.7 | 4.42 |
| Formate of compound 32 | 52.23 | 4.93 | Compound 58 | 103.5 | 5.01 |
| Compound 33 | 18.91 | 4.14 | Compound 59 | 166.0 | 5.35 |
| Formate of compound 34 | 44.67 | 4.09 | Compound 60 | 55.5 | 4.95 |
| Compound 35 | 17.46 | 4.44 | Compound 61 | 53.76 | 4.99 |
| Formate of compound 36 | 46.51 | 3.20 | Formate of compound 62 | 13.46 | 4.37 |
| Compound 38 | 50.77 | 4.40 | Formate of compound 63 | 141.80 | 6.40 |
| Formate of compound 39 | 11.93 | 4.62 | Compound 64 | 16.51 | 3.98 |
| Formate of compound 40 | 46.06 | 3.72 | / | / | / |

Experimental conclusion: The compounds of the present disclosure can inhibit the interaction of PD-1/PD-L1 at the cellular level, thereby significantly activating the NFAT signaling pathway of T cells.

### Experimental example 4: Pharmacokinetic Test

### Experimental purpose: To study the pharmacokinetics of compounds in C57BL/6 mice.

Experimental materials: C57BL/6 mice (male, 8 weeks old, body weight 25g-30g)

Experimental operation: The pharmacokinetic characteristics of rodents after intravenous (IV) and oral (PO) administration of compounds were tested in standard protocols. In the experiment, the candidate compounds were formulated into 1 mg/mL clear solutions, and administered to mice via a single intravenous injection and oral administration. The vehicle of intravenous and oral administration was 5% DMSO/5% polyethylene glycol 15-hydroxystearate (Solutol)/90% aqueous solution. Four male C57BL/6 mice were used in the experiment, wherein two mice were administered intravenously at a dose of 1 mg/kg, and the plasma samples were collected 0.0833, 0.25, 0.5, 1, 2, 4, 6, 8 and 24 h after administration, the other two mice were given oral gavage at a dose of 10 mg/kg, and the plasma samples were collected 0.25, 0.5, 1, 2, 4, 6, 8, and 24 h after administration. Whole blood samples within 24 hours were collected, centrifuged at 3000g for 15 minutes, and the supernatant was separated to obtain plasma samples. Acetonitrile solution containing internal standard was added thereto to precipitate proteins, the mixture was thoroughly mixed and centrifuged to obtain the supernatant for injection. The plasma concentration was quantified by LC-MS/MS analysis method, and the pharmacokinetic parameters were calculated, such as peak concentration (Cmax), clearance (CL), half-life (T_{1/2}), tissue distribution (Vdss), area under the drug-time curve (AUC0-last), bioavailability (F) and so on.

The pharmacokinetic-related parameters of the compounds of the examples of the present disclosure in mice are shown in the following table.

**Table 4: Pharmacokinetic test results**

| **Compound** | **Formate of compound 21** | **Formate of compound 42** | **Formate of compound 50** |
|---|---|---|---|
| **Peak concentration Cₘₐₓ (nM)** | 117 | 260 | 221 |
| **Clearance CL (mL/min/kg)** | 98.4 | 36.6 | 85.5 |
| **Tissue distribution V_{dss} (L/kg)** | 65.6 | 40.8 | 18.3 |
| **Half-life T_{1/2} (IV, h)** | 11.6 | 16.4 | 3.72 |
| **Area under the drug-time curve AUC₀₋ₗₐₛₜ PO (nM.hr)** | 796 | 3105 | 1658 |
| **Bioavailability F (%)** | 34.9 | 61.6 | 63.5 |

Experimental conclusion: The compounds of the present disclosure have good pharmacokinetic properties, including good oral bioavailability, oral exposure, half-life and clearance rate.

## Claims

1. A compound represented by formula (I), an isomer or a pharmaceutically acceptable salt thereof, 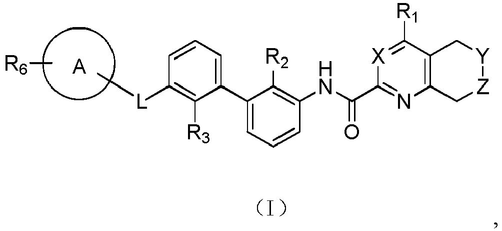 wherein,
ring A is selected from phenyl, 5- to 10- membered heteroaryl and 5- to 10- membered heterocyclenyl, the phenyl, 5- to 10- membered heteroaryl and 5- to 10- membered heterocyclenyl are optionally substituted by 1, 2 or 3 Rₐ;
L is selected from single bond, -C(=O)NH-, -CH=CH-, -O-CH₂- and -NH-;
X is selected from -CH- and N;
when Y is selected from single bond, -CH₂- and -CH(CH₃)-, then Z is selected from NR₄;
or, when Y is selected from NR₅, then Z is selected from-CH₂-;
R₁ is selected from H, C₁₋₃ alkyl, C₁₋₃ alkyl-O-, pyridyl-C₁₋₃ alkyl-O- and C₁₋₃ alkyl-NH-, the C₁₋₃ alkyl, C₁₋₃ alkyl-O-, pyridyl-C₁₋₃ alkyl-O- and C₁₋₃ alkyl-NH- are optionally substituted by 1, 2 or 3 R_{b};
R₂ and R₃ are independently selected from H, F, Cl, Br, I, CN, C₁₋₃ alkyl, C₂₋₆ alkenyl and C₂₋₆ alkynyl, the C₁₋₃ alkyl, C₂₋₆ alkenyl and C₂₋₆ alkynyl are optionally substituted by 1, 2 or 3 R_{c};
R₆ is selected from H, C₁₋₆ alkyl, -C₀₋₆ alkyl-C₃₋₆ cycloalkyl, -C₀₋₆ alkyl-3- to 10-membered heterocycloalkyl, -C₀₋₆ alkyl-NH- C₁₋₆ alkyl, -C₀₋₆ alkyl-NH-C₀₋₆ alkyl-C₃₋₆cycloalkyl, -C₀₋₆ alkyl-NH-C₀₋₆ alkyl-3- to 6- membered heterocycloalkyl and -C₀₋₆ alkyl-O-C₁₋₆ alkyl, the C₁₋₆ alkyl, -C₀₋₆ alkyl-C₃₋₆ cycloalkyl, -C₀₋₆ alkyl-3- to 6-membered heterocycloalkyl, -C₀₋₆ alkyl-NH- C₁₋₆ alkyl, -C₀₋₆ alkyl-NH-C₀₋₆ alkyl-C₃₋₆cycloalkyl, -C₀₋₆ alkyl-NH-C₀₋₆ alkyl-3- to 6- membered heterocycloalkyl and -C₀₋₆ alkyl-O-C₁₋₆ alkyl are optionally substituted by 1, 2 or 3 R_{d};
R₄ and R₅ are independently selected from H, C₁₋₆ alkyl, C₁₋₆ alkyl-NH-, C₃₋₈ cycloalkyl, -C₀₋₆ alkyl-C₃₋₆ cycloalkyl and 3- to 8- membered heterocycloalkyl, the C₁₋₆ alkyl, C₁₋₆ alkyl-NH-, C₃₋₈ cycloalkyl, -C₀₋₆ alkyl-C₃₋₆ cycloalkyl and 3- to 8- membered heterocycloalkyl are optionally substituted with 1, 2 or 3 Rₑ;
Rₐ, R_{b}, R_{c}, R_{d} and Rₑ are independently selected from H, F, Cl, Br, I, OH, CN, NH₂, COOH, 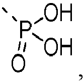 C₁₋₆ alkyl, -O-C₁₋₆ alkyl, -C₀₋₆ alkyl-NH-C₁₋₆ alkyl, -C₀₋₆ alkyl-NH-C₀₋₆ alkyl-3- to 6-membered heterocycloalkyl, -N(C₁₋₆ alkyl)₂, -C₀₋₆ alkyl-C₃₋₆ cycloalkyl and -C₀₋₆ alkyl-3- to 6- membered heterocycloalkyl, the C₁₋₆ alkyl, -O-C₁₋₆ alkyl, -C₀₋₆ alkyl-NH-C₁₋₆ alkyl, -C₀₋₆ alkyl-NH-C₀₋₆ alkyl-3- to 6- membered heterocycloalkyl, - N(C₁₋₆ alkyl)₂, -C₀₋₆ alkyl-C₃₋₆ cycloalkyl and -C₀₋₆ alkyl-3- to 6- membered heterocycloalkyl are optionally substituted by 1, 2 or 3 R;
R is independently selected from F, Cl, Br, I, OH, CN, NH₂, COOH, CH₃, CH₃CH₂, CH₃O, NHCH₃, N(CH₃)₂ and CH₃(CH₃)₂;
the 5- to 10- membered heteroaryl, 5- to 10- membered heterocycloalkenyl, 3- to 6-membered heterocycloalkyl, 3- to 8- membered heterocycloalkyl and 3- to 10-membered heterocycloalkyl independently comprise 1, 2, 3 or 4 heteroatoms or heteroatom groups independently selected from -NH-, N, -S- and -O-.

2. The compound, the isomer or the pharmaceutically acceptable salt thereof according to claim 1, which is selected from 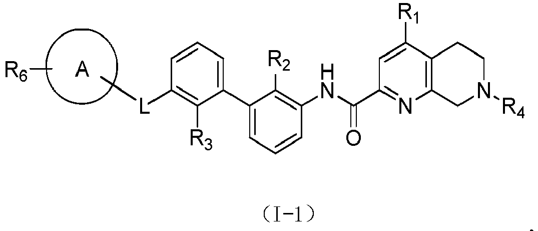 wherein R₁, R₂, R₂, R₃, R₄, R₆, L and ring A are as defined in claim 1.

3. The compound, the isomer or the pharmaceutically acceptable salt thereof according to claim 2, which is selected from 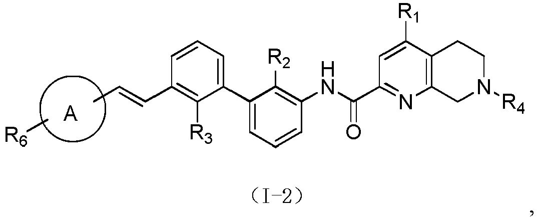 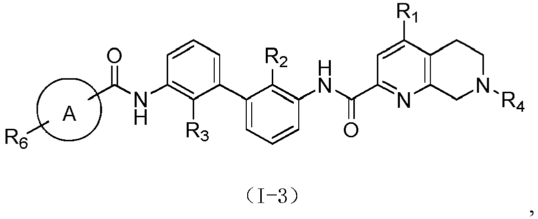 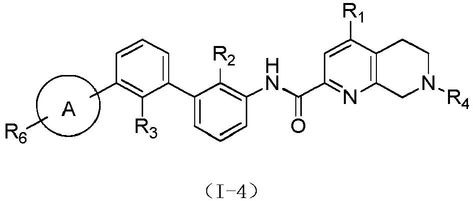 and 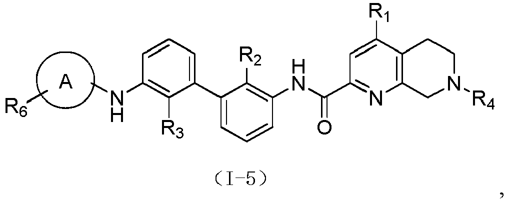 wherein R₁, R₂, R₂, R₃, R₄, R₆, L and ring A are as defined in claim 2.

4. The compound, the isomer or the pharmaceutically acceptable salt thereof according to claim 3, which is selected from 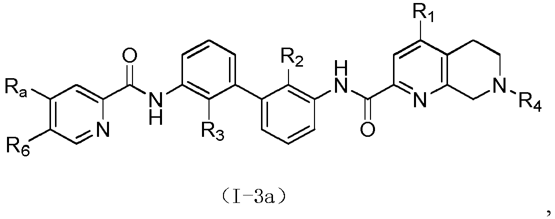 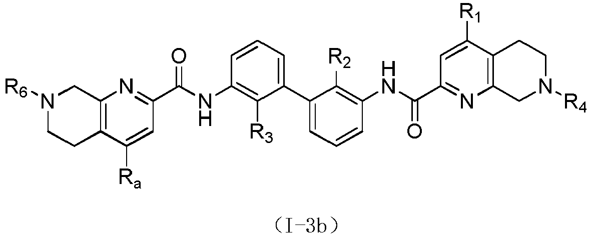 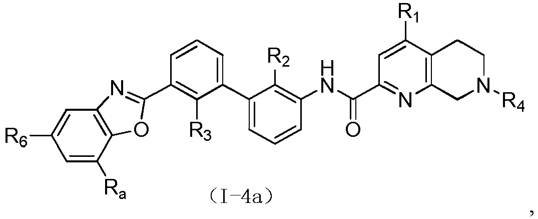 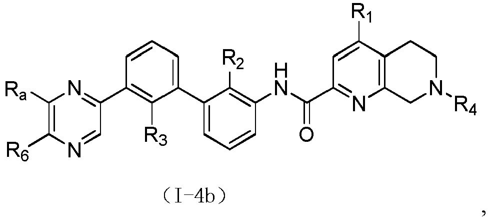 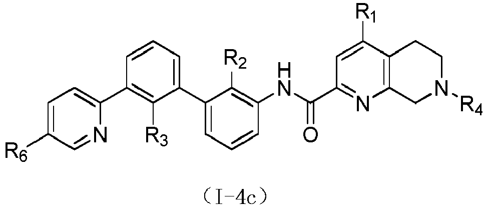 and 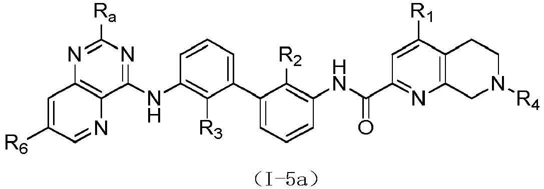 wherein R₁, R₂, R₂, R₃, R₄, and R₆ are as defined in claim 3, Rₐ is as defined in claim 1.

5. The compound, the isomer or the pharmaceutically acceptable salt thereof according to claim 4, which is selected from 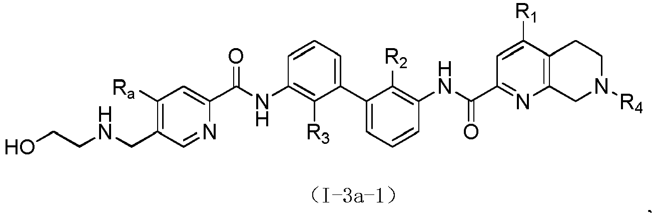 wherein R₁, R₂, R₂, R₃, R₄, and Rₐ are as defined in claim 4.

6. The compound, the isomer or the pharmaceutically acceptable salt thereof according to any one of claims 1-5, wherein, Rₐ, R_{b}, R_{c} and Rₑ are independently selected from H, F, Cl, Br, I, OH, CN, NH₂, COOH, CH₃, CF₃, CHF₂, CH₂F, CH₃CH₂, CH₂OH, 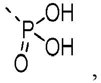 CH₃O, NHCH₃, N(CH₃)₂, CH₃(CH₃)₂, 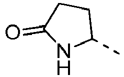 and

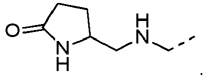

7. The compound, the isomer or the pharmaceutically acceptable salt thereof according to any one of claims 1-4, wherein, R_{d} is selected from F, Cl, Br, I, OH, CN, NH₂, COOH, CH₃, CH₃CH₂, CH₂OH, CH₃O, NHCH₃, N(CH₃)₂ and CH₃(CH₃)₂.

8. The compound, the isomer or the pharmaceutically acceptable salt thereof according to any one of claims 1-7, wherein, R₁ is selected from H, CN, CH₃, CH₃O-, pyridyl-CH₂O- and CH₃CH₂NH-, the CH₃, CH₃O-, pyridyl-CH₂O- and CH₃CH₂-NH-are optionally substituted by 1, 2 or 3 R_{b}.

9. The compound, the isomer or the pharmaceutically acceptable salt thereof according to claim 8, wherein, R₁ is selected from CN, CF₃, -OCHF₂, CH₃, CH₃O-, - NHCH₂CH₂OH,

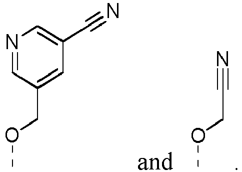

10. The compound, the isomer or the pharmaceutically acceptable salt thereof according to any one of claims 1-5, wherein, R₂ and R₃ are independently selected from Cl, CN and CH₃.

11. The compound, the isomer or the pharmaceutically acceptable salt thereof according to any one of claims 1-4, wherein, R₆ is selected from H, -CH₂CH₃, 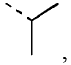 -CH₂NHCH₃, -CH₂NHCH₂CH₃, 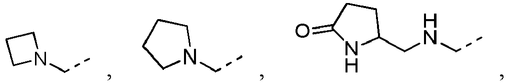 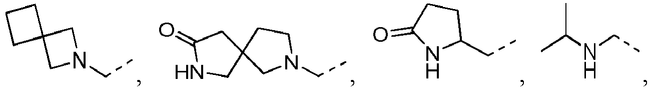 the -CH₂CH₃, 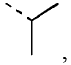 -CH₂NHCH₃, -CH₂NHCH₂CH₃, 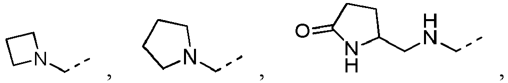 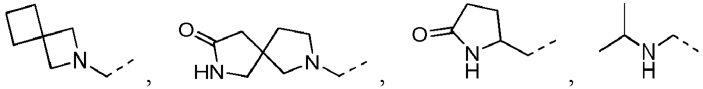 are optionally substituted by 1, 2 or 3 R_{d}.

12. The compound, the isomer or the pharmaceutically acceptable salt thereof according to claim 11, wherein, R₆ is selected from H, 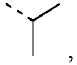 -CH₂CH₂OH, - CH₂NHCH₂CH₂OH, 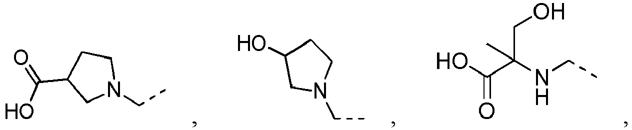 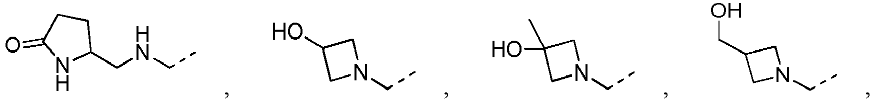 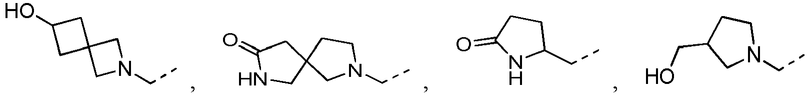 and

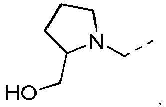

13. The compound, the isomer or the pharmaceutically acceptable salt thereof according to any one of claims 1-5, wherein, R₄ and R₅ are independently selected from H, CH₃, CH₂CH₃, 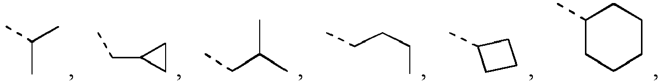 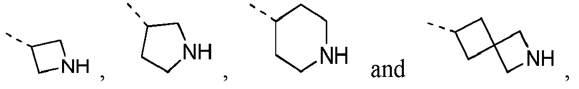 the CH₃, CH₂CH₃, 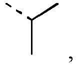 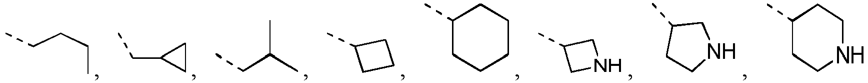 and 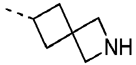 are optionally substituted with 1, 2 or 3 Rₑ.

14. The compound, the isomer or the pharmaceutically acceptable salt thereof according to claim 13, wherein, R₄ and R₅ are independently selected from H, CH₃, CH₂CH₃, 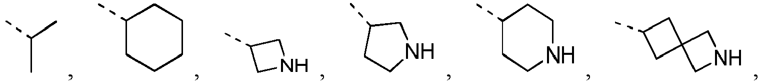 CH₂CH₂OH, 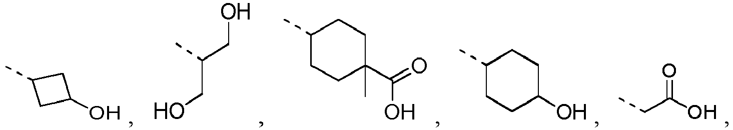 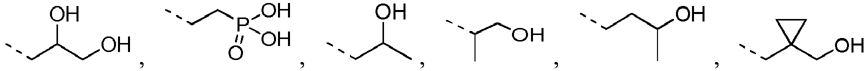 and

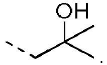

15. The compound, the isomer or the pharmaceutically acceptable salt thereof according to any one of claims 1-5, wherein, the structural moiety 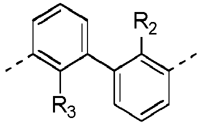 is selected from

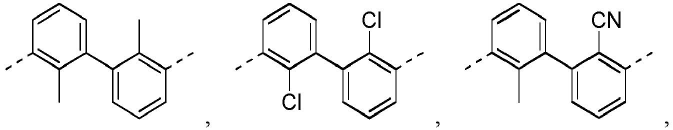

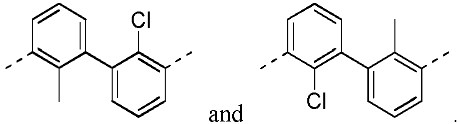

16. The compound, the isomer or the pharmaceutically acceptable salt thereof according to any one of claims 1-3, wherein, ring A is selected from benzo[*d]*oxazolyl, pyrrolidine-2-keto, pyridyl, 5,6,7,8-tetrahydro-1,7-diazanaphthyl, phenyl, 4,5,6,7-tetrahydro-2*H*-pyrazolo[3,4-*c*]pyridyl, 4,5,6,7-tetrahydrothiazolo[5,4-*c*]pyridyl, pyrazinyl and pyrido[3,2-*d*]pyrimidinyl, the benzo[*d*]oxazolyl, pyrrolidine-2-keto, pyridyl and 5,6,7,8-tetrahydro-1,7-diazanaphthyl, phenyl, 4,5,6,7-tetrahydro-2*H-*pyrazolo[3,4-*c*]pyridyl, 4,5,6,7-tetrahydrothiazolo[5,4-*c*]pyridyl, pyrazinyl and pyrido[3,2-*d*]pyrimidinyl are optionally substituted by 1, 2 or 3 Rₐ.

17. The compound, the isomer or the pharmaceutically acceptable salt thereof according to claim 16, wherein, ring A is selected from 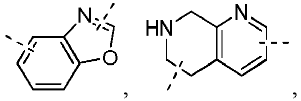 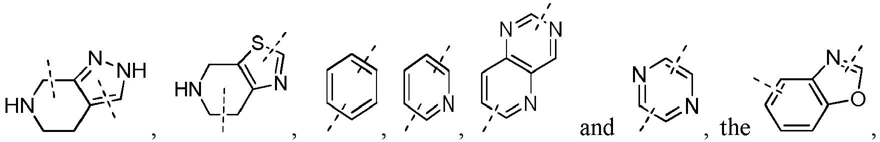 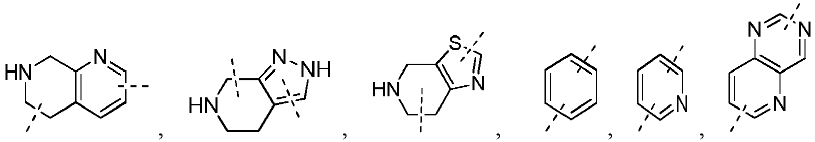 and 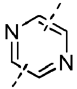 are optionally substituted by 1, 2 or 3 Rₐ.

18. The compound, the isomer or the pharmaceutically acceptable salt thereof according to claim 17, wherein, ring A is selected from

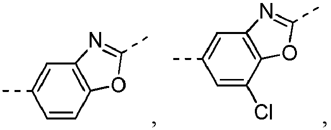

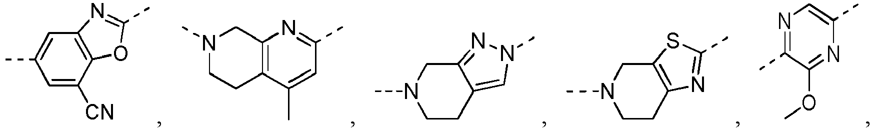

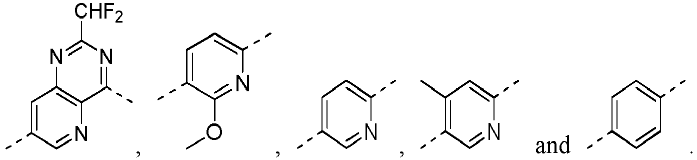

19. The compound, the isomer or the pharmaceutically acceptable salt thereof according to any one of claims 1-3, wherein, the structural moiety 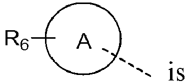 selected from

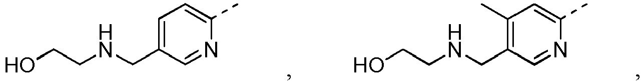

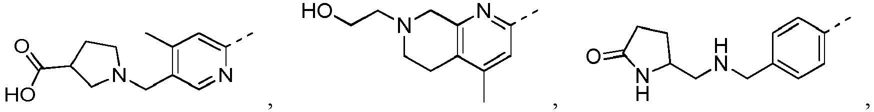

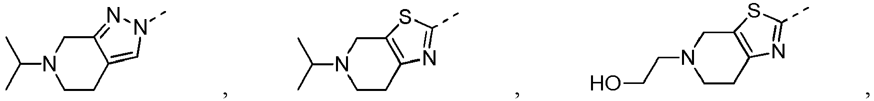

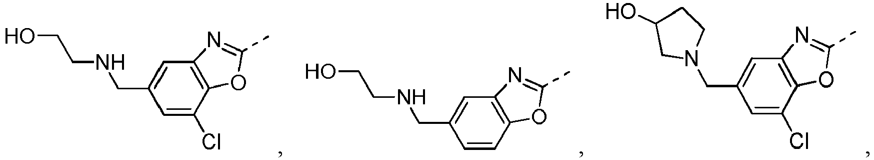

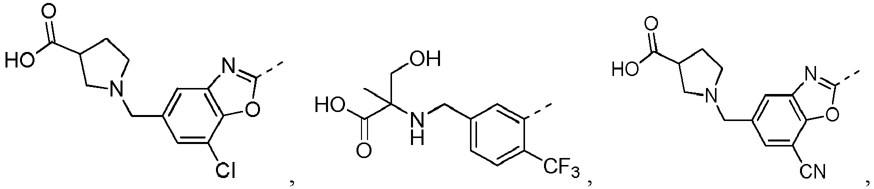

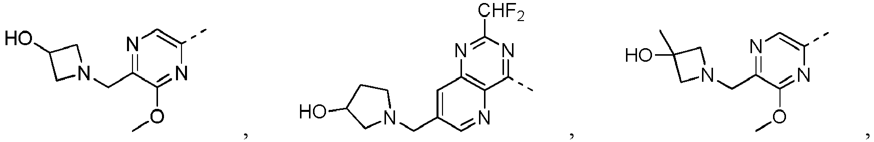

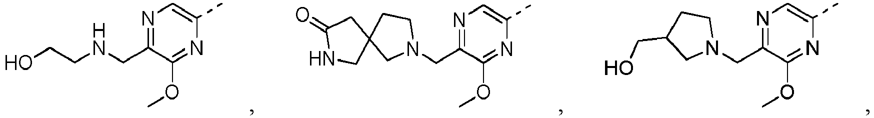

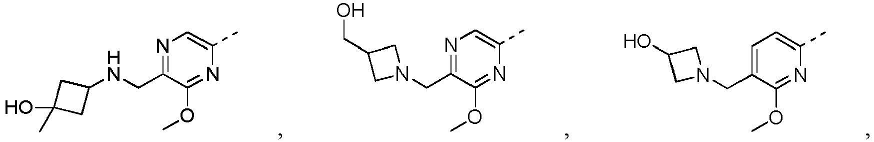

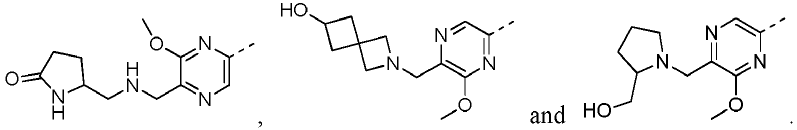

20. A compound represented by the following formula, an isomer or a pharmaceutically acceptable salt thereof,

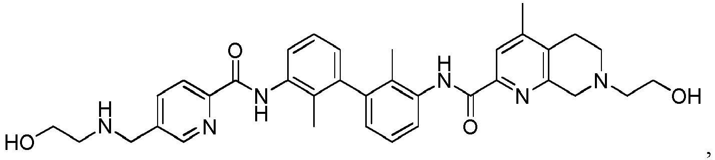

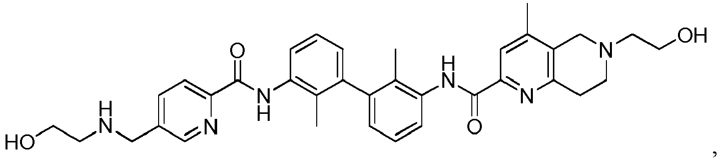

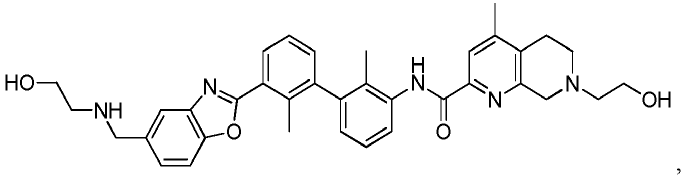

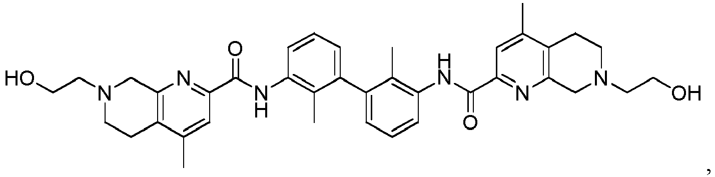

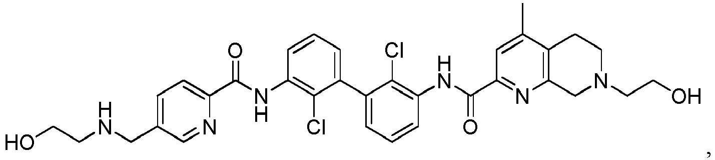

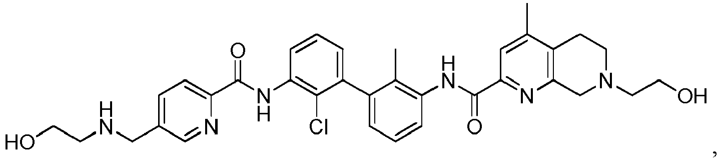

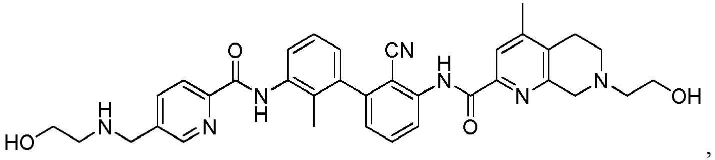

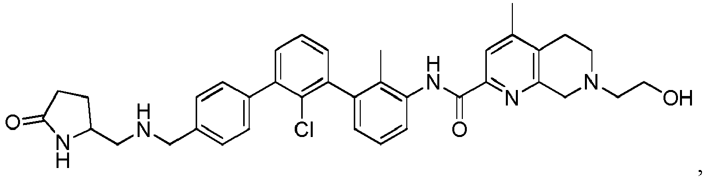

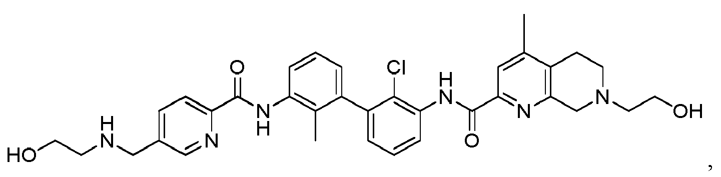

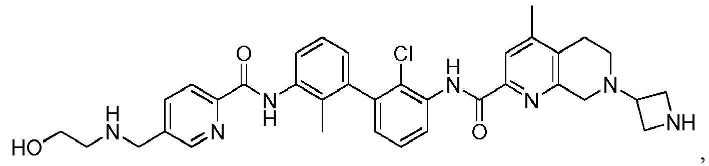

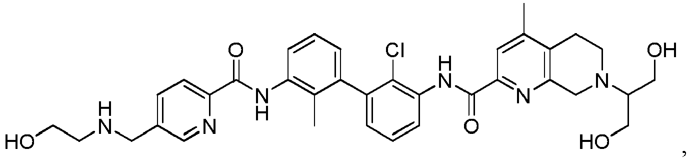

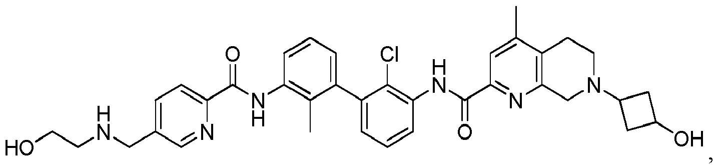

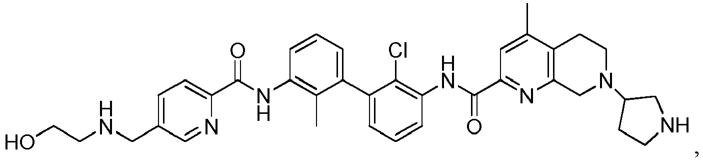

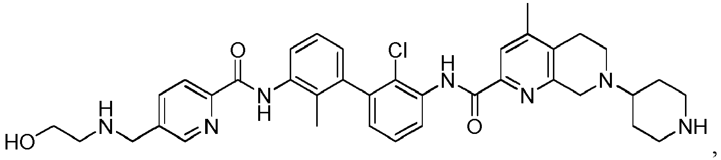

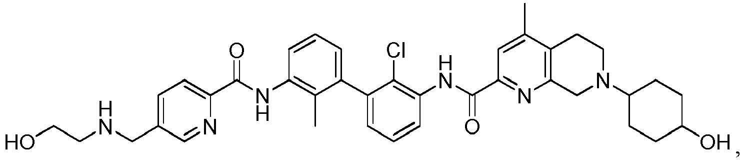

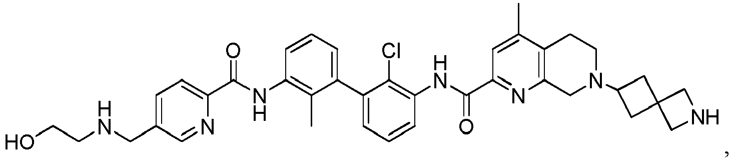

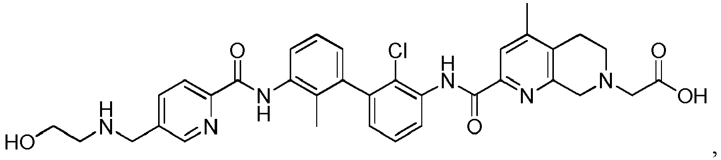

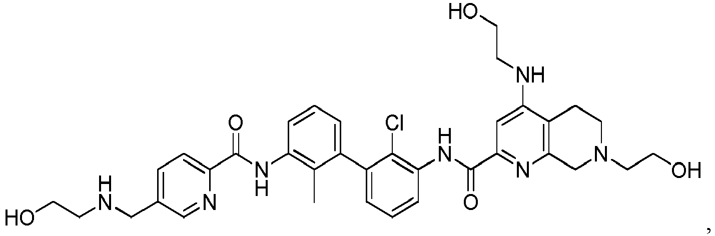

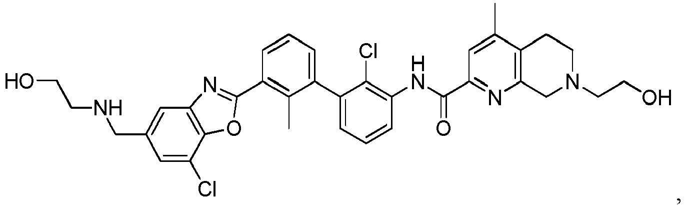

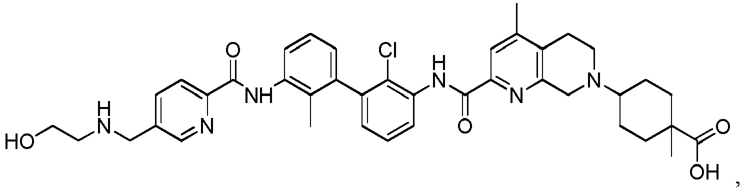

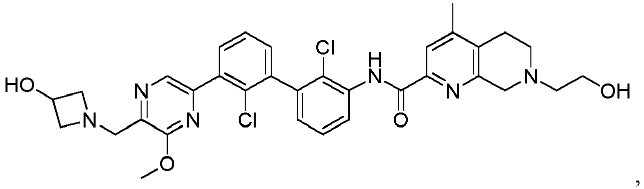

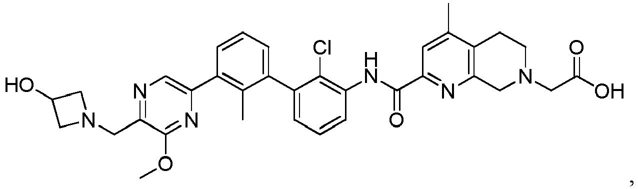

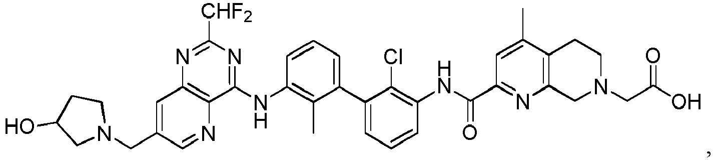

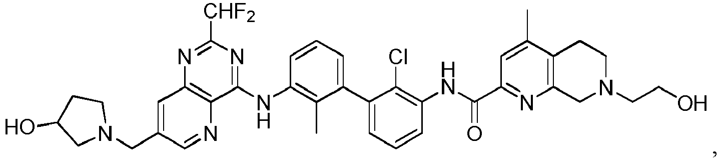

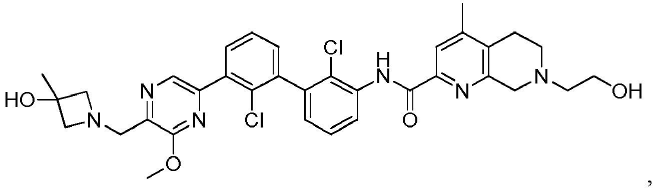

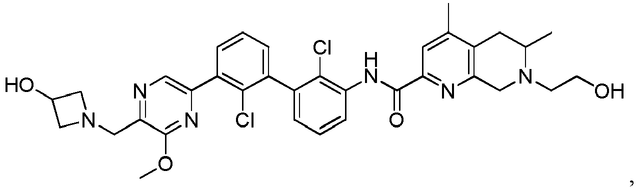

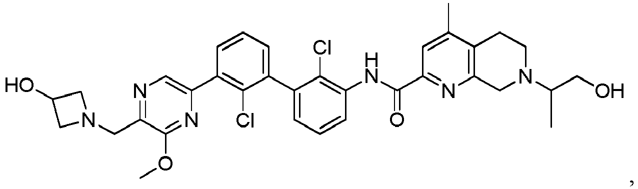

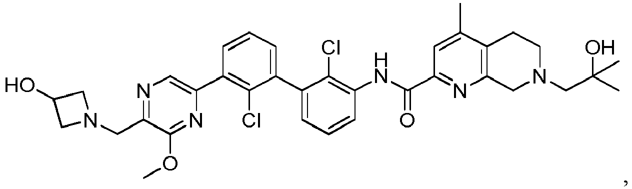

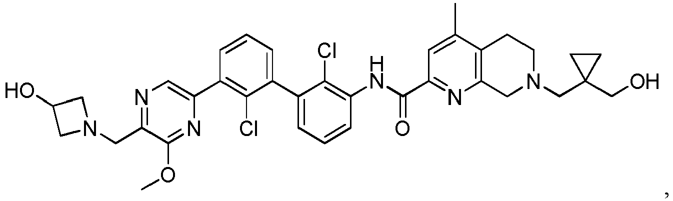

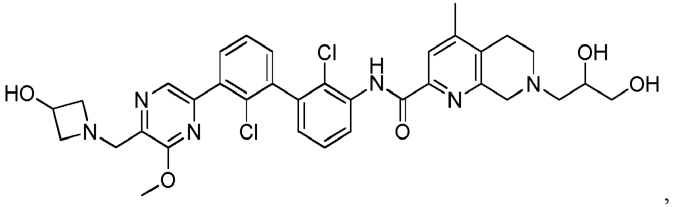

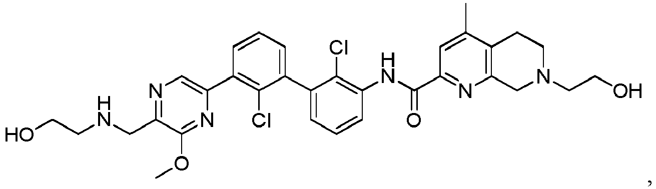

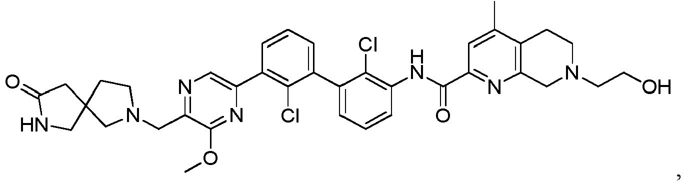

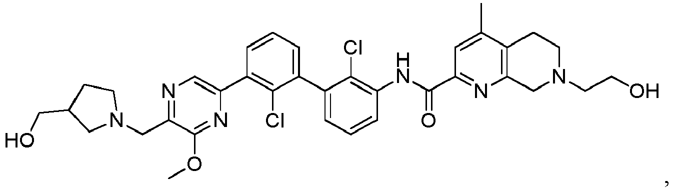

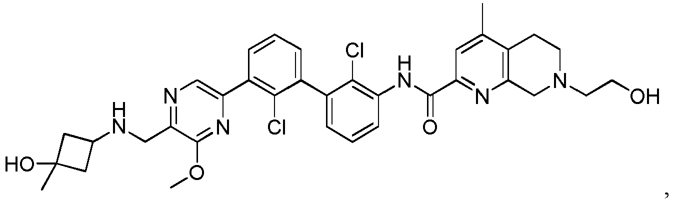

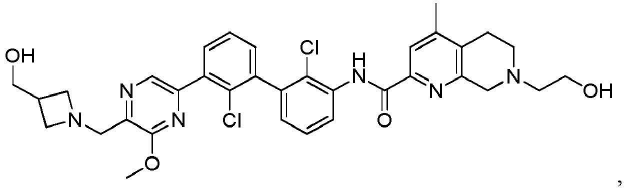

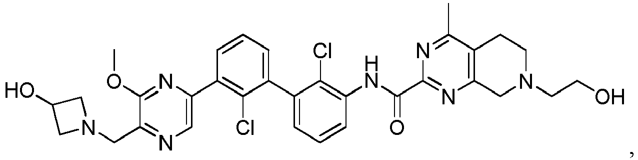

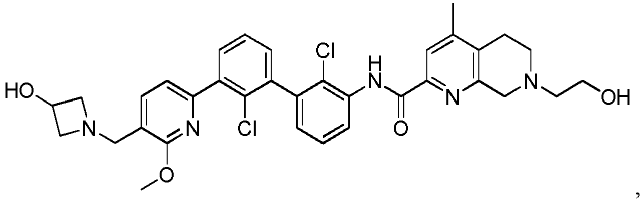

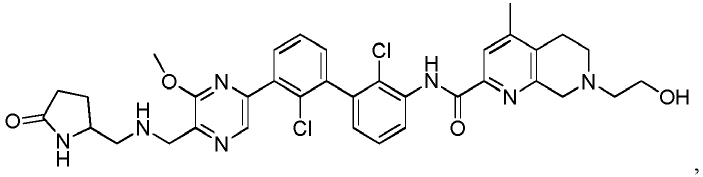

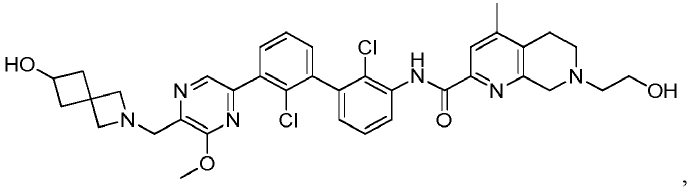

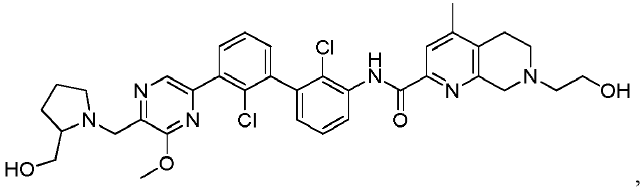

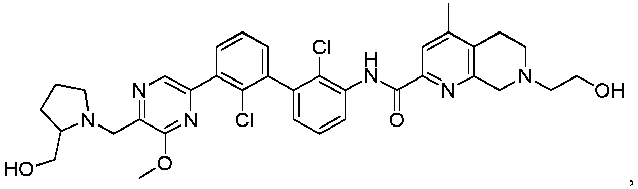

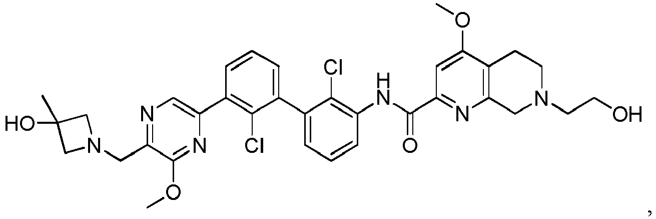

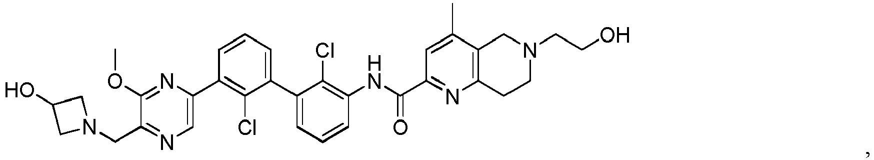

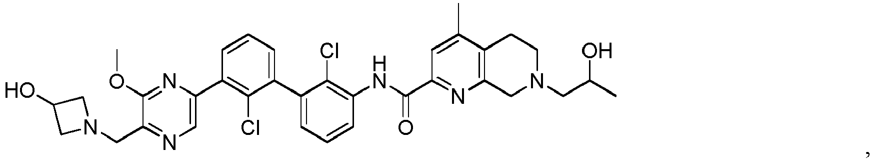

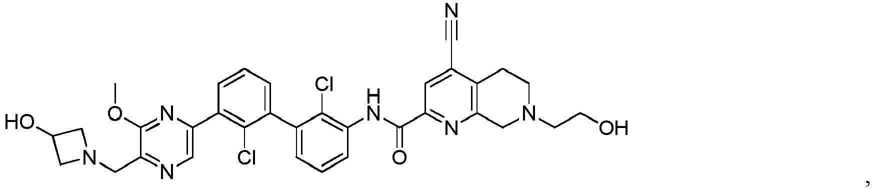

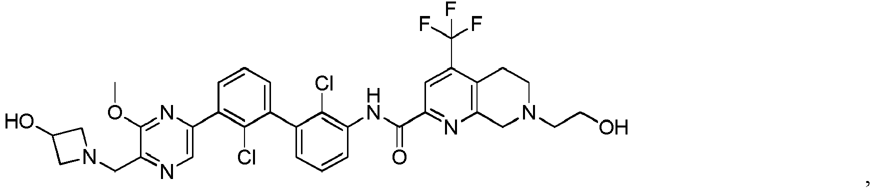

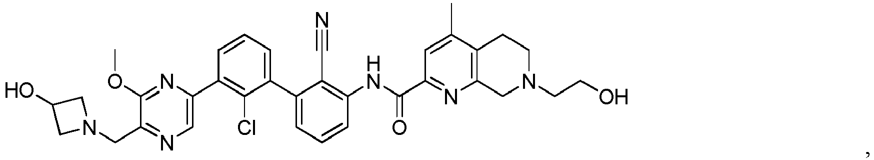

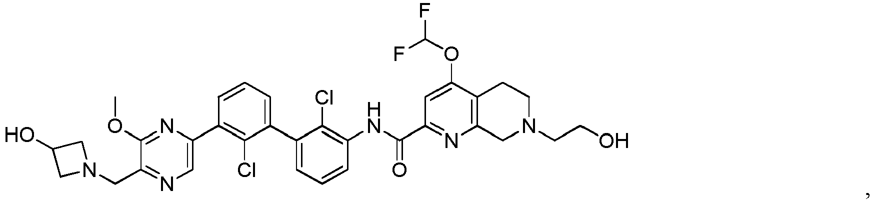

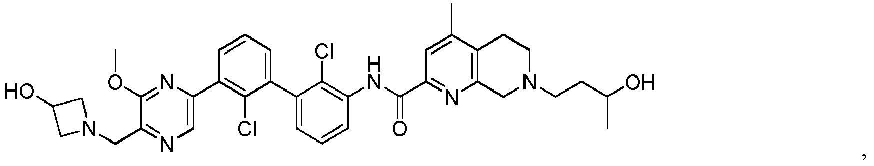

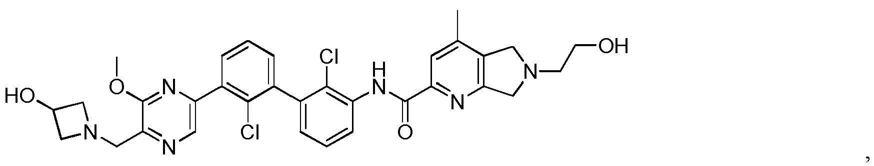

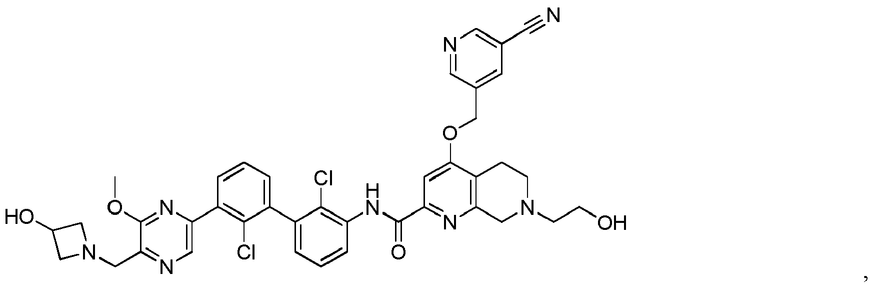

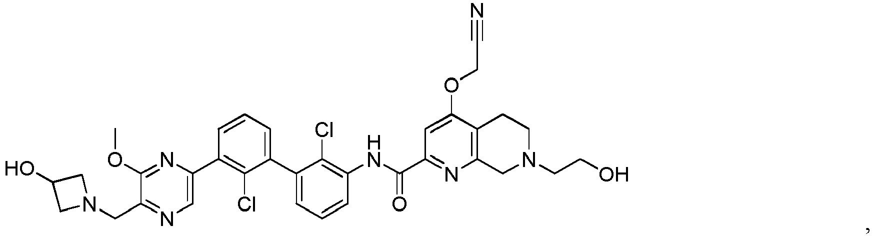

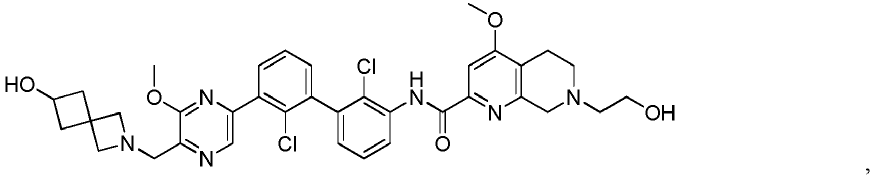

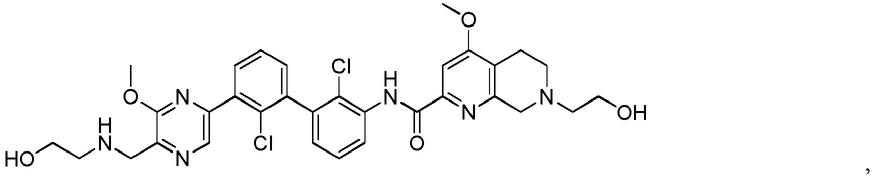

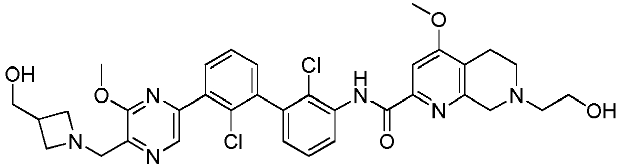

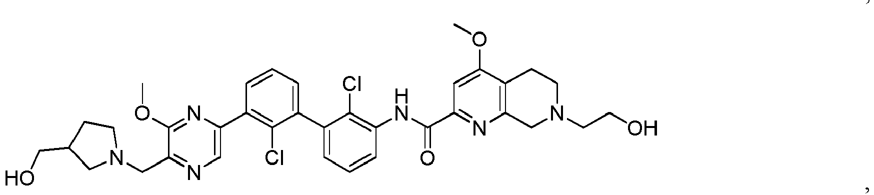

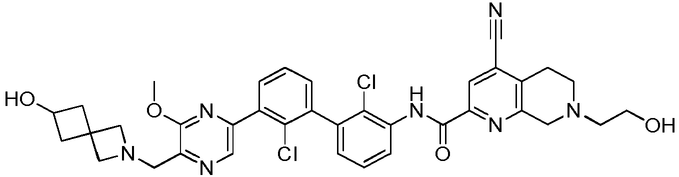

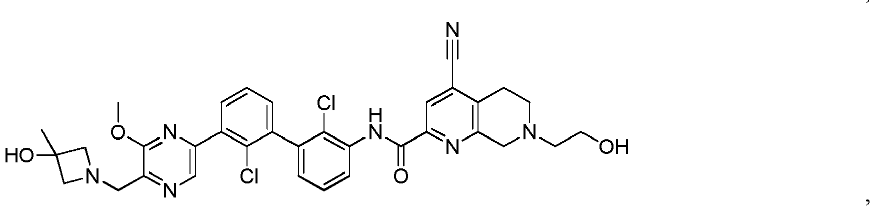

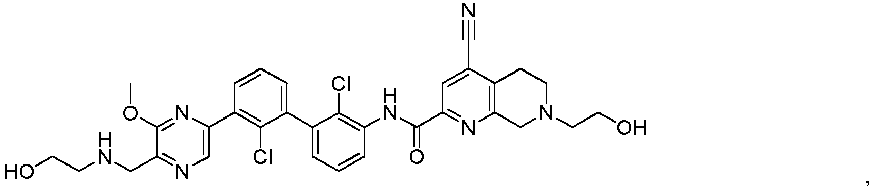

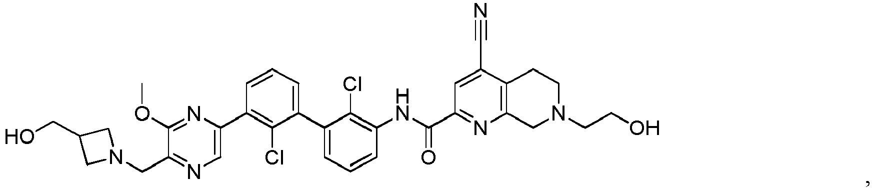

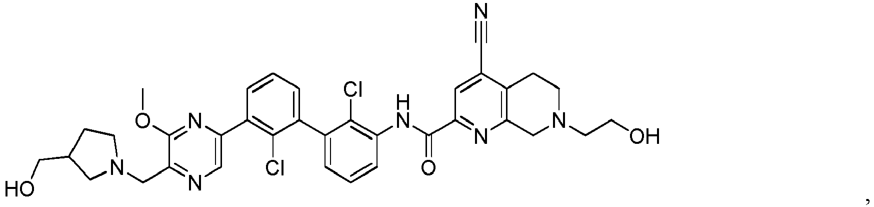

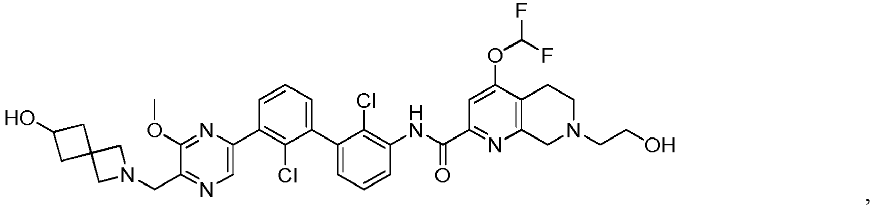

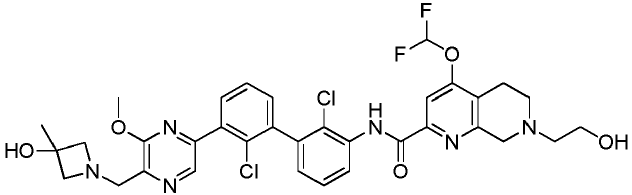

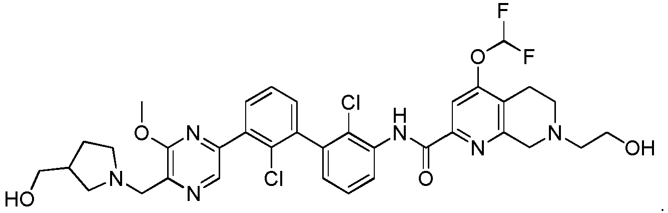

21. The compound, the isomer or the pharmaceutically acceptable salt thereof according to claim 20, which is selected from

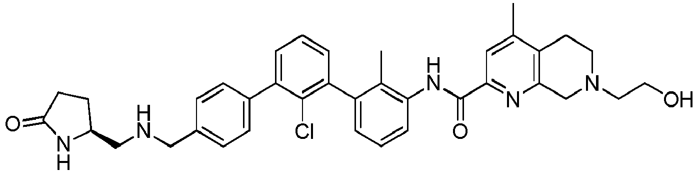

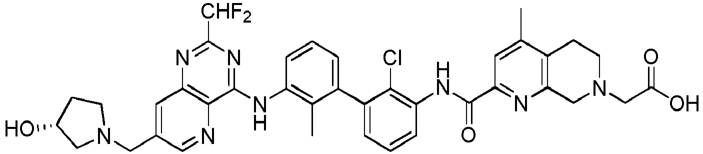

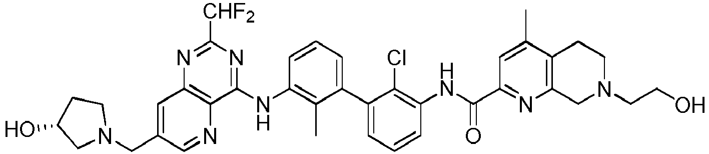

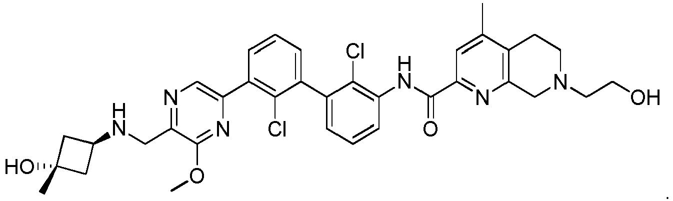

22. A pharmaceutical composition comprising a therapeutically effective amount of the compound or the pharmaceutically acceptable salt thereof according to any one of claims 1-21 as an active ingredient and a pharmaceutically acceptable carrier.

23. A use of the compound or the pharmaceutically acceptable salt thereof according to any one of claims 1-21 or the pharmaceutical composition according to claim 20 in the manufacture of a PD-1/PD-L1 inhibitor.

24. The use according to claim 23, wherein the PD-1/PD-L1 inhibitor is an anti-tumor drug.
